# EUROPEAN PATENT APPLICATION

(11) **EP 1 396 488 A1**
(43) Date of publication of application: **10.03.2004**
(21) Application number: 02726463.9
(22) Date of filing: 23.05.2002
(51) Int. Cl.: C07D 217/24

(54) **FUSED HETEROCYCLIC COMPOUND AND MEDICINAL USE THEREOF**

(30) Priority: 23.05.2001 JP 2001154571
(71) Applicant: Mitsubishi Pharma Corporation, Osaka-shi, Osaka 541-0046 (JP)
(72) Inventor: FUJIO, Masakazu, MITSUBISHI PHARMA CORPORATION, Chuo-ku, Tokyo 103-8405 (JP); SATOH, Hiroyuki, MITSUBISHI PHARMA CORPORATION, Chuo-ku, Tokyo 103-8405 (JP); NUMATA, Atushi, MITSUBISHI PHARMA CORPORATION, Chuo-ku, Tokyo 103-8405 (JP); TAKANASHI, Shinichi, MITSUBISHI PHARMA CORPORATION, Chuo-ku, Tokyo 103-8405 (JP); EGI, Yasuhiro, MITSUBISHI PHARMA CORPORATION, Chuo-ku, Tokyo 103-8405 (JP); TATSUMI, Ryou, MITSUBISHI PHARMA CORPORATION, Chuo-ku, Tokyo 103-8405 (JP)
(74) Representative: von Kreisler, Alek, Dipl.-Chem.
(86) International application number: PCT/JP2002/004995
(87) International publication number: WO 2002/094790

(57) **Abstract**

The fused heterocyclic compound of the present invention, which is represented by the formula (I): 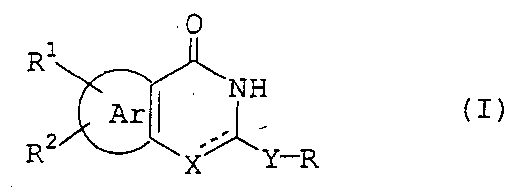 wherein each symbol is as defined in the specification, an optically active form thereof, a pharmaceutically acceptable salt thereof, a hydrate thereof and a water adduct thereof show poly(ADP-ribose) synthase inhibitory action and are useful as therapeutic drugs for cerebral infarction.

## Description

### Technical Field

The present invention relates to a poly(ADP-ribose) synthase inhibitor represented by the above-mentioned formula (I) and a cerebral infarction treatment drug represented by the above-mentioned formula (I).

### Background Art

Poly(ADP-ribose) synthase (poly(ADP-ribose) polymerase; hereinafter abbreviated as PARP) having nicotinic acid amide nucleotide (NAD) as a substrate is an intranuclear enzyme that cleaves the bond between nicotinic acid amide and ribose, transfers ADP-ribose residue to a protein, and causes addition polymerization of plural ADP-ribose residues. This enzyme is attracting attention as an apoptosis-related enzyme, whose major action is to be activated by recognizing the nick of DNA damaged by a free radical, such as nitrogen oxide, active oxygen and the like, which is produced at the lesion on ischemia, and aid DNA repair.

In recent years, it is considered that the activation of PARP decreases intracellular NAD, and a large amount of ATP is consumed to compensate for the decrease, which in turn depletes the intracellular energy to cause cell death. It has been clarified in experiments using PARP knock out mouse that cultured nerve cells show resistance to disorders caused by nitrogen oxide and excitatory amino acids such as NMDA (N-methyl-D-aspartate) and the like, and show a remarkable protective effect to suppress 80% or above of cerebral infarction caused by cerebral ischemia (Eliasson MJL. et. al., Nature Med., 3, 1089-95 (1997)).

However, none of the PARP inhibitors reported heretofore has been subjected to clinical tests as a cerebral infarction treatment drug. As the PARP inhibitors reported until today, for example, 5-substituted-3,4-dihydro-2H-isoquinoline derivative (JP-A-2-124874), 1,11b-dihydrobenzopyrano[4.3.2-de]isoquinolin-3-one derivative (WO99/11645), 3,4-dihydro-5-[4-(1-piperidinyl)-butoxy]-1(2H)-isoquinoline (WO99/08680, WO99/11649), pyrimidine derivative (WO00/42025), benzimidazole derivative (WO00/64878, WO00/68206), phthalazine derivative (WO00/67734, WO00/44726) and the like are known, but the PARP inhibitory activity thereof is not very strong.

Moreover, JP-B-46-12454 discloses isoquinoline derivatives having analgesic action and hypoglycemic action, US patent Nos. 1174272 and 1062357 disclose quinazoline derivatives having hypotensive action, UK patent Nos. GB 1,174,272, GB1,062,357 and German patent DE2121031 disclose quinazoline derivatives having hypotensive action, and JP-A-64-42472 discloses quinazoline derivatives having cerebral function disorder improving action, but none of them took note of the PARP inhibitory action.

The present invention aims to provide a compound having a PARP inhibitory action and useful as a cerebral infarction treatment drug, particularly as a treatment drug for an acute phase of cerebral infarction.

### Disclosure of the Invention

The present inventors have conducted intensive studies and found that a fused heterocyclic compound represented by the following formula (I), an optically active form thereof, a pharmaceutically acceptable salt thereof, a hydrate thereof and a water adduct thereof have potent PARP inhibitory action, which resulted in the completion of the present invention.

Therefore, the compound of the present invention can be useful as a cerebral infarction treatment drug, particularly as an acute phase cerebral infarction treatment drug. Accordingly, the present invention provides the following.
1. A fused heterocyclic compound represented by the formula (I): 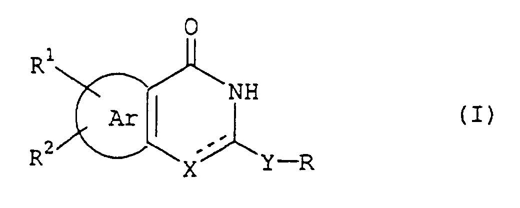 wherein
   - a dotted line part: is a single bond or a double bond;
   - ring Ar: is a benzene ring, a naphthalene ring or an aromatic heterocycle;
   - X: is a carbon atom optionally substituted by an alkyl, an aromatic heterocyclic group optionally having substituent(s) or a phenyl optionally having substituent(s), or a nitrogen atom;
   - Y: is
   -(CH₂)ₘ-,
   -(CH₂)ₘ-N(R⁴)-CO-(CH₂)ₙ-,
   -(CH₂)ₘ-CO-N(R⁴)-(CH₂)ₙ-,
   -(CH₂)ₘ-CO-O-(CH₂)ₙ-,
   -(CH₂)ₘ-O-CO-(CH₂)ₙ-,
   -(CH₂)ₘ-O-(CH₂)ₙ- or
   - (CH₂)ₘ-CO-(CH₂)ₙ-
   wherein m and n are the same or different and each is 0 or an integer of 1-10, R⁴ is a hydrogen or an alkyl, and -(CH₂)ₘ- is linked with a parent-nucleus;
   - R¹ and R²: are the same or different and each is a hydrogen, a halogen, an alkyl, an alkoxy, a haloalkyl, a hydroxy, an amino, a dialkylamino, a nitro, a cyano, an acyl, a carboxy, an ester, a carbamoyl, an N-alkylcarbamoyl, an N,N-dialkylcarbamoyl, an acylamino, a diacylamino, a thiol, an alkylthio, an alkoxycarbonylamino, a sulfamoyl, an N-alkylsulfamoyl, an N,N-dialkylsulfamoyl or an alkoxyalkyloxy; and
   - R: is an amino, a monoalkylamino, a dialkylamino, a morpholino or a thiomorpholino, or represented by the following formula (a)-(e): 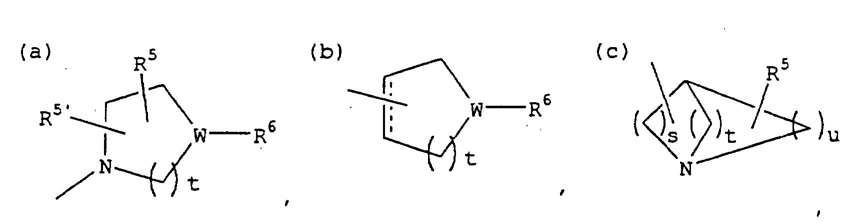 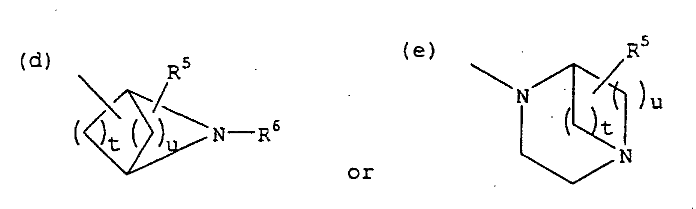
   wherein a dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, monoalkylamino, dialkylamino, alkyl, alkoxycarbonyl, alkylsulfonyl, acyl, acylamino, benzoylamino optionally having substituent(s), hydroxy, arylalkyl, sulfamoyl or alkylsulfonylamino, or represented by the following formula (f)-(i): 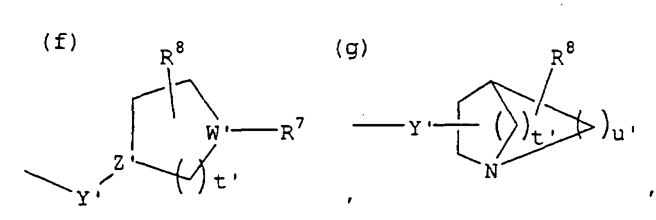 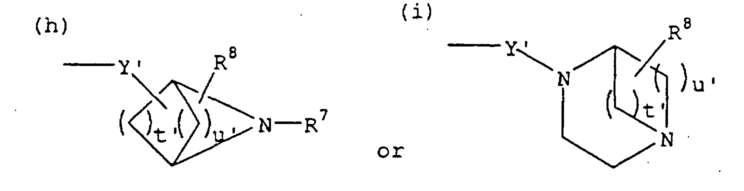 wherein Y' is as defined for Y above, Z' is CH or a nitrogen atom, W' is CH, a nitrogen atom or an oxygen atom, t' is an integer of 1-3, u' is an integer of 1-3, provided that when the above-mentioned formula (a) is piperazine, then R⁶ may be hydroxyalkyl, R⁷ is hydrogen, amino, monoalkylamino, dialkylamino, alkyl, alkoxycarbonyl, alkylsulfonyl, acyl, hydroxyalkyl, acylamino or benzoylamino optionally having substituent(s), provided that when W' is an oxygen atom, then R⁷ should be absent, and R⁸ is hydrogen, alkyl or hydroxyalkyl,
   provided that (1) when X is the unsubstituted carbon atom, the ring Ar is a benzene ring, Y is -(CH₂)ₘ- (m=0) and R is monoalkylamino, dialkylamino, piperidinyl, 3-methyl-1-piperidino, piperazin-1-yl, 4-methylpiperazin-1-yl, 1-piperidino, 4-morpholino or 4-(2-hydroxyethyl)piperazin-1-yl, then R¹ should be halogen, alkyl, alkoxy, haloalkyl, hydroxy, amino, dialkylamino, nitro, cyano, acyl, carboxy, ester, carbamoyl, N-alkylcarbamoyl, N,N-dialkylcarbamoyl, acylamino, diacylamino, thiol, alkylthio, alkoxycarbonylamino, sulfamoyl, N-alkylsulfamoyl or N,N-dialkylsulfamoyl, and (2) when X is a nitrogen atom and Y is -(CH₂)ₘ- (m=0), then R should be represented by any of the above-mentioned formulas (b)-(d), an optically active form thereof, a pharmaceutically acceptable salt thereof, a hydrate thereof or a water adduct thereof.
2. The fused heterocyclic compound of the above-mentioned 1, wherein, in the formula (I),
   X is a carbon atom optionally substituted by alkyl, aromatic heterocyclic group optionally having substituent(s) or phenyl optionally having substituent(s),
   an optically active form thereof, a pharmaceutically acceptable salt thereof, a hydrate thereof or a water adduct thereof.
3. The fused heterocyclic compound of the above-mentioned 1, wherein, in the formula (I),
   R¹ is halogen, alkyl, alkoxy, haloalkyl, hydroxy, amino, dialkylamino, nitro, cyano, acyl, carboxy, ester, carbamoyl, N-alkylcarbamoyl, N,N-dialkylcarbamoyl, acylamino, diacylamino, thiol, alkylthio, alkoxycarbonylamino, sulfamoyl, N-alkylsulfamoyl, N,N-dialkylsulfamoyl or alkoxyalkyloxy, and
   R² is hydrogen,
   an optically active form thereof, a pharmaceutically acceptable salt thereof, a hydrate thereof or a water adduct thereof.
4. The fused heterocyclic compound of the above-mentioned 1, wherein, in the formula (I),
   the dotted line part is a single bond or a double bond,
   ring Ar is a benzene ring, a naphthalene ring or an aromatic heterocycle,
   X is a carbon atom optionally substituted by alkyl or phenyl optionally having substituent(s), or a nitrogen atom,
   Y is
   -(CH₂)ₘ-,
   - (CH₂)ₘ-N(R⁴)-CO-(CH₂)ₙ- or
   - (CH₂)ₘ-CO-(CH₂)ₙ-
   wherein m and n are the same or different and each is 0 or an integer of 1-10, R⁴ is hydrogen, and -(CH₂)ₘ- is linked with a parent-nucleus,
   R¹ and R² are the same or different and each is hydrogen, halogen, alkyl, alkoxy, haloalkyl, hydroxy, amino, dialkylamino, nitro, cyano, carboxy, N,N-dialkylcarbamoyl, alkylthio or alkoxyalkyloxy, and
   R is dialkylamino or morpholino, or represented by the following formula (a)-(d): 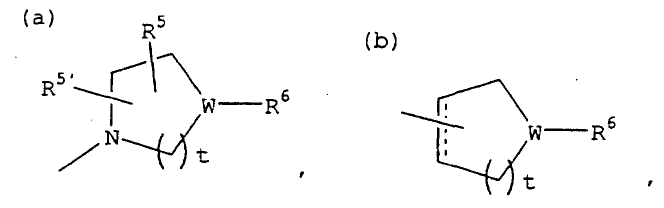 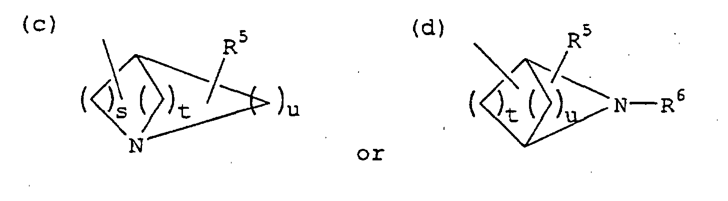 wherein a dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, dialkylamino, alkyl, alkoxycarbonyl, alkylsulfonyl, acylamino, hydroxy, arylalkyl, sulfamoyl or alkylsulfonylamino, or represented by the following formula (f): 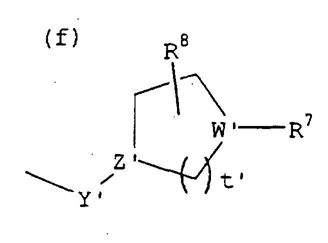 wherein Y' is as defined for Y above, Z' is CH or a nitrogen atom, W' is CH, a nitrogen atom or an oxygen atom, t' is an integer of 1-3, provided that, when the above-mentioned formula (a) is piperazine, then R⁶ may be hydroxyalkyl, R⁷ is hydrogen or alkyl, provided that when W' is an oxygen atom, then R⁷ should be absent, and R⁸ is hydrogen,
   an optically active form thereof, a pharmaceutically acceptable salt thereof, a hydrate thereof or a water adduct thereof.
5. The fused heterocyclic compound of the above-mentioned 1, wherein, in the formula (I),
   the dotted line part is a single bond or a double bond,
   ring Ar is a benzene ring, a naphthalene ring, or an aromatic heterocycle selected from pyridine, pyrazole and thiophene,
   X is a carbon atom optionally substituted by alkyl or phenyl optionally having substituent(s) selected from the group consisting of halogen, alkyl and alkoxy, or a nitrogen atom,
   Y is
   -(CH₂)ₘ-,
   -(CH₂)ₘ-N(R⁴)-CO-(CH₂)ₙ- or
   -(CH₂)ₘ-CO-(CH₂)ₙ-
   wherein m and n are the same or different and each is 0 or an integer of 1-5, R⁴ is hydrogen, and -(CH₂)ₘ- is linked with a parent-nucleus,
   R¹ and R² are the same or different and each is hydrogen, halogen, alkyl, alkoxy, haloalkyl, hydroxy, amino, dialkylamino, nitro, cyano, carboxy, N,N-dialkylcarbamoyl, alkylthio or alkoxyalkyloxy, and
   R is dialkylamino or morpholino, or represented by the following formula (a)-(d): 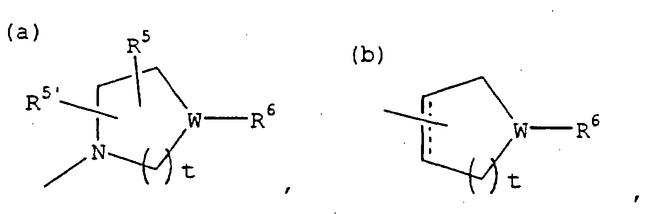 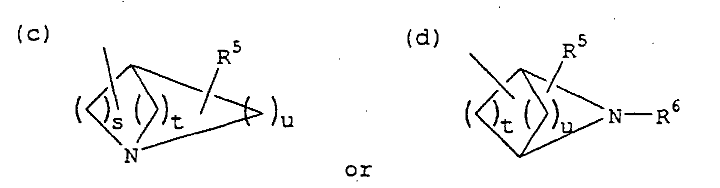 wherein the dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, dialkylamino, alkyl, alkoxycarbonyl, alkylsulfonyl, acylamino, hydroxy, arylalkyl, sulfamoyl or alkylsulfonylamino, or represented by the following formula (f): 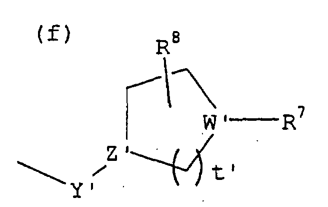 wherein Y' is as defined for Y above, Z' is nitrogen atom, W' is CH, a nitrogen atom or an oxygen atom, t' is an integer of 1-3, provided that when the above-mentioned formula (a) is piperazine, then R⁶ may be hydroxyalkyl, R⁷ is hydrogen or alkyl, provided that when W' is an oxygen atom, then R⁷ should be absent, and R⁸ is hydrogen;
   provided that when X is a nitrogen atom, then R should be represented by the above-mentioned formula (b),
   an optically active form thereof, a pharmaceutically acceptable salt thereof, a hydrate thereof or a water adduct thereof.
6. The fused heterocyclic compound of the above-mentioned 1, which is selected from
   (1) 5-methyl-3-(4-methylpiperazin-1-yl)-2H-isoquinolin-1-one,
   (2) 3-(3-dimethylaminopyrrolidin-1-yl)-2H-isoquinolin-1-one,
   (3) 3-(4-dimethylaminopiperidin-1-yl)-2H-isoquinolin-1-one,
   (4) 3-(3-hydroxymethyl-4-methylpiperazin-1-yl)-2H-isoquinolin-1-one,
   (5) 3-(4-aminopiperazin-1-yl)-2H-isoquinolin-1-one,
   (6) 3-(4-dimethylaminopiperazin-1-yl)-2H-isoquinolin-1-one,
   (7) 3-(4-propylpiperazin-1-yl)-2H-isoquinolin-1-one,
   (8) 3-(4-methanesulfonylpiperazin-1-yl)-2H-isoquinolin-1-one,
   (9) 3-(4-ethoxycarbonylpiperazin-1-yl)-2H-isoquinolin-1-one,
   (10) 3-(4-methylhomopiperazin-1-yl)-2H-isoquinolin-1-one,
   (11) 5-methyl-3-(4-methylhomopiperazin-1-yl)-2H-isoquinolin-1-one,
   (12) 5-methyl-3-(4-dimethylaminopiperid-in-1-yl)-2H-isoquinolin-1-one,
   (13) 3-(3-dimethylaminopyrrolidin-1-yl)-5-methyl-2H-isoquinolin-1-one,
   (14) 5-methyl-3-(4-morpholino)-2H-isoquinolin-1-one,
   (15) 3-(4-aminopiperazin-1-yl)-5-methyl-2H-isoquinolin-1-one,
   (16) 3-(4-dimethylaminopiperazin-1-yl)-5-methyl-2H-isoquinolin-1-one,
   (17) 3-(4-hydroxypiperidin-1-yl)-5-methyl-2H-isoquinolin-1-one,
   (18) 5-methoxy-3-(4-methylpiperazin-1-yl)-2H-isoquinolin-1-one,
   (19) 5-hydroxy-3-(4-methylpiperazin-1-yl)-2H-isoquinolin-1-one,
   (20) 5-fluoro-3-(4-methylpiperazin-1-yl)-2H-isoquinolin-1-one,
   (21) 5-chloro-3-(4-methylpiperazin-1-yl)-2H-isoquinolin-1-one,
   (22) 5-bromo-3-(4-methylpiperazin-1-yl)-2H-isoquinolin-1-one,
   (23) 8-chloro-3-(4-methylpiperazin-1-yl)-2H-isoquinolin-1-one,
   (24) 7-methyl-3-(4-methylpiperazin-1-yl)-2H-isoquinolin-1-one,
   (25) 7-bromo-3-(4-methylpiperazin-1-yl)-2H-isoquinolin-1-one,
   (26) 3-(4-dimethylaminopiperidin-1-yl)-5-methoxy-2H-isoquinolin-1-one,
   (27) 5-hydroxy-3-(4-dimethylaminopiperidin-1-yl)-2H-isoquinolin-1-one,
   (28) 5-fluoro-3-(4-dimethylaminopiperidin-1-yl)-2H-isoquinolin-1-one,
   (29) 5-chloro-3-(4-dimethylaminopiperidin-1-yl)-2H-isoquinolin-1-one,
   (30) 6-chloro-3-(4-methylpiperazin-1-yl)-2H-isoquinolin-1-one,
   (31) 7-bromo-3-(4-dimethylaminopiperidin-1-yl)-2H-isoquinolin-1-one,
   (32) 5-bromo-3-(4-dimethylaminopiperidin-1-yl)-2H-isoquinolin-1-one,
   (33) 5-fluoro-3-(4-(2-hydroxyethyl)piperazin-1-yl)-2H-isoquinolin-1-one,
   (34) 6-methyl-3-(4-methylpiperazin-1-yl)-2H-isoquinolin-1-one,
   (35) 3-(4-(2-hydroxyethyl)piperazin-1-yl)-6-methyl-2H-isoquinolin-1-one,
   (36) 8-methyl-3-(4-methylpiperazin-1-yl)-2H-isoquinolin-1-one,
   (37) 7-bromo-3-(4-(2-hydroxyethyl)piperazin-1-yl)-2H-isoquinolin-1-one,
   (38) 3-(4-methylpiperazin-1-yl)-5-nitro-2H-isoquinolin-1-one,
   (39) 5-amino-3-(4-methylpiperazin-1-yl)-2H-isoquinolin-1-one 1 water adduct,
   (40) 5-cyano-3-(4-methylpiperazin-1-yl)-2H-isoquinolin-1-one,
   (41) 3-[4-(2-hydroxyethyl)piperazin-1-yl]-8-methyl-2H-isoquinolin-1-one,
   (42) 3-(4-methylpiperazin-1-yl)-5-trifluoromethyl-2H-isoquinolin-1-one,
   (43) 3-[4-(2-hydroxyethyl)piperazin-1-yl]-7-methyl-1H-isoquinolin-1-one,
   (44) 3-(4-methylpiperazin-1-yl)-5-methylthio-2H-isoquinolin-1-one,
   (45) 5-dimethylamino-3-(4-methylpiperazin-1-yl)-2H-isoquinolin-1-one,
   (46) 3-(4-dimethylaminopiperidin-1-yl)-5-nitro-2H-isoquinolin-1-one,
   (47) 5-amino-3-(4-dimethylaminopiperidin-1-yl)-2H-isoquinolin-1-one,
   (48) 3-(4-dimethylaminopiperidin-1-yl)-5-trifluoromethyl-2H-isoquinolin-1-one,
   (49) 3-(4-dimethylaminopiperidin-1-yl)-5-methylthio-2H-isoquinolin-1-one,
   (50) 5-cyano-3-(4-dimethylaminopiperidin-1-yl)-2H-isoquinolin-1-one,
   (51) 5,7-dimethyl-3-(4-methylpiperazin-1-yl)-2H-isoquinolin-1-one,
   (52) 5,7-dichloro-3-(4-methylpiperazin-1-yl)-2H-isoquinolin-1-one,
   (53) 5,7-dibromo-3-(4-methylpiperazin-1-yl)-2H-isoquinolin-1-one,
   (54) 5,7-difluoro-3-(4-methylpiperazin-1-yl)-2H-isoquinolin-1-one,
   (55) 5-chloro-7-fluoro-3-(4-methylpiperazin-1-yl)-2H-isoquinolin-1-one,
   (56) 6,7-dihydroxy-3-(4-methylpiperazin-1-yl)-2H-isoquinolin-1-one,
   (57) 5,7-dichloro-3-(4-dimethylaminopiperidin-1-yl)-2H-isoquinolin-1-one,
   (58) 5,7-dibromo-3-(4-dimethylaminopiperidin-1-yl)-2H-isoquinolin-1-one,
   (59) 5-bromo-7-chloro-3-(4-dimethylaminopiperidin-1-yl)-2H-isoquinolin-1-one,
   (60) 6,7-dihydroxy-3-(4-dimethylaminopiperidin-1-yl)-2H-isoquinolin-1-one,
   (61) 3-[4-(4-morpholino)piperidin-1-yl]-2H-isoquinolin-1-one,
   (62) 3-{4-[2-(piperidin-1-yl)ethyl]piperazin-1-yl}-2H-isoquinolin-1-one,
   (63) 3-{4-[3-(piperidin-1-yl)propyl]piperazin-1-yl}-2H-isoquinolin-1-one,
   (64) 3-{4-[4-(4-morpholino)butyl]piperazin-1-yl}-2H-isoquinolin-1-one,
   (65) 3-{4-[4-(piperidin-1-yl)butyl]piperazin-1-yl}-2H-isoquinolin-1-one,
   (66) 3-{4-[5-(piperidin-1-yl)pentyl]piperazin-1-yl}-2H-isoquinolin-1-one,
   (67) 3-(4-(4-(4-methylpiperazin-1-yl)butyl)piperazin-1-yl)-2H-isoquinolin-1-one,
   (69) 5-methyl-3-[4-(4-morpholino)piperidin-1-yl]-2H-isoquinolin-1-one,
   (70) 5-methyl-3-{4-[2-(piperidin-1-yl)ethyl]piperazin-1-yl}-2H-isoquinolin-1-one,
   (71) 5-methyl-3-{4-[3-(piperidin-1-yl)propyl]piperazin-1-yl}-2H-isoquinolin-1-one,
   (72) 5-methyl-3-{4-[5-(piperidin-1-yl)pentyl]piperazin-1-yl}-2H-isoquinolin-1-one,
   (73) 5-methyl-3-{4-[4-(piperidin-1-yl)butyl]piperazin-1-yl}-2H-isoquinolin-1-one,
   (74) 5-methyl-3-{4-[4-(4-morpholino)butyl]piperazin-1-yl}-2H-isoquinolin-1-one,
   (75) 5-methyl-3-(4-(4-(4-methylpiperazin-1-yl)butyl)piperazin-1-yl)-2H-isoquinolin-1-one,
   (76) 7-bromo-3-{4-[4-(piperidin-1-yl)butyl]piperazin-1-yl}-2H-isoquinolin-1-one,
   (77) 5-chloro-3-{4-[4-(piperidin-1-yl)butyl]piperazin-1-yl}-2H-isoquinolin-1-one,
   (78) 5-bromo-3-{4-[4-(piperidin-1-yl)butyl]piperazin-1-yl}-2H-isoquinolin-1-one,
   (80) 5-chloro-3-{4-[4-(4-morpholino)butyl]piperazin-1-yl}-2H-isoquinolin-1-one,
   (81) 3-(piperidin-4-yl)-2H-isoquinolin-1-one hydrobromide,
   (82) 3-(1-methylpiperidin-4-yl)-2H-isoquinolin-1-one 1/5 water adduct,
   (83) 3-((4-methylpiperazin-1-yl)carbonyl)-2H-isoquinolin-1-one,
   (84) 3-(2-(dimethylamino)ethyl)-2H-isoquinolin-1-one,
   (85) 3-(3-(dimethylamino)propyl)-2H-isoquinolin-1-one,
   (86) 3-(1-azabicyclo[2.2.2]octan-3-yl)-2H-isoquinolin-1-one,
   (87) 3-((1-azabicyclo[2.2.2]octan-3-yl)methyl)-2H-isoquinolin-1-one,
   (88) 3-(8-methyl-8-azabicyclo[3.2.1]octan-3-yl)-2H-isoquinolin-1-one,
   (89) 5-methyl-3-(2-(dimethylamino)ethyl)-2H-isoquinolin-1-one,
   (90) 3-(3-(dimethylamino)propyl)-5-methyl-2H-isoquinolin-1-one,
   (91) 3-(1-azabicyclo[2.2.2]octan-3-yl)-5-methyl-2H-isoquinolin-1-one,
   (92) 3-((1-azabicyclo[2.2.2]octan-3-yl)methyl)-5-methyl-2H-isoquinolin-1-one,
   (93) 3-(piperidin-4-yl)-5-methyl-2H-isoquinolin-1-one hydrochloride,
   (94) 5-methyl-3-(8-methyl-8-azabicyclo[3.2.1]octan-3-yl)-2H-isoquinolin-1-one,
   (95) 5-chloro-3-(1-methylpiperidin-4-yl)-2H-isoquinolin-1-one,
   (96) 5-bromo-3-(1-methylpiperidin-4-yl)-2H-isoquinolin-1-one,
   (97) 4-phenyl-3-(piperidin-4-yl)-2H-isoquinolin-1-one hydrobromide,
   (98) 3-(1-methylpiperidin-4-yl)-4-phenyl-2H-isoquinolin-1-one 1/5 water adduct,
   (99) 4-(4-methoxyphenyl)-3-(1-methylpiperidin-4-yl)-2H-isoquinolin-1-one,
   (100) 4-(4-chlorophenyl)-3-(1-methylpiperidin-4-yl)-2H-isoquinolin-1-one,
   (101) 5,7-dibromo-3-(piperidin-4-yl)-2H-isoquinolin-1-one,
   (102) 5-methoxy-3-(piperidin-4-yl)-2H-isoquinolin-1-one,
   (103) 5-hydroxy-3-(piperidin-4-yl)-2H-isoquinolin-1-one,
   (104) 5-fluoro-3-(piperidin-4-yl)-2H-isoquinolin-1-one,
   (105) 3-(1-methylpiperidin-4-yl)-5-trifluoromethyl-2H-isoquinolin-1-one,
   (106) 5-fluoro-3-(1-methylpiperidin-4-yl)-2H-isoquinolin-1-one,
   (107) 5-methoxy-3-(1-methylpiperidin-4-yl)-2H-isoquinolin-1-one,
   (108) 5-hydroxy-3-(1-methylpiperidin-4-yl)-2H-isoquinolin-1-one,
   (109) 5-chloro-3-(1-methylpiperidin-4-yl)-4-phenyl-2H-isoquinolin-1-one,
   (110) 5-(4-methylpiperazin-1-yl)-6H-thieno[2,3-c]pyridin-7-one,
   (111) 5-(4-dimethylaminopiperidin-1-yl)-6H-thieno[2,3-c]pyridin-7-one,
   (112) 6-(4-methylpiperazin-1-yl)-5H-thieno[3,2-c]pyridin-4-one,
   (113) 6-(4-dimethylaminopiperidin-1-yl)-5H-thieno[3,2-c]pyridin-4-one,
   (114) 3-(4-methylpiperazin-1-yl)-2H-benz[f]isoquinolin-1-one,
   (115) 3-(4-dimethylaminopiperidin-1-yl)-2H-benz[f]isoquinolin-1-one,
   (116) 3-(4-methylpiperazin-1-yl)-2H-benz[h]isoquinolin-1-one,
   (117) 3-(4-dimethylaminopiperidin-1-yl)-2H-benz[h]isoquinolin-1-one,
   (118) 7-(4-methylpiperazin-1-yl)-6H-1,6-naphthyridin-5-one,
   (119) 7-(4-dimethylaminopiperidin-1-yl)-6H-1,6-naphthyridin-5-one,
   (120) 8-methyl-2-(piperidin-4-yl)-3H-quinazolin-4-one,
   (121) 2-(1-methylpiperidin-4-yl)-8-methyl-3H-quinazolin-4-one,
   (123) 8-methoxy-2-(1-methylpiperidin-4-yl)-3H-quinazolin-4-one,
   (124) 8-hydroxy-2-(1-methylpiperidin-4-yl)-3H-quinazolin-4-one,
   (125) 8-fluoro-2-(1-methylpiperidin-4-yl)-3H-quinazolin-4-one,
   (126) 8-chloro-2-(1-methylpiperidin-4-yl)-3H-quinazolin-4-one,
   (127) 8-bromo-2-(1-methylpiperidin-4-yl)-3H-quinazolin-4-one,
   (128) 8-methoxy-2-(4-dimethylaminocyclohexan-1-yl)-3H-quinazolin-4-one,
   (129) 8-hydroxy-2-(4-dimethylaminocyclohexan-1-yl)-3H-quinazolin-4-one,
   (130) 8-fluoro-2-(4-dimethylaminocyclohexan-1-yl)-3H-quinazolin-4-one,
   (131) 8-chloro-2-(4-dimethylaminocyclohexan-1-yl)-3H-quinazolin-4-one,
   (132) 8-bromo-2-(4-dimethylaminocyclohexan-1-yl)-3H-quinazolin-4-one,
   (133) 2-(1-azabicyclo[2.2.2]octan-3-yl)-3H-quinazolin-4-one,
   (134) 2-((1-azabicyclo[2.2.2]octan-3-yl)methyl)-3H-quinazolin-4-one,
   (135) 2-(8-methyl-8-azabicyclo[3.2.1]octan-3-yl)-3H-quinazolin-4-one,
   (136) 2-(4-dimethylaminocyclohexan-1-yl)-3H-quinazolin-4-one,
   (137) 8-methyl-2-(4-dimethylaminocyclohexan-1-yl)-3H-quinazolin-4-one,
   (138) 2-(1-azabicyclo[2.2.2]octan-3-yl)-8-methyl-3H-quinazolin-4-one,
   (139) 2-((1-azabicyclo[2.2.2]octan-3-yl)methyl)-8-methyl-3H-quinazolin-4-one,
   (140) 2-(2-(dimethylamino)ethyl)-3H-quinazolin-4-one,
   (141) 2-(3-(dimethylamino)propyl)-3H-quinazolin-4-one,
   (142) 2-(5-(dimethylamino)pentyl)-3H-quinazolin-4-one,
   (143) 8-methyl-2-(8-methyl-8-azabicyclo[3.2.1]octan-3-yl)-3H-quinazolin-4-one,
   (144) 8-methyl-2-(2-(dimethylamino)ethyl)-3H-quinazolin-4-one,
   (145) 8-methyl-2-(3-(dimethylamino)propyl)-3H-quinazolin-4-one,
   (146) 8-methyl-2-(5-(dimethylamino)pentyl)-3H-quinazolin-4-one,
   (147) 3-(4-(dimethylamino)cyclohexan-1-yl)-2H-isoquinolin-1-one, and
   (148) 3-(3-(4-methylpiperazin-1-yl)propyl)-2H-isoquinolin-1-one,
      an optically active form thereof, a pharmaceutically acceptable salt thereof, a hydrate thereof or a water adduct thereof.
7. The fused heterocyclic compound of the above-mentioned 1, which is selected from
   (151) (R)-3-(2-hydroxymethyl-4-methylpiperazin-1-yl)-2H-isoquinolin-1-one,
   (152) (S)-3-(2-hydroxymethyl-4-methylpiperazin-1-yl)-2H-isoquinolin-1-one,
   (153) 3-(3-hydroxymethyl-4-methylpiperazin-1-yl)-5-methyl-2H-isoquinolin-1-one,
   (154) 3-(3-ethoxycarbonyl-4-methylpiperazin-1-yl)-2H-isoquinolin-1-one,
   (155) 3-(3-methylpiperazin-1-yl)-2H-isoquinolin-1-one,
   (156) (S)-3-(3-hydroxymethyl-4-methylpiperazin-1-yl)-2H-isoquinolin-1-one,
   (157) (R)-3-(3-hydroxymethyl-4-methylpiperazin-1-yl)-2H-isoquinolin-1-one,
   (158) 3-(1-methylpiperidin-4-yl)-3,4-dihydro-2H-isoquinolin-1-one,
   (159) 8-methyl-2-[2-(diethylamino)ethyl]-3H-quinazolin-4-one,
   (162) 3-(3,5-dimethylpiperazin-1-yl)-2H-isoquinolin-1-one,
   (163) 4-(4-methylphenyl)-3-(1-methylpiperidin-4-yl)-2H-isoquinolin-1-one,
   (164) 4-(4-fluorophenyl)-3-(1-methylpiperidin-4-yl)-2H-isoquinolin-1-one,
   (165) 8-methyl-2-(2-piperidinoethyl)-3H-quinazolin-4-one,
   (166) 8-methyl-2-[2-(morpholin-4-yl)ethyl]-3H-quinazolin-4-one,
   (167) 4-(2-methoxyphenyl)-3-(1-methylpiperidin-4-yl)-2H-isoquinolin-1-one,
   (168) 4-(3-methylphenyl)-3-(1-methylpiperidin-4-yl)-2H-isoquinolin-1-one,
   (169) 4-(2-methylphenyl)-3-(1-methylpiperidin-4-yl)-2H-isoquinolin-1-one,
   (170) 4-(3-methoxyphenyl)-3-(1-methylpiperidin-4-yl)-2H-isoquinolin-1-one,
   (171) 5-methoxymethyloxy-3-(1-methylpiperidin-4-yl)-2H-isoquinolin-1-one,
   (173) 8-methyl-2-[5-(diethylamino)pentyl]-3H-quinazolin-4-one,
   (174) 8-methyl-2-[4-(diethylamino)butyl]-3H-quinazolin-4-one,
   (175) 8-methyl-2-[4-(dimethylamino)butyl]-3H-quinazolin-4-one,
   (176) 8-methyl-2-[3-(pyrrolidin-1-yl)propyl]-3H-quinazolin-4-one,
   (177) 7-(1-methylpiperidin-4-yl)-6H-1,6-naphthyridin-5-one 1/10 water adduct,
   (178) 5-methyl-3-(1-methylpiperidin-4-yl)-2H-isoquinolin-1-one,
   (179) 4-methyl-3-(1-methylpiperidin-4-yl)-2H-isoquinolin-1-one,
   (180) 5-(dimethylamino)-3-(1-methylpiperidin-4-yl)-2H-isoquinolin-1-one,
   (181) 5-amino-3-(1-methylpiperidin-4-yl)-2H-isoquinolin-1-one,
   (182) 4-(2-fluorophenyl)-3-(1-methylpiperidin-4-yl)-2H-isoquinolin-1-one,
   (183) 7-chloro-3-(1-methylpiperidin-4-yl)-2H-isoquinolin-1-one,
   (184) 5-hydroxy-3-(1-methylpiperidin-4-yl)-3,4-dihydro-2H-isoquinolin-1-one,
   (185) 5-methoxymethyloxy-3-(3-dimethylaminopropyl)-2H-isoquinolin-1-one,
   (186) 5-hydroxy-3-(3-dimethylaminopropyl)-2H-isoquinolin-1-one hydrochloride,
   (187) 5-methoxymethyloxy-3-(4-dimethylaminobutyl)-2H-isoquinolin-1-one,
   (188) 5-hydroxy-3-(4-dimethylaminobutyl)-2H-isoquinolin-1-one hydrochloride,
   (189) 5-hydroxy-3-(2-(piperidin-1-yl)ethyl)-2H-isoquinolin-1-one,
   (190) 3-(1-methyl-1,2,3,6-tetrahydropyridin-4-yl)-2H-isoquinolin-1-one,
   (191) 3-(1-benzylpiperidin-3-yl)-2H-isoquinolin-1-one,
   (192) 3-(1-methylpiperidin-3-yl)-2H-isoquinolin-1-one,
   (193) 3-(1-methyl-1,2,3,6-tetrahydropyridin-5-yl)-2H-isoquinolin-1-one,
   (194) 3-(3-hydroxymethylpiperazin-1-yl)-2H-isoquinolin-1-one,
   (195) 3-(4-ethyl-3-hydroxymethylpiperazin-1-yl)-2H-isoquinolin-1-one,
   (196) 3-(3-hydroxymethyl-4-propylpiperazin-1-yl)-2H-isoquinolin-1-one,
   (197) 3-(4-benzyl-3-hydroxymethylpiperazin-1-yl)-2H-isoquinolin-1-one,
   (198) 5-bromo-3-(3-hydroxymethylpiperazin-1-yl)-2H-isoquinolin-1-one,
   (199) 5-bromo-3-(3-hydroxymethyl-4-methylpiperazin-1-yl)-2H-isoquinolin-1-one,
   (200) 3-(4-piperidinopiperidin-1-yl)-2H-isoguinolin-1-one,
   (201) 3-(3-hydroxymethylpiperidin-1-yl)-2H-isoquinolin-1-one,
   (202) 3-(3-(dimethylcarbamoyl)piperidin-1-yl)-2H-isoquinolin-1-one,
   (203) 3-(3-hydroxymethyl-4-isobutylpiperazin-1-yl)-2H-isoquinolin-1-one,
   (204) 3-[4-(dimethylamino)butyl]-2H-isoquinolin-1-one,
   (205) 5-fluoro-3-(3-hydroxymethyl-4-methylpiperazin-1-yl)-2H-isoquinolin-1-one,
   (206) 3-(3-(dimethylaminomethyl)piperidin-1-yl)-2H-isoquinolin-1-one,
   (207) 6-methyl-3-(1-methylpiperidin-4-yl)-2H-isoquinolin-1-one,
   (208) 7-methyl-3-(1-methylpiperidin-4-yl)-2H-isoquinolin-1-one,
   (209) 8-methyl-3-(1-methylpiperidin-4-yl)-2H-isoquinolin-1-one,
   (210) 7-methoxymethyloxy-3-(1-methylpiperidin-4-yl)-2H-isoquinolin-1-one,
   (211) 7-hydroxy-3-(1-methylpiperidin-4-yl)-2H-isoquinolin-1-one hydrochloride,
   (212) (S)-3-(3-hydroxymethyl-4-methylpiperazin-1-yl)-5-methyl-2H-isoquinolin-1-one,
   (213) 3-(3-(pyrrolidin-1-yl)propyl)-2H-isoquinolin-1-one,
   (214) 5-chloro-3-(3-hydroxymethyl-4-methylpiperazin-1-yl)-2H-isoquinolin-1-one,
   (216) 3-[3-(piperidin-1-yl)propyl]-2H-isoquinolin-1-one,
   (217) 5-hydroxy-3-(3-(pyrrolidin-1-yl)propyl)-2H-isoquinolin-1-one,
   (218) 5-methyl-3-[2-(piperidin-1-yl)ethyl]-2H-isoquinolin-1-one,
   (219) 3-[2-(piperidin-1-yl)ethyl]-2H-isoquinolin-1-one,
   (220) 3-[2-(pyrrolidin-1-yl)ethyl]-2H-isoquinolin-1-one,
   (221) 5-methyl-3-[2-(pyrrolidin-1-yl)ethyl]-2H-isoquinolin-1-one,
   (222) 5-methyl-3-[3-(pyrrolidin-1-yl)propyl-1-yl]-2H-isoquinolin-1-one,
   (223) 1,5-dihydro-6-(1-methyl-1,2,3,6-tetrahydropyridin-4-yl)pyrazolo[4,3-c]pyridin-4-one,
   (224) N,N-dimethyl-3-(1-methylpiperidin-1-yl)-2H-1-oxoisoquinoline-5-carboxamide 1/4 water adduct,
   (225) 5-methyl-3-(octahydroindolizin-7-yl)-2H-isoquinolin-1-one 3/4 water adduct,
   (226) 5-methyl-3-(octahydroindolizin-7-yl)-2H-isoquinolin-1-one 1/2 water adduct,
   (227) 3-(1-methylpiperidin-1-yl)-2H-1-oxoisoquinoline-5-carboxylic acid hydrochloride,
   (228) 5-methyl-3-[3-(piperidin-1-yl)propyl]-2H-isoquinolin-1-one,
   (229) 3-(dimethylamino)methyl-2H-isoquinolin-1-one,
   (230) 3-[(4-methylpiperazin-1-yl)methyl]-2H-isoquinolin-1-one,
   (231) 3-(piperidinomethyl)-2H-isoquinolin-1-one,
   (232) 3-[(morpholin-1-yl)methyl]-2H-isoquinolin-1-one,
   (233) 3-[(homopiperidin-1-yl)methyl]-2H-isoquinolin-1-one,
   (234) 3-[3-(homopiperidin-1-yl)propyl]-2H-isoquinolin-1-one,
   (235) 3-(1-sulfamoylpiperidin-4-yl)-5-methyl-2H-isoquinolin-1-one 1/4 water adduct,
   (236) 3-(4-methyl-3-oxopiperazin-1-yl)-5-methyl-2H-isoquinolin-1-one 1/10 water adduct,
   (237) 3-(1-aminopiperidin-4-yl)-5-methyl-2H-isoquinolin-1-one,
   (238) 3-(1-(methanesulfonylamino)piperidin-4-yl)-5-methyl-2H-isoquinolin-1-one,
   (239) 3-(1-trifluoroacetaminopiperidin-4-yl)-5-methyl-2H-isoquinolin-1-one,
   (240) 3-[2-(homopiperidin-1-yl)ethyl]-2H-isoquinolin-1-one,
   (241) N-(5-methyl-2H-1-oxoisoquinolin-3-yl)-2-(dimethylamino)acetamide,
   (243) N-(5-methyl-2H-1-oxoisoquinolin-3-yl)-3-(dimethylamino)propanamide,
   (244) 3-(1-dimethylaminopiperidin-4-yl)-5-methyl-2H-isoquinolin-1-one,
   (245) N-(5-methyl-2H-1-oxoisoquinolin-3-yl)-4-(dimethylamino)butanamide,
   (246) N-(2H-1-oxoisoquinolin-3-yl)-4-(dimethylamino)butanamide,
   (247) 3-(4-methyl-2-oxopiperazin-1-yl)-2H-isoquinolin-1-one,
   (248) 5-methyl-3-(1-methylpyrrolidin-3-yl)-2H-isoquinolin-1-one, and
   (249) 3-(1-methylpyrrolidin-3-yl)-2H-isoquinolin-1-one, an optically active form thereof, a pharmaceutically acceptable salt thereof, a hydrate thereof or a water adduct thereof.
8. The fused heterocyclic compound of the above-mentioned 1, wherein, in the formula (I),
   the dotted line part is a single bond or a double bond,
   ring Ar is a benzene ring, a naphthalene ring or an aromatic heterocycle,
   X is a carbon atom optionally substituted by alkyl or phenyl optionally having substituent(s), or a nitrogen atom,
   Y is
   - (CH₂)ₘ-,
   - (CH₂)ₘ-N(R⁴)-CO-(CH₂)ₙ- or
   - (CH₂)ₘ-CO-(CH₂)ₙ-
   wherein m and n are the same or different and each is 0 or an integer of 1-10, R⁴ is hydrogen, and -(CH₂)ₘ- is linked with a parent-nucleus,
   R¹ and R² are the same or different and each is hydrogen, halogen, alkyl, alkoxy, hydroxy, amino, dialkylamino, nitro, carboxy, N,N-dialkylcarbamoyl or alkoxyalkyloxy, and
   R is dialkylamino or morpholino, or represented by the following formula (a)-(c): 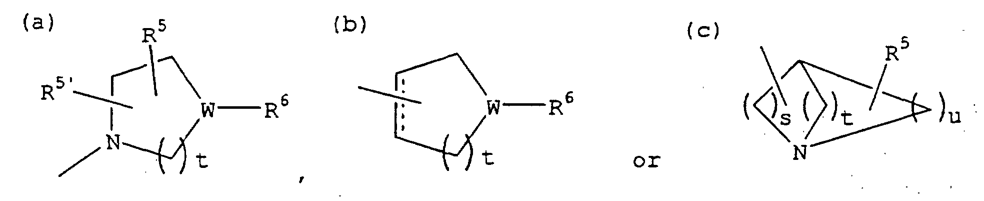 wherein the dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, dialkylamino, alkyl, alkoxycarbonyl, alkylsulfonyl, acylamino, hydroxy, arylalkyl, sulfamoyl or alkylsulfonylamino, or represented by the following formula (f): 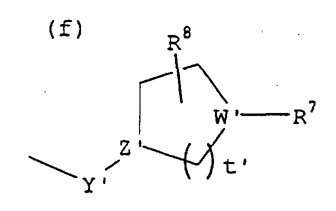 wherein Y' is as defined for Y above, Z' is a nitrogen atom, W' is CH, a nitrogen atom or an oxygen atom, t' is an integer of 1-3, provided that when the above-mentioned formula (a) is piperazine, then R⁶ may be hydroxyalkyl, R⁷ is hydrogen or alkyl, provided that when W' is an oxygen atom, then R⁷ should be absent, and R⁸ is hydrogen,
   an optically active form thereof, a pharmaceutically acceptable salt thereof, a hydrate thereof or a water adduct thereof.
9. The fused heterocyclic compound of the above-mentioned 1, wherein, in the formula (I),
   the dotted line part is a single bond or a double bond, ring Ar is a benzene ring or a naphthalene ring, or an aromatic heterocycle selected from the group consisting of pyridine, pyrazole and thiophene,
   X is a carbon atom optionally substituted by phenyl optionally having substituent(s) selected from the group consisting of halogen, alkyl and alkoxy, or a nitrogen atom,
   Y is
   -(CH₂)ₘ-,
   -(CH₂)ₘ-N(R⁴)-CO-(CH₂)ₙ- or
   -(CH₂)ₘ-CO-(CH₂)ₙ-
   wherein m and n are the same or different and each is 0 or an integer of 1-5, R⁴ is hydrogen, and -(CH₂)ₘ- is linked with a parent-nucleus,
   R¹ and R² are the same or different and each is hydrogen, halogen, alkyl, alkoxy, hydroxy, amino, dialkylamino, carboxy, N,N-dialkylcarbamoyl or alkoxyalkyloxy, and
   R is dialkylamino or morpholino, or represented by the following formula (a)-(c): 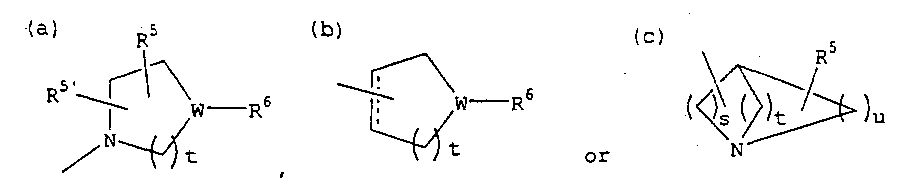 wherein the dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, dialkylamino, alkyl, alkoxycarbonyl, alkylsulfonyl, acylamino, hydroxy, arylalkyl or alkylsulfonylamino, or represented by the following formula (f) : 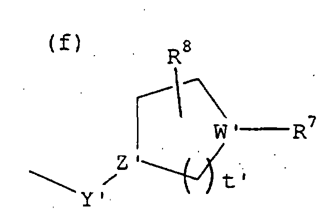 wherein Y' is as defined for Y above, Z' is a nitrogen atom, W' is CH, a nitrogen atom or an oxygen atom, t' is an integer of 1-3, provided that when the above-mentioned formula (a) is piperazine, then R⁶ may be hydroxyalkyl, R⁷ is hydrogen or alkyl, provided that when W' is an oxygen atom, then R⁷ should be absent, and R⁸ is hydrogen,
   provided that when X is a nitrogen atom, then R should be represented by the above-mentioned formula (b), an optically active form thereof, a pharmaceutically acceptable salt thereof, a hydrate thereof or a water adduct thereof.
10. The fused heterocyclic compound of the above-mentioned 1, which is selected from
   (1) 5-methyl-3-(4-methylpiperazin-1-yl)-2H-isoquinolin-1-one,
   (2) 3-(3-dimethylaminopyrrolidin-1-yl)-2H-isoquinolin-1-one,
   (3) 3-(4-dimethylaminopiperidin-1-yl)-2H-isoquinolin-1-one,
   (4) 3-(3-hydroxymethyl-4-methylpiperazin-1-yl)-2H-isoquinolin-1-one,
   (7) 3-(4-propylpiperazin-1-yl)-2H-isoquinolin-1-one,
   (8) 3-(4-methanesulfonylpiperazin-1-yl)-2H-isoquinolin-1-one,
   (9) 3-(4-ethoxycarbonylpiperazin-1-yl)-2H-isoquinolin-1-one,
   (10) 3-(4-methylhomopiperazin-1-yl)-2H-isoquinolin-1-one,
   (11) 5-methyl-3-(4-methylhomopiperazin-1-yl)-2H-isoquinolin-1-one,
   (12) 5-methyl-3-(4-dimethylaminopiperidin-1-yl)-2H-isoquinolin-1-one,
   (13) 3-(3-dimethylaminopyrrolidin-1-yl)-5-methyl-2H-isoquinolin-1-one,
   (14) 5-methyl-3-(4-morpholino)-2H-isoquinolin-1-one,
   (17) 3-(4-hydroxypiperidin-1-yl)-5-methyl-2H-isoquinolin-1-one,
   (18) 5-methoxy-3-(4-methylpiperazin-1-yl)-2H-isoquinolin-1-one,
   (19) 5-hydroxy-3-(4-methylpiperazin-1-yl)-2H-isoquinolin-1-one,
   (20) 5-fluoro-3-(4-methylpiperazin-1-yl)-2H-isoquinolin-1-one,
   (21) 5-chloro-3-(4-methylpiperazin-1-yl)-2H-isoquinolin-1-one,
   (22) 5-bromo-3-(4-methylpiperazin-1-yl)-2H-isoquinolin-1-one,
   (23) 8-chloro-3-(4-methylpiperazin-1-yl)-2H-isoquinolin-1-one,
   (24) 7-methyl-3-(4-methylpiperazin-1-yl)-2H-isoquinolin-1-one,
   (25) 7-bromo-3-(4-methylpiperazin-2-yl)-2H-isoquinolin-1-one,
   (28) 5-fluoro-3-(4-dimethylaminopiperidin-1-yl)-2H-isoquinolin-1-one,
   (29) 5-chloro-3-(4-dimethylaminopiperidin-1-yl)-2H-isoquinolin-1-one,
   (30) 6-chloro-3-(4-methylpiperazin-1-yl)-2H-isoquinolin-1-one,
   (31) 7-bromo-3-(4-dimethylaminopiperidin-1-yl)-2H-isoquinolin-1-one,
   (32) 5-bromo-3-(4-dimethylaminopiperidin-1-yl)-2H-isoquinolin-1-one,
   (33) 5-fluoro-3-(4-(2-hydroxyethyl)piperazin-1-yl)-2H-isoquinolin-1-one,
   (34) 6-methyl-3-(4-methylpiperazin-1-yl)-2H-isoquinolin-1-one,
   (35) 3-(4-(2-hydroxyethyl)piperazin-1-yl)-6-methyl-2H-isoquinolin-1-one,
   (36) 8-methyl-3-(4-methylpiperazin-1-yl)-2H-isoquinolin-1-one,
   (37) 7-bromo-3-(4-(2-hydroxyethyl)piperazin-1-yl)-2H-isoquinolin-1-one,
   (38) 3-(4-methylpiperazin-1-yl)-5-nitro-2H-isoquinolin-1-one,
   (39) 5-amino-3-(4-methylpiperazin-1-yl)-2H-isoquinolin-1-one 1 water adduct,
   (41) 3-[4-(2-hydroxyethyl)piperazin-1-yl]-8-methyl-2H-isoquinolin-1-one,
   (43) 3-[4-(2-hydroxyethyl)piperazin-1-yl]-7-methyl-1H-isoquinolin-1-one,
   (62) 3-{4-[2-(piperidin-1-yl)ethyl]piperazin-1-yl}-2H-isoquinolin-1-one,
   (63) 3-{4-[3-(piperidin-1-yl)propyl]piperazin-1-yl}-2H-isoquinolin-1-one,
   (65) 3-{4-[4-(piperidin-1-yl)butyl]piperazin-1-yl}-2H-isoquinolin-1-one,
   (66) 3-{4-[5-(piperidin-1-yl)pentyl]piperazin-1-yl}-2H-isoquinolin-1-one,
   (69) 5-methyl-3-[4-(4-morpholino)piperidin-1-yl]-2H-isoquinolin-1-one,
   (70) 5-methyl-3-{4-[2-(piperidin-1-yl)ethyl]piperazin-1-yl}-2H-isoquinolin-1-one,
   (71) 5-methyl-3-{4-[3-(piperidin-1-yl)propyl]piperazin-1-yl}-2H-isoquinolin-1-one,
   (72) 5-methyl-3-{4-[5-(piperidin-1-yl)pentyl]piperazin-1-yl}-2H-isoquinolin-1-one,
   (73) 5-methyl-3-{4-[4-(piperidin-1-yl)butyl]piperazin-1-yl}-2H-isoquinolin-1-one,
   (74) 5-methyl-3-{4-[4-(4-morpholino)butyl]piperazin-1-yl}-2H-isoquinolin-1-one,
   (75) 5-methyl-3-(4-(4-(4-methylpiperazin-1-yl)butyl)piperazin-1-yl)-2H-isoquinolin-1-one,
   (76) 7-bromo-3-{4-[4-(piperidin-1-yl)butyl]piperazin-1-yl}-2H-isoquinolin-1-one,
   (77) 5-chloro-3-{4-[4-(piperidin-1-yl)butyl]piperazin-1-yl}-2H-isoquinolin-1-one,
   (78) 5-bromo-3-{4-[4-(piperidin-1-yl)butyl]piperazin-1-yl}-2H-isoquinolin-1-one,
   (80) 5-chloro-3-{4-[4-(4-morpholino)butyl]piperazin-1-yl}-2H-isoquinolin-1-one,
   (81) 3-(piperidin-4-yl)-2H-isoquinolin-1-one hydrobromide,
   (82) 3-(1-methylpiperidin-4-yl)-2H-isoquinolin-1-one 1/5 water adduct,
   (83) 3-((4-methylpiperazin-1-yl)carbonyl)-2H-isoquinolin-1-one,
   (90) 3-(3-(dimethylamino)propyl)-5-methyl-2H-isoquinolin-1-one,
   (93) 3-(piperidin-4-yl)-5-methyl-2H-isoquinolin-1-one hydrochloride,
   (95) 5-chloro-3-(1-methylpiperidin-4-yl)-2H-isoquinolin-1-one,
   (97) 4-phenyl-3-(piperidin-4-yl)-2H-isoquinolin-1-one hydrobromide,
   (98) 3-(1-methylpiperidin-4-yl)-4-phenyl-2H-isoquinolin-1-one 1/5 water adduct,
   (99) 4-(4-methoxyphenyl)-3-(1-methylpiperidin-4-yl)-2H-isoquinolin-1-one,
   (100) 4-(4-chlorophenyl)-3-(1-methylpiperidin-4-yl)-2H-isoquinolin-1-one,
   (106) 5-fluoro-3-(1-methylpiperidin-4-yl)-2H-isoquinolin-1-one,
   (107) 5-methoxy-3-(1-methylpiperidin-4-yl)-2H-isoquinolin-1-one,
   (108) 5-hydroxy-3-(1-methylpiperidin-4-yl)-2H-isoquinolin-1-one,
   (110) 5-(4-methylpiperazin-1-yl)-6H-thieno[2,3-c]pyridin-7-one,
   (112) 6-(4-methylpiperazin-1-yl)-5H-thieno[3,2-c]pyridin-4-one,
   (114) 3-(4-methylpiperazin-1-yl)-2H-benz[f]isoquinolin-1-one,
   (115) 3-(4-dimethylaminopiperidin-1-yl)-2H-benz[f]isoquinolin-1-one,
   (116) 3-(4-methylpiperazin-1-yl)-2H-benz[h]isoquinolin-1-one,
   (117) 3-(4-dimethylaminopiperidin-1-yl)-2H-benz[h]isoquinolin-1-one,
   (120) 8-methyl-2-(piperidin-4-yl)-3H-quinazolin-4-one,
   (121) 2-(1-methylpiperidin-4-yl)-8-methyl-3H-quinazolin-4-one,
   (123) 8-methoxy-2-(1-methylpiperidin-4-yl)-3H-quinazolin-4-one,
   (137) 8-methyl-2-(4-dimethylaminocyclohexan-1-yl)-3H-quinazolin-4-one,
   (138) 2-(1-azabicyclo[2.2.2]octan-3-yl)-8-methyl-3H-quinazolin-4-one,
   (139) 2-((1-azabicyclo[2.2.2]octan-3-yl)methyl)-8-methyl-3H-quinazolin-4-one,
   (144) 8-methyl-2-(2-(dimethylamino)ethyl)-3H-quinazolin-4-one,
   (145) 8-methyl-2-(3-(dimethylamino)propyl)-3H-quinazolin-4-one,
   (146) 8-methyl-2-(5-(dimethylamino)pentyl)-3H-quinazolin-4-one,
   (147) 3-(4-(dimethylamino)cyclohexan-1-yl)-2H-isoquinolin-1-one, and
   (148) 3-(3-(4-methylpiperazin-1-yl)propyl)-2H-isoquinolin-1-one,
   an optically active form thereof, a pharmaceutically acceptable salt thereof, a hydrate thereof or a water adduct thereof.
11. The fused heterocyclic compound of the above-mentioned 1, which is selected from
   (151) (R)-3-(2-hydroxymethyl-4-methylpiperazin-1-yl)-2H-isoquinolin-1-one,
   (152) (S)-3-(2-hydroxymethyl-4-methylpiperazin-1-yl)-2H-isoquinolin-1-one,
   (153) 3-(3-hydroxymethyl-4-methylpiperazin-1-yl)-5-methyl-2H-isoquinolin-1-one,
   (154) 3-(3-ethoxycarbonyl-4-methylpiperazin-1-yl)-2H-isoquinolin-1-one,
   (155) 3-(3-methylpiperazin-1-yl)-2H-isoquinolin-1-one,
   (156) (S)-3-(3-hydroxymethyl-4-methylpiperazin-1-yl)-2H-isoquinolin-1-one,
   (157) (R)-3-(3-hydroxymethyl-4-methylpiperazin-1-yl)-2H-isoquinolin-1-one,
   (158) 3-(1-methylpiperidin-4-yl)-3,4-dihydro-2H-isoquinolin-1-one,
   (159) 8-methyl-2-[2-(diethylamino)ethyl]-3H-quinazolin-4-one,
   (162) 3-(3,5-dimethylpiperazin-1-yl)-2H-isoquinolin-1-one,
   (163) 4-(4-methylphenyl)-3-(1-methylpiperidin-4-yl)-2H-isoquinolin-1-one,
   (164) 4-(4-fluorophenyl)-3-(1-methylpiperidin-4-yl)-2H-isoquinolin-1-one,
   (165) 8-methyl-2-(2-piperidinoethyl)-3H-quinazolin-4-one,
   (166) 8-methyl-2-[2-(morpholin-4-yl)ethyl]-3H-quinazolin-4-one,
   (167) 4-(2-methoxyphenyl)-3-(1-methylpiperidin-4-yl)-2H-isoquinolin-1-one,
   (168) 4-(3-methylphenyl)-3-(1-methylpiperidin-4-yl)-2H-isoquinolin-1-one,
   (169) 4-(2-methylphenyl)-3-(2-methylpiperidin-4-yl)-2H-isoquinolin-1-one,
   (170) 4-(3-methoxyphenyl)-3-(1-methylpiperidin-4-yl)-2H-isoquinolin-1-one,
   (171) 5-methoxymethyloxy-3-(1-methylpiperidin-4-yl)-2H-isoquinolin-1-one,
   (173) 8-methyl-2-[5-(diethylamino)pentyl]-3H-quinazolin-4-one,
   (175) 8-methyl-2-[4-(dimethylamino)butyl]-3H-quinazolin-4-one,
   (176) 8-methyl-2-[3-(pyrrolidin-1-yl)propyl]-3H-quinazolin-4-one,
   (177) 7-(1-methylpiperidin-4-yl)-6H-1,6-naphthyridin-5-one 1/10 water adduct,
   (178) 5-methyl-3-(1-methylpiperidin-4-yl)-2H-isoquinolin-1-one,
   (179) 4-methyl-3-(1-methylpiperidin-4-yl)-2H-isoquinolin-1-one,
   (180) 5-(dimethylamino)-3-(1-methylpiperidin-4-yl)-2H-isoquinolin-1-one,
   (181) 5-amino-3-(1-methylpiperidin-4-yl)-2H-isoquinolin-1-one,
   (182) 4-(2-fluorophenyl)-3-(1-methylpiperidin-4-yl)-2H-isoquinolin-1-one,
   (183) 7-chloro-3-(1-methylpiperidin-4-yl)-2H-isoquinolin-1-one,
   (184) 5-hydroxy-3-(1-methylpiperidin-4-yl)-3,4-dihydro-2H-isoquinolin-1-one,
   (185) 5-methoxymethyloxy-3-(3-dimethylaminopropyl)-2H-isoquinolin-1-one,
   (186) 5-hydroxy-3-(3-dimethylaminopropyl)-2H-isoquinolin-1-one hydrochloride,
   (187) 5-methoxymethyloxy-3-(4-dimethylaminobutyl)-2H-isoquinolin-1-one,
   (188) 5-hydroxy-3-(4-dimethylaminobutyl)-2H-isoquinolin-1-one hydrochloride,
   (189) 5-hydroxy-3-(2-(piperidin-1-yl)ethyl)-2H-isoquinolin-1-one,
   (190) 3-(1-methyl-1,2,3,6-tetrahydropyridin-4-yl)-2H-isoquinolin-1-one,
   (191) 3-(1-benzylpiperidin-3-yl)-2H-isoquinolin-1-one,
   (192) 3-(1-methylpiperidin-3-yl)-2H-isoquinolin-1-one,
   (193) 3-(1-methyl-1,2,3,6-tetrahydropyridin-5-yl)-2H-isoquinolin-1-one,
   (194) 3-(3-hydroxymethylpiperazin-1-yl)-2H-isoquinolin-1-one,
   (195) 3-(4-ethyl-3-hydroxymethylpiperazin-1-yl)-2H-isoquinolin-1-one,
   (196) 3-(3-hydroxymethyl-4-propylpiperazin-1-yl)-2H-isoquinolin-1-one,
   (197) 3-(4-benzyl-3-hydroxymethylpiperazin-1-yl)-2H-isoquinolin-1-one,
   (198) 5-bromo-3-(3-hydroxymethylpiperazin-1-yl)-2H-isoquinolin-1-one,
   (199) 5-bromo-3-(3-hydroxymethyl-4-methylpiperazin-1-yl)-2H-isoquinolin-1-one,
   (200) 3-(4-piperidinopiperidin-1-yl)-2H-isoquinolin-1-one,
   (201) 3-(3-hydroxymethylpiperidin-1-yl)-2H-isoquinolin-1-one,
   (202) 3-(3-(dimethylcarbamoyl)piperidin-1-yl)-2H-isoquinolin-1-one,
   (203) 3-(3-hydroxymethyl-4-isobutylpiperazin-1-yl)-2H-isoquinolin-1-one,
   (204) 3-[4-(dimethylamino)butyl]-2H-isoquinolin-1-one,
   (205) 5-fluoro-3-(3-hydroxymethyl-4-methylpiperazin-1-yl)-2H-isoquinolin-1-one,
   (206) 3-(3-(dimethylaminomethyl)piperidin-1-yl)-2H-isoquinolin-1-one,
   (207) 6-methyl-3-(1-methylpiperidin-4-yl)-2H-isoquinolin-1-one,
   (208) 7-methyl-3-(1-methylpiperidin-4-yl)-2H-isoquinolin-1-one,
   (209) 8-methyl-3-(1-methylpiperidin-4-yl)-2H-isoquinolin-1-one,
   (210) 7-methoxymethyloxy-3-(1-methylpiperidin-4-yl)-2H-isoquinolin-1-one,
   (211) 7-hydroxy-3-(1-methylpiperidin-4-yl)-2H-isoquinolin-1-one hydrochloride,
   (212) (S)-3-(3-hydroxymethyl-4-methylpiperazin-1-yl)-5-methyl-2H-isoquinolin-1-one,
   (213) 3-(3-(pyrrolidin-1-yl)propyl)-2H-isoquinolin-1-one,
   (214) 5-chloro-3-(3-hydroxymethyl-4-methylpiperazin-1-yl)-2H-isoquinolin-1-one,
   (216) 3-[3-(piperidin-1-yl)propyl]-2H-isoquinolin-1-one,
   (217) 5-hydroxy-3-(3-(pyrrolidin-1-yl)propyl)-2H-isoquinolin-1-one,
   (218) 5-methyl-3-[2-(piperidin-1-yl)ethyl]-2H-isoquinolin-1-one,
   (219) 3-[2-(piperidin-1-yl)ethyl]-2H-isoquinolin-1-one,
   (220) 3-[2-(pyrrolidin-1-yl)ethyl]-2H-isoquinolin-1-one,
   (221) 5-methyl-3-[2-(pyrrolidin-1-yl)ethyl]-2H-isoquinolin-1-one,
   (222) 5-methyl-3-[3-(pyrrolidin-1-yl)propyl-1-yl]-2H-isoquinolin-1-one,
   (223) 1,5-dihydro-6-(1-methyl-1,2,3,6-tetrahydropyridin-4-yl)pyrazolo[4,3-c]pyridin-4-one,
   (224) N,N-dimethyl-3-(1-methylpiperidin-1-yl)-2H-1-oxoisoquinoline-5-carboxamide 1/4 water adduct,
   (225) 5-methyl-3-(octahydroindolizin-7-yl)-2H-isoquinolin-1-one 3/4 water adduct,
   (226) 5-methyl-3-(octahydroindolizin-7-yl)-2H-isoquinolin-1-one 1/2 water adduct,
   (227) 3-(1-methylpiperidin-1-yl)-2H-1-oxoisoquinoline-5-carboxylic acid hydrochloride,
   (228) 5-methyl-3-[3-(piperidin-1-yl)propyl]-2H-isoquinolin-1-one,
   (229) 3-(dimethylamino)methyl-2H-isoquinolin-1-one,
   (230) 3-[(4-methylpiperazin-1-yl)methyl]-2H-isoquinolin-1-one,
   (231) 3-(piperidinomethyl)-2H-isoquinolin-1-one,
   (232) 3-[(morpholin-1-yl)methyl]-2H-isoquinolin-1-one,
   (233) 3-[(homopiperidin-1-yl)methyl]-2H-isoquinolin-1-one,
   (234) 3-[3-(homopiperidin-1-yl)propyl]-2H-isoquinolin-1-one,
   (235) 3-(1-sulfamoylpiperidin-4-yl)-5-methyl-2H-isoquinolin-1-one 1/4 water adduct,
   (236) 3-(4-methyl-3-oxopiperazin-1-yl)-5-methyl-2H-isoquinolin-1-one 1/10 water adduct,
   (237) 3-(1-aminopiperidin-4-yl)-5-methyl-2H-isoguinolin-1-one,
   (238) 3-(1-(methanesulfonylamino)piperidin-4-yl)-5-methyl-2H-isoquinolin-1-one,
   (239) 3-(1-trifluoroacetaminopiperidin-4-yl)-5-methyl-2H-isoquinolin-1-one,
   (240) 3-[2-(homopiperidin-1-yl)ethyl]-2H-isoquinolin-1-one,
   (241) N-(5-methyl-2H-1-oxoisoquinolin-3-yl)-2-(dimethylamino)acetamide,
   (243) N-(5-methyl-2H-1-oxoisoquinolin-3-yl)-3-(dimethylamino)propanamide,
   (244) 3-(1-dimethylaminopiperidin-4-yl)-5-methyl-2H-isoquinolin-1-one,
   (245) N-(5-methyl-2H-1-oxoisoquinolin-3-yl)-4-(dimethylamino)butanamide,
   (246) N-(2H-1-oxoisoquinolin-3-yl)-4-(dimethylamino)butanamide,
   (247) 3-(4-methyl-2-oxopiperazin-1-yl)-2H-isoquinolin-1-one,
   (248) 5-methyl-3-(1-methylpyrrolidin-3-yl)-2H-isoquinolin-1-one, and
   (249) 3-(1-methylpyrrolidin-3-yl)-2H-isoquinolin-1-one, an optically active form thereof, a pharmaceutically acceptable salt thereof, a hydrate thereof or a water adduct thereof.
12. The fused heterocyclic compound of the above-mentioned 1, wherein, in the formula (I),
   the dotted line part is a double bond,
   ring Ar is a benzene ring,
   X is a carbon atom optionally substituted by phenyl optionally having substituent(s), or a nitrogen atom,
   Y is -(CH₂)ₘ- wherein m is 0 or an integer of 1-10,
   R¹ and R² are the same or different and each is hydrogen, alkyl, hydroxy or amino, and
   R is dialkylamino, or represented by the following formula (a) or (b) : 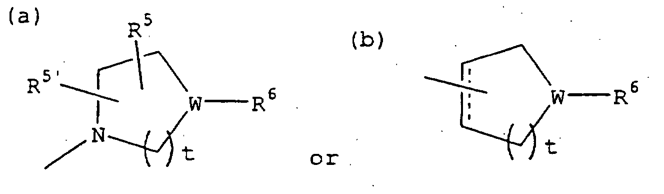 wherein a dotted line part is a single bond, W is CH or a nitrogen atom, t is an integer of 0-3, R⁵ and R^{5'} are the same or different and each is hydroxyalkyl, and R⁶ is hydrogen, alkyl or dialkylamino,
   an optically active form thereof, a pharmaceutically acceptable salt thereof, a hydrate thereof or a water adduct thereof.
13. The fused heterocyclic compound of the above-mentioned 1, wherein, in the formula (I),
   the dotted line part is a double bond,
   ring Ar is a benzene ring,
   X is a carbon atom optionally substituted by phenyl optionally having substituent(s) selected from the group consisting of halogen, alkyl and alkoxy, or a nitrogen atom,
   Y is -(CH₂)ₘ- wherein m is 0 or an integer of 1-3,
   R¹ is alkyl, hydroxy or amino,
   R² is hydrogen, and
   R is dialkylamino, or represented by the following formula (a): 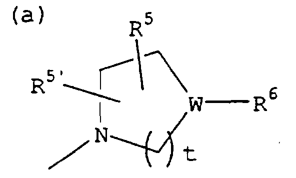 wherein W is CH or a nitrogen atom, t is an integer of 1 or 2, R⁵ is hydroxyalkyl, R^{5'} is hydrogen, and R⁶ is hydrogen, alkyl or dialkylamino, or
   the following formula (b): 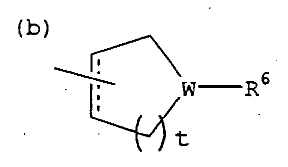 wherein a dotted line part is a single bond, W is a nitrogen atom, t is an integer of 2, and R⁶ is alkyl,
   an optically active form thereof, a pharmaceutically acceptable salt thereof, a hydrate thereof or a water adduct thereof.
14. The fused heterocyclic compound of the above-mentioned 1, which is selected from
   (1) 5-methyl-3-(4-methylpiperazin-1-yl)-2H-isoquinolin-1-one,
   (3) 3-(4-dimethylaminopiperidin-1-yl)-2H-isoquinolin-1-one,
   (4) 3-(3-hydroxymethyl-4-methylpiperazin-1-yl)-2H-isoquinolin-1-one,
   (12) 5-methyl-3-(4-dimethylaminopiperidin-1-yl)-2H-isoquinolin-1-one,
   (19) 5-hydroxy-3-(4-methylpiperazin-1-yl)-2H-isoquinolin-1-one,
   (82) 3-(1-methylpiperidin-4-yl)-2H-isoquinolin-1-one 1/5 water adduct,
   (90) 3-(3-(dimethylamino)propyl)-5-methyl-2H-isoquinolin-1-one,
   (98) 3-(1-methylpiperidin-4-yl)-4-phenyl-2H-isoquinolin-1-one 1/5 water adduct,
   (108) 5-hydroxy-3-(1-methylpiperidin-4-yl)-2H-isoquinolin-1-one, and
   (121) 2-(1-methylpiperidin-4-yl)-8-methyl-3H-quinazolin-4-one,
   an optically active form thereof, a pharmaceutically acceptable salt thereof, a hydrate thereof or a water adduct thereof.
15. The fused heterocyclic compound of the above-mentioned 1, which is selected from
   (156) (S)-3-(3-hydroxymethyl-4-methylpiperazin-1-yl)-2H-isoquinolin-1-one,
   (178) 5-methyl-3-(1-methylpiperidin-4-yl)-2H-isoquinolin-1-one,
   (181) 5-amino-3-(1-methylpiperidin-4-yl)-2H-isoquinolin-1-one,
   (186) 5-hydroxy-3-(3-dimethylaminopropyl)-2H-isoquinolin-1-one hydrochloride,
   (189) 5-hydroxy-3-(2-(piperidin-1-yl)ethyl)-2H-isoquinolin-1-one, and
   (212) (S)-3-(3-hydroxymethyl-4-methylpiperazin-1-yl)-5-methyl-2H-isoquinolin-1-one,
   an optically active form thereof, a pharmaceutically acceptable salt thereof, a hydrate thereof or a water adduct thereof.
16. The fused heterocyclic compound of the above-mentioned 1, which is selected from
   (82) 3-(1-methylpiperidin-4-yl)-2H-isoquinolin-1-one 1/5 water adduct,
   (90) 3-(3-(dimethylamino)propyl)-5-methyl-2H-isoquinolin-1-one, and
   (108) 5-hydroxy-3-(1-methylpiperidin-4-yl)-2H-isoquinolin-1-one,
   an optically active form thereof, a pharmaceutically acceptable salt thereof, a hydrate thereof or a water adduct thereof.
17. The fused heterocyclic compound of the above-mentioned 1, wherein, in the formula (I),
   the dotted line part is a double bond,
   ring Ar is a benzene ring,
   X is a carbon atom optionally substituted by phenyl optionally having substituent(s), or a nitrogen atom,
   Y is -(CH₂)ₘ- wherein m is 0 or an integer of 1-10,
   R¹ and R² are the same or different and each is hydrogen, alkyl, hydroxy or amino, and
   R is dialkylamino, or represented by the following formula (b) : 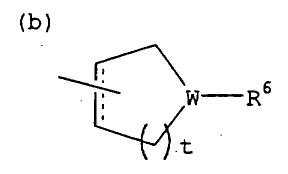 wherein a dotted line part is a single bond, W is CH or a nitrogen atom, t is an integer of 0-3, and R⁶ is hydrogen, dialkylamino or alkyl,
   an optically active form thereof, a pharmaceutically acceptable salt thereof, a hydrate thereof or a water adduct thereof.
18. The fused heterocyclic compound of the above-mentioned 1, wherein, in the formula (I),
   the dotted line part is a double bond,
   ring Ar is a benzene ring,
   X is a carbon atom optionally substituted by phenyl optionally having substituent(s), or a nitrogen atom,
   Y is -(CH₂)ₘ- wherein m is 0 or an integer of 1-10,
   R¹ and R² are the same or different and each is hydrogen, alkyl, hydroxy or amino, and
   R is dialkylamino, or represented by the following formula (a) : 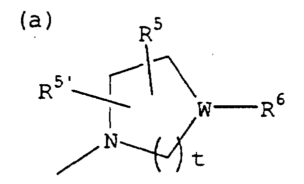 wherein W is CH or a nitrogen atom, t is an integer of 0-3, R⁵ and R^{5'} are the same or different and each is hydroxyalkyl, and R⁶ is hydrogen, dialkylamino or alkyl,
   an optically active form thereof, a pharmaceutically acceptable salt thereof, a hydrate thereof or a water adduct thereof.
19. The fused heterocyclic compound of the above-mentioned 1, which is selected from
   (82) 3-(1-methylpiperidin-4-yl)-2H-isoquinolin-1-one 1/5 water adduct,
   (108) 5-hydroxy-3-(1-methylpiperidin-4-yl)-2H-isoquinolin-1-one, and
   (121) 2-(1-methylpiperidin-4-yl)-8-methyl-3H-quinazolin-4-one,
   an optically active form thereof, a pharmaceutically acceptable salt thereof, a hydrate thereof or a water adduct thereof.
20. The fused heterocyclic compound of the above-mentioned 1, which is selected from
   (156) (S)-3-(3-hydroxymethyl-4-methylpiperazin-1-yl)-2H-isoquinolin-1-one and
   (212) (S)-3-(3-hydroxymethyl-4-methylpiperazin-1-yl)-5-methyl-2H-isoquinolin-1-one,
   an optically active form thereof, a pharmaceutically acceptable salt thereof, a hydrate thereof or a water adduct thereof.
21. The fused heterocyclic compound of the above-mentioned 1, wherein, in the formula (I),
   the dotted line part is a double bond,
   ring Ar is a benzene ring,
   X is a carbon atom optionally substituted by phenyl optionally having substituent(s), or a nitrogen atom,
   Y is -(CH₂)ₘ- wherein m is 0 or an integer of 1-10,
   R¹ is alkyl, hydroxy or amino,
   R² is hydrogen, and
   R is dialkylamino, or represented by the following formula (a) : 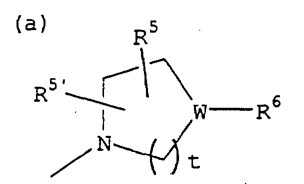 wherein W is CH or a nitrogen atom, t is an integer of 0-3, R⁵ and R^{5'} are the same or different and each is hydroxyalkyl, and R⁶ is hydrogen, dialkylamino or alkyl,
   an optically active form thereof, a pharmaceutically acceptable salt thereof, a hydrate thereof or a water adduct thereof.
22. The fused heterocyclic compound of the above-mentioned 1, which is (1) 5-methyl-3-(4-methylpiperazin-1-yl)-2H-isoquinolin-1-one,
   an optically active form thereof, a pharmaceutically acceptable salt thereof, a hydrate thereof or a water adduct thereof.
23. A pharmaceutical agent comprising the fused heterocyclic compound of any of the above-mentioned 1 to 22, an optically active form thereof, a pharmaceutically acceptable salt thereof, a hydrate thereof or a water adduct thereof.
24. A prophylactic and/or therapeutic drug for a disease caused by functional promotion of poly(ADP-ribose) synthase, which comprises a fused heterocyclic compound represented by the formula (I): 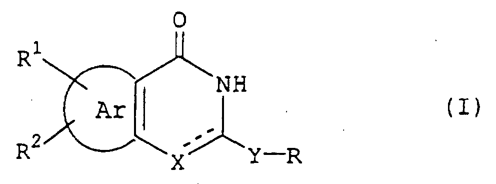 wherein
   - a dotted line part: is a single bond or a double bond;
   - ring Ar: is a benzene ring, a naphthalene ring or an aromatic heterocycle;
   - X: is a carbon atom optionally substituted by an alkyl, an aromatic heterocyclic group optionally having substituent(s) or a phenyl optionally having substituent(s), or a nitrogen atom;
   - Y is: - (CH₂)ₘ-,
   - (CH₂)ₘ-N(R⁴)-CO-(CH₂)ₙ-,
   - (CH₂)ₘ-CO-N(R⁴)-(CH₂)ₙ-,
   - (CH₂)ₘ-CO-O-(CH₂)ₙ-,
   - (CH₂)ₘ-O-CO-(CH₂)ₙ-,
   - (CH₂)ₘ-O-(CH₂)ₙ- or
   - (CH₂)ₘ-CO-(CH₂)ₙ-
   wherein m and n are the same or different and each is 0 or an integer of 1-10, R⁴ is hydrogen or alkyl, and -(CH₂)ₘ- is linked with a parent-nucleus;
   - R¹ and R²: are the same or different and each is a hydrogen, a halogen, an alkyl, an alkoxy, a haloalkyl, a hydroxy, an amino, a dialkylamino, a nitro, a cyano, an acyl, a carboxy, an ester, a carbamoyl, an N-alkylcarbamoyl, an N,N-dialkylcarbamoyl, an acylamino, a diacylamino, a thiol, an alkylthio, an alkoxycarbonylamino, a sulfamoyl, an N-alkylsulfamoyl, an N,N-dialkylsulfamoyl or an alkoxyalkyloxy; and
   - R: is an amino, a monoalkylamino, a dialkylamino, a morpholino or a thiomorpholino, or represented by the following formula (a)-(e): 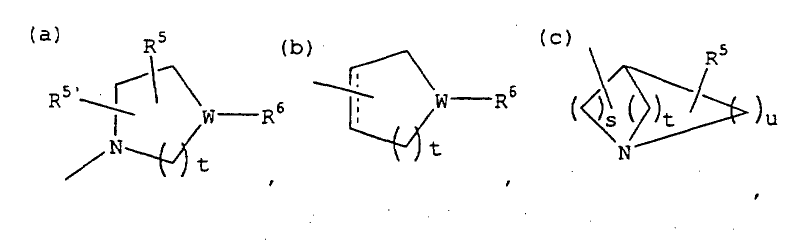 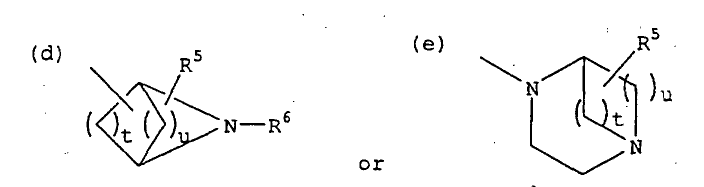
   wherein a dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, monoalkylamino, dialkylamino, alkyl, alkoxycarbonyl, alkylsulfonyl, acyl, acylamino, benzoylamino optionally having substituent(s), hydroxy, arylalkyl, sulfamoyl or alkylsulfonylamino, or represented by the following formula (f)-(i): 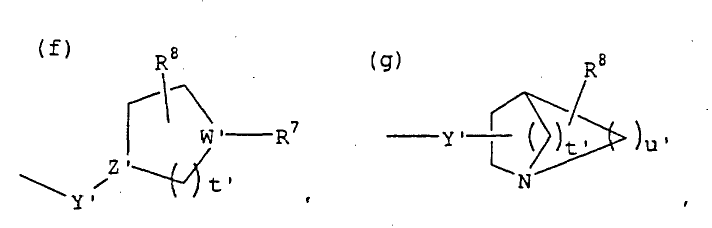 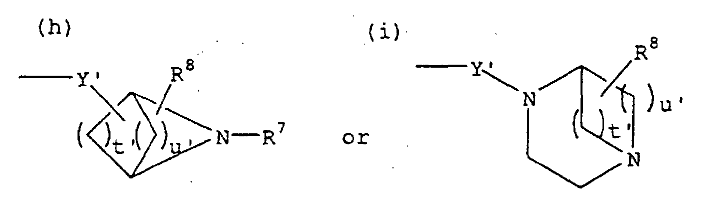 wherein Y' is as defined for Y above, Z' is CH or a nitrogen atom, W' is CH, a nitrogen atom or an oxygen atom, t' is an integer of 1-3, u' is an integer of 1-3, provided that when the above-mentioned formula (a) is piperazine, then R⁶ may be hydroxyalkyl, R⁷ is hydrogen, amino, monoalkylamino, dialkylamino, alkyl, alkoxycarbonyl, alkylsulfonyl, acyl, hydroxyalkyl, acylamino or benzoylamino optionally having substituent(s), provided that when W' is an oxygen atom, then R⁷ should be absent, and R⁸ is hydrogen, alkyl or hydroxyalkyl,
   an optically active form thereof, a pharmaceutically acceptable salt thereof, a hydrate thereof or a water adduct thereof.
25. A prophylactic and/or therapeutic drug for a disease caused by functional promotion of poly(ADP-ribose) synthase, which comprises a fused heterocyclic compound represented by the formula (I): 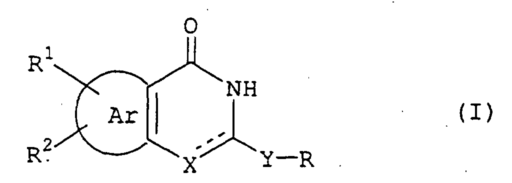 wherein
   - a dotted line part: is a single bond or a double bond;
   - ring Ar: is a benzene ring, a naphthalene ring or an aromatic heterocycle;
   - X: is a carbon atom optionally substituted by an alkyl, an aromatic heterocyclic group optionally having substituent(s) or a phenyl optionally having substituent(s), or a nitrogen atom;
   - Y: is
   - (CH₂)ₘ-,
   - (CH₂)ₘ-N(R⁴)-CO- (CH₂)ₙ-,
   - (CH₂)ₘ-CO-N(R⁴)-(CH₂)ₙ-,
   - (CH₂)ₘ-CO-O-(CH₂)ₙ- or
   - (CH₂)ₘ-O-(CH₂)ₙ-
   wherein m and n are the same or different and each is 0 or an integer of 1-10, R⁴ is hydrogen or alkyl, and - (CH₂)ₘ- is linked with a parent-nucleus;
   - R¹ and R²: are the same or different and each is a hydrogen, a halogen, an alkyl, an alkoxy, a haloalkyl, a hydroxy, an amino, a dialkylamino, a nitro, a cyano, an acyl, a carboxy, an ester, a carbamoyl, an N-alkylcarbamoyl, an N,N-dialkylcarbamoyl, an acylamino, a diacylamino, a thiol, an alkylthio, an alkoxycarbonylamino, a sulfamoyl, an N-alkylsulfamoyl or an N,N-dialkylsulfamoyl; and
   - R: is an amino, a monoalkylamino, a dialkylamino, a morpholino or a thiomorpholino, or represented by the following formula (a)-(e): 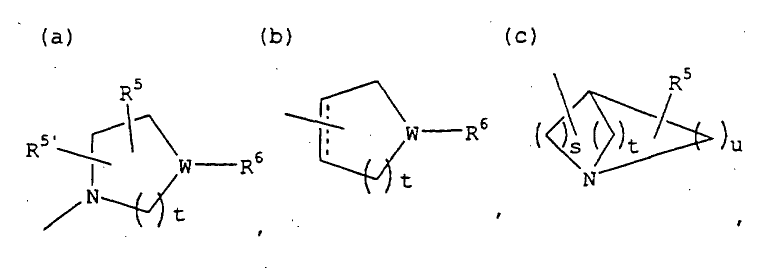 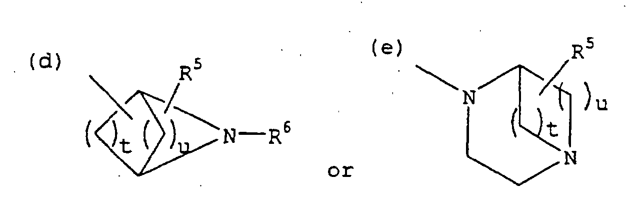 wherein a dotted line part is a single bond or a double bond, W is CH or a nitrogen atom, s is an integer of 1-3, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, monoalkylamino, dialkylamino, alkyl, alkoxycarbonyl, alkylsulfonyl, acyl, acylamino, benzoylamino optionally having substituent(s), hydroxy, arylalkyl, sulfamoyl or alkylsulfonylamino, or represented by the following formula (f)-(i): 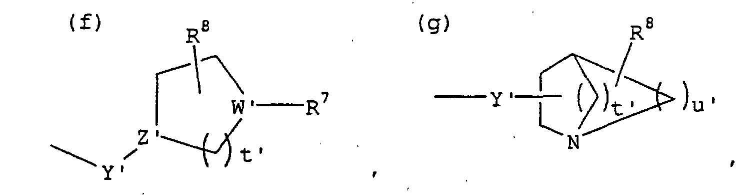 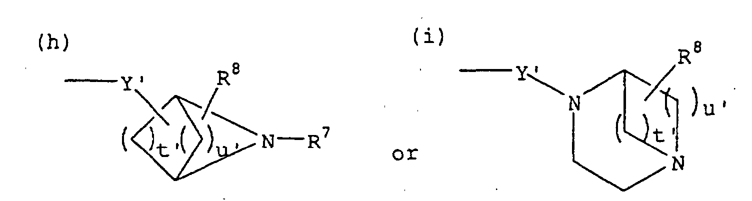
   wherein Y' is as defined for Y above, Z' is CH or a nitrogen atom, W' is CH or a nitrogen atom, t' is an integer of 1-3, u' is an integer of 1-3, R⁷ is hydrogen, amino, monoalkylamino, dialkylamino, alkyl, alkoxycarbonyl, alkylsulfonyl, acyl, hydroxyalkyl, acylamino or benzoylamino optionally having substituent(s), and R⁸ is hydrogen, alkyl or hydroxyalkyl,
   provided that (1) when Y is -(CH₂)ₘ- (m=0) and R is 4-methylpiperazin-1-yl, 1-piperidino, 4-morpholino or 4-(2-hydroxyethyl)piperazin-1-yl, then R¹ should be halogen, alkyl, alkoxy, haloalkyl, hydroxy, amino, dialkylamino, nitro, cyano, acyl, carboxy, ester, carbamoyl, N-alkylcarbamoyl, N,N-dialkylcarbamoyl, acylamino, diacylamino, thiol, alkylthio, alkoxycarbonylamino, sulfamoyl, N-alkylsulfamoyl or N,N-dialkylsulfamoyl, and (2) when X is a nitrogen atom and Y is - (CH₂)ₘ- (m=0), then R should be represented by any of the formulas (b)-(d) and Z should be CH,
   an optically active form thereof, a pharmaceutically acceptable salt thereof, a hydrate thereof or a water adduct thereof.
26. The prophylactic and/or therapeutic drug of the above-mentioned 24 or 25, which is used for cerebral infarction.
27. The prophylactic and/or therapeutic drug of any of the above-mentioned 24 to 26, which is used for an acute phase of cerebral infarction.
28. A poly(ADP-ribose) synthase inhibitor comprising a fused heterocyclic compound represented by the formula (I): 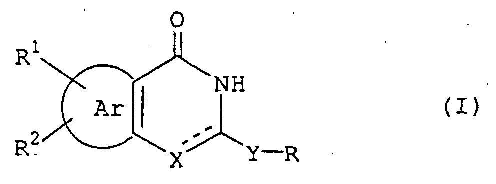 wherein
   - a dotted line part: is a single bond or a double bond;
   - ring Ar: is a benzene ring, a naphthalene ring or an aromatic heterocycle;
   - X: is a carbon atom optionally substituted by an alkyl, an aromatic heterocyclic group optionally having substituent(s) or a phenyl optionally having substituent(s), or a nitrogen atom;
   - Y: is
   - (CH₂)ₘ-,
   - (CH₂)ₘ-N(R⁴)-CO-(CH₂)ₙ-,
   - (CH₂)ₘ-CO-N(R⁴)-(CH₂)ₙ-,
   - (CH₂)ₘ-CO-O-(CH₂)ₙ-,
   - (CH₂)ₘ-O-CO-(CH₂)ₙ-,
   - (CH₂)ₘ-O-(CH₂)ₙ- or
   - (CH₂)ₘ-CO-(CH₂)ₙ-
   wherein m and n are the same or different and each is 0 or an integer of 1-10, R⁴ is hydrogen or alkyl, and - (CH₂)ₘ- is linked with a parent-nucleus;
   - R¹ and R²: are the same or different and each is a hydrogen, a halogen, an alkyl, an alkoxy, a haloalkyl, a hydroxy, an amino, a dialkylamino, a nitro, a cyano, an acyl, a carboxy, an ester, a carbamoyl, an N-alkylcarbamoyl, an N,N-dialkylcarbamoyl, an acylamino, a diacylamino, a thiol, an alkylthio, an alkoxycarbonylamino, a sulfamoyl, an N-alkylsulfamoyl, an N,N-dialkylsulfamoyl or an alkoxyalkyloxy; and
   - R: is an amino, a monoalkylamino, a dialkylamino, a morpholino or a thiomorpholino, or represented by the following formula (a)-(e): 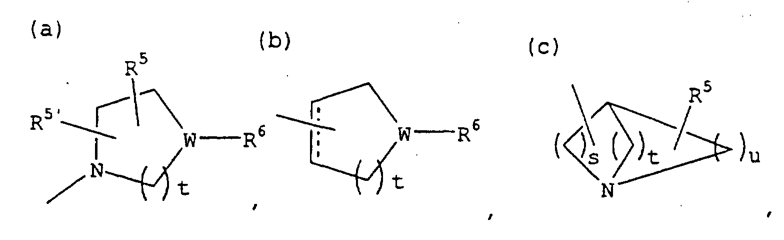 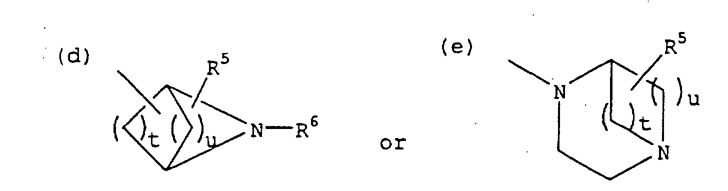 wherein a dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, monoalkylamino, dialkylamino, alkyl, alkoxycarbonyl, alkylsulfonyl, acyl, acylamino, benzoylamino optionally having substituent(s), hydroxy, arylalkyl, sulfamoyl or alkylsulfonylamino, or represented by the following formula (f)-(i): 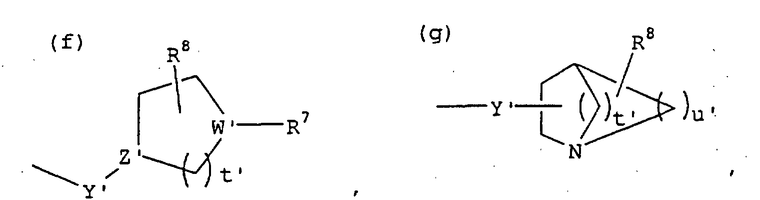 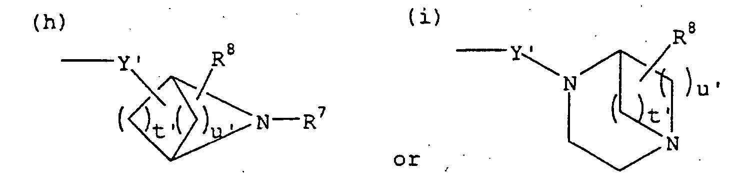
   wherein Y' is as defined for Y above, Z' is CH or a nitrogen atom, W' is CH, a nitrogen atom or an oxygen atom, t' is an integer of 1-3, u' is an integer of 1-3, provided that when the above-mentioned formula (a) is piperazine, then R⁶ may be hydroxyalkyl, R⁷ is hydrogen, amino, monoalkylamino, dialkylamino, alkyl, alkoxycarbonyl, alkylsulfonyl, acyl, hydroxyalkyl, acylamino or benzoylamino optionally having substituent(s), provided that when W' is an oxygen atom, then R⁷ should be absent, and R⁸ is hydrogen, alkyl or hydroxyalkyl,
   an optically active form thereof, a pharmaceutically acceptable salt thereof, a hydrate thereof or a water adduct thereof.
29. A poly(ADP-ribose) synthase inhibitor comprising a fused heterocyclic compound represented by the formula (I): 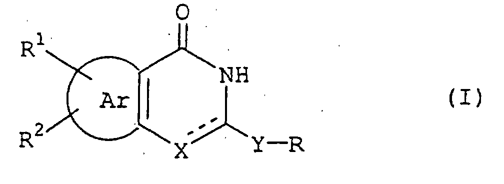
wherein
- a dotted line part: is a single bond or a double bond;
- ring Ar: is a benzene ring, a naphthalene ring or an aromatic heterocycle;
- X: is a carbon atom optionally substituted by an alkyl, an aromatic heterocyclic group optionally having substituent(s) or a phenyl optionally having substituent(s), or a nitrogen atom;
- Y: is
- (CH₂)ₘ-,
- (CH₂)ₘ-N(R⁴)-CO-(CH₂)ₙ-,
- (CH₂)ₘ-CO-N(R⁴)-(CH₂)ₙ-,
-(CH₂)ₘ-CO-O-(CH₂)ₙ- or
-(CH₂)ₘ-O-(CH₂)ₙ-
wherein m and n are the same or different and each is 0 or an integer of 1-10, R⁴ is hydrogen or alkyl, and -(CH₂)ₘ- is linked with a parent-nucleus;
- R¹ and R²: are the same or different and each is a hydrogen, a halogen, an alkyl, an alkoxy, a haloalkyl, a hydroxy, an amino, a dialkylamino, a nitro, a cyano, an acyl, a carboxy, an ester, a carbamoyl, an N-alkylcarbamoyl, an N,N-dialkylcarbamoyl, an acylamino, a diacylamino, a thiol, an alkylthio, an alkoxycarbonylamino, a sulfamoyl, an N-alkylsulfamoyl or an N,N-dialkylsulfamoyl; and
- R: is an amino, a monoalkylamino, a dialkylamino, a morpholino or a thiomorpholino, or represented by the following formula (a)-(e): 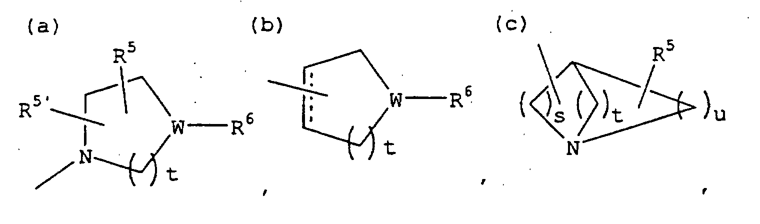 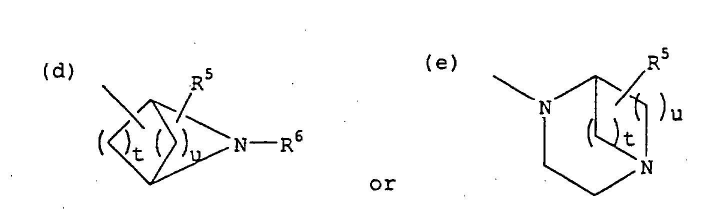 wherein a dotted line part is a single bond or a double bond, W is CH or a nitrogen atom, s is an integer of 1-3, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, monoalkylamino, dialkylamino, alkyl, alkoxycarbonyl, alkylsulfonyl, acyl, acylamino, benzoylamino optionally having substituent(s), hydroxy, arylalkyl, sulfamoyl or alkylsulfonylamino, or represented by the following formula (f)-(i): 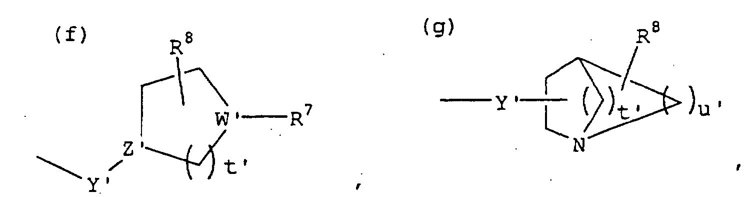 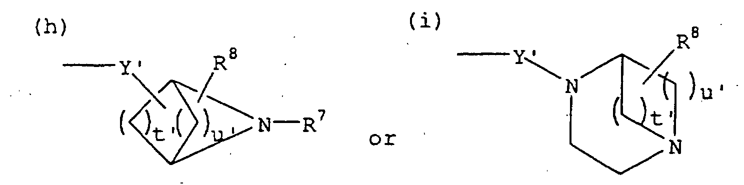
wherein Y' is as defined for Y above, Z' is CH or a nitrogen atom, W' is CH or a nitrogen atom, t' is an integer of 1-3, u' is an integer of 1-3, and R⁷ is hydrogen, amino, monoalkylamino, dialkylamino, alkyl, alkoxycarbonyl, alkylsulfonyl, acyl, hydroxyalkyl, acylamino or benzoylamino optionally having substituent(s), and R⁸ is hydrogen, alkyl or hydroxyalkyl,
provided that (1) when Y is -(CH₂)ₘ- (m=0) and R is 4-methylpiperazin-1-yl, 1-piperidino, 4-morpholino or 4-(2-hydroxyethyl)piperazin-1-yl, then R¹ should be halogen, alkyl, alkoxy, haloalkyl, hydroxy, amino, dialkylamino, nitro, cyano, acyl, carboxy, ester, carbamoyl, N-alkylcarbamoyl, N,N-dialkylcarbamoyl, acylamino, diacylamino, thiol, alkylthio, alkoxycarbonylamino, sulfamoyl, N-alkylsulfamoyl or N,N-dialkylsulfamoyl, and (2) when X is a nitrogen atom and Y is - (CH₂)ₘ- (m=0), then R should be represented by any of the above formulas (b)-(d) and Z should be CH,
an optically active form thereof, a pharmaceutically acceptable salt thereof, a hydrate thereof or a water adduct thereof.

The "parent-nucleus" in the present specification is a part represented by 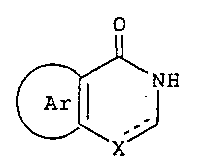 The "dotted line part" refers to the part represented by -----, which consists of a bond represented by a dotted line and a bond represented by a solid line. By "the dotted line part is a double bond" is meant that the bond represented by the dotted line is a single bond, and by "the dotted line part is a single bond" is meant that the bond represented by the dotted line does not exist.

In the present specification, "thiol" means an -SH group.

The compound of the formula (I) can take the form of a tautomer as shown in the following formula (II). The present invention encompasses both tautomers.

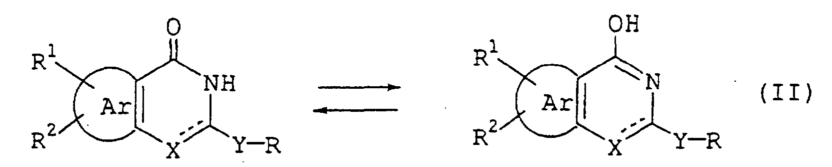

Specific examples of respective groups in the above-mentioned formula (I) are as follows.

The aromatic heterocycle for ring Ar means a 5-membered or 6-membered aromatic ring having 1 or 2 hetero atoms, such as nitrogen, oxygen and sulfur in the ring, which is exemplified by pyridine, furan, thiophene, pyrimidine, oxazole, thiazole, isoxazole, isothiazole, pyrazole and the like, with preference given to pyridine, thiophene and pyrazole.

Specific examples of the substituent for R¹ and R² are as follows, which can be substituted on an optional carbon atom of ring Ar.
(1) halogen: fluorine, chlorine, bromine and iodine, of which fluorine, chlorine and bromine are preferable.
(2) alkyl: linear or branched chain alkyl having 1 to 4 carbon atoms, such as methyl, ethyl, propyl, isopropyl, butyl, isobutyl, tertiary butyl and the like, of which methyl is preferable.
(3) alkoxy: alkoxy consisting of linear or branched chain alkyl having 1 to 4 carbon atoms and oxygen atom, which is exemplified by methoxy, ethoxy, propoxy, isopropoxy, butoxy, tertiary butoxy and the like, of which methoxy is preferable.
(4) haloalkyl: linear or branched chain alkyl having 1 to 4 carbon atoms, which is substituted by 1 or more halogen atoms, where halogen atom is exemplified by those similar to the above-mentioned (1), such as fluoromethyl, difluoromethyl, trifluoromethyl, 2-fluoroethyl, 2,2-difluoroethyl, 2,2,2-trifluoroethyl and the like, of which trifluoromethyl is preferable.
(5) hydroxy.
(6) amino.
(7) dialkylamino: dialkylamino wherein alkyl moieties are the same or different and each is independently linear or branched chain alkyl having 1 to 4 carbon atoms, and the alkyl moiety may form a ring. Examples thereof include dimethylamino, diethylamino, N-methyl-N-ethylamino, pyrrolidin-1-yl, piperidin-1-yl and the like, of which dimethylamino is preferable.
(8) nitro.
(9) cyano.
(10) acyl: acyl having 1 to 4 carbon atoms in total, which consists of linear or branched chain alkyl and carbonyl, which is exemplified by formyl, acetyl, propionyl, 2-methylpropionyl, butyryl and the like.
(11) carboxy.
(12) ester: ester consisting of linear or branched chain alkoxy having 1 to 4 carbon atoms and carbonyl, which is exemplified by methoxycarbonyl, ethoxycarbonyl, propoxycarbonyl, isopropoxycarbonyl, butoxycarbonyl, tertiary butoxycarbonyl and the like.
(13) carbamoyl.
(14-1) N-alkylcarbamoyl: N-alkylcarbamoyl consisting of monoalkylamino having 1 to 4 carbon atoms and carbonyl, which is exemplified by N-methylcarbamoyl, N-ethylcarbamoyl, N-propylcarbamoyl, N-butylcarbamoyl and the like.
(14-2) N,N-dialkylcarbamoyl: N,N-dialkylcarbamoyl consisting of dialkylamino (as defined in the above-mentioned (7)) and carbonyl, which is exemplified by N,N-dimethylcarbamoyl, N,N-diethylcarbamoyl, N,N-dipropylcarbamoyl, N,N-dibutylcarbamoyl and the like.
(15-1) acylamino: acylamino consisting of acyl (as defined in the above-mentioned (10)) and amino, which is exemplified by formylamino, acetylamino, propionylamino, butyrylamino and the like.
(15-2) diacylamino: diacylamino consisting of two acyls (as defined in the above-mentioned (10)) and amino, wherein the acyl moieties may be independently the same or different, which is exemplified by N,N-diacetylamino, N,N-dipropionylamino, N,N-dibutyrylamino and the like.
(16) thiol.
(17) alkylthio: alkylthio consisting of linear or branched chain alkyl having 1 to 4 carbon atoms and sulfur atom, which is exemplified by methylthio, ethylthio, propylthio, butylthio and the like, of which methylthio is preferable.
(18) alkoxycarbonylamino: alkoxycarbonylamino consisting of ester (as defined in the above-mentioned (12)) and amino, which is exemplified by methoxycarbonylamino, ethoxycarbonylamino, propoxycarbonylamino, butoxycarbonylamino and the like.
(19) sulfamoyl.
(20-1) N-alkylsulfamoyl: N-alkylsulfamoyl consisting of monoalkylamino, wherein the alkyl moiety is as defined in the above-mentioned (2), and sulfone, which is exemplified by N-methylsulfamoyl, N-ethylsulfamoyl, N-propylsulfamoyl, N-butylsulfamoyl and the like.
(20-2) N,N-dialkylsulfamoyl: N,N-dialkylsulfamoyl consisting of dialkylamino (as defined in the above-mentioned (7)) and sulfone, which is exemplified by N,N-dimethylsulfamoyl, N,N-diethylsulfamoyl, N,N-dipropylsulfamoyl, N,N-dibutylsulfamoyl and the like.
(21) alkoxyalkyloxy: alkoxyalkyloxy consisting of alkoxy, alkyl and oxygen, wherein alkoxy and alkyl are as defined in the aforementioned (3) and (2), respectively, which is exemplified by methoxymethyloxy, ethoxymethyloxy and the like, of which methoxymethyloxy is preferable.

The substituent for X is now explained. As the alkyl, those similar to alkyl for R¹ can be mentioned, with preference given to methyl. As the aromatic heterocyclic group, monovalent groups of those similar to aromatic heterocycle for ring Ar can be mentioned. As the substituent optionally substituting phenyl and aromatic heterocyclic group, those similar to R¹ and R² can be mentioned, of which alkyl (preferably methyl), halogen (preferably chlorine, fluorine) and alkoxy (preferably methoxy) are preferable.

As the alkyl for R⁴, those similar to alkyl for R¹ can be mentioned.

As the monoalkylamino and dialkylamino for R, those similar to amino substituted by alkyl for R¹ and dialkylamino for R¹, respectively, can be mentioned. Specific examples of dialkylamino preferably include dimethylamino and diethylamino.

As alkyl and alkoxy, which are various substituents or constituent factors of substituents for R⁵ and R^{5'}, those similar to the aforementioned (e.g., those for R¹) can be mentioned. As the hydroxyalkyl, hydroxymethyl is preferable, as the dialkylcarbamoyl, dimethylcarbamoyl is preferable, as the dialkylaminoalkyl, dimethylaminomethyl is preferable, and as the alkoxycarbonyl, ethoxycarbonyl is preferable.

The substituent for R⁶ is now explained. As the monoalkylamino, dialkylamino, alkyl, alkoxycarbonyl and acyl, those similar to the aforementioned monoalkylamino, dialkylamino, alkyl, alkoxycarbonyl (as defined in the above-mentioned (12)), and acyl can be mentioned. As the alkyl, methyl, ethyl, propyl and isobutyl are preferable, as the dialkylamino, dimethylamino is preferable, and as the alkoxycarbonyl, ethoxycarbonyl is preferable. As the acylamino, a group consisting of acyl having 1 to 4 carbon atoms and amino group can be mentioned, which is selected from formylamino, acetylamino, propionylamino, 2-methylpropionylamino, butyrylamino and the like. The acyl moiety may have a substituent, and preferably halogen (particularly, fluorine) can be mentioned. The substituted acylamino is exemplified by trifluoroacetylamino. The alkylsulfonyl is alkylsulfonyl consisting of alkyl as defined by the above-mentioned (2), and sulfonyl, and, for example, methanesulfonyl is preferable. The alkylsulfonylamino consists of the aforementioned alkylsulfonyl and amino, such methylsulfonylamino, ethylsulfonylamino and the like, of which methylsulfonylamino is preferable. As the substituent(s) for benzoylamino optionally having substituent(s), those similar to the substituents for R¹ can be mentioned. As the arylalkyl, for example, benzyl, phenethyl and the like can be mentioned, of which benzyl is preferable. When the formula (a) is piperazine, R⁶ may be hydroxyalkyl, and as the hydroxyalkyl, a group consisting of linear or branched chain alkyl having 1 to 4 carbon atoms and hydroxyl group can be mentioned, such as hydroxymethyl, 2-hydroxyethyl, 3-hydroxypropyl, 2-hydroxy-2-methylpropyl, 4-hydroxybutyl and the like, of which hydroxyethyl is preferable.

As alkyl and acyl, which are various substituents or constituent factors of substituents for R⁷, those as defined above can be mentioned, of which methyl is preferable as alkyl. As the substituent for benzoylamino optionally having substituent(s), those similar to the substituent for R¹ can be mentioned.

As the alkyl for R⁸, those similar to the aforementioned can be mentioned.

A fused heterocyclic compound represented by the formula (I) : 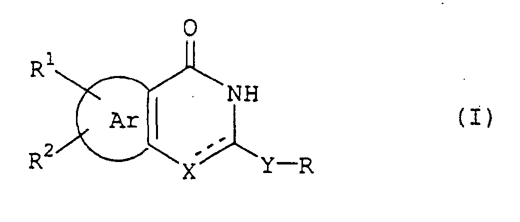 wherein
- a dotted line part: is a single bond or a double bond;
- ring Ar: is a benzene ring, a naphthalene ring or an aromatic heterocycle;
- X: is a carbon atom optionally substituted by an alkyl, an aromatic heterocyclic group optionally having substituent(s) or a phenyl optionally having substituent(s), or a nitrogen atom;
- Y: is
-(CH₂)ₘ-,
-(CH₂)ₘ-N(R⁴)-CO-(CH₂)ₙ-,
- (CH₂)ₘ-CO-N(R⁴)-(CH₂)ₙ-,
- (CH₂)ₘ-CO-O-(CH₂)ₙ-,
- (CH₂)ₘ-O-CO-(CH₂)ₙ-,
-(CH₂)ₘ-O-(CH₂)ₙ- or
- (CH₂)ₘ-CO-(CH₂)ₙ-
wherein m and n are the same or different and each is 0 or an integer of 1-10, R⁴ is hydrogen or alkyl, and -(CH₂)ₘ- is linked with a parent-nucleus;
- R¹ and R²: are the same or different and each is a hydrogen, a halogen, an alkyl, an alkoxy, a haloalkyl, a hydroxy, an amino, a dialkylamino, a nitro, a cyano, an acyl, a carboxy, an ester, a carbamoyl, an N-alkylcarbamoyl, an N,N-dialkylcarbamoyl, an acylamino, a diacylamino, a thiol, an alkylthio, an alkoxycarbonylamino, a sulfamoyl, an N-alkylsulfamoyl, an N,N-dialkylsulfamoyl or an alkoxyalkyloxy; and
- R: is an amino, a monoalkylamino, a dialkylamino, a morpholino or a thiomorpholino, or represented by the following formula (a)-(e): 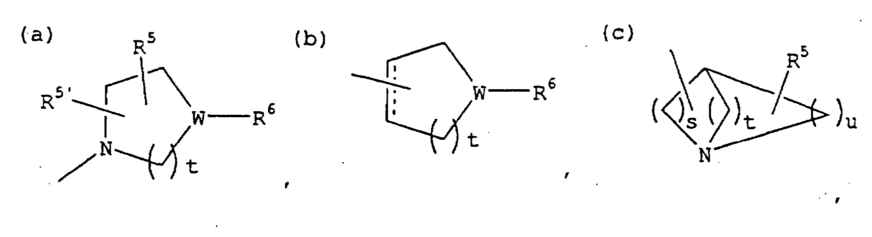 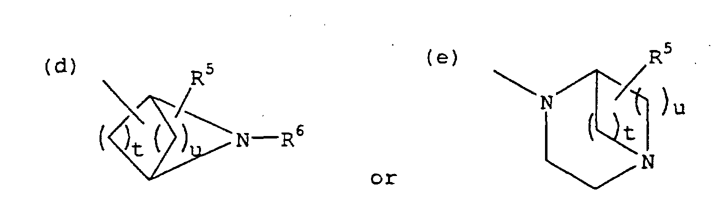 wherein a dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, monoalkylamino, dialkylamino, alkyl, alkoxycarbonyl, alkylsulfonyl, acyl, acylamino, benzoylamino optionally having substituent(s), hydroxy, arylalkyl, sulfamoyl or alkylsulfonylamino, or represented by the following formula (f)-(i): 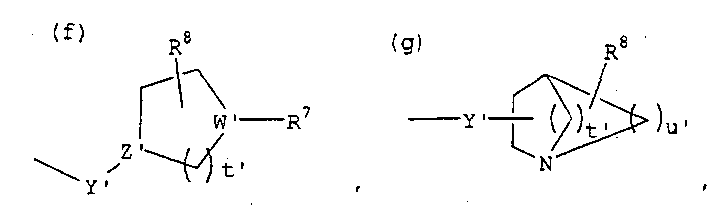 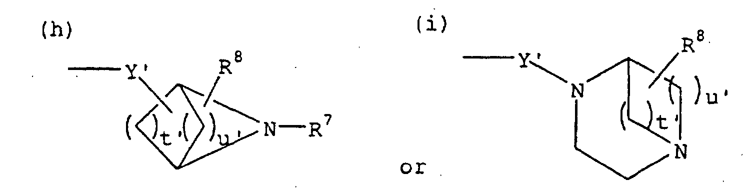
wherein Y' is as defined for Y above, Z' is CH or a nitrogen atom, W' is CH, a nitrogen atom or an oxygen atom, t' is an integer of 1-3, u' is an integer of 1-3, provided that when the above-mentioned formula (a) is piperazine, then R⁶ may be hydroxyalkyl, R⁷ is hydrogen, amino, monoalkylamino, dialkylamino, alkyl, alkoxycarbonyl, alkylsulfonyl, acyl, hydroxyalkyl, acylamino or benzoylamino optionally having substituent(s), provided that when W' is an oxygen atom, then R⁷ should be absent, and R⁸ is hydrogen, alkyl or hydroxyalkyl,
an optically active form thereof, a pharmaceutically acceptable salt thereof, a hydrate thereof and a water adduct thereof (hereinafter those are collectively abbreviated as a fused heterocyclic compound unless particularly specified) show a high PARP inhibitory action, are useful as prophylactic and/or therapeutic drugs for diseases caused by functional promotion of poly(ADP-ribose) synthase, and can be used particularly for cerebral infarction (acute phase). In addition, a fused heterocyclic compound is also useful as a poly(ADP-ribose) synthase inhibitor.

In the fused heterocyclic compounds, a compound wherein, (1) when X is an unsubstituted carbon atom, ring Ar is a benzene ring, Y is -(CH₂)ₘ- (m=0) and R is monoalkylamino, dialkylamino, piperidinyl, 3-methyl-1-piperidino, piperazin-1-yl, 4-methylpiperazin-1-yl, 1-piperidino, 4-morpholino or 4-(2-hydroxyethyl)piperazin-1-yl, then R¹ should be halogen, alkyl, alkoxy, haloalkyl, hydroxy, amino, dialkylamino, nitro, cyano, acyl, carboxy, ester, carbamoyl, N-alkylcarbamoyl, N,N-dialkylcarbamoyl, acylamino, diacylamino, thiol, alkylthio, alkoxycarbonylamino, sulfamoyl, N-alkylsulfamoyl or N,N-dialkylsulfamoyl, and (2) when X is a nitrogen atom and Y is -(CH₂)ₘ- (m=0), then R should be represented by any of the above-mentioned formulas (b)-(d), is a novel compound.

As an embodiment of the fused heterocyclic compound, for example, a compound of the formula (I), wherein
[1]
   - Y: is
   -(CH₂)ₘ-,
   - (CH₂)ₘ-N(R⁴)-CO-(CH₂)ₙ-,
   - (CH₂)ₘ-CO-N (R⁴)-(CH₂)ₙ-,
   - (CH₂)ₘ-CO-O-(CH₂)ₙ- or
   - (CH₂)ₘ-O-(CH₂)ₙ-
   wherein m and n are the same or different and each is 0 or an integer of 1-10, R⁴ is hydrogen or alkyl, and - (CH₂)ₘ- is linked with a parent-nucleus,
   - R¹ and R²: are the same or different and each is a hydrogen, a halogen, an alkyl, an alkoxy, a haloalkyl, a hydroxy, an amino, a dialkylamino, a nitro, a cyano, an acyl, a carboxy, an ester, a carbamoyl, an N-alkylcarbamoyl, an N,N-dialkylcarbamoyl, an acylamino, a diacylamino, a thiol, an alkylthio, an alkoxycarbonylamino, a sulfamoyl, an N-alkylsulfamoyl or an N,N-dialkylsulfamoyl,
   s is an integer of 1-4,

   W' is CH or a nitrogen atom, and provided that (1) when Y is -(CH₂)ₘ- (m=0) and R is 4-methylpiperazin-1-yl, 1-piperidino, 4-morpholino or 4-(2-hydroxyethyl)piperazin-1-yl, then R¹ should be halogen, alkyl, alkoxy, haloalkyl, hydroxy, amino, dialkylamino, nitro, cyano, acyl, carboxy, ester, carbamoyl, N-alkylcarbamoyl, N,N-dialkylcarbamoyl, acylamino, diacylamino, thiol, alkylthio, alkoxycarbonylamino, sulfamoyl, N-alkylsulfamoyl or N,N-dialkylsulfamoyl, (2) when X is a nitrogen atom and Y is - (CH₂)ₘ- (m=0), then R should be represented by any of the formulas (b)-(d) and Z should be CH, and (3) when the above-mentioned formula (a) is piperazine, then R⁶ should not be hydroxyalkyl;
[2] a compound of the formula (I) wherein X is a carbon atom optionally substituted by alkyl, aromatic heterocyclic group optionally having substituent(s) or phenyl optionally having substituent(s);
[3] a compound of the formula (I) wherein R¹ is halogen, alkyl, alkoxy, haloalkyl, hydroxy, amino, dialkylamino, nitro, cyano, acyl, carboxy, ester, carbamoyl, N-alkylcarbamoyl, N,N-dialkylcarbamoyl, acylamino, diacylamino, thiol, alkylthio, alkoxycarbonylamino, sulfamoyl, N-alkylsulfamoyl, N,N-dialkylsulfamoyl or alkoxyalkyloxy, and R² is hydrogen; and the like can be mentioned.

As the fused heterocyclic compound, for example, a compound of the formula (I), wherein
[A] the dotted line part is a single bond or a double bond, ring Ar is a benzene ring, a naphthalene ring or an aromatic heterocycle,
   X is a carbon atom optionally substituted by alkyl or phenyl optionally having substituent(s), or a nitrogen atom,
   Y is
   - (CH₂)ₘ-,
   - (CH₂)ₘ-N(R⁴)-CO- (CH₂)ₙ- or
   - (CH₂)ₘ-CO-(CH₂)ₙ-
   wherein m and n are the same or different and each is 0 or an integer of 1-10, R⁴ is hydrogen, and -(CH₂)ₘ- is linked with a parent-nucleus,
   R¹ and R² are the same or different and each is hydrogen, halogen, alkyl, alkoxy, haloalkyl, hydroxy, amino, dialkylamino, nitro, cyano, carboxy, N,N-dialkylcarbamoyl, alkylthio or alkoxyalkyloxy, and
   R is dialkylamino or morpholino, or represented by the above-mentioned formulas (a)-(d)
   wherein a dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, dialkylamino, alkyl, alkoxycarbonyl, alkylsulfonyl, acylamino, hydroxy, arylalkyl, sulfamoyl or alkylsulfonylamino, or represented by the above-mentioned formula (f)
   wherein Y' is as defined for Y above, Z' is CH or a nitrogen atom, W' is CH, a nitrogen atom or an oxygen atom, t' is an integer of 1-3, provided that when the above-mentioned formula (a) is piperazine, then R⁶ may be hydroxyalkyl, R⁷ is hydrogen or alkyl, provided that when W' is an oxygen atom, then R⁷ should be absent, and R⁸ is hydrogen,
   is preferable;
[B] a compound of the formula (I) wherein
   the dotted line part is a single bond or a double bond,
   ring Ar is a benzene ring, a naphthalene ring, or an aromatic heterocycle selected from pyridine, pyrazole and thiophene,
   X is a carbon atom optionally substituted by alkyl or phenyl optionally having substituent(s) selected from the group consisting of halogen, alkyl and alkoxy, or a nitrogen atom,
   Y is
   -(CH₂)ₘ-,
   - (CH₂)ₘ-N(R⁴)-CO- (CH₂)ₙ- or
   - (CH₂)ₘ-CO-(CH₂)ₙ-
   wherein m and n are the same or different and each is 0 or an integer of 1-5, R⁴ is hydrogen, and -(CH₂)ₘ- is linked with a parent-nucleus,
   R¹ and R² are the same or different and each is hydrogen, halogen, alkyl, alkoxy, haloalkyl, hydroxy, amino, dialkylamino, nitro, cyano, carboxy, N,N-dialkylcarbamoyl, alkylthio or alkoxyalkyloxy, and
   R is dialkylamino or morpholino, or represented by the above-mentioned formulas (a)-(d)
   wherein the dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, dialkylamino, alkyl, alkoxycarbonyl, alkylsulfonyl, acylamino, hydroxy, arylalkyl, sulfamoyl or alkylsulfonylamino, or represented by the above-mentioned formula (f)
   wherein Y' is as defined for Y above, Z' is a nitrogen atom, W' is CH, a nitrogen atom or an oxygen atom, t' is an integer of 1-3, provided that when the above-mentioned formula (a) is piperazine, then R⁶ may be hydroxyalkyl, R⁷ is hydrogen or alkyl, provided that when W' is an oxygen atom, then R⁷ should be absent, and R⁸ is hydrogen,
   provided that when X is a nitrogen atom, then R should be represented by the above-mentioned formula (b), is more preferable;
[C] a compound of the formula (I) wherein
   the dotted line part is a single bond or a double bond,
   ring Ar is a benzene ring, a naphthalene ring or an aromatic heterocycle,
   X is a carbon atom optionally substituted by alkyl or phenyl optionally having substituent(s), or a nitrogen atom,
   Y is
   - (CH₂)ₘ-,
   - (CH₂)ₘ-N(R⁴)-CO-(CH₂)ₙ- or
   - (CH₂)ₘ-CO-(CH₂)ₙ-
   wherein m and n are the same or different and each is 0 or an integer of 1-10, R⁴ is hydrogen, and -(CH₂)ₘ- is linked with a parent-nucleus,
   R¹ and R² are the same or different and each is hydrogen, halogen, alkyl, alkoxy, hydroxy, amino, dialkylamino, nitro, carboxy, N,N-dialkylcarbamoyl or alkoxyalkyloxy, and
   R is dialkylamino or morpholino, or represented by the following formulas (a)-(c)
   wherein the dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, dialkylamino, alkyl, alkoxycarbonyl, alkylsulfonyl, acylamino, hydroxy, arylalkyl, sulfamoyl or alkylsulfonylamino, or represented by the above-mentioned formula (f)
   wherein Y' is as defined for Y above, Z' is a nitrogen atom, W' is CH, a nitrogen atom or an oxygen atom, t' is an integer of 1-3, provided that when the above-mentioned formula (a) is piperazine, then R⁶ may be hydroxyalkyl, R⁷ is hydrogen or alkyl, provided that when W' is an oxygen atom, then R⁷ should be absent, and R⁸ is hydrogen,
   is still more preferable; of which
[D] a compound of the formula (I) wherein
   the dotted line part is a single bond or a double bond,
   ring Ar is a benzene ring or a naphthalene ring, or an aromatic heterocycle selected from the group consisting of pyridine, pyrazole and thiophene,
   X is a carbon atom optionally substituted by phenyl optionally having substituent(s) selected from the group consisting of halogen, alkyl and alkoxy, or a nitrogen atom,
   Y is
   -(CH₂)ₘ-,
   -(CH₂)ₘ-N(R⁴)-CO-(CH₂)ₙ- or
   -(CH₂)ₘ-CO-(CH₂)ₙ-
   wherein m and n are the same or different and each is 0 or an integer of 1-5, R⁴ is hydrogen, and -(CH₂)ₘ- is linked with a parent-nucleus,
   R¹ and R² are the same or different and each is hydrogen, halogen, alkyl, alkoxy, hydroxy, amino, dialkylamino, carboxy, N,N-dialkylcarbamoyl or alkoxyalkyloxy, and
   R is dialkylamino or morpholino, or represented by the following formulas (a)-(c)
   wherein the dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, dialkylamino, alkyl, alkoxycarbonyl, alkylsulfonyl, acylamino, hydroxy, arylalkyl or alkylsulfonylamino, or represented by the above-mentioned formula (f)
   wherein Y' is as defined for Y above, Z' is a nitrogen atom, W' is CH, a nitrogen atom or an oxygen atom, t' is an integer of 1-3, provided that when the above-mentioned formula (a) is piperazine, then R⁶ may be hydroxyalkyl, R⁷ is hydrogen or alkyl, provided that when W' is an oxygen atom, then R⁷ should be absent, and R⁸ is hydrogen,
   provided that when X is a nitrogen atom, then R should be represented by the above-mentioned formula (b), is still more preferable; and moreover,
[E] a compound of the formula (I) wherein
   the dotted line part is a double bond,
   ring Ar is a benzene ring,
   X is a carbon atom optionally substituted by phenyl optionally having substituent(s), or a nitrogen atom,
   Y is -(CH₂)ₘ- wherein m is 0 or an integer of 1-10,
   R¹ and R² are the same or different and each is hydrogen, alkyl, hydroxy or amino, and
   R is dialkylamino, or represented by the above-mentioned formula (a) or (b)
   wherein a dotted line part is a single bond, W is CH or a nitrogen atom, t is an integer of 0-3, R⁵ and R^{5'} are the same or different and each is hydroxyalkyl, and R⁶ is hydrogen, alkyl or dialkylamino,
   is preferable, particularly
[F] a compound of the formula (I) wherein
   the dotted line part is a double bond,
   ring Ar is a benzene ring,
   X is a carbon atom optionally substituted by phenyl optionally having substituent(s) selected from the group consisting of halogen, alkyl and alkoxy, or a nitrogen atom,
   Y is -(CH₂)ₘ- wherein m is 0 or an integer of 1-3,
   R¹ is alkyl, hydroxy or amino,
   R² is hydrogen, and
   R is dialkylamino, or represented by the above-mentioned formula (a)
   wherein W is CH or a nitrogen atom, t is an integer of 1 or 2, R⁵ is hydroxyalkyl, R^{5'} is hydrogen, and R⁶ is hydrogen, alkyl or dialkylamino, or
   the above-mentioned formula (b)
   wherein a dotted line part is a single bond, W is a nitrogen atom, t is an integer of 2, and R⁶ is alkyl,
   is preferable.

Specific examples of the fused heterocyclic compound include Example compounds of the present invention, and of those,
(1) 5-methyl-3-(4-methylpiperazin-1-yl)-2H-isoquinolin-1-one,
(2) 3-(3-dimethylaminopyrrolidin-1-yl)-2H-isoquinolin-1-one,
(3) 3-(4-dimethylaminopiperidin-1-yl)-2H-isoquinolin-1-one,
(4) 3-(3-hydroxymethyl-4-methylpiperazin-1-yl)-2H-isoquinolin-1-one,
(7) 3-(4-propylpiperazin-1-yl)-2H-isoquinolin-1-one,
(8) 3-(4-methanesulfonylpiperazin-1-yl)-2H-isoquinolin-1-one,
(9) 3-(4-ethoxycarbonylpiperazin-1-yl)-2H-isoquinolin-1-one,
(10) 3-(4-methylhomopiperazin-1-yl)-2H-isoquinolin-1-one,
(11) 5-methyl-3-(4-methylhomopiperazin-1-yl)-2H-isoquinolin-1-one,
(12) 5-methyl-3-(4-dimethylaminopiperidin-1-yl)-2H-isoquinolin-1-one,
(13) 3-(3-dimethylaminopyrrolidin-1-yl)-5-methyl-2H-isoquinolin-1-one,
(14) 5-methyl-3-(4-morpholino)-2H-isoquinolin-1-one,
(17) 3-(4-hydroxypiperidin-1-yl)-5-methyl-2H-isoquinolin-1-one,
(18) 5-methoxy-3-(4-methylpiperazin-1-yl)-2H-isoquinolin-1-one,
(19) 5-hydroxy-3-(4-methylpiperazin-1-yl)-2H-isoquinolin-1-one,
(20) 5-fluoro-3-(4-methylpiperazin-1-yl)-2H-isoquinolin-1-one,
(21) 5-chloro-3-(4-methylpiperazin-1-yl)-2H-isoquinolin-1-one,
(22) 5-bromo-3-(4-methylpiperazin-1-yl)-2H-isoquinolin-1-one,
(23) 8-chloro-3-(4-methylpiperazin-1-yl)-2H-isoquinolin-1-one,
(24) 7-methyl-3-(4-methylpiperazin-1-yl)-2H-isoquinolin-1-one,
(25) 7-bromo-3-(4-methylpiperazin-1-yl)-2H-isoquinolin-1-one,
(28) 5-fluoro-3-(4-dimethylaminopiperidin-1-yl)-2H-isoquinolin-1-one,
(29) 5-chloro-3-(4-dimethylaminopiperidin-1-yl)-2H-isoquinolin-1-one,
(30) 6-chloro-3-(4-methylpiperazin-1-yl)-2H-isoquinolin-1-one,
(31) 7-bromo-3-(4-dimethylaminopiperidin-1-yl)-2H-isoquinolin-1-one,
(32) 5-bromo-3-(4-dimethylaminopiperidin-1-yl)-2H-isoquinolin-1-one,
(33) 5-fluoro-3-(4-(2-hydroxyethyl)piperazin-1-yl)-2H-isoquinolin-1-one,
(34) 6-methyl-3-(4-methylpiperazin-1-yl)-2H-isoquinolin-1-one,
(35) 3-(4-(2-hydroxyethyl)piperazin-1-yl)-6-methyl-2H-isoquinolin-1-one,
(36) 8-methyl-3-(4-methylpiperazin-1-yl)-2H-isoquinolin-1-one,
(37) 7-bromo-3-(4-(2-hydroxyethyl)piperazin-1-yl)-2H-isoquinolin-1-one,
(38) 3-(4-methylpiperazin-1-yl)-5-nitro-2H-isoquinolin-1-one,
(39) 5-amino-3-(4-methylpiperazin-1-yl)-2H-isoquinolin-1-one 1 water adduct,
(41) 3-[4-(2-hydroxyethyl)piperazin-1-yl]-8-methyl-2H-isoquinolin-1-one,
(43) 3-[4-(2-hydroxyethyl)piperazin-1-yl]-7-methyl-1H-isoquinolin-1-one,
(62) 3-{4-[2-(piperidin-1-yl)ethyl]piperazin-1-yl}-2H-isoquinolin-1-one,
(63) 3-{4-[3-(piperidin-1-yl)propyl]piperazin-1-yl}-2H-isoquinolin-1-one,
(65) 3-{4-[4-(piperidin-1-yl)butyl]piperazin-1-yl}-2H-isoquinolin-1-one,
(66) 3-{4-[5-(piperidin-1-yl)pentyl]piperazin-1-yl}-2H-isoquinolin-1-one,
(69) 5-methyl-3-[4-(4-morpholino)piperidin-1-yl]-2H-isoquinolin-1-one,
(70) 5-methyl-3-{4-[2-(piperidin-1-yl)ethyl]piperazin-1-yl}-2H-isoquinolin-1-one,
(71) 5-methyl-3-{4-[3-(piperidin-1-yl)propyl]piperazin-1-yl}-2H-isoquinolin-1-one,
(72) 5-methyl-3-{4-[5-(piperidin-1-yl)pentyl]piperazin-1-yl}-2H-isoquinolin-1-one,
(73) 5-methyl-3-{4-[4-(piperidin-1-yl)butyl]piperazin-1-yl}-2H-isoquinolin-1-one,
(74) 5-methyl-3-{4-[4-(4-morpholino)butyl]piperazin-1-yl}-2H-isoquinolin-1-one,
(75) 5-methyl-3-(4-(4-(4-methylpiperazin-1-yl)butyl)piperazin-1-yl)-2H-isoquinolin-1-one,
(76) 7-bromo-3-{4-[4-(piperidin-1-yl)butyl]piperazin-1-yl}-2H-isoquinolin-1-one,
(77) 5-chloro-3-{4-[4-(piperidin-1-yl)butyl]piperazin-1-yl}-2H-isoquinolin-1-one,
(78) 5-bromo-3-{4-[4-(piperidin-1-yl)butyl]piperazin-1-yl}-2H-isoquinolin-1-one,
(80) 5-chloro-3-{4-[4-(4-morpholino)butyl]piperazin-1-yl}-2H-isoquinolin-1-one,
(81) 3-(piperidin-4-yl)-2H-isoquinolin-1-one hydrobromide,
(82) 3-(1-methylpiperidin-4-yl)-2H-isoquinolin-1-one 1/5 water adduct,
(83) 3-((4-methylpiperazin-1-yl)carbonyl)-2H-isoquinolin-1-one,
(90) 3-(3-(dimethylamino)propyl)-5-methyl-2H-isoquinolin-1-one,
(93) 3-(piperidin-4-yl)-5-methyl-2H-isoquinolin-1-one hydrochloride,
(95) 5-chloro-3-(1-methylpiperidin-4-yl)-2H-isoquinolin-1-one,
(97) 4-phenyl-3-(piperidin-4-yl)-2H-isoquinolin-1-one hydrobromide,
(98) 3-(1-methylpiperidin-4-yl)-4-phenyl-2H-isoquinolin-1-one 1/5 water adduct,
(99) 4-(4-methoxyphenyl)-3-(1-methylpiperidin-4-yl)-2H-isoquinolin-1-one,
(100) 4-(4-chlorophenyl)-3-(1-methylpiperidin-4-yl)-2H-isoquinolin-1-one,
(106) 5-fluoro-3-(1-methylpiperidin-4-yl)-2H-isoquinolin-1-one,
(107) 5-methoxy-3-(1-methylpiperidin-4-yl)-2H-isoquinolin-1-one,
(108) 5-hydroxy-3-(1-methylpiperidin-4-yl)-2H-isoquinolin-1-one,
(110) 5-(4-methylpiperazin-1-yl)-6H-thieno[2,3-c]pyridin-7-one,
(112) 6-(4-methylpiperazin-1-yl)-5H-thieno[3,2-c]pyridin-4-one,
(114) 3-(4-methylpiperazin-1-yl)-2H-benz[f]isoquinolin-1-one,
(115) 3-(4-dimethylaminopiperidin-1-yl)-2H-benz[f]isoquinolin-1-one,
(116) 3-(4-methylpiperazin-1-yl)-2H-benz[h]isoquinolin-1-one,
(117) 3-(4-dimethylaminopiperidin-1-yl)-2H-benz[h]isoquinolin-1-one,
(120) 8-methyl-2-(piperidin-4-yl)-3H-quinazolin-4-one,
(121) 2-(1-methylpiperidin-4-yl)-8-methyl-3H-quinazolin-4-one,
(123) 8-methoxy-2-(1-methylpiperidin-4-yl)-3H-quinazolin-4-one,
(137) 8-methyl-2-(4-dimethylaminocyclohexan-1-yl)-3H-quinazolin-4-one,
(138) 2-(1-azabicyclo[2.2.2]octan-3-yl)-8-methyl-3H-quinazolin-4-one,
(139) 2-((1-azabicyclo[2.2.2]octan-3-yl)methyl)-8-methyl-3H-quinazolin-4-one,
(144) 8-methyl-2-(2-(dimethylamino)ethyl)-3H-quinazolin-4-one,
(145) 8-methyl-2-(3-(dimethylamino)propyl)-3H-quinazolin-4-one,
(146) 8-methyl-2-(5-(dimethylamino)pentyl)-3H-quinazolin-4-one,
(147) 3-(4-(dimethylamino)cyclohexan-1-yl)-2H-isoquinolin-1-one, and
(148) 3-(3-(4-methylpiperazin-1-yl)propyl)-2H-isoquinolin-1-one; as well as
(151) (R)-3-(2-hydroxymethyl-4-methylpiperazin-1-yl)-2H-isoquinolin-1-one,
(152) (S)-3-(2-hydroxymethyl-4-methylpiperazin-1-yl)-2H-isoquinolin-1-one,
(153) 3-(3-hydroxymethyl-4-methylpiperazin-1-yl)-5-methyl-2H-isoquinolin-1-one,
(154) 3-(3-ethoxycarbonyl-4-methylpiperazin-1-yl)-2H-isoquinolin-1-one,
(155) 3-(3-methylpiperazin-1-yl)-2H-isoquinolin-1-one,
(156) (S)-3-(3-hydroxymethyl-4-methylpiperazin-1-yl)-2H-isoquinolin-1-one,
(157) (R)-3-(3-hydroxymethyl-4-methylpiperazin-1-yl)-2H-isoquinolin-1-one,
(158) 3-(1-methylpiperidin-4-yl)-3,4-dihydro-2H-isoquinolin-1-one,
(159) 8-methyl-2-[2-(diethylamino)ethyl]-3H-quinazolin-4-one,
(162) 3-(3,5-dimethylpiperazin-1-yl)-2H-isoquinolin-1-one,
(163) 4-(4-methylphenyl)-3-(1-methylpiperidin-4-yl)-2H-isoquinolin-1-one,
(164) 4-(4-fluorophenyl)-3-(1-methylpiperidin-4-yl)-2H-isoquinolin-1-one,
(165) 8-methyl-2-(2-piperidinoethyl)-3H-quinazolin-4-one,
(166) 8-methyl-2-[2-(morpholin-4-yl)ethyl]-3H-quinazolin-4-one,
(167) 4-(2-methoxyphenyl)-3-(1-methylpiperidin-4-yl)-2H-isoquinolin-1-one,
(168) 4-(3-methylphenyl)-3-(1-methylpiperidin-4-yl)-2H-isoquinolin-1-one,
(169) 4-(2-methylphenyl)-3-(1-methylpiperidin-4-yl)-2H-isoquinolin-1-one,
(170) 4-(3-methoxyphenyl)-3-(1-methylpiperidin-4-yl)-2H-isoquinolin-1-one,
(171) 5-methoxymethyloxy-3-(1-methylpiperidin-4-yl)-2H-isoquinolin-1-one,
(173) 8-methyl-2-[5-(diethylamino)pentyl]-3H-quinazolin-4-one,
(175) 8-methyl-2-[4-(dimethylamino)butyl]-3H-quinazolin-4-one,
(176) 8-methyl-2-[3-(pyrrolidin-1-yl)propyl]-3H-quinazolin-4-one,
(177) 7-(1-methylpiperidin-4-yl)-6H-1,6-naphthyridin-5-one 1/10 water-g, product,
(178) 5-methyl-3-(1-methylpiperidin-4-yl)-2H-isoquinolin-1-one,
(179) 4-methyl-3-(1-methylpiperidin-4-yl)-2H-isoquinolin-1-one,
(180) 5-(dimethylamino)-3-(1-methylpiperidin-4-yl)-2H-isoquinolin-1-one,
(181) 5-amino-3-(1-methylpiperidin-4-yl)-2H-isoquinolin-1-one,
(182) 4-(2-fluorophenyl)-3- (1-methylpiperidin-4-yl)-2H-isoquinolin-1-one,
(183) 7-chloro-3-(1-methylpiperidin-4-yl)-2H-isoquinolin-1-one,
(184) 5-hydroxy-3-(1-methylpiperidin-4-yl)-3,4-dihydro-2H-isoquinolin-1-one,
(185) 5-methoxymethyloxy-3-(3-dimethylaminopropyl)-2H-isoquinolin-1-one,
(186) 5-hydroxy-3-(3-dimethylaminopropyl)-2H-isoquinolin-1-one hydrochloride,
(187) 5-methoxymethyloxy-3-(4-dimethylaminobutyl)-2H-isoquinolin-1-one,
(188) 5-hydroxy-3-(4-dimethylaminobutyl)-2H-isoquinolin-1-one hydrochloride,
(189) 5-hydroxy-3-(2-(piperidin-1-yl)ethyl)-2H-isoquinolin-1-one,
(190) 3-(1-methyl-1,2,3,6-tetrahydropyridin-4-yl)-2H-isoquinolin-1-one,
(191) 3-(1-benzylpiperidin-3-yl)-2H-isoquinolin-1-one,
(192) 3-(1-methylpiperidin-3-yl)-2H-isoquinolin-1-one,
(193) 3-(1-methyl-1,2,3,6-tetrahydropyridin-5-yl)-2H-isoquinolin-1-one,
(194) 3-(3-hydroxymethylpiperazin-1-yl)-2H-isoquinolin-1-one,
(195) 3-(4-ethyl-3-hydroxymethylpiperazin-1-yl)-2H-isoquinolin-1-one,
(196) 3-(3-hydroxymethyl-4-propylpiperazin-1-yl)-2H-isoquinolin-1-one,
(197) 3-(4-benzyl-3-hydroxymethylpiperazin-1-yl)-2H-isoquinolin-1-one,
(198) 5-bromo-3-(3-hydroxymethylpiperazin-1-yl)-2H-isoquinolin-1-one,
(199) 5-bromo-3-(3-hydroxymethyl-4-methylpiperazin-1-yl)-2H-isoquinolin-1-one,
(200) 3-(4-piperidinopiperidin-1-yl)-2H-isoquinolin-1-one,
(201) 3-(3-hydroxymethylpiperidin-1-yl)-2H-isoquinolin-1-one,
(202) 3-(3-(dimethylcarbamoyl)piperidin-1-yl)-2H-isoquinolin-1-one,
(203) 3-(3-hydroxymethyl-4-isobutylpiperazin-1-yl)-2H-isoquinolin-1-one,
(204) 3-[4-(dimethylamino)butyl]-2H-isoquinolin-1-one,
(205) 5-fluoro-3-(3-hydroxymethyl-4-methylpiperazin-1-yl)-2H-isoquinolin-1-one,
(206) 3-(3-(dimethylaminomethyl)piperidin-1-yl)-2H-isoquinolin-1-one,
(207) 6-methyl-3-(1-methylpiperidin-4-yl)-2H-isoquinolin-1-one,
(208) 7-methyl-3-(1-methylpiperidin-4-yl)-2H-isoquinolin-1-one,
(209) 8-methyl-3-(1-methylpiperidin-4-yl)-2H-isoquinolin-1-one,
(210) 7-methoxymethyloxy-3-(1-methylpiperidin-4-yl)-2H-isoquinolin-1-one,
(211) 7-hydroxy-3-(1-methylpiperidin-4-yl)-2H-isoquinolin-1-one hydrochloride,
(212) (S)-3-(3-hydroxymethyl-4-methylpiperazin-1-yl)-5-methyl-2H-isoquinolin-1-one,
(213) 3-(3-(pyrrolidin-1-yl)propyl)-2H-isoquinolin-1-one,
(214) 5-chloro-3-(3-hydroxymethyl-4-methylpiperazin-1-yl)-2H-isoquinolin-1-one,
(216) 3-[3-(piperidin-1-yl)propyl]-2H-isoquinolin-1-one,
(217) 5-hydroxy-3-(3-(pyrrolidin-1-yl)propyl)-2H-isoquinolin-1-one,
(218) 5-methyl-3-[2-(piperidin-1-yl)ethyl]-2H-isoquinolin-1-one,
(219) 3-[2-(piperidin-1-yl)ethyl]-2H-isoquinolin-1-one,
(220) 3-[2-(pyrrolidin-1-yl)ethyl]-2H-isoquinolin-1-one,
(221) 5-methyl-3-[2-(pyrrolidin-1-yl)ethyl]-2H-isoquinolin-1-one,
(222) 5-methyl-3-[3-(pyrrolidin-1-yl)propyl-1-yl]-2H-isoquinolin-1-one,
(223) 1,5-dihydro-6-(1-methyl-1,2,3,6-tetrahydropyridin-4-yl)pyrazolo[4,3-c]pyridin-4-one,
(224) N,N-dimethyl-3-(1-methylpiperidin-1-yl)-2H-1-oxoisoquinoline-5-carboxamide 1/4 water adduct,
(225) 5-methyl-3-(octahydroindolizin-7-yl)-2H-isoquinolin-1-one 3/4 water adduct,
(226) 5-methyl-3-(octahydroindolizin-7-yl)-2H-isoquinolin-1-one 1/2 water adduct,
(227) 3-(1-methylpiperidin-1-yl)-2H-1-oxoisoquinoline-5-carboxylic acid hydrochloride,
(228) 5-methyl-3-[3-(piperidin-1-yl)propyl]-2H-isoquinolin-1-one,
(229) 3-(dimethylamino)methyl-2H-isoquinolin-1-one,
(230) 3-[(4-methylpiperazin-1-yl)methyl]-2H-isoquinolin-1-one,
(231) 3-(piperidinomethyl)-2H-isoquinolin-1-one,
(232) 3-[(morpholin-1-yl)methyl]-2H-isoquinolin-1-one,
(233) 3-[(homopiperidin-1-yl)methyl]-2H-isoquinolin-1-one,
(234) 3-[3-(homopiperidin-1-yl)propyl]-2H-isoquinolin-1-one,
(235) 3-(1-sulfamoylpiperidin-4-yl)-5-methyl-2H-isoquinolin-1-one 1/4 water adduct,
(236) 3-(4-methyl-3-oxopiperazin-1-yl)-5-methyl-2H-isoquinolin-1-one 1/10 water adduct,
(237) 3-(1-aminopiperidin-4-yl)-5-methyl-2H-isoquinolin-1-one,
(238) 3-(1-(methanesulfonylamino)piperidin-4-yl)-5-methyl-2H-isoquinolin-1-one,
(239) 3-(1-trifluoroacetaminopiperidin-4-yl)-5-methyl-2H-isoquinolin-1-one,
(240) 3-[2-(homopiperidin-1-yl)ethyl]-2H-isoquinolin-1-one,
(241) N-(5-methyl-2H-1-oxoisoquinolin-3-yl)-2-(dimethylamino)acetamide,
(243) N-(5-methyl-2H-1-oxoisoquinolin-3-yl)-3-(dimethylamino)propanamide,
(244) 3-(1-dimethylaminopiperidin-4-yl)-5-methyl-2H-isoquinolin-1-one,
(245) N-(5-methyl-2H-1-oxoisoquinolin-3-yl)-4-(dimethylamino)butanamide,
(246) N-(2H-1-oxoisoquinolin-3-yl)-4-(dimethylamino)butanamide,
(247) 3-(4-methyl-2-oxopiperazin-1-yl)-2H-isoquinolin-1-one,
(248) 5-methyl-3-(1-methylpyrrolidin-3-yl)-2H-isoquinolin-1-one, and
(249) 3-(1-methylpyrrolidin-3-yl)-2H-isoquinolin-1-one are preferable. The numbers in the parentheses show Example numbers.

More preferable specific examples include
(1) 5-methyl-3-(4-methylpiperazin-1-yl)-2H-isoquinolin-1-one,
(3) 3-(4-dimethylaminopiperidin-1-yl)-2H-isoquinolin-1-one,
(4) 3-(3-hydroxymethyl-4-methylpiperazin-1-yl)-2H-isoquinolin-1-one,
(12) 5-methyl-3-(4-dimethylaminopiperidin-1-yl)-2H-isoquinolin-1-one,
(19) 5-hydroxy-3-(4-methylpiperazin-1-yl)-2H-isoquinolin-1-one,
(82) 3-(1-methylpiperidin-4-yl)-2H-isoquinolin-1-one 1/5 water adduct,
(90) 3-(3-(dimethylamino)propyl)-5-methyl-2H-isoquinolin-1-one,
(98) 3-(1-methylpiperidin-4-yl)-4-phenyl-2H-isoquinolin-1-one 1/5 water adduct,
(108) 5-hydroxy-3-(1-methylpiperidin-4-yl)-2H-isoquinolin-1-one, and
(121) 2-(1-methylpiperidin-4-yl)-8-methyl-3H-quinazolin-4-one; as well as
(156) (S)-3-(3-hydroxymethyl-4-methylpiperazin-1-yl)-2H-isoquinolin-1-one,
(178) 5-methyl-3-(1-methylpiperidin-4-yl)-2H-isoquinolin-1-one,
(181) 5-amino-3-(1-methylpiperidin-4-yl)-2H-isoquinolin-1-one,
(186) 5-hydroxy-3-(3-dimethylaminopropyl)-2H-isoquinolin-1-one hydrochloride,
(189) 5-hydroxy-3-(2-(piperidin-1-yl)ethyl)-2H-isoquinolin-1-one, and
(212) (S)-3-(3-hydroxymethyl-4-methylpiperazin-1-yl)-5-methyl-2H-isoquinolin-1-one, and of these,
(82) 3-(1-methylpiperidin-4-yl)-2H-isoquinolin-1-one 1/5 water adduct,
(90) 3-(3-(dimethylamino)propyl)-5-methyl-2H-isoquinolin-1-one, and
(108) 5-hydroxy-3-(1-methylpiperidin-4-yl)-2H-isoquinolin-1-one; as well as
(156) (S)-3-(3-hydroxymethyl-4-methylpiperazin-1-yl)-2H-isoquinolin-1-one,
(178) 5-methyl-3-(1-methylpiperidin-4-yl)-2H-isoquinolin-1-one,
(181) 5-amino-3-(1-methylpiperidin-4-yl)-2H-isoquinolin-1-one,
(186) 5-hydroxy-3-(3-dimethylaminopropyl)-2H-isoquinolin-1-one hydrochloride,
(189) 5-hydroxy-3-(2-(piperidin-1-yl)ethyl)-2H-isoquinolin-1-one and
(212) (S)-3-(3-hydroxymethyl-4-methylpiperazin-1-yl)-5-methyl-2H-isoquinolin-1-one are preferable.

While the fused heterocyclic compound of the present invention is stable in aqueous solutions, a compound of the formula (I), wherein
the dotted line part is a double bond,
ring Ar is a benzene ring,
X is a carbon atom optionally substituted by phenyl optionally having substituent(s), or a nitrogen atom,
Y is -(CH₂)ₘ- wherein m is 0 or an integer of 1-10,
R¹ and R² are the same or different and each is hydrogen, alkyl, hydroxy or amino, and
R is dialkylamino, or represented by the above-mentioned formula (b)
wherein a dotted line part is a single bond, W is CH or a nitrogen atom, t is an integer of 0-3, and R⁶ is hydrogen, dialkylamino or alkyl,
is still superior in stability, and of these compounds,
(82) 3-(1-methylpiperidin-4-yl)-2H-isoquinolin-1-one 1/5 water adduct,
(108) 5-hydroxy-3-(1-methylpiperidin-4-yl)-2H-isoquinolin-1-one, and
(121) 2-(1-methylpiperidin-4-yl)-8-methyl-3H-quinazolin-4-one
are preferable.

In addition, the fused heterocyclic compounds of the present invention have lower affinity for adrenergic α1 receptor, of which compounds of the formula (I), wherein
the dotted line part is a double bond,
ring Ar is a benzene ring,
X is a carbon atom optionally substituted by phenyl optionally having substituent(s), or a nitrogen atom,
Y is -(CH₂)ₘ- wherein m is 0 or an integer of 1-10,
R¹ and R² are the same or different and each is hydrogen, alkyl, hydroxy or amino, and
R is dialkylamino, or represented by the above-mentioned formula (a)
wherein W is CH or a nitrogen atom, t is an integer of 0-3, R⁵ and R^{5'} are the same or different and each is hydroxyalkyl, and R⁶ is hydrogen, dialkylamino or alkyl,
show still lower affinity for adrenergic α1 receptor, and of these compounds,
(156) (S)-3-(3-hydroxymethyl-4-methylpiperazin-1-yl)-2H-isoquinolin-1-one and
(212) (S)-3-(3-hydroxymethyl-4-methylpiperazin-1-yl)-5-methyl-2H-isoquinolin-1-one
are preferable.

Compounds of the formula (I), wherein
the dotted line part is a double bond,
ring Ar is a benzene ring,
X is a carbon atom optionally substituted by phenyl optionally having substituent(s), or a nitrogen atom,
Y is -(CH₂)ₘ- wherein m is 0 or an integer of 1-10,
R¹ is alkyl, hydroxy or amino,
R² is hydrogen, and
R is dialkylamino, or represented by the above-mentioned formula (a)
wherein W is CH or a nitrogen atom, t is an integer of 0-3, R⁵ and R^{5'} are the same or different and each is hydroxyalkyl, and R⁶ is hydrogen, dialkylamino or alkyl,
are superior to conventional compounds in PARP inhibitory action, and of these compounds,
(1) 5-methyl-3-(4-methylpiperazin-1-yl)-2H-isoquinolin-1-one is particularly preferable.

The preferable position of substitution of R¹ when ring Ar is a benzene ring is as shown in the following formula (III)

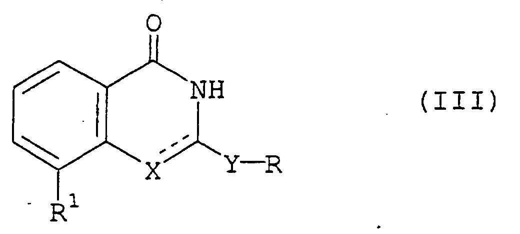

As the compound of the formula (I) and a pharmaceutically acceptable salt thereof, an acid addition salt with inorganic acid (e.g., hydrochloric acid, hydrobromic acid) or organic acid can be mentioned. In addition, the compound can be also converted to oxalate for crystallization of the compound.

The compound of the formula (I) and a pharmaceutically acceptable salt thereof may be present as a water adduct, hydrate or a solvate. Such water adduct (1/2 water adduct, 1/4 water adduct, 1/5 water adduct, 1/10 water adduct, 3/4 water adduct, 1 water adduct and the like), hydrate and solvate are also encompassed in the present invention. When the compound of the formula (I) has an asymmetric atom, at least two kinds of optical isomers are present. Such optical isomers and mixtures thereof (including racemate) are encompassed in the present invention.

The compounds of the present invention encompassed in the formula (I) can be synthesized by the following methods. In the reaction formula groups below, each symbol is as defined above unless particularly specified.

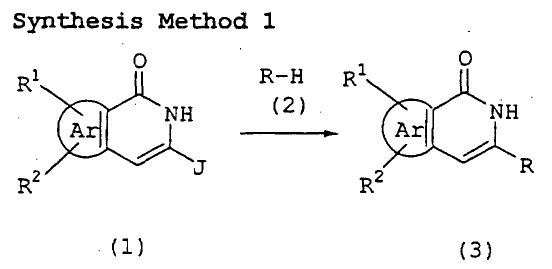

A compound of the formula (1) described in Berichte (Chem. Ber.), vol. 102, pp. 3656-3665, 1969, wherein J is a leaving group generally used in organic synthetic chemistry, such as a chlorine atom, a bromine atom, an iodine atom, a methanesulfonyloxy, a p-toluenesulfonyloxy and the like, and a compound of the formula (2) are reacted in the presence or absence of a suitable base generally used in organic synthetic chemistry, such as potassium carbonate, potassium hydrogencarbonate, sodium carbonate, sodium hydrogencarbonate, sodium acetate, potassium acetate, sodium hydroxide, potassium hydroxide, sodium hydride and the like, in a suitable solvent that does not inhibit the progress of the reaction (alcohol such as methanol, ethanol and the like, benzene, toluene, xylene, dimethylformamide, dimethyl sulfoxide, N-methyl-2-pyrrolidone, or a mixed solvent thereof and the like) or without solvent, at a temperature of from room temperature to reflux temperature of the solvent for 0.1 (6 min) - 48 hr to give a compound of the formula (3).

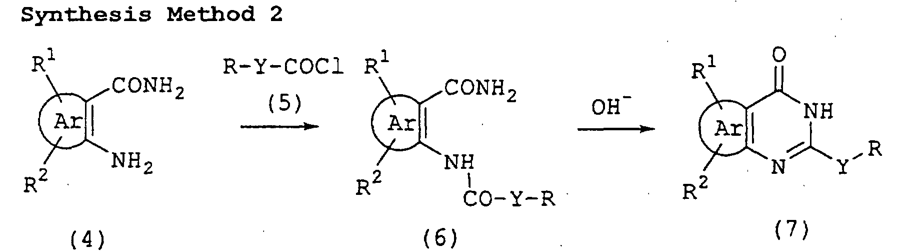

A compound of the formula (4), wherein ring Ar, R¹ and R² are as defined above, is reacted with acid chloride (5) in a suitable solvent that does not inhibit the progress of the reaction (methylene chloride, chloroform, benzene, toluene, xylene, dimethylformamide, dimethyl sulfoxide, N-methyl-2-pyrrolidone, or a mixed solvent thereof and the like) at a temperature of from room temperature to reflux temperature of the solvent for 0.1 (6 min) - 48 hr to give a compound of the formula (6). Then, the compound of the formula (6) is reacted in the presence of a suitable base generally used in organic synthetic chemistry, such as sodium methoxide, sodium ethoxide, potassium methoxide, potassium ethoxide, potassium tertiary butoxide, sodium, potassium, potassium carbonate, potassium hydrogencarbonate, sodium carbonate, sodium hydrogencarbonate, sodium acetate, potassium acetate, sodium hydroxide, potassium hydroxide, sodium hydride, butyllithium and the like, in a suitable solvent that does not inhibit the progress of the reaction (benzene, toluene, xylene, dimethylformamide, dimethyl sulfoxide, N-methyl-2-pyrrolidone, pyridine, or a mixed solvent thereof and the like), at a temperature of from room temperature to reflux temperature of the solvent for 0.1 (6 min) - 48 hr to give a compound of the formula (7).

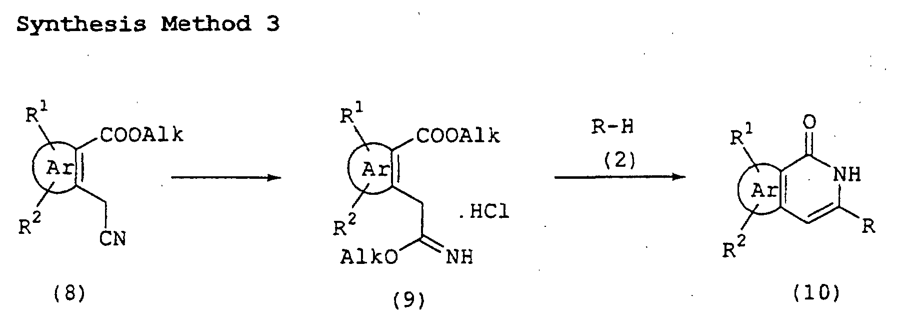

A compound of the formula (8), wherein Alk is alkyl having 1-4 carbon atoms, is reacted by blowing in a hydrochloric acid gas or by adding hydrochloric acid-methanol, hydrochloric acid-ethanol, hydrochloric acid-propanol, hydrochloric acid-isopropanol, hydrochloric acid-butanol, hydrochloric acid-diethyl ether, hydrochloric acid-diisopropyl ether, hydrochloric acid-tetrahydrofuran, or hydrochloric acid-1,4-dioxane solution, in a suitable solvent that does not inhibit the progress of the reaction (methanol, ethanol, propanol, isopropanol, butanol, diethyl ether, diisopropyl ether, tetrahydrofuran, 1,4-dioxane, hexane, cyclohexane, pentane or an optional mixed solvent thereof and the like) at a temperature of from -78°c to room temperature for 0.1 (6 min) - 48 hr to give a compound of the formula (9). The compound of the formula (9) is reacted with a compound of the formula (2) in a suitable solvent that does not inhibit the progress of the reaction (methanol, ethanol, propanol, isopropanol, butanol, diethyl ether, diisopropyl ether, tetrahydrofuran, 1,4-dioxane, hexane, cyclohexane, pentane or an optional mixed solvent thereof and the like) at a temperature of from -78°C to the refluxing temperature of the solvent for 0.1 (6 min) - 48 hr to give a compound of the formula (10).

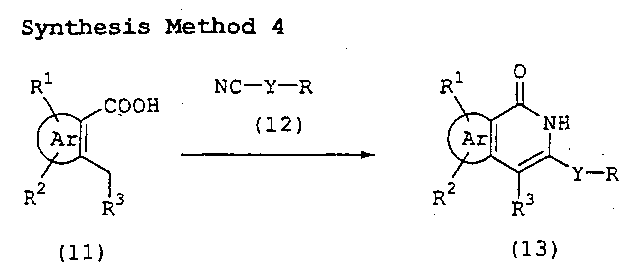

A compound of the formula (11) wherein R³ is hydrogen, alkyl, phenyl optionally having substituent(s) or an aromatic heterocyclic group optionally having substituent(s); as the alkyl, those similar to alkyl for R¹ can be mentioned, with preference given to methyl; as the aromatic heterocyclic group, monovalent groups of those similar to the aromatic heterocycle for ring Ar can be mentioned; as the substituent that may substitute phenyl and aromatic heterocyclic group, those similar to R¹ and R² can be mentioned, particularly alkyl (preferably methyl), halogen (preferably chlorine, fluorine) and alkoxy (preferably methoxy)) is reacted with a compound of the formula (12) in the presence of a suitable base generally used in organic synthetic chemistry, such as n-butyllithium, lithium diisopropylamide, lithium diethylamide, lithium bistrimethylsilylamide and the like, in a suitable solvent that does not inhibit the progress of the reaction (diethyl ether, diisopropyl ether, tetrahydrofuran, 1,4-dioxane, or an optional mixed solvent thereof and the like) at a temperature from -78°C to the refluxing temperature of the solvent for 0.1 (6 min) - 48 hr to give a compound of the formula (13).

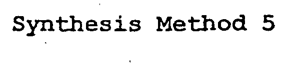

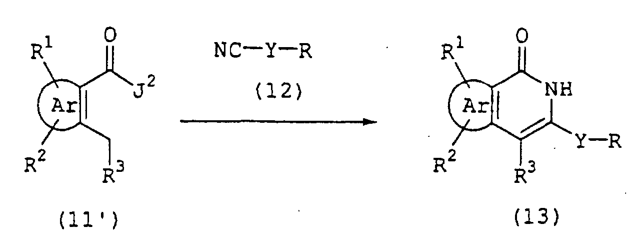

A compound of the formula (11') wherein J² is hydroxy, amino, monoalkylamino (as defined for amino substituted by alkyl for R¹) or dialkylamino (as defined for dialkylamino for R²) is reacted with a compound of the formula (12) in the presence of a suitable base generally used in organic synthetic chemistry, such as n-butyllithium, lithium diisopropylamide, lithium diethylamide, lithium bistrimethylsilylamide and the like, in a suitable solvent that does not inhibit the progress of the reaction (diethyl ether, diisopropyl ether, tetrahydrofuran, 1,4-dioxane, or an optional mixed solvent thereof and the like) at a temperature from -78°C to the refluxing temperature of the solvent for 0.1 (6 min) - 48 hr to give a compound of the formula (13).

The compound of the present invention thus obtained can be isolated and purified by a conventional method.

The compound of the formula (I) obtained by the above-mentioned method, an optical isomer thereof or a pharmaceutically acceptable salt thereof has potent PARP inhibitory action and are useful as a therapeutic drug of cerebral infarction, particularly a therapeutic drug in acute phase of cerebral infarction.

When the fused heterocyclic compound, an optical isomer thereof or a pharmaceutically acceptable salt thereof of the present invention is used as a pharmaceutical agent, the compound of the present invention is admixed with a pharmaceutically acceptable carrier (excipient, binder, disintegrant, flavoring agent, odor improving agent, emulsifier, diluent, dissolution aids and the like) to give a pharmaceutical composition or preparation (tablet, pill, capsule, granule, powder, syrup, emulsion, elixir, suspension, solution, injection, drop, suppository and the like), which can be administered orally or parenterally. A pharmaceutical composition can be prepared according to a conventional method. In the present specification, "parenteral" includes subcutaneous injection, intravenous injection, intramuscular injection, intraperitoneal injection, infusion and the like. A preparation for injection can be prepared according to a method known in the pertinent field. Suppository for rectal administration can be produced by admixing the drug and a suitable excipient and the like. As the dosage form of a solid preparation for oral administration, those mentioned above, such as powder, granule, tablet, pill, capsule and the like, can be mentioned. As the liquid for oral administration, a pharmaceutically acceptable emulsion, syrup, elixir, suspension, solution and the like can be mentioned.

The dose is determined in consideration of age, body weight, general health conditions, sex, diet, administration time, administration method, clearance rate, combination of drugs, the severity of the disease state for which patient is under treatment, and other factors. The compound of the present invention, an optical isomer thereof and a pharmaceutically acceptable salt thereof can be used safely at low toxicity. While the daily dose varies depending on the condition and body weight of patient, the kind of compound, administration route and the like, it is desirably administered parenterally in an amount of about 0.01 - 50 mg/person/day, preferably 0.01 - 20 mg/person/day by subcutaneous, intravenous, intramuscular or intarectal administration, and orally in an amount of about 0.01 - 150 mg/person/day, preferably 0.1-100 mg/person/day.

### Examples

The present invention is explained in detail in the following by Example, Formulation Examples and Experimental Examples, which are not to be construed as limitative. The unit of the J value shown here is Hz.

### Example 1

3-Chloro-5-methyl-2H-isoquinolin-1-one (1.0 g) was dissolved in 1-methylpiperazine (2 ml), and the mixture was stirred under heating at 120°C for 4 hr. After the completion of the reaction, reaction solution was dissolved in chloroform, washed with an aqueous potassium carbonate solution and dried over potassium carbonate. The solvent was concentrated, and the obtained residue was purified by silica gel column chromatography. A chloroform:methanol=20:1 effluent fraction was concentrated, and diisopropyl ether was added to the obtained residue. The precipitated crystals were collected by filtration to give 5-methyl-3-(4-methylpiperazin-1-yl)-2H-isoquinolin-1-one (0.373 g). melting point: 186-188°C/decomposition. ¹H-NMR(400MHz,CDCl₃) d: 2.41(3H,s), 2.47 (3H,s) , 2.60-2.65(4H,m), 3.30-3.37(4H,m), 5.78(1H,s), 7.18(1H,t,J=8Hz), 7.40(1H,d,J=8Hz), 8.13(1H,d,J=8Hz), 11.39(1H,brS).

### Example 2

In the same manner as in Example 1, the reaction was carried out using 3-chloro-2H-isoquinolin-1-one (1.0 g) and 3-dimethylaminopyrrolidine (2 ml) to give 3-(3-dimethylaminopyrrolidin-1-yl)-2H-isoquinolin-1-one (195 mg). melting point: 233-234°C/decomposition. ¹H-NMR(400MHz,CDCl₃)δ: 2.29-2.33(2H,m), 2.40 (6H,s) , 2.98(1H,brS), 3.40-3.50(2H,m), 3.57-3.70(2H,m), 5.38(1H,s), 7.08(1H,t,J=8Hz), 7.26-7.29(1H,m), 7.43-7.47(1H,m), 8.14(1H,d,J=8Hz), 9.95(1H,brS).

### Example 3

In the same manner as in Example 1, the reaction was carried out using 3-chloro-2H-isoquinolin-1-one (1.0 g) and 4-dimethylaminopiperidine (2 ml) to give 3-(4-dimethylaminopiperidin-1-yl)-2H-isoquinolin-1-one (383 mg). melting point: 204-205°C/decomposition. ¹H-NMR(400MHz,CDCl₃)δ: 1.71-1.74(4H,m), 1.97-2.00(2H,m), 2.36 (6H,s) , 2.76-2.82(2H,m), 3.70-7.73(2H,m), 5.76(1H,s), 7.38(1H,d,J=8Hz), 7.54(1H,t,J=8Hz), 8.25(1H,d,J=8Hz), 9.98(1H,brS).

### Example 4

In the same manner as in Example 1, the reaction was carried out using 3-chloro-2H-isoquinolin-1-one (1.0 g) and 2-hydroxymethyl-1-methylpiperazine (2.0 g) to give 3-(3-hydroxymethyl-4-methylpiperazin-1-yl)-2H-isoquinolin-1-one (59 mg), melting point: 193-194°C/decomposition. ¹H-NMR(400MHz,CDCl₃)δ: 2.50(3H,s), 2.64-2.70(2H,m), 2.95-3.05(2H,m), 3.30-3.35(2H,m), 3.43-3.46(2H,m), 3.73(1H,d,J=12Hz), 4.07-4.11(1H,m), 5.85(1H,s), 7.31(1H,t,J=8Hz), 7.42(1H,d,J=8Hz), 7.55-7.58(1H,m), 8.26(1H,d,J=8Hz), 11.89(1H,brS).

### Example 5

In the same manner as in Example 1, the reaction is carried out using 3-chloro-2H-isoquinolin-1-one (1.0 g) and 4-aminopiperazine (2 ml) to give 3-(4-aminopiperazin-1-yl)-2H-isoquinolin-1-one (1.0 g).

### Example 6

In the same manner as in Example 1, the reaction is carried out using 3-chloro-2H-isoquinolin-1-one (1.0 g) and 4-dimethylaminopiperazine (2 ml) to give 3-(4-dimethylaminopiperazin-1-yl)-2H-isoquinolin-1-one.

### Example 7

In the same manner as in Example 1, the reaction was carried out using 3-chloro-2H-isoquinolin-1-one (1.0 g) and 4-propylpiperazine (2 ml) to give 3-(4-propylpiperazin-1-yl)-2H-isoquinolin-1-one (81 mg). melting point: 208-209°C/decomposition. ¹H-NMR(400MHz,CDCl₃)δ: 0.97(3H,t,J=8Hz), 1.56-1.60 (4H,m) , 2.68-2.69(4H,m), 3.27-3.29(4H,m), 5.77(1H,s), 7.27-7.31(1H,m), 7.41(1H,d,J=4Hz), 7.54-7.58(1H,m), 8.25(1H,d,J=4Hz), 10.30(1H,brS).

### Example 8

In the same manner as in Example 1, the reaction was carried out using 3-chloro-2H-isoquinolin-1-one (1.0 g) and 4-methanesulfonylpiperazine (2 ml) to give 3-(4-methanesulfonylpiperazin-1-yl)-2H-isoquinolin-1-one (49 mg). melting point: 257-259°C/decomposition. ¹H-NMR(400MHz,CDCl₃)δ: 2.90(3H,s), 3.39(4H,brS), 3.51(4H,brS), 5.84(1H,s), 7.32-7.36(1H,m), 7.44(1H,d,J=8Hz), 7.60(1H,d,J=8Hz), 8.20(1H,d,J=8Hz), 11.6(1H,brS).

### Example 9

In the same manner as in Example 1, the reaction was carried out using 3-chloro-2H-isoquinolin-1-one (1.0 g) and 4-ethoxycarbonylpiperazine (2 ml) to give 3-(4-ethoxycarbonylpiperazin-1-yl)-2H-isoquinolin-1-one (895 mg). melting point: 191-192°C/decomposition. ¹H-NMR(400MHz,CDCl₃)δ: 1.30(3H,t,J=4Hz), 3.26-3.29(4H,m), 3.56-3.57(4H,m), 4.17-4.22(2H,m), 5.84(1H,s), 7.33(1H,t,J=8Hz), 7.44(1H,d,J=4Hz), 7.58-7.87(1H,m), 8.24(1H,d,J=8Hz), 12.05(1H,brS).

### Example 10

In the same manner as in Example 1, the reaction was carried out using 3-chloro-2H-isoquinolin-1-one (1.0 g) and 4-methylhomopiperazine (2 ml) to give 3-(4-methylhomopiperazin-1-yl)-2H-isoquinolin-1-one (623 mg). melting point: 171-172°C/decomposition. ¹H-NMR(400MHz,CDCl₃)δ: 2.47(3H,s), 2.52-2.54 (2H,m) , 2.71-2.74 (2H,m) , 2.89-2.91(2H,m), 3.50-3.59 (2H,m) , 3.71(2H,m), 5.57(1H,s), 7.14(1H,d,J=8Hz), 7.29(1H,t,J=8Hz), 7.48(1H,t,J=8Hz), 8.14(1H,d,J=8Hz), 10.36(1H,brS).

### Example 11

In the same manner as in Example 1, the reaction was carried out using 3-chloro-5-methyl-2H-isoquinolin-1-one (1.0 g) and 4-methylhomopiperazine (2 ml) to give 5-methyl-3-(4-methylhomopiperazin-1-yl)-2H-isoquinolin-1-one (0.37 g). ¹H-NMR(CDCl₃) d: 2.00-2.09(2H,m), 2.41(3H+3H,S), 2.58-2.65(2H,m), 2.75-2.83(2H,m), 3.55-3.60(2H,m), 3.61-3.68(2H,m), 5.50(1H,s), 7.03(1H,t,J=8Hz), 7.33(1H,d,J=7Hz), 8.06(1H,d,J=8Hz), 10.10(1H,brS).

### Example 12

In the same manner as in Example 1, the reaction was carried out using 3-chloro-5-methyl-2H-isoquinolin-1-one (1.0 g) and 4-dimethylaminopiperidine (2 ml) to give 5-methyl-3-(4-dimethylaminopiperidin-1-yl)-2H-isoquinolin-1-one (1.17 g). melting point: 217-218°C melting point/decomposition. ¹H-NMR(400MHz,DMSO-d₆) d: 1.44-1.55(2H,m), 1.76-1.84(2H,m), 2.19(6H,s), 2.15-2.22(1H,m), 2.39(3H,s), 2.65-2.72(2H,m), 3.65-3.72(2H,m), 5.65(1H,s), 7.19(1H,t,J=8Hz), 7.39(1H,d,J=7Hz), 7.88(1H,d,J=8Hz), 11.13(1H,brS).

### Example 13

In the same manner as in Example 1, the reaction was carried out using 3-chloro-5-methyl-2H-isoquinolin-1-one (1.0 g) and 3-dimethylaminopyrrolidine (2 ml) to give 3-(3-dimethylaminopyrrolidin-1-yl)-5-methyl-2H-isoquinolin-1-one (0.15 g). ¹H-NMR(DMSO-d₆) d: 1.72-1.83(1H,m), 2.01-2.16(1H,m), 2.18(6H,s), 2.32(3H,s), 2.70-2.78(1H,m), 3.14-3.19(1H,m), 3.29-3.38(1H,m), 3.46-3.59(2H,m), 5.22(1H,s), 6.90(1H,t,J=8Hz), 7.28(1H,d,J=7Hz), 7.80(1H,d,J=8Hz), 10.64(1H,brS).

### Example 14

In the same manner as in Example 1, the reaction was carried out using 3-chloro-5-methyl-2H-isoquinolin-1-one (1.0 g) and morpholine (2 ml) to give 5-methyl-3-(4-morpholino)-2H-isoquinolin-1-one (0.43 g). ¹H-NMR(CDCl₃) d: 1.68-1.80(2H,m), 1.95-2.05(2H,m), 2.35-2.43(1H,m), 2.46(3H,s), 2.56-2.63(4H,m), 2.76-2.85(2H,m), 3.70-3.80(6H,m), 5.78(1H,s), 7.16(1H,t,J=8Hz), 7.39(1H,d,J=7Hz), 8.13(1H,d,J=8Hz), 11.64(1H,brS).

### Example 15

In the same manner as in Example 1, the reaction is carried out using 3-chloro-5-methyl-2H-isoquinolin-1-one (1.0 g) and 4-aminopiperazine (2 ml) to give 3-(4-aminopiperazin-1-yl)-5-methyl-2H-isoquinolin-1-one.

### Example 16

In the same manner as in Example 1, the reaction is carried out using 3-chloro-5-methyl-2H-isoquinolin-1-one (1.0 g) and 4-dimethylaminopiperazine (2 ml) to give 3-(4-dimethylaminopiperazin-1-yl)-5-methyl-2H-isoquinolin-1-one.

### Example 17

In the same manner as in Example 1, the reaction was carried out using 3-chloro-5-methyl-2H-isoquinolin-1-one (1.0 g) and 4-hydroxypiperidine (2 g) to give 3-(4-hydroxypiperidin-1-yl)-5-methyl-2H-isoquinolin-1-one (0.21 g). ¹H-NMR(DMSO-d₆) d: 1.40-1.55(2H,m), 1.75-1.80(2H,m), 2.37(3H,s), 2.80-2.90(2H,m), 3.40-3.55(2H,m), 3.56-3.65(1H,m), 4.70(1H,d,J=4Hz), 5.64(1H,s), 7.07(1H,t,J=8Hz), 7.37(1H,d,J=7Hz), 7.86(1H,d,J=8Hz), 11.10(1H,brS).

### Example 18

Ethyl 2-cyanomethyl-3-methoxybenzoate (10.0 g) was dissolved in chloroform (50 ml) and methanol (50 ml), and hydrochloric acid gas was blown thereinto under ice-cooling. After the completion of the reaction, the solvent was concentrated, and diisopropyl ether was added to the obtained residue. The precipitated crystals were collected by filtration to give methyl 2-ethoxycarbonyl-6-methoxyphenylacetimidate hydrochloride (12 g). Methyl 2-ethoxycarbonyl-6-methoxyphenylacetimidate hydrochloride (6 g) was dissolved in methanol (50 mL), and a solution of 1-methylpiperazine (4.6 ml) in methanol was added dropwise under ice-cooling. The mixture was stirred for one day at room temperature. After the completion of the reaction, the solvent was concentrated and the residue was purified by silica gel chromatography to give 5-methoxy-3-(4-methylpiperazin-1-yl)-2H-isoquinolin-1-one (3.0 g). ¹H-NMR(400MHz,CDCl₃) δ: 2.44(3H,s), 2.70-2.80(4H,m), 3.30-3.40(4H,m), 3.94(3H,s), 6.12(1H,s), 6.98(1H,d,J=8Hz), 7.21(1H,t,J=8Hz), 7.83(1H,d,J=8Hz), 11.23(1H,brS).

### Example 19

5-Methoxy-3-(4-methylpiperazin-1-yl)-2H-isoquinolin-1-one (2.0 g) obtained in Example 18 was dissolved in methylene chloride (40 ml), and boron tribromide (4.2 ml) was added at - 78°C. The mixture was stirred at room temperature for 2 days and poured into an aqueous potassium carbonate solution. The aqueous layer was concentrated and purified by silica gel column chromatography. A chloroform:methanol=10:1 effluent fraction was concentrated and further purified by silica gel column chromatography. A chloroform:methanol=10:1 effluent fraction was concentrated. Finally, the fraction was purified by silica gel column chromatography. A chloroform:methanol=10:1 effluent fraction was concentrated. The obtained crystals were collected by filtration using ethyl acetate to give 5-hydroxy-3-(4-methylpiperazin-1-yl)-2H-isoquinolin-1-one (0.1 g). ¹H-NMR(400MHz,DMSO-d₆) d: 2.21(3H,s), 2.39-2.49(4H,m), 3.05-3.12(4H,m), 5.84(1H,s), 6.95(1H,d,J=8Hz), 7.04(1H,t,J=8Hz), 7.48(1H,d,J=8Hz), 9.87(1H,brS), 11.06(1H,s).

### Example 20

In the same manner as in Example 1, the reaction was carried out using 3-chloro-5-fluoro-2H-isoquinolin-1-one (1.0 g) and 4-methylpiperazine (2 g) to give 5-fluoro-3-(4-methylpiperazin-1-yl)-2H-isoquinolin-1-one (578 mg). melting point: 238-239°C/decomposition. ¹H-NMR(400MHz,CDCl₃)δ: 2.41(3H,s), 2.65-2.67(4H,m), 3.62-3.50(4H,m), 5.92(1H,s), 7.17-7.29(3H,m), 8.02(1H,d,J=8Hz), 10.87(1H,brS).

### Example 21

In the same manner as in Example 1, the reaction was carried out using 3,5-dichloro-2H-isoquinolin-1-one (1.0 g) and 4-methylpiperazine (2 g) to give 5-chloro-3-(4-methylpiperazin-1-yl)-2H-isoquinolin-1-one (618 mg). melting point: 230-231°C/decomposition. ¹H-NMR(400MHz,CDCl₃)δ: 2.42(3H,s), 2.66-2.68(4H,m), 3.36-3.50(4H,m), 6.07(1H,s), 7.18(1H,t,J=8Hz), 7.63(1H,d,J=8Hz), 8.16(1H,d,J=8Hz), 11.12(1H,brS).

### Example 22

In the same manner as in Example 1, the reaction was carried out using 5-bromo-3-chloro-2H-isoquinolin-1-one (1.0 g) and 4-methylpiperazine (2 g) to give 5-bromo-3-(4-methylpiperazin-1-yl)-2H-isoquinolin-1-one. melting point: 240-241°C/decomposition. ¹H-NMR (400MHz, CDCl₃)δ: 2.42(3H,s), 2.66-2.80(4H,m), 3.37-3.39(4H,m), 6.05(1H,s), 7.12(1H,dd,J=8,4Hz), 7.82(1H,d,J=8Hz), 8.20(1H,d,J=8Hz), 11.24(1H,brS).

### Example 23

In the same manner as in Example 1, the reaction was carried out using 3,8-dichloro-2H-isoquinolin-1-one (1.0 g) and 4-methylpiperazine (2 g) to give 8-chloro-3-(4-methylpiperazin-1-yl)-2H-isoquinolin-1-one (0.56 g). ¹H-NMR(400MHz,CDCl₃)δ: 2.40(3H,s), 2.63-2.66(4H,m), 3.35-3.42 (4H,m) , 5.70(1H,s), 7.21(1H,dd,J=1Hz,7Hz), 7.26(1H,dd,J=1Hz,8Hz), 7.36(1H,t,J=8Hz), 11.14(1H,brS).

### Example 24

In the same manner as in Example 1, the reaction was carried out using 3-chloro-7-methyl-2H-isoquinolin-1-one (1.0 g) and 4-methylpiperazine (2 g) to give 7-methyl-3-(4-methylpiperazin-1-yl)-2H-isoquinolin-1-one (0.23 g). ¹H-NMR(400MHz,CDCl₃)δ: 2.45 (3H,s) , 2.49(3H,s), 2.68-2.92(4H,m), 3.30-3.45(4H,m), 5.77(1H,s), 7.32(1H,d,J=8Hz), 7.40(1H,d,J=8Hz), 8.02(1H,s), 10.79(1H,brS).

### Example 25

In the same manner as in Example 1, the reaction was carried out using 7-bromo-3-chloro-2H-isoquinolin-1-one (1.0 g) and 4-methylpiperazine (2 g) to give 7-bromo-3-(4-methylpiperazin-1-yl)-2H-isoquinolin-1-one (0.66 g). ¹H-NMR(400MHz,DMSO-d₆)δ: 2.21 (3H,s) , 2.39-2.48(4H,m), 3.06-3.15(4H,m), 5.80(1H,s), 7.41(1H,d,J=8Hz), 7.65(1H,d,J=9Hz), 8.06(1H,s), 11.29(1H,brS).

### Example 26

In the same manner as in Example 18 using 4-dimethylaminopiperidine instead of 4-methylpiperazine, 3-(4-dimethylaminopiperidin-1-yl)-5-methoxy-2H-isoquinolin-1-one is obtained.

### Example 27

In the same manner as in Example 19 using 5-methoxy-3-(4-dimethylaminopiperidin-1-yl)-2H-isoquinolin-1-one instead of 5-methoxy-3-(4-methylpiperazin-1-yl)-2H-isoquinolin-1-one, 5-hydroxy-3-(4-dimethylaminopiperidin-1-yl)-2H-isoquinolin-1-one is obtained.

### Example 28

In the same manner as in Example 1, the reaction was carried out using 3-chloro-5-fluoro-2H-isoguinolin-1-one (1.0 g) and 4-dimethylaminopiperidine (2 g) to give 5-fluoro-3-(4-dimethylaminopiperidin-1-yl)-2H-isoquinolin-1-one (0.24 g). ¹H-NMR(400MHz,CDCl₃)δ: 1.71-1.80(2H,m), 2.00-2.05(2H,m), 2.37(6H,s), 2.35-2.40(1H,m), 2.80-2.90(2H,m), 3.80-3.86(2H,m), 5.91(1H,s), 7.13-7.26(2H,m), 8.02(1H,d,J=8Hz), 10.93(1H,brS).

### Example 29

In the same manner as in Example 1, the reaction was carried out using 3,5-dichloro-2H-isoquinolin-1-one (1.0 g) and 4-dimethylaminopiperidine (2 g) to give 5-chloro-3-(4-dimethylaminopiperidin-1-yl)-2H-isoquinolin-1-one (249 mg). melting point: 210-211°C/decomposition. ¹H-NMR(400MHz,CDCl₃)δ: 1.74-1.80(2H,m), 2.01-2.04(2H,m), 2.29-2.31(7H,m), 2.85-2.91(2H,m), 3.81-3.85(2H,m), 6.08(1H,s), 7.17(1H,dd,J=8.4Hz), 7.62(1H,d,J=8Hz), 8.18(1H,d,J=8Hz), 10.63(1H,brS).

### Example 30

In the same manner as in Example 1, the reaction was carried out using 3,6-dichloro-2H-isoquinolin-1-one (1.0 g) and 1-methylpiperazine (2 g) to give 6-chloro-3-(4-methylpiperazin-1-yl)-2H-isoquinolin-1-one (0.25 g). ¹H-NMR(400MHz,CDCl₃)δ: 2.43(3H,s), 2.60-2.65(4H,m), 3.30-3.34(4H,m), 5.68(1H,s), 7.21(1H,dd,J=2Hz,9Hz), 7.38(1H,d,J=2Hz), 8.15(1H,d,J=9Hz), 11.06(1H,brS).

### Example 31

In the same manner as in Example 1, the reaction was carried out using 7-bromo-3-chloro-2H-isoquinolin-1-one (1.0 g) and 4-dimethylaminopiperidine (2 g) to give 7-bromo-3-(4-dimethylaminopiperidin-1-yl)-2H-isoquinolin-1-one (145 mg). melting point: 227-228°C/decomposition. ¹H-NMR(400MHz,CDCl₃)δ: 1.76-1.78(2H,m), 12.04-2.06(2H,m), 2.42-2.44(7H,m), 2.80-2.85(2H,m), 3.74-3.78(2H,m), 5.70(1H,s), 7.24(1H,s), 7.60(1H,dd,J=8,4Hz), 10.4-10.6(1H,brS).

### Example 32

In the same manner as in Example 1, the reaction was carried out using 5-bromo-3-chloro-2H-isoquinolin-1-one (1.0 g) and 4-dimethylaminopiperidine (2 g) to give 5-bromo-3-(4-dimethylaminopiperidin-1-yl)-2H-isoquinolin-1-one (373 mg). melting point: 214-215°C/decomposition. ¹H-NMR(400MHz,CDCl₃) δ: 1.72-1.82(2H,m), 2.01-2.04(2H,m), 2.31-2.32(7H,m), 2.54-2.91(2H,m), 3.96-3.89(2H,m), 6.07(1H,s), 7.08(1H,t,J=8Hz), 7.89(1H,d,J=8Hz), 8.22(1H,d,J=8Hz), 11.31(1H,brS).

### Example 33

In the same manner as in Example 1, the reaction was carried out using 3-chloro-5-fluoro-2H-isoquinolin-1-one (1.0 g) and 4-(2-hydroxyethyl)piperazine (2 g) to give 5-fluoro-3-(4-(2-hydroxyethyl)piperazin-1-yl)-2H-isoquinolin-1-one (161 mg). melting point: 207-208°C/decomposition. ¹H-NMR(400MHz,CDCl₃)δ: 2.64-2.66(3H,m), 2.72-2.74(4H,m), 3.29-3.31(4H,m), 3.67-3.69(2H,m), 5.90(1H,s), 7.15-7.27 (2H,m), 7.98(1H,d,J=8Hz), 10.54(1H,brS).

### Example 34

In the same manner as in Example 1, the reaction was carried out using 3-chloro-6-methyl-2H-isoquinolin-1-one (1.0 g) and 1-methylpiperazine (2 g) to give 6-methyl-3-(4-methylpiperazin-1-yl)-2H-isoquinolin-1-one (124 mg). melting point: 242-243°C/decomposition. ¹H-NMR(400MHz,CDCl₃)δ: 2.37(3H,s), 2.41(3H,s), 2.62-2.73(4H,m), 2.20-2.24(4H,m), 5.67(1H,s), 7.08(1H,d,J=8Hz), 7.16(1H,s), 8.10(1H,d,J=8Hz), 10.30(1H,brS).

### Example 35

In the same manner as in Example 1, the reaction was carried out using 3-chloro-6-methyl-2H-isoquinolin-1-one (1.0 g) and 1-(2-hydroxyethyl)piperazine (2 g) to give 3-(4-(2-hydroxyethyl)piperazin-1-yl)-6-methyl-2H-isoquinolin-1-one (138 mg). melting point: 236-237°C/decomposition. ¹H-NMR(400MHz,CDCl₃) δ: 2.43(3H,s), 2.66-2.68(3H,m), 2.75-2.77(4H,m), 3.27-3.29(4H,m), 3.68-3.70(2H,m), 5.69(1H,m), 7.10(1H,d,J=8Hz), 7.19(1H,s), 8.11(1H,d,J=8Hz), 11.12(1H,brS).

### Example 36

In the same manner as in Example 1, the reaction was carried out using 3-chloro-8-methyl-2H-isoquinolin-1-one (1.0 g) and 1-methylpiperazine (2 g) to give 8-methyl-3-(4-methylpiperazin-1-yl)-2H-isoquinolin-1-one (292 mg). melting point: 224-225°C/decomposition. ¹H-NMR(400MHz,CDCl₃)δ: 2:35 (3H,s) , 2.58-2.60(4H,m), 2.86(3H,s), 3.26-3.27(4H,m), 5.67(1H,s), 6.97(1H,d,J=4Hz), 7.18(1H,d,J=8Hz), 7.33(1H,d,J=8Hz), 10.78(1H,brS).

### Example 37

In the same manner as in Example 1, the reaction was carried out using 7-bromo-3-chloro-2H-isoquinolin-1-one (1.0 g) and 1-(2-hydroxyethyl)piperazine (2 g) to give 7-bromo-3-(4-(2-hydroxyethyl)piperazin-1-yl)-2H-isoquinolin-1-one (0.46 g). ¹H-NMR(400MHz,CDCl₃)δ: 2.68(2H,t,J=5Hz), 2.68-2.78(4H,m), 3.28-3.32(4H,m), 3.70(2H,t,J=5Hz), 5.71(1H,s), 7.27(1H,d,J=7Hz), 7.61(1H,dd,J=2Hz,9Hz), 8.33(1H,d,J=2Hz), 10.75(1H, brS).

### Example 38

In the same manner as in Example 1, the reaction was carried out using 3-chloro-5-nitro-2H-isoquinolin-1-one (1.0 g) and 1-methylpiperazine (2 mL) to give 3-(4-methylpiperazin-1-yl)-5-nitro-2H-isoquinolin-1-one (0.78 g). ¹H-NMR(DMSO-d₆)δ: 2.21(3H,s), 2.35-2.45(4H,m), 3.22-3.32(4H,m), 6.37(1H,s), 7.26(1H,t,J=8Hz), 8.34-8.37(2H,m), 11.58(1H,brs).

### Example 39

3-(4-Methylpiperazin-1-yl)-5-nitro-2H-isoquinolin-1-one (1.0 g) was dissolved in methanol (10 mL), and activated carbon (0.3 g) and ferric chloride (0.05 g) were added. Hydrated hydrazine (0.5 mL) was added dropwise to the mixture with heating under reflux. The mixture was heated under reflux for 2 hr. After the completion of the reaction, the reaction solution was filtered. The filtrate was concentrated, and the obtained residue was dissolved in chloroform and washed with an aqueous potassium carbonate solution. The organic layer was dried over potassium carbonate and concentrated. The obtained residue was purified by silica gel column chromatography, and a chloroform:methanol=10:1 effluent fraction was concentrated. Isopropyl ether was added to the obtained residue, and the precipitated crystals were collected by filtration to give 5-amino-3-(4-methylpiperazin-1-yl)-2H-isoquinolin-1-one 1 water adduct (0.4 g). ¹H-NMR(DMSO-d₆)δ: 2.20(3H,s), 2.35-2.45(4H,m), 3.02-3.12(4H,m), 5.40(2H,s), 5.80(1H,s), 6.74(1H,d,J=8Hz), 6.91(1H,t,J=8Hz), 7.26(1H,d,J=8Hz), 10.97(1H,brs).

### Example 40

Sandmeyer reaction using 5-amino-3-(4-methylpiperazin-1-yl)-2H-isoquinolin-1-one (1.0 g) gives 5-cyano-3-(4-methylpiperazin-1-yl)-2H-isoquinolin-1-one.

### Example 41

In the same manner as in Example 1, the reaction was carried out using 3-chloro-8-methyl-2H-isoquinolin-1-one (1.0 g) and 4-(2-hydroxyethyl)piperazine (2 g) to give 3-[4-(2-hydroxyethyl)piperazin-1-yl]-8-methyl-2H-isoquinolin-1-one (365 mg). melting point: 209-210°C/decomposition. ¹H-NMR(400MHz,CDCl₃)δ: 2.60-2.63 (3H,m) , 2.68-2.71(4H,m), 2.85(3H,s), 3.27-3.28(4H,m), 3.64-3.66(2H,m), 5.67(1H,s), 6.97(1H,d,J=8Hz), 7.19(1H,d,J=8Hz), 7.34(1H,d,J=8Hz), 10.89(1H,brS).

### Example 42

In the same manner as in Example 1, the reaction is carried out using 3-chloro-5-fluoromethyl-2H-isoquinolin-1-one (1.0 g) and 1-methylpiperazine (2 g) to give 3- (4-methylpiperazin-1-yl)-5-trifluoromethyl-2H-isoquinolin-1-one.

### Example 43

In the same manner as in Example 1, the reaction was carried out using 3-chloro-7-methyl-2H-isoquinolin-1-one (1.0 g) and 1-(2-hydroxyethyl)piperazine (2 g) to give 3-[4-(2-hydroxyethyl)piperazin-1-yl]-7-methyl-1H-isoquinolin-1-one (235 mg). ¹H-NMR(400MHz,CDCl₃) δ: 2.44 (3H,s) , 2.67(2H,t,J=5Hz), 2.68-2.78(4H,m), 3.25-3.29(4H,m), 3.60-3.68(2H,m), 5.75(1H,s), 7.31(1H,d,J=8Hz), 7.38(1H,dd,J=2Hz,8Hz), 8.03(1H,s), 10.86(1H,brS).

### Example 44

In the same manner as in Example 1, the reaction is carried out using 3-chloro-5-methylthio-2H-isoquinolin-1-one (1.0 g) and 1-methylpiperazine (2 g) to give 3-(4-methylpiperazin-1-yl)-5-methylthio-2H-isoquinolin-1-one.

### Example 45

In the same manner as in Example 1, the reaction is carried out using 3-chloro-5-dimethylamino-2H-isoquinolin-1-one (1.0 g) and 1-methylpiperazine (2 g) to give 5-dimethylamino-3-(4-methylpiperazin-1-yl)-2H-isoquinolin-1-one.

### Example 46

In the same manner as in Example 1, the reaction was carried out using 3-chloro-5-nitro-2H-isoquinolin-1-one (1.0 g) and 4-dimethylaminopiperidine (2 g) to give 3-(4-dimethylaminopiperidin-1-yl)-5-nitro-2H-isoquinolin-1-one.

### Example 47

3-(4-Dimethylaminopiperidin-1-yl)-5-nitro-2H-isoquinolin-1-one (1.0 g) is reduced using Fe to give 5-amino-3-(4-dimethylaminopiperidin-1-yl)-2H-isoquinolin-1-one.

### Example 48

In the same manner as in Example 1, the reaction is carried out using 3-chloro-5-trifluoromethyl-2H-isoquinolin-1-one (1.0 g) and 4-dimethylaminopiperidine (2 g) to give 3-(4-dimethylaminopiperidin-1-yl)-5-trifluoromethyl-2H-isoquinolin-1-one.

### Example 49

In the same manner as in Example 1, the reaction is carried out using 3-chloro-5-methylthio-2H-isoquinolin-1-one (1.0 g) and 4-dimethylaminopiperidine (2 g) to give 3-(4-dimethylaminopiperidin-1-yl)-5-methylthio-2H-isoquinolin-1-one.

### Example 50

5-Amino-3-(4-dimethylaminopiperidin-1-yl)-2H-isoquinolin-1-one (1.0 g) to be obtained in Example 47 is subjected to Sandmeyer reaction to give 5-cyano-3-(4-dimethylaminopiperidin-1-yl)-2H-isoquinolin-1-one.

### Example 51

In the same manner as in Example 1, the reaction is carried out using 3-chloro-5,7-dimethyl-2H-isoquinolin-1-one (1.0 g) and 1-methylpiperazine (2 g) to give 5,7-dimethyl-3-(4-methylpiperazin-1-yl)-2H-isoquinolin-1-one.

### Example 52

In the same manner as in Example 1, the reaction is carried out using 3,5,7-trichloro-2H-isoquinolin-1-one (1.0 g) and 1-methylpiperazine (2 g) to give 5,7-dichloro-3-(4-methylpiperazin-1-yl)-2H-isoquinolin-1-one.

### Example 53

In the same manner as in Example 1, the reaction is carried out using 5,7-dibromo-3-chloro-2H-isoquinolin-1-one (1.0 g) and 1-methylpiperazine (2 g) to give 5,7-dibromo-3-(4-methylpiperazin-1-yl)-2H-isoquinolin-1-one.

### Example 54

In the same manner as in Example 1, the reaction is carried out using 3-chloro-5,7-difluoro-2H-isoquinolin-1-one (1.0 g) and 1-methylpiperazine (2 g) to give 5,7-difluoro-3-(4-methylpiperazin-1-yl)-2H-isoquinolin-1-one.

### Example 55

In the same manner as in Example 1, the reaction is carried out using 3,5-dichloro-7-fluoro-2H-isoquinolin-1-one (1.0 g) and 1-methylpiperazine (2 g) to give 5-chloro-7-fluoro-3-(4-methylpiperazin-1-yl)-2H-isoquinolin-1-one.

### Example 56

In the same manner as in Example 1, the reaction is carried out using 3-chloro-6,7-dihydroxy-2H-isoquinolin-1-one (1.0 g) and 1-methylpiperazine (2 g) to give 6,7-dihydroxy-3-(4-methylpiperazin-1-yl)-2H-isoquinolin-1-one.

### Example 57

In the same manner as in Example 1, the reaction is carried out using 3,5,7-trichloro-2H-isoquinolin-1-one (1.0 g) and 4-dimethylaminopiperidine (2 g) to give 5,7-dichloro-3-(4-dimethylaminopiperidin-1-yl)-2H-isoquinolin-1-one.

### Example 58

In the same manner as in Example 1, the reaction is carried out using 5,7-dibromo-3-chloro-2H-isoquinolin-1-one (1.0 g) and 4-dimethylaminopiperidine (2 g) to give 5,7-dibromo-3-(4-dimethylaminopiperidin-1-yl)-2H-isoquinolin-1-one.

### Example 59

In the same manner as in Example 1, the reaction is carried out using 5-bromo-3,7-dichloro-2H-isoquinolin-1-one (1.0 g) and 4-dimethylaminopiperidine (2 g) to give 5-bromo-7-chloro-3-(4-dimethylaminopiperidin-1-yl)-2H-isoquinolin-1-one.

### Example 60

In the same manner as in Example 1, the reaction is carried out using 3-chloro-6,7-dihydroxy-2H-isoquinolin-1-one (1.0 g) and 4-dimethylaminopiperidine (2 g) to give 6,7-dihydroxy-3-(4-dimethylaminopiperidin-1-yl)-2H-isoquinolin-1-one.

### Example 61

In the same manner as in Example 1, the reaction is carried out using 3-chloro-2H-isoquinolin-1-one (1.0 g) and 4-(4-morpholino)-piperidine (2 g) to give 3-[4-(4-morpholino)piperidin-1-yl]-2H-isoquinolin-1-one.

### Example 62

In the same manner as in Example 1, the reaction was carried out using 3-chloro-2H-isoquinolin-1-one (1.0 g) and 4-[2-(piperidin-1-yl)ethyl]piperazine (2 g) to give 3-{4-[2-(piperidin-1-yl)ethyl]piperazin-1-yl}-2H-isoquinolin-1-one (0.42 g). ¹H-NMR(CDCl₃) δ: 1.45-1.60(6H,m), 2.40-2.75(12H,m), 3.26-3.29(4H,m), 5.74(1H,s), 7.25-7.29(1H,m), 7.38(1H,d,J=8Hz), 7.52-7.56(1H,m), 8.22(1H,d,J=8Hz), 11.19(1H,brS).

### Example 63

In the same manner as in Example 1, the reaction was carried out using 3-chloro-2H-isoquinolin-1-one (1.0 g) and 4-[3-(piperidin-1-yl)propyl]piperazine (2 g) to give 3-{4-[3-(piperidin-1-yl)propyl]piperazin-1-yl}-2H-isoquinolin-1-one (0.26 g). ¹H-NMR(CDCl₃)δ: 1.39-1.95(8H,m), 2.41-2.68(12H,m), 3.25-3.29(4H,m), 5.75(1H,s), 7.27-7.30(1H,m), 7.39(1H,d,J=8Hz), 7.55(1H,d,J=8Hz), 8.24(1H,d,J=8Hz), 10.05(1H,brS).

### Example 64

In the same manner as in Example 1, the reaction is carried out using 3-chloro-2H-isoquinolin-1-one (1.0 g) and 4-[4-(4-morpholino)butyl]piperazine (2 g) to give 3-{4-[4-(4-morpholino)butyl]piperazin-1-yl}-2H-isoquinolin-1-one.

### Example 65

In the same manner as in Example 1, the reaction was carried out using 3-chloro-2H-isoquinolin-1-one (1.0 g) and 4-[4-(piperidin-2-yl)butyl]piperazine (2 g) to give 3-{4-[4-(piperidin-1-yl)butyl]piperazin-1-yl}-2H-isoquinolin-1-one (0.28 g). ¹H-NMR(400MHz,CDCl₃)δ: 1.40-1.68(10H,m), 2.28-2.50(8H,m), 2.60-2.68(4H,m), 3.26-3.32(4H,m), 5.76(1H,s), 7.28(1H,t,J=8Hz), 7.40(1H,d,J=8Hz), 7.53-7.57(1H,m), 8.24(1H,d,J=8Hz), 10.99(1H,brS).

### Example 66

In the same manner as in Example 1, the reaction was carried out using 3-chloro-2H-isoquinolin-1-one (1.0 g) and 4-[5-(piperidin-1-yl)pentyl]piperazine (2 g) to give 3-{4-[5-(piperidin-1-yl)pentyl]piperazin-1-yl}-2H-isoquinolin-1-one (0.32 g). ¹H-NMR(400MHz,CDCl₃)δ: 1.35-1.75(12H,m), 2.42-2.70(12H,m), 3.24-3.29(4H,m), 5.75(1H,s), 7.26-7.30(1H,m), 7.39(1H,d,J=8Hz), 7.53-7.57(1H,m), 8.24(1H,d,J=8Hz), 10.51 (1H,brS).

### Example 67

In the same manner as in Example 1, the reaction is carried out using 3-chloro-2H-isoquinolin-1-one (1.0 g) and 4-(4-(4-methylpiperazin-1-yl)butyl]piperazine (2 g) to give 3-(4-(4-(4-methylpiperazin-1-yl)butyl)piperazin-1-yl)-2H-isoquinolin-1-one.

### Example 69

In the same manner as in Example 1, the reaction was carried out using 3-chloro-5-methyl-2H-isoquinolin-1-one (1.0 g) and 4-(4-morpholino)-piperidine (2 g) to give 5-methyl-3-[4-(4-morpholino)piperidin-1-yl]-2H-isoquinolin-1-one (0.15 g). ¹H-NMR(CDCl₃) d: 1.68-1.80(2H,m), 1.95-2.05(2H,m), 2.35-2.43(1H,m), 2.46(3H,s), 2.56-2.63(4H,m), 2.76-2.85(2H,m), 3.70-3.80(6H,m), 5.78(1H,s), 7.16(1H,t,J=8Hz), 7.39(1H,d,J=7Hz), 8.13(1H,d,J=8Hz), 11.64(1H,brS).

### Example 70

In the same manner as in Example 1, the reaction was carried out using 3-chloro-5-methyl-2H-isoquinolin-1-one (1.0 g) and 4-[2-(piperidin-1-yl)ethyl]piperazine (2 g) to give 5-methyl-3-{4-[2-(piperidin-1-yl)ethyl]piperazin-1-yl}-2H-isoquinolin-1-one (0.12 g). ¹H-NMR(CDCl₃) d: 1.45-1.65(6H,m), 2.46(3H,s), 2.46-2.72(12H,m), 3.24-3.30(4H,m), 5.76(1H,s), 7.18(1H,t,J=8Hz), 7.40(1H,d,J=7Hz), 8.12(1H,d,J=8Hz), 10.55-10.70(1H,m).

### Example 71

In the same manner as in Example 1, the reaction was carried out using 3-chloro-5-methyl-2H-isoquinolin-1-one (1.0 g) and 4-[3-(piperidin-1-yl)propyl]piperazine (2 g) to give 5-methyl-3-{4-[3-(piperidin-1-yl)propyl]piperazin-1-yl}-2H-isoquinolin-1-one (0.56 g). ¹H-NMR(CDCl₃) d: 1.42-1.85(8H,m), 2.38-2.50(8H,m), 2.47(3H,s), 2.68-2.71(4H,m), 3.30-3.33(4H,m), 5.77(1H,s), 7.19(1H,t,J=7Hz), 7.41(1H,d,J=7Hz), 8.13(1H,d,J=7Hz), 11.13(1H,brS).

### Example 72

In the same manner as in Example 1, the reaction was carried out using 3-chloro-5-methyl-2H-isoquinolin-1-one (1.0 g) and 4-[5-(piperidin-1-yl)pentyl]piperazine (2 g) to give 5-methyl-3-{4-[5-(piperidin-1-yl)pentyl]piperazin-1-yl}-2H-isoquinolin-1-one (0.35 g). ¹H-NMR(CDCl₃) d: 1.38-1.72(12H,m), 2.32-2.51(8H,m), 2.47(3H,s), 2.68-2.71(4H,m), 3.30-3.33(4H,m), 5.77(1H,s), 7.18(1H,t,J=7Hz), 7.40(1H,d,J=7Hz), 8.13(1H,d,J=8Hz), 10.97(1H,brS).

### Example 73

In the same manner as in Example 1, the reaction was carried out using 3-chloro-5-methyl-2H-isoquinolin-1-one (1.0 g) and 4-[4-(piperidin-1-yl)butyl]piperazine (2 g) to give 5-methyl-3-{4-[4-(piperidin-1-yl)butyl]piperazin-1-yl}-2H-isoquinolin-1-one (0.67 g). ¹H-NMR(400MHz,CDCl₃)δ: 1.40-1.63(10H,m), 2.26-2.50(8H,m), 2.48(3H,s), 2.60-2.65(4H,m), 3.22-3.30(4H,m), 5.77(1H,s), 7.19(1H,t,J=7Hz), 7.41(1H,d,J=7Hz), 8.14(1H,d,J=8Hz), 10.60-10.98(1H,m).

### Example 74

In the same manner as in Example 1, the reaction was carried out using 3-chloro-5-methyl-2H-isoquinolin-1-one (1.0 g) and 4-[4-(4-morpholino)butyl]piperazine (2 g) to give 5-methyl-3-{4-[4-(4-morpholino)butyl]piperazin-1-yl}-2H-isoquinolin-1-one (625 mg). melting point: 177-178°C/decomposition. ¹H-NMR(400MHz,CDCl₃)δ: 1.76-1.78(8H,brS), 2.46(3H,s), 2.48-2.50(4H,brS), 2.69-2.71(4H,brS), 3.29-3.31(4H,brS), 3.74-3.76(4H,brS), 5.77(1H,s), 7.18(1H,t,J=8Hz), 7.40(1H,d,J=8Hz), 8.12(1H,d,J=8Hz).

### Example 75

In the same manner as in Example 1, the reaction was carried out using 3-chloro-5-methyl-2H-isoquinolin-1-one (1.0 g) and 4-[4-(4-methylpiperazin-1-yl)butyl]piperazine (2 g) to give 5-methyl-3-(4-(4-(4-methylpiperazin-1-yl)butyl)piperazin-1-yl)-2H-isoquinolin-1-one (59 mg). melting point: 202-203°C/decomposition. ¹H-NMR(400MHz,CDCl₃)δ: 1.60-1.62(8H,brS), 2.18(3H,s), 2.37-2.39(8Hz,brS), 2.47(3H,s), 2.64-2.66(4H,brS), 3.24-3.26(4H,brS), 5.77(1H,s), 7.18(1H,t,J=8Hz), 7.41(1H,d,J=8Hz), 8.13(1H,d,J=8Hz).

### Example 76

In the same manner as in Example 1, the reaction was carried out using 7-bromo-3-chloro-2H-isoquinolin-1-one (1.0 g) and 4-[4-(piperidin-1-yl)butyl]piperazine (2 g) to give 7-bromo-3-{4-[4-(piperidin-1-yl)butyl]piperazin-1-yl}-2H-isoquinolin-1-one (175 mg). melting point: 211-212°C/decomposition. ¹H-NMR(400MHz,CDCl₃)δ: 1.20-1.80(12H,m), 2.35-2.45(6H,m), 2.60-2.62(4H,m), 3.21-3.31(4H,m), 5.66(1H,s), 7.21-7.24(1H,m), 7.58(1H,dd,J=8,4Hz), 8.32(1H,d,J=4Hz), 9.85(1H,brS).

### Example 77

In the same manner as in Example 1, the reaction was carried out using 3,5-dichloro-2H-isoquinolin-1-one (1.0 g) and 4-[4-(piperidin-1-yl)butyl]piperazine (2 g) to give 5-chloro-3-{4-[4-(piperidin-1-yl)butyl]piperazin-1-yl}-2H-isoquinolin-1-one (166 mg). melting point: 172-173°C/decomposition. ¹H-NMR(400MHz,CDCl₃)δ: 1.57-1.63(12H,m), 2.44-2.47(6H,m), 2.62-2.65(4H,m), 3.28-3.29(4H,m), 6.04(1H,s), 7.17(1H,t,J=4Hz), 7.62(1H,d,J=8Hz), 8.15(1H,d,J=8Hz), 9.66(1H,brS).

### Example 78

In the same manner as in Example 1, the reaction was carried out using 5-bromo-3-chloro-2H-isoquinolin-1-one (1.0 g) and 4-[4-(piperidin-1-yl)butyl]piperazine (2 g) to give 5-bromo-3-{4-[4-(piperidin-1-yl)butyl]piperazin-1-yl}-2H-isoquinolin-1-one (39 mg). melting point: 165-166°C/decomposition. ¹H-NMR(400MHz,CDCl₃)δ: 1.20-1.25(8Hz,m), 2.38-2.63(12H,m), 3.27-3.29(4H,m), 4.02-4.06(2H,m), 6.01(1H,s), 7.08(1H,t,J=8Hz), 7.78(1H,d,J=8Hz), 8.17(1H,d,J=8Hz), 9.98(1H,brS).

### Example 80

In the same manner as in Example 1, the reaction was carried out using 3,5-dichloro-2H-isoquinolin-1-one (1.0 g) and 4-[4-(4-morpholino)butyl]piperazine (2 g) to give 5-chloro-3-{4-[4-(4-morpholino)butyl]piperazin-1-yl}-2H-isoquinolin-1-one (603 mg). melting point: 195-196°C/decomposition. ¹H-NMR(400MHz,CDCl₃)δ: 1.65-1.73(4H,m), 2.47-2.55(8H,m), 2.75-2.78(4H,m), 3.43-3.45(4H,m), 3.81-3.84(4H,m), 6.14(1H,s), 7.26(1H,t,J=8Hz), 7.71(1H,d,J=8Hz), 8.24(1H,d,J=8Hz), 10.99(1H,brS).

### Example 81

Diethylamine (1.4 ml) was dissolved in tetrahydrofuran (26 ml), and a solution (1.59 M, 42 ml) of n-butyllithium in hexane was added dropwise at -78°C. The mixture was stirred at 0°C for 30 min and cooled to -78°C. A solution (26 ml) of 2-methylbenzoic acid (4.1 g) in tetrahydrofuran was added dropwise to the reaction solution. The mixture was stirred at 0°C for 30 min and cooled to -78°C. A solution (26 ml) of 1-benzyloxycarbonyl-4-cyanopiperidine (7.2 g) in tetrahydrofuran was added dropwise to the reaction solution, and the mixture was stirred at -78°C for one day. After the completion of the reaction, water was added to the reaction solution, and the mixture was extracted with ethyl acetate. The extract was dried over magnesium sulfate, and the solvent was concentrated. The obtained residue was subjected to silica gel column chromatography. A chloroform:methanol=30:1 effluent fraction was concentrated, and diisopropyl ether was added to the precipitated crystals. The crystals were collected by filtration. Recrystallization from isopropanol gave 3-(1-benzyloxycarbonylpiperidin-4-yl)-2H-isoquinolin-1-one (1.2 g) as white crystals. melting point: 179-181°C. 3-(1-Benzyloxycarbonylpiperidin-4-yl)-2H-isoquinolin-1-one (1.2 g) was suspended in chloroform (10 ml), and a solution (5 ml) of hydrobromic acid in acetic acid was added dropwise. After the completion of the reaction, the solvent was concentrated, and acetone was added to the obtained residue. The precipitated crystals were collected by filtration to give 3-(piperidin-4-yl)-2H-isoquinolin-1-one hydrobromide (1.1 g). melting point: >270°C.

### Example 82

3-(Piperidin-4-yl)-2H-isoquinolin-1-one hydrobromide (1.1 g) obtained in Example 81 was suspended in acetonitrile (20 ml), and 37% formaldehyde (2.8 ml) was added. Thereto was added sodium cyanoborohydride (0.66 g) under ice-cooling. Acetic acid (0.36 ml) was added dropwise, and the mixture was reacted overnight. After the completion of the reaction, the solvent was concentrated, and an aqueous potassium carbonate solution was added to the obtained residue. The mixture was extracted with chloroform. The extract was dried over magnesium sulfate, and the solvent was concentrated. The precipitated crystals were recrystallized from isopropyl alcohol-methanol to give 3-(1-methylpiperidin-4-yl)-2H-isoquinolin-1-one 1/5 water adduct (0.18 g). ¹H-NMR(400MHz,DMSO-d₆)δ: 1.60-1.69(2H,m), 1.86-1.94(4H,m), 2.19(3H,s), 2.34-2.41(1H,m), 2.82-2.91(2H,m), 6.36(1H,s), 7.41(1H,t,J=7Hz), 7.59(1H,d,J=7Hz), 7.63-7.67(1H,m), 8.12(1H,d,J=8Hz), 11.21(1H,brS).

### Different synthesis method

2,2,6,6-Tetramethylpyridine (9.0 mL) was dissolved in tetrahydrofuran (80 mL), and n-butyllithium (1.6 mol/L, 40 mL) was added dropwise under ice-cooling. The mixture was stirred under ice-cooling for 30 min and cooled to -78°C. A solution (80 mL) of N,N-diethyl-2-methylbenzamide (10 g) in tetrahydrofuran was added dropwise to the reaction solution, and the mixture was stirred at 0°C for 1 hr. The reaction solution was cooled to -78°C, and a solution (80 mL) of 4-cyano-1-methylpiperidine (5.0 g) in tetrahydrofuran was added dropwise. The reaction solution was warmed to room temperature as it was. After the completion of the reaction, an aqueous potassium carbonate solution was added to the reaction solution, and the mixture was extracted with ethyl acetate. The organic layer was washed with saturated brine and then dried over magnesium sulfate. The solvent was concentrated, and the precipitated crystals were washed with diisopropyl ether to give crude crystals of 3-(1-methylpiperidin-4-yl)-2H-isoquinolin-1-one. The crude crystals were dissolved in 1 mol/L aqueous hydrochloric acid and neutralized with an aqueous potassium carbonate solution. The precipitated crystals were collected by filtration to give 3-(1-methylpiperidin-4-yl)-2H-isoquinolin-1-one (5.3 g). melting point: 255-257°C. ¹H-NMR(400MHz,DMSO-d₆)δ: 1.60-1.69(2H,m), 1.86-1.94(4H,m), 2.19(3H,s), 2.34-2.41(1H,m), 2.82-2.91(2H,m), 6.36(1H,s), 7.41(1H,t,J=7Hz), 7.59(1H,d,J=7Hz), 7.63-7.67(1H,m), 8.12(1H,d,J=8Hz), 11.21(1H,brS). MS(EI)242(M+).

### Example 83

1-Oxo-2H-isoquinoline-3-carboxylic acid (0.56 g), 4-methylpiperazine (0.33 g) and triethylamine (0.84 ml) were dissolved in dimethylformamide (10 ml) , and benzotriazol-1-yloxy-tris(dimethylamino)phosphonium hexafluorophosphate (1.45 g) was added at room temperature. After the completion of the reaction, water was added to the reaction solution. The mixture was extracted with ethyl acetate, then washed with an aqueous potassium carbonate solution and saturated brine and dried over potassium carbonate. The solvent was concentrated, and the obtained residue was subjected to silica gel column chromatography. A chloroform:methanol=10:1 effluent fraction was concentrated, and ethyl acetate and diisopropyl ether were added to the obtained residue. The precipitated crystals were collected by filtration to give 3-((4-methylpiperazin-1-yl)carbonyl)-2H-isoquinolin-1-one (0.29 g). melting point: 170-172°C

### Example 84

The reaction is carried out in the same manner as in Example 81 using 2-methylbenzoic acid and 3-dimethylaminopropionitrile to give 3-(2-(dimethylamino)ethyl)-2H-isoquinolin-1-one.

### Example 85

The reaction is carried out in the same manner as in Example 81 using 2-methylbenzoic acid and 4-dimethylaminobutyronitrile to give 3-(3-(dimethylamino)propyl)-2H-isoquinolin-1-one.

### Example 86

The reaction is carried out in the same manner as in Example 81 using 2-methylbenzoic acid and 3-cyano-1-azabicyclo[2.2.2]octane to give 3-(1-azabicyclo[2.2.2]octan-3-yl)-2H-isoquinolin-1-one.

### Example 87

The reaction is carried out in the same manner as in Example 81 using 2-methylbenzoic acid and 3-cyanomethyl-1-azabicyclo[2.2.2]octane to give 3-((1-azabicyclo[2.2.2]octan-3-yl)methyl)-2H-isoquinolin-1-one.

### Example 88

The reaction is carried out in the same manner as in Example 81 using 2-methylbenzoic acid and 3-cyano-8-methyl-8-azabicyclo[3.2.1]octane to give 3-(8-methyl-8-azabicyclo[3.2.1]octan-3-yl)-2H-isoquinolin-1-one.

### Example 89

The reaction is carried out in the same manner as in Example 81 using 2,3-dimethylbenzoic acid and 3-dimethylaminopropionitrile to give 5-methyl-3-(2-(dimethylamino)ethyl)-2H-isoquinolin-1-one.

### Example 90

4-(Dimethylamino)butyronitrile (4.4 g) was dissolved in tetrahydrofuran (40 mL), and a 1 mol/L borane/tetrahydrofuran solution (40 mL) was added dropwise under ice-cooling. After the completion of the reaction, water was added to the reaction solution. The mixture was extracted with ethyl acetate, washed with saturated brine and dried over magnesium sulfate. The solvent was concentrated, and the obtained residue was purified by silica gel column chromatography. A hexane:ethyl acetate=1:1 effluent fraction was concentrated to give (4-(dimethylamino)butyronitrile-N1)trihydroboron (2.8 g).

The reaction was carried out in the same manner as in Example 82 using N,N-dimethyl-2,3-dimethylbenzamide (3.9 g) and (4-(dimethylamino)butyronitrile-N1)trihydroboron to give (3-(3-(dimethylamino)propyl)-5-methyl-2H-isoquinolin-1-one-N1)trihydroboron (2.0 g). (3-(3-(Dimethylamino)propyl)-5-methyl-2H-isoquinolin-1-one-N1)trihydroboron (2.0 g) was dissolved in acetone (20 mL), and conc. hydrochloric acid (1 mL) was added at room temperature. After the completion of the reaction, the solvent was concentrated. Toluene was added to the obtained residue, and the mixture was extracted with water. Potassium carbonate was added to basify the aqueous layer, and the mixture was extracted with chloroform and dried over magnesium sulfate. The solvent was concentrated, and the obtained residue was purified by silica gel column chromatography (NH silica gel, Fuji Silysia Chemical Ltd.). A chloroform effluent fraction was concentrated, and diisopropyl ether was added to the obtained residue. The precipitated crystals were collected by filtration to give 3-(3-(dimethylamino)propyl)-5-methyl-2H-isoquinolin-1-one (1.5 g). melting point: 105-106°C.¹H-NMR(CDCl₃) δ: 1.79-1.89(2H,m), 2.34(6H,s), 2.39(2H,t,J=6Hz), 2.49(3H,s), 2.69(2H,t,J=6Hz), 6.34(1H,s), 7.31(1H,t,J=8Hz), 7.42(1H,dd,J=1Hz,7Hz), 8.23(1H,d,J=8Hz), 11.37(1H,brs).

### Example 91

The reaction is carried out in the same manner as in Example 81 using 2,3-dimethylbenzoic acid and 3-cyano-1-azabicyclo[2.2.2]octane to give 3-(1-azabicyclo[2.2.2]octan-3-yl)-5-methyl-2H-isoquinolin-1-one.

### Example 92

The reaction is carried out in the same manner as in Example 81 using 2,3-dimethylbenzoic acid and 3-cyanomethyl-1-azabicyclo[2.2.2]octane to give 3-((1-azabicyclo[2.2.2]octan-3-yl)methyl)-5-methyl-2H-isoquinolin-1-one.

### Example 93

The reaction was carried out in the same manner as in Example 82 using N,N-dimethyl-2,3-dimethylbenzamide (7.6 g) and 4-cyano-1-t-butoxycarbonylpiperidine (9.0 g) to give 3-(1-t-butoxycarbonylpiperidin-4-yl)-5-methyl-2H-isoquinolin-1-one (6.4 g). ¹H-NMR(CDCl₃)δ: 1.50(9H,s), 1.62-1.82(2H,m), 1.96-2.07(2H,m), 2.53(3H,s), 2.63-2.95(3H,m), 6.42(1H,s), 7.34(1H,t,J=8Hz), 7.48(1H,d,J=7Hz), 8.24(1H,d,J=8Hz), 11.30(1H,brs).

3-(1-t-Butoxycarbonylpiperidin-4-yl)-5-methyl-2H-isoquinolin-1-one (9.4 g) was suspended in acetone (100 mL), and conc. hydrochloric acid (10 mL) was added at room temperature. After the completion of the reaction, the solvent was concentrated, and the obtained crystals were washed with acetone to give 3-(piperidin-4-yl)-5-methyl-2H-isoquinolin-1-one hydrochloride (6.5 g). ¹H-NMR (DMSO-d₆) δ: 1.79-1.95(2H,m), 2.09-2.17(2H,m), 2.48(3H,s), 2.73-3.17(3H,m), 3.33-3.43(2H,m), 6.29(1H,s), 7.33(1H,t,J=8Hz), 7.53(1H,d,J=8Hz), 8.01(1H,d,J=8Hz), 9.00(1H,brs), 9.24(1H,brs), 11.36(1H,brs). MS(EI):242(M+).

### Example 94

The reaction is carried out in the same manner as in Example 81 using 2,3-dimethylbenzoic acid and 3-cyano-8-methyl-8-azabicyclo[3.2.1]octane to give 5-methyl-3-(8-methyl-8-azabicyclo[3.2.1]octan-3-yl)-2H-isoquinolin-1-one.

### Example 95

In the same manner as in Example 82, the reaction was carried out using N,N-diethyl-3-chloro-2-methylbenzamide (1.0 g) and 4-cyano-1-methylpiperidine (0.7 g) to give 5-chloro-3-(1-methylpiperidin-4-yl)-2H-isoquinolin-1-one (1.2 g). melting point: 227-229°C. ¹H-NMR(400MHz,CDCl₃)δ: 10.36(brs,1H), 8.27(d,J=7.8Hz,1H), 7.68(dd,J=6.6Hz,1.1,1H), 7.33(t,J=8.1Hz,1H), 6.70(s,1H), 3.03(d,J=11.5Hz,2H), 2.59-2.45(m,1H), 2.35(s,3H), 2.20-1.75 (m,6H). MS (EI) : 276 (M+)

### Example 96

The reaction is carried out in the same manner as in Example 81 using 3-bromo-2-methylbenzoic acid and 4-cyano-1-methylpiperidine to give 5-bromo-3-(1-methylpiperidin-4-yl)-2H-isoquinolin-1-one.

### Example 97

Diisopropylamine (6.2 ml) was dissolved in tetrahydrofuran (50 ml), and a solution (1.59 M, 28 ml) of n-butyllithium in hexane was added dropwise at -78°C. The mixture was stirred at 0°C for 30 min and cooled to -78°C. A solution (20 ml) of 2-benzylbenzoic acid (4.2 g) in tetrahydrofuran was added dropwise to the reaction solution, and the mixture was stirred at 0°C for 30 min and cooled to - 78°C. A solution (20 ml) of 1-benzyloxycarbonyl-4-cyanopiperidine (5.3 g) in tetrahydrofuran was added dropwise to the reaction solution, and the mixture was stirred at -78°C for one day. After the completion of the reaction, water was added to the reaction solution, and the mixture was extracted with ethyl acetate. The extract was dried over magnesium sulfate, and the solvent was concentrated. The obtained residue was subjected to silica gel column chromatography. A chloroform:methanol=40:1 effluent fraction was concentrated, and diisopropyl ether was added to the precipitated crystals. The crystals were collected by filtration to give 3-(1-benzyloxycarbonylpiperidin-4-yl)-4-phenyl-2H-isoquinolin-1-one (2.1 g) as white crystals (melting point: 276-278°C). 3- (1-Benzyloxycarbonylpiperidin-4-yl)-4-phenyl-2H-isoquinolin-1-one (2.0 g) was suspended in chloroform (10 ml), and a solution (5 ml) of hydrobromic acid in acetic acid was added dropwise. After the completion of the reaction, the solvent was concentrated, and acetone was added to the obtained residue. The precipitated crystals were collected by filtration to give 4-phenyl-3-(piperidin-4-yl)-2H-isoquinolin-1-one hydrobromide (1.5 g) . melting point: >270°C.
¹H-NMR(DMSO-d₆) d: 1.71-1.75(2H,m), 2.05-2.15(2H,m), 2.54-2.68(2H,m), 3.23-3.29(2H,m), 6.84(1H,d,J=8Hz), 7.26(2H,d,J=6Hz), 7.42-7.58(5H,m), 8.20-8.22(1H,m), 8.69(1H,brS), 11. 09 (1H,brS).

### Example 98

4-Phenyl-3-(piperidin-4-yl)-2H-isoquinolin-1-one hydrobromide (2.2 g) obtained in Example 97 was suspended in acetonitrile (40 ml), and 37% formaldehyde (2.3 ml) was added. Sodium cyanoborohydride (0.0.54 g) was added to the mixture under ice-cooling. Acetic acid (0.28 ml) was added dropwise, and the mixture was allowed to react overnight. After the completion of the reaction, the solvent was concentrated, and an aqueous potassium carbonate solution was added to the obtained residue. The mixture was extracted with chloroform and dried over magnesium sulfate. The solvent was concentrated and ethyl acetate was added to the precipitated crystals. The crystals were collected by filtration to give 3-(1-methylpiperidin-4-yl)-4-phenyl-2H-isoquinolin-1-one 1/5 water adduct (0.18 g). melting point: 258-260°C/decomposition. ¹H-NMR(CDCl₃) d: 1.65-1.82(6H,m), 2.24(3H,s), 2.39-2.44(1H,m), 2.83-2.89(2H,m), 7.02(1H,d,J=8Hz), 7.23-7.26(2H,m), 7.41-7.53(5H,m), 8.43(1H,d,J=7Hz), 8.45(1H,brS).

### Example 99

In the same manner as in Example 82, the reaction was carried out using N,N-diethyl-2-(4-methoxybenzyl)benzamide (1.0 g) and 4-cyano-1-methylpiperidine (0.6 g) to give 4-(4-methoxyphenyl)-3-(1-methylpiperidin-4-yl)-2H-isoquinolin-1-one (0.4 g). melting point: 209-211°C. ¹H-NMR(400MHz,CDCl₃) δ: 8.82(brs,1H), 8.41(d,J=7.8Hz,1H), 7.57-7.38(m,2H), 7.20-6.95(m,5H), 3.88(s,3H), 2.87(d,J=9.8Hz,2H), 2.50-2.40(m,1H), 2.23(s,3H), 1.97-1.75(m,4H), 1.66(d,J=11.4Hz,2H).

### Example 100

In the same manner as in Example 82, the reaction was carried out using N,N-diethyl-2-(4-chlorobenzyl)benzamide (1.0 g) and 4-cyano-1-methylpiperidine (0.6 g) to give 4-(4-chlorophenyl)-3-(1-methylpiperidin-4-yl)-2H-isoquinolin-1-one (0.4 g). melting point: 254-256°C. ¹H-NMR(400MHz,CDCl₃) δ: 8.56(brs,1H), 8.41(d,J=8.1Hz,1H), 7.57-7.30(m,4H), 7.17(d,J=8.3Hz,2H), 6.96(d,J=8.0Hz,1H), 2.87(d,J=6.8Hz,2H), 2.43-2.30(m,1H), 2.23(s,3H), 1.90-1.58(m,6H).

### Example 101

The reaction is carried out in the same manner as in Example 81 using 3,5-dibromo-2-methylbenzoic acid and 1-benzyloxycarbonyl-4-cyanopiperidine to give 5,7-dibromo-3-(piperidin-4-yl)-2H-isoquinolin-1-one.

### Example 102

The reaction is carried out in the same manner as in Example 81 using 3-methoxy-2-methylbenzoic acid and 1-benzyloxycarbonyl-4-cyanopiperidine to give 5-methoxy-3-(piperidin-4-yl)-2H-isoquinolin-1-one.

### Example 103

The reaction is carried out in the same manner as in Example 19 using 5-methoxy-3-(piperidin-4-yl)-2H-isoquinolin-1-one obtained in Example 102 to give 5-hydroxy-3-(piperidin-4-yl)-2H-isoquinolin-1-one.

### Example 104

The reaction is carried out in the same manner as in Example 81 using 3-fluoro-2-methylbenzoic acid and 1-benzyloxycarbonyl-4-cyanopiperidine to give 5-fluoro-3-(piperidin-4-yl)-2H-isoquinolin-1-one.

### Example 105

The reaction is carried out in the same manner as in Example 81 using 3-trifluoromethyl-2-methylbenzoic acid and 4-cyano-1-methylpiperidine to give 3-(1-methylpiperidin-4-yl)-5-trifluoromethyl-2H-isoquinolin-1-one.

### Example 106

In the same manner as in Example 82, the reaction was carried out using N,N-diethyl-3-fluoro-2-methylbenzamide (1.0 g) and 4-cyano-1-methylpiperidine (0.7 g) to give 5-fluoro-3-(1-methylpiperidin-4-yl)-2H-isoquinolin-1-one (0.5 g). melting point: 220-222°C. ¹H-NMR(400MHz,CDCl₃) δ: 10.24(brs,1H), 8.12(d,J=7.8Hz,1H), 7.40-7.25(m,2H), 6.53(s,1H), 3.02(d,J=11.7Hz,2H), 2.57-2.41(m,1H), 2.34(s,3H), 2.11(t,J=12.0Hz,2H), 2.02(d,J=13.4Hz,2H), 1.90-1.75(m,2H). MS (EI) : 260 (M+)

### Example 107

In the same manner as in Example 82, the reaction was carried out using N,N-diethyl-3-methoxy-2-methylbenzamide (1.0 g) and 4-cyano-1-methylpiperidine (0.6 g) to give 5-methoxy-3-(1-methylpiperidin-4-yl)-2H-isoquinolin-2-one (0.2 g). melting point: 239-242°C. ¹H-NMR(400MHz,DMSO-d₆) δ: 11.24(brs,1H), 7.68(d,J=8.1Hz,1H), 7.33(t,J=8.1Hz,1H), 7.18(d,J=7.8Hz,1H), 6.44(s,1H), 3.89(s,3H), 2.85(d,J=11.5Hz,2H), 2.43-2.28(m,1H), 2.17(s,3H), 1.95-1.78(m,4H), 1.65-1.50(m,2H). MS(EI): 272 (M+)

### Example 108

In the same manner as in Example 19, the reaction was carried out using 5-methoxy-3-(1-methylpiperidin-4-yl)-2H-isoquinolin-1-one (1.0 g) to give 5-hydroxy-3-(1-methylpiperidin-4-yl)-2H-isoquinolin-1-one (0.1 g). melting point: >280/dec. ¹H-NMR(400MHz,DMSO-d₆) δ: 11.11,(s,1H), 10.02(s,1H), 7.56(d,J=8.0Hz,1H), 7.18(t,J=7.8Hz,1H), 7.03(d,J=6.6Hz,1H), 6.44(s,1H), 2.85(d,J=11.4Hz,2H), 2.45-2.25(m,1H), 2.17(s,3H), 1.99-1.80 (m, 4H) , 1. 70-1. 50 (m, 2H) . MS (EI) : 258 (M+)

### Different synthesis method

5-Methoxymethyloxy-3-(1-methylpiperidin-4-yl)-2H-isoquinolin-1-one (3.0 g) was dissolved in methanol (30 mL), and 30% hydrochloric acid/methanol (3 mL) was added to the mixture at room temperature. After the completion of the reaction, solvent was concentrated, and the precipitated crystals were collected by filtration to give 5-hydroxy-3-(1-methylpiperidin-4-yl)-2H-isoquinolin-1-one hydrochloride (2.9 g). melting point: 269-271°C. ¹H-NMR (400MHz,DMSO-d₆) δ: 11.28(brs,1H), 10.23(brs,1H), 7.57(d,J=8.1Hz,1H), 7.22(t,J=7.8Hz,1H), 7.08(d,J=7.6Hz,1H), 6.42(s,1H), 3.49(d,J=11.7Hz,2H), 3.08-2.91(m,2H), 2.76(s,3H), 2.70-2.60(m,1H), 2.16(d,J=13.2Hz,2H), 1.93-1.78(m,2H). MS(EI): 258(M+)

### Example 109

The reaction is carried out in the same manner as in Example 97 using 3-chloro-2-benzylbenzoic acid and 4-cyano-1-methylpiperidine to give 5-chloro-3-(1-methylpiperidin-4-yl)-4-phenyl-2H-isoquinolin-1-one.

### Example 110

The reaction was carried out in the same manner as in Example 1 using 5-chloro-6H-thieno[2,3-c]pyridin-7-one (1 g) and 1-methylpiperazine (2 ml) to give 5-(4-methylpiperazin-1-yl)-6H-thieno[2,3-c]pyridin-7-one (53 mg). ¹H-NMR(400MHz,CDCl₃) δ: 2.38(3H,s), 2.58-2.63(4H,m), 3.23-3.30(4H,m), 5.95 (1H,s), 7.08(1H,d,J=5Hz), 7.62(1H,d,J=5Hz), 11.35(1H,brS).

### Example 111

The reaction is carried out in the same manner as in Example 1 using 5-chloro-6H-thieno[2,3-c]pyridin-7-one and 4-dimethylaminopiperidine (2 ml) to give 5-(4-dimethylaminopiperidin-1-yl)-6H-thieno[2,3-c]pyridin-7-one.

### Example 112

The reaction was carried out in the same manner as in Example 1 using 6-chloro-5H-thieno[3,2-c]pyridin-4-one (1 g) and 1-methylpiperazine (5 ml) to give 6-(4-methylpiperazin-1-yl)-5H-thieno[3,2-c]pyridin-4-one (35 mg). ¹H-NMR(400MHz,CDCl₃) δ: 2.39(3H,s), 2.55-2.70(4H,m), 3.20-3.30(4H,m), 6.02(1H,s), 7.03(1H,d,J=5Hz), 7.45(1H,d,J=5Hz), 11.35(1H,brS).

### Example 113

The reaction is carried out in the same manner as in Example 1 using 6-chloro-5H-thieno[3,2-c]pyridin-4-one and 4-dimethylaminopiperidine(2 ml) to give 6-(4-dimethylaminopiperidin-1-yl)-5H-thieno[3,2-c]pyridin-4-one.

### Example 114

The reaction was carried out in the same manner as in Example 1 using 3-chloro-2H-benz[f]isoquinolin-1-one (1 g) and 4-methylpiperazine (5 ml) to give 3-(4-methylpiperazin-1-yl)-2H-benz[f]isoquinolin-1-one (0.21 g). melting point: 253-254°C/decomposition. ¹H-NMR(400MHz,CDCl₃) δ: 2.36 (3H,s) , 2.71-2.72(4H,brS), 3.46-3.47(4H,brS), 6.55(1H,s), 7.58-7.66(3H,m), 7.88(1H,d,J=8Hz), 8.22(1H,d,J=8Hz), 8.39(1H,d,J=8Hz).

### Example 115

The reaction was carried out in the same manner as in Example 1 using 3-chloro-2H-benz[f]isoquinolin-1-one (1 g) and 4-dimethylaminopiperidine (5 ml) to give 3-(4-dimethylaminopiperidin-1-yl)-2H-benz[f]isoquinolin-1-one (77 mg). melting point: 232-233°C/decomposition. ¹H-NMR(400MHz,CDCl₃) δ: 1.83-1.89(2H,m), 2.05-2.08(2H,m), 2.39-2.41(7H,m), 2.93-2.99(2H,m), 3.98-4.00(2H,m), 6.55(1H,s), 7.57-7.65(3H,m), 7.87(1H,d,J=8Hz), 8.23(1H,d,J=8Hz), 8.38(1H,d,J=8Hz).

### Example 116

The reaction was carried out in the same manner as in Example 1 using 3-chloro-2H-benz[h]isoquinolin-1-one (1 g) and 4-methylpiperazine (5 ml) to give 3-(4-methylpiperazin-1-yl)-2H-benz[h]isoquinolin-1-one (0.35 g). ¹H-NMR(400MHz,CDCl₃) δ: 2.44(3H,s), 2.70-2.76(4H,m), 3.44-3.50(4H,m), 5.92(1H,s), 7.40(1H,d,J=9Hz), 7.49(1H,t,J=7Hz), 7.62(1H,t,J=8Hz), 7.81(1H,d,J=8Hz), 7.87(1H,d,J=9Hz), 10.03(1H,d,J=8Hz), 12.22(1H,brS).

### Example 117

The reaction was carried out in the same manner as in Example 1 using 3-chloro-2H-benz[h]isoquinolin-1-one (1 g) and 4-dimethylaminopiperidine (2 ml) to give 3-(4-dimethylaminopiperidin-1-yl)-2H-benz[h]isoquinolin-1-one (0.56 g). ¹H-NMR(400MHz,CDCl₃) δ: 1.78-1.88(2H,m), 2.01-2.06(2H,m), 2.34(6H,s), 2.34-2.42(1H,m), 2.38-2.45(2H,m), 4.02-4.06(2H,m), 5.91(1H,s), 7.38(1H,d,J=9Hz), 7.47(1H,t,J=7Hz), 7.64(1H,t,J=7Hz), 7.79(1H,d,J=7Hz), 7.85(1H,d,J=9Hz), 10.06(1H,d,J=8Hz), 12.04(1H,brS).

### Example 118

The reaction is carried out in the same manner as in Example 1 using 7-chloro-6H-1,6-naphthyridin-5-one and 1-methylpiperazine to give 7-(4-methylpiperazin-1-yl)-6H-1,6-naphthyridin-5-one.

### Example 119

The reaction is carried out in the same manner as in Example 1 using 7-chloro-6H-1,6-naphthyridin-5-one and 4-dimethylaminopiperidine to give 7-(4-dimethylaminopiperidin-1-yl)-6H-1,6-naphthyridin-5-one.

### Example 120

2-Amino-3-methylbenzamide (6.0 g) and triethylamine (11.2 ml) were dissolved in dimethylformamide (60 ml), and 1-(benzyloxycarbonyl)piperidin-4-ylcarbonyl chloride (16.9 g) was added to the mixture under ice-cooling. The mixture was stirred at room temperature for 2 hr, and water and chloroform were added. The precipitated crystals were collected by filtration to give 2-((1-(benzyloxycarbonyl)piperidin-4-yl)carbonyl)amino-3-methylbenzamide (6.8 g). The filtrate was extracted with chloroform and dried over magnesium sulfate. The solvent was concentrated, and the precipitated crystals were collected by filtration with chloroform to give 2.2 g of the compound. The filtrate was concentrated and purified by silica gel column chromatography to give 2.0 g of the compound. A total of 11 g was obtained.

This compound was suspended in a mixed solvent of pyridine (28 ml) and water (28 ml), and 2N aqueous sodium hydroxide (2.8 ml) was added. The mixture was stirred at room temperature for 4 days. After the completion of the reaction, the precipitated crystals were collected by filtration and washed with water to give 2-(1-(benzyloxycarbonyl)piperidin-4-yl)-8-methyl-3H-quinazolin-4-one (8.7 g).

This compound (8.7 g) was dissolved in a solution of hydrobromic acid in acetic acid, and the mixture was stirred overnight. After the completion of the reaction, the precipitated crystals were collected by filtration to give hydrobromide. The crystals were alkalized and recrystallized from ethanol to give 8-methyl-2-(piperidin-4-yl)-3H-quinazolin-4-one (5 g). ¹H-NMR(400MHz,DMSO-d₆) d: 1.62-1.83(4H,m), 2.42-2.56(4H,m), 2.51(3H,S), 2.60-2.68(1H,m), 2.95-3.05(2H,m), 3.32(1H,brS), 7.31(1H,t,J=8Hz), 7.62(1H,d,J=8Hz), 7.90(1H,d,J=8Hz).

### Example 121

The reaction was carried out in the same manner as in Example 82 using 2-(piperidin-4-yl)-8-methyl-3H-quinazolin-4-one (0.9 g) obtained in Example 120 to give 2-(1-methylpiperidin-4-yl)-8-methyl-3H-quinazolin-4-one (0.42 g). ¹H-NMR(400MHz,CDCl₃) δ: 2.00-2.15(6H,m), 2.33(3H,S), 2.55-2.64(1H,m), 2.58(3H,s), 2.96-3.04(2H,m), 7.32(1H,t,J=8Hz), 7.58(1H,d,J=7Hz), 8.09(1H,d,J=8Hz), 10.81(1H,brS).

### Example 123

The reaction was carried out in the same manner as in Examples 120 and 82 using 2-amino-3-methoxybenzamide (2.0 g) and 1-(benzyloxycarbonyl)piperidin-4-ylcarbonyl chloride (6.2 g) to give 8-methoxy-2-(1-methylpiperidin-4-yl)-3H-quinazolin-4-one (0.22 g). ¹H-NMR(400MHz,DMSO-d₆) d: 1.78-1.92(6H,m), 2.18(3H,s), 2.47-2.52(1H,m), 2.80-2.88(2H,m), 3.89(3H,s), 7.31(1H,t,J=8Hz), 7.38(1H,d,J=8Hz), 7.63(1H,d,J=7Hz), 12.14(1H,brS).

### Example 124

The reaction is carried out in the same manner as in Example 19 using 8-methoxy-2-(1-methylpiperidin-4-yl)-3H-quinazolin-4-one obtained in Example 123 to give 8-hydroxy-2-(1-methylpiperidin-4-yl)-3H-quinazolin-4-one.

### Example 125

The reaction is carried out in the same manner as in Examples 120 and 82 using 2-amino-3-fluorobenzamide and 1-(benzyloxycarbonyl)piperidin-4-ylcarbonyl chloride to give 8-fluoro-2-(1-methylpiperidin-4-yl)-3H-quinazolin-4-one.

### Example 126

The reaction is carried out in the same manner as in Examples 120 and 82 using 2-amino-3-chlorobenzamide and 1-(benzyloxycarbonyl)piperidin-4-ylcarbonyl chloride to give 8-chloro-2-(1-methylpiperidin-4-yl)-3H-quinazolin-4-one.

### Example 127

, The reaction is carried out in the same manner as in Examples 120 and 82 using 2-amino-3-bromobenzamide and 1-(benzyloxycarbonyl)piperidin-4-ylcarbonyl chloride to give 8-bromo-2-(1-methylpiperidin-4-yl)-3H-quinazolin-4-one.

### Example 128

The reaction is carried out in the same manner as in Example 120 using 2-amino-3-methoxybenzamide and 4-dimethylaminocyclohexanecarbonyl chloride to give 8-methoxy-2-(4-dimethylaminocyclohexan-1-yl)-3H-quinazolin-4-one.

### Example 129

The reaction is carried out in the same manner as in Example 19 using 8-methoxy-2-(4-dimethylaminocyclohexan-1-yl)-3H-quinazolin-4-one obtained in Example 128 to give 8-hydroxy-2-(4-dimethylaminocyclohexan-1-yl)-3H-quinazolin-4-one.

### Example 130

The reaction is carried out in the same manner as in Example 120 using 2-amino-3-fluorobenzamide and 4-dimethylaminocyclohexanecarbonyl chloride to give 8-fluoro-2-(4-dimethylaminocyclohexan-1-yl)-3H-quinazolin-4-one.

### Example 131

The reaction is carried out in the same manner as in Example 120 using 2-amino-3-chlorobenzamide and 4-dimethylaminocyclohexanecarbonyl chloride to give 8-chloro-2-(4-dimethylaminocyclohexan-1-yl)-3H-quinazolin-4-one.

### Example 132

The reaction is carried out in the same manner as in Example 120 using 2-amino-3-bromobenzamide and 4-dimethylaminocyclohexanecarbonyl chloride to give 8-bromo-2-(4-dimethylaminocyclohexan-1-yl)-3H-quinazolin-4-one.

### Example 133

The reaction is carried out in the same manner as in Example 120 using 2-aminobenzamide and (1-azabicyclo[2.2.2]octan-3-yl)carbonyl chloride to give 2-(1-azabicyclo[2.2.2]octan-3-yl)-3H-quinazolin-4-one.

### Example 134

The reaction is carried out in the same manner as in Example 120 using 2-aminobenzamide and (1-azabicyclo[2.2.2]octan-3-yl)acetyl chloride to give 2-((1-azabicyclo[2.2.2]octan-3-yl)methyl)-3H-quinazolin-4-one.

### Example 135

The reaction is carried out in the same manner as in Example 120 using 2-aminobenzamide and (8-methyl-8-azabicyclo[3.2.1]octan-3-yl)carbonyl chloride to give 2-(8-methyl-8-azabicyclo[3.2.1]octan-3-yl)-3H-quinazolin-4-one.

### Example 136

The reaction is carried out in the same manner as in Example 120 using 2-aminobenzamide and 4-dimethylaminocyclohexanecarbonyl chloride to give 2-(4-dimethylaminocyclohexan-1-yl)-3H-quinazolin-4-one.

### Example 137

The reaction was carried out in the same manner as in Example 120 using 2-amino-3-methylbenzamide and 4-dimethylaminocyclohexanecarbonyl chloride to give 8-methyl-2-(4-dimethylaminocyclohexan-1-yl)-3H-quinazolin-4-one (4.6 mg). melting point: 181-183°C/decomposition. ¹H-NMR(400MHz,CDCl₃) δ: 1.83-1.85(6H,m), 2.05-2.35(10Hz,m), 2.61(3H,s), 2.94-2.96(1H,m), 7.34(1H,t,J=8Hz), 7.60(1H,d,J=8Hz), 8.11(1H,d,J=8Hz).

### Example 138

The reaction was carried out in the same manner as in Example 120 using 2-amino-3-methylbenzamide and (1-azabicyclo[2.2.2]octan-3-yl)carbonyl chloride to give 2-(1-azabicyclo[2.2.2]octan-3-yl)-8-methyl-3H-quinazolin-4-one (17.7 mg). melting point: 230-231°C/decomposition. ¹H-NMR(400MHz,CDCl₃) δ: 1.12-1.14(1H,m), 1.76-1.92(4H,m), 2.29-2.32(1H,m), 2.65(3H,S), 2.94-2.96(1H,m), 3.05-3.09(3H,m), 3.24-3.26(2H,m), 4.02-4.04(1H,m), 7.37(1H,t,J=8Hz), 7.64(1H,d,J=8Hz), 8.11(1H,d,J=8Hz).

### Example 139

The reaction was carried out in the same manner as in Example 120 using 2-amino-3-methylbenzamide and (1-azabicyclo[2.2.2]octan-3-yl)acetyl chloride to give 2-((1-azabicyclo[2.2.2]octan-3-yl)methyl)-8-methyl-3H-quinazolin-4-one (27 mg). melting point: 187-188°C/decomposition. ¹H-NMR(400MHz,CDCl₃) δ: 1.65-2.10(5H,m), 2.56-2.59(5H,m), 2.80-2.85(1H,m), 3.00-3.14(SH,m), 3.35-3.41(2H,m), 7.29-7.33(1H,t,J=8Hz), 7.57(1H,d,J=8Hz), 8.03(1H,d,J=8Hz).

### Example 140

The reaction is carried out in the same manner as in Example 120 using 2-aminobenzamide and 3-dimethylaminopropionyl chloride to give 2-(2-(dimethylamino)ethyl)-3H-quinazolin-4-one.

### Example 141

The reaction is carried out in the same manner as in Example 120 using 2-aminobenzamide and 4-dimethylaminobutyryl chloride to give 2-(3-(dimethylamino)propyl)-3H-quinazolin-4-one.

### Example 142

The reaction is carried out in the same manner as in Example 120 using 2-aminobenzamide and 6-dimethylaminohexanoyl chloride to give 2-(5-(dimethylamino)pentyl)-3H-quinazolin-4-one.

### Example 143

The reaction is carried out in the same manner as in Example 120 using 2-amino-3-methylbenzamide and (8-methyl-8-azabicyclo[3.2.1]octan-3-yl)carbonyl chloride to give 8-methyl-2-(8-methyl-8-azabicyclo[3.2.1]octan-3-yl)-3H-quinazolin-4-one.

### Example 144

The reaction was carried out in the same manner as in Example 120 using 2-amino-3-methylbenzamide (3.24 g) and 3-(dimethylamino)propanoic acid hydrochloride (3.38 g) to give 8-methyl-2-[2-(dimethylamino)ethyl]-3H-quinazolin-4-one (936 mg, 50%).
¹H-NMR(DMSO-d₆) d: 2.18(6H,s), 2.69-3.20(7H,m), 7.32(1H,t,J=7.7Hz), 7.62(1H,d,J=7.3Hz), 7.89(1H,d,J=8.1Hz).

### Example 145

The reaction was carried out in the same manner as in Example 120 using 2-amino-3-methylbenzamide (3.24 g) and 4-(dimethylamino)butanoic acid hydrochloride (3.69 g) to give 8-methyl-2-[3-(dimethylamino)propyl]-3H-quinazolin-4-one (177 mg, 3%).
¹H-NMR(DMSO-d₆) d: 1.91-1.97(2H,m), 2.40(6H,s), 2.49-2.53(2H,m), 2.59(3H,s), 2.88-2.91(2H,m), 7.26-7.73(1H,m), 7.55(1H,d,J=7.3Hz), 8.10(1H,d,J=8.0Hz).

### Example 146

The reaction was carried out in the same manner as in Example 120 using 2-amino-3-methylbenzamide (3.26 g) and 6-(dimethylamino)hexanoic acid hydrochloride (2.49 g) to give 8-methyl-2-[5-(dimethylamino)pentyl]-3H-quinazolin-4-one (622 mg, 14%).
¹H-NMR(DMSO-d₆) d: 1.44-1.49(4H,m), 1.80-1.88(2H,m), 2.24(6H,s), 2.31-2.34(2H,m), 2.67(3H,s), 2.66-2.71(2H,m), 7.26-7.29(1H,m), 7.54(1H,d,J=7.3Hz), 8.06(1H,d,J=7.3Hz).

### Example 147

The reaction was carried out in the same manner as in Example 81 using 2-methylbenzoic acid (1.8 g) and 1-cyano-4-(dimethylamino)cyclohexane (2.0 g) to give 3-(4-(dimethylamino)cyclohexan-1-yl)-2H-isoquinolin-1-one (0.39 g). melting point: 256-258°C. ¹H-NMR(CDCl₃) δ: 1.40-1.46(2H,m), 1.55-1.65(2H,m), 2.07-2.14(4H,m), 2.25-2.35(1H,m), 2.35(6H,s), 2.42-2.51(1H,m), 6.32(1H,s), 7.43(1H,t,J=7Hz), 7.49(1H,d,J=8Hz), 7.62(1H,t,J=7Hz), 8.37(1H,t,d,J=8Hz), 10.31-10.80(1H,m).

### Example 148

The reaction was carried out in the same manner as in Example 81 using 2-methylbenzoic acid (1.8 g) and 4-(4-methylpiperazin-1-yl)butyronitrile (2.2 g) to give 3-(3-(4-methylpiperazin-1-yl)propyl)-2H-isoquinolin-1-one (0.027 g). melting point: 120-122°C. ¹H-NMR(CDCl₃) δ: 1.83-1.88(2H,m), 2.37(3H,s), 2.42-2.66(12H,m), 6.22(1H,s), 7.36-7.43 (2H,m) , 7.55-7.59(1H,m), 8.33(1H,d,J=8Hz), 11.35(1H,brS)

### Example 151

In the same manner as in Example 1, the reaction was carried out using 3-chloro-2H-isoquinolin-1-one (1.0 g) and (R)-3-hydroxymethyl-1-methylpiperazine (2.0 g) to give (R)-3-(2-hydroxymethyl-4-methylpiperazin-1-yl)-2H-isoquinolin-1-one (0.3 g). melting point: 172-174°C. ¹H-NMR(400MHz,CDCl₃) δ:11.35(brs,1H), 8.16(d,J=8.0Hz,1H), 7.54(t,J=8.0Hz,1H), 7.37(d,J=8.0Hz,1H), 7.30-7.20(m,1H), 5.89(s,1H), 4.10-3.90(m,3H), 3.70-3.60(m,1H), 3.49-3.40(m,1H), 3.27-3.18(m,1H), 3.14-2.95(m,1H), 2.75-2.20(m,6H)

### Example 152

In the same manner as in Example 1, the reaction was carried out using 3-chloro-2H-isoquinolin-1-one (1.0 g) and (S)-3-hydroxymethyl-1-methylpiperazine (2.0 g) to give (S)-3-(2-hydroxymethyl-4-methylpiperazin-1-yl)-2H-isoquinolin-1-one (0.3 g). melting point: 169-171°C. ¹H-NMR(400MHz,CDCl₃) δ:11.41(brs,1H), 8.15(d,J=8.0Hz,1H), 7.54(t,J=8.0Hz,1H), 7.37(d,J=8.0Hz,1H), 7.30-7.20(m,1H), 5.88(s,1H), 4.15-3.91(m,3H), 3.70-3.60(m,1H), 3.49-3.40(m,1H), 3.27-3.18(m,1H), 3.14-2.95(m,1H), 2.75-2.20(m,6H)

### Example 153

In the same.manner as in Example 1, the reaction was carried out using 3-chloro-5-methyl-2H-isoquinolin-1-one (1.0 g) and 2-hydroxymethyl-1-methylpiperazine (1.4 g) to give 3-(3-hydroxymethyl-4-methylpiperazin-1-yl)-5-methyl-2H-isoquinolin-1-one (0.3 g). melting point: 232-234°C. ¹H-NMR(400MHz,DMSO-d₆) δ: 2.13(1H,br.s), 2.20-2.35(1H,m), 2.24(3H,s), 2.38(3H,s), 2.58(1H,t,J=10Hz), 2.70-2.85(2H,m), 3.30-3.70(4H,m), 4.63(1H,t,J=5Hz), 5.64(1H,s), 7.09(1H,t,J=8Hz), 7.39(1H,d,J=8Hz), 7.87(1H,d,J=8Hz), 11.17(1H,s). MS (EI) :287(M⁺)

### Example 154

In the same manner as in Example 18, the reaction was carried out using methyl 2-methoxycarbonylphenylacetimidate hydrochloride (1.0 g) and 2-ethoxycarbonyl-1-methylpiperazine (0.7 g) to give 3-(3-ethoxycarbonyl-4-methylpiperazin-1-yl)-2H-isoquinolin-1-one (0.5 g). melting point: 105-109°C. ¹H-NMR(400MHz,DMSO-d₆) δ:11.15(s,1H), 7.99(d,J=8.1Hz,1H), 7.53(t,J=8.0Hz,1H), 7.43(d,J=7.8Hz,1H), 7.20(t,J=7.8Hz,1H), 5.80(s,1H), 3.52(d,J=10.5Hz,1H), 4.12(q,J=7.1Hz,2H), 3.16(d,J=5.1Hz,1H), 3.06-2.90(m,4H), 2.35-2.27(m,1H), 2.25(s,3H), 1.20(t,J=7.1Hz,3H).

### Example 155

In the same manner as in Example 18, the reaction was carried out using methyl 2-methoxycarbonylphenylacetimidate hydrochloride (1.0 g) and 2-methylpiperazine (0.6 g) to give 3-(3-methylpiperazin-1-yl)-2H-isoquinolin-1-one (0.3 g). melting point: 164-167°C. ¹H-NMR(400MHz,DMSO-d₆) δ:11.03(brs,1H), 7.98(d,J=8.0Hz,1H), 7.51(t,J=6.8Hz,1H), 7.41(d,J=7.8Hz,1H), 7.17(t,J=7.1Hz,1H), 5.72(s,1H), 3.48(d,J=11.5Hz,2H), 2.90(d,J=12.0Hz,1H), 2.80-2.70(m,2H), 2.60-2.40(m,2H), 2.21(t,J=11.0Hz,1H), 0.99(d,J=6.4Hz,3H).

### Example 156

In the same manner as in Example 18, the reaction was carried out using methyl 2-methoxycarbonylphenylacetimidate hydrochloride (1.0 g) and (S)-2-(hydroxymethyl)-1-methylpiperazine (0.7 g) to give (S)-3-(3-hydroxymethyl-4-methylpiperazin-1-yl)-2H-isoquinolin-1-one (0.3 g). melting point: 173-174°C. ¹H-NMR (400MHz,DMSO-d₆) δ: 11.10(s,1H), 7.99(d,J=7.8Hz,1H), 7.52(t,J=7.1Hz,1H), 7.42(d,J=7.8Hz,1H), 7.19(t,J=7.3Hz,1H), 5.74(s,1H), 4.57(brs,1H), 3.67-3.49(m,3H), 3.45-3.28(m,2H), 2.77-2.15(m,2H), 2.56(t,J=10.5Hz,1H), 2.24(s,3H), 2.12(brs,1H)

### Different synthesis method

The reaction was carried out in the same manner as in Example 1 using 3-chloro-2H-isoquinolin-1-one (4.0 g) and (S)-2-hydroxymethylpiperazine (5.5 g) to give (S)-3-(3-hydroxymethylpiperazin-1-yl)-2H-isoquinolin-1-one (4.5 g). (S)-3-(3-Hydroxymethylpiperazin-1-yl)-2H-isoquinolin-1-one (4.5 g) was dissolved in acetonitrile (80 mL) and 37% aqueous formalin solution (8.2 mL), and sodium cyanoborohydride (3.4 g) and acetic acid (0.85 mL) were added thereto under ice-cooling. After the completion of the reaction, the reaction solution was concentrated, and an aqueous potassium carbonate solution was added to the obtained residue. The mixture was extracted with chloroform. The organic layer was dried over magnesium sulfate, concentrated and purified by silica gel column chromatography. A chloroform:methanol=4:1 effluent fraction was concentrated, and the precipitated crystals were washed with diisopropyl ether to give (S)-3-(3-hydroxymethyl-4-methylpiperazin-1-yl)-2H-isoquinolin-1-one (3.3 g).

### Example 157

In the same manner as in Example 18, the reaction was carried out using methyl 2-methoxycarbonylphenylacetimidate hydrochloride (1.0 g) and (R)-2-(hydroxymethyl)-1-methylpiperazine (0.7 g) to give (R)-3-(3-hydroxymethyl-4-methylpiperazin-1-yl)-2H-isoquinolin-1-one (0.4 g). melting point: 171-173°C. ¹H-NMR(400MHz,DMSO-d₆) δ:11.10(s,1H), 7.99(d,J=7.8Hz,1H), 7.52(t,J=7.5Hz,1H), 7.43(d,J=7.8Hz,1H), 7.19(t,J=7.8Hz,1H), 5.74(s,1H), 4.57(brs,1H), 3.67-3.49(m,3H), 3.45-3.28(m,2H), 2.77-2.15(m,2H), 2.56(t,J=10.5Hz,1H), 2.25(s,3H), 2.12(brs,1H)

### Example 158

3-(1-Methylpiperidin-4-yl)-2H-isoquinolin-1-one (2.0 g) was dissolved in methanol (20 mL) and hydrogenated at 70°C in the presence of 10% palladium-carbon (1.0 g) at 40 atm. After the completion of the reaction, the reaction solution was filtered, and the filtrate was concentrated and purified by silica gel column chromatography. A chloroform:methanol=10:1 effluent fraction was concentrated, and isopropyl ether was added to the obtained residue. The precipitated crystals were collected by filtration to give 3-(1-methylpiperidin-4-yl)-3,4-dihydro-2H-isoquinolin-1-one (0.1 g). melting point: 139-141°C. ¹H-NMR (400MHz,CDCl₃) δ: 8.08(d,J=7.6Hz,1H), 7.48(t,J=7.3Hz,1H), 7.38(t,J=7.6Hz,1H), 7.23(d,J=7.3Hz,1H), 6.32(brs,1H), 3.52(brs,1H), 3.15-2.90(m,4H), 2.30(s,3H), 2.16(brs,1H), 2.00-1.70(m,4H), 1.58-1.30(m,2H).

### Example 159

The reaction was carried out in the same manner as in Example 120 using 2-amino-3-methylbenzamide (3.24 g) and 3-(diethylamino)propanoic acid hydrochloride (4.00 g) to give 8-methyl-2-[2-(diethylamino)ethyl]-3H-quinazolin-4-one (82 mg) (8%).
¹H-NMR(DMSO-d₆) d: 1.13 (6H,m) , 2.60 (3H,s) , 2.65-2.71(4H,m), 2.81-2.87(4H,m), 7.24-7.29(1H,m), 7.53(1H,d,J=7.3Hz), 8.08(1H,d,J=8.0Hz), 12.58(1H,m).

### Example 162

In the same manner as in Example 18, the reaction was carried out using methyl 2-methoxycarbonylphenylacetimidate hydrochloride (1.0 g) and 2,6-dimethylpiperazine (0.6 g) to give 3-(3,5-dimethylpiperazin-1-yl)-2H-isoquinolin-1-one (0.3 g). melting point: 208-210°C. ¹H-NMR (400MHz,DMSO-d₆) δ: 10.98(brs,1H), 7.97(d,J=7.8Hz,1H), 7.50(t,J=7.1Hz,1H), 7.41(d,J=8.1Hz,1H), 7.16(t,J=7.1Hz,1H), 5.72(s,1H), 3.51(d,J=10.5Hz,2H). 3.40-3.25(m,1H), 2.90-2.78(m,2H), 2.15(t,J=11.0Hz,2H), 0.99(d,J=6.4Hz, 6H).

### Example 163

In the same manner as in Example 82, the reaction was carried out using N,N-diethyl-2-(4-methylbenzyl)benzamide (1.0 g) and 4-cyano-1-methylpiperidine (0.6 g) to give 4-(4-methylphenyl)-3-(1-methylpiperidin-4-yl)-2H-isoquinolin-1-one (0.3 g). melting point: 244-246°C. ¹H-NMR (400MHz,CDCl₃) δ: 8.49(brs,1H), 8.40(d,J=8.0Hz,1H), 7.47(t,J=6.6Hz,1H), 7.40(t,J=8.3Hz,1H), 7.26(d,J=7.6Hz,2H), 7.09(d,J=8.0Hz,2H), 7.02(d,J=7.6Hz,1H), 2.86(d,J=5.6Hz,2H), 2.50-2.38(m,1H), 2.44(s,3H), 2.22(s,3H), 1.90-1.60(m,6H).

### Example 164

In the same manner as in Example 82, the reaction was carried out using N,N-diethyl-2-(4-fluorobenzyl)benzamide (1.0 g) and 4-cyano-1-methylpiperidine (0.6 g) to give 4-(4-fluorophenyl)-3-(1-methylpiperidin-4-yl)-2H-isoquinolin-1-one (0.4 g). melting point: 269-271°C. ¹H-NMR (400MHz,CDCl₃) δ: 8.53(brs,1H), 8.41(q,J=6.8Hz,1H), 7.55-7.44(m,1H), 7.43-7.38(m,1H), 7.24-7.10(m,4H), 6.97(d,J=7.6Hz,1H), 2.87(d,J=8.8Hz,2H), 2.44-2.30(m,1H), 2.22(s,3H), 1.90-1.60(m,6H).

### Example 165

The reaction was carried out in the same manner as in Example 120 using 2-amino-3-methylbenzamide (3.38 g) and 3-piperidinopropanoic acid hydrochloride (4.26 g) to give 8-methyl-2-(2-piperidinoethyl)-3H-quinazolin-4-one (1.04 g, 55%).
¹H-NMR(DMSO-d₆) δ: 1.51(2H,m), 1.68-1.74(4H,m), 2.56(7H,m), 2.72-2.77(2H,m), 2.81-2.86(2H,m), 7.23-7.29(1H,m), 7.52(1H,d,J=7.1Hz), 8.89(1H,d,J=7.8Hz).

### Example 166

The reaction is carried out in the same manner as in Example 120 using 2-amino-3-methylbenzamide (3.24 g) and 3-(morpholin-4-yl)propanoic acid hydrochloride (3.19 g) to give 8-methyl-2-[2-(morpholin-4-yl)ethyl]-3H-quinazolin-4-one (518 mg, 22%).
¹H-NMR(DMSO-d₆) δ: 2.56-2.58(4H,m), 2.64(3H,s), 2.83-2.85(2H,m), 2.89-2.92(2H,m), 3.66-3.85(4H,m), 7.31(1H,t,J=7.6Hz), 7.57(1H,d,J=7.3Hz), 8.11(1H,d,J=7.8Hz), 12.02(1H,m).

### Example 167

In the same manner as in Example 82, the reaction was carried out using N,N-diethyl-2-(2-methoxybenzyl)benzamide (1.0 g) and 4-cyano-1-methylpiperidine (0.6 g) to give 4-(2-methoxyphenyl)-3-(1-methylpiperidin-4-yl)-2H-isoquinolin-1-one (0.2 g). melting point: 153-155°C. ¹H-NMR(400MHz,CDCl₃) δ: 8.44(brs,1H), 8.39(d,J=7.8Hz,1H), 7.50-7.32(m,3H), 7.11(d,J=5.6,1H), 7.09-6.98(m,2H), 6.94(d,J=8.0Hz,1H), 3.68(s,3H), 2.92-2.80(m,2H), 2.40-2.26(m,1H), 2.21(s,3H), 1.82-1.50(m,6H).

### Example 168

In the same manner as in Example 82, the reaction was carried out using N,N-diethyl-2-(3-methylbenzyl)benzamide (1.0 g) and 4-cyano-1-methylpiperidine (0.6 g) to give 4-(3-methylphenyl)-3-(1-methylpiperidin-4-yl)-2H-isoquinolin-1-one (0.3 g). melting point: 214-216°C. ¹H-NMR (400MHz,CDCl₃) δ: 8.69(brs,1H), 8.41(d,J=6.6Hz,1H), 7.48(t,J=7.1Hz,1H), 7.43-7.28(m,2H), 7.27-7.10(m,1H), 7.08-6.95(m,3H), 2.95-2.80(m,2H), 2.50-2.38(m,1H), 2.40(s,3H), 2.23(s,3H), 2.10-1.60(m,6H).

### Example 169

In the same manner as in Example 82, the reaction was carried out using N,N-diethyl-2-(2-methylbenzyl)benzamide (1.0 g) and 4-cyano-1-methylpiperidine (0.6 g) to give 4-(2-methylphenyl)-3-(1-methylpiperidin-4-yl)-2H-isoquinolin-1-one (0.3 g). melting point: 249-251°C. ¹H-NMR (400MHz,CDCl₃) δ: 8.62(brs,1H), 8.42(d,J=7.1Hz,1H), 7.50-7.20(m,5H), 7.09(d,J=7.1Hz,1H), 6.83(d,J=7.3Hz (1H), 2.90-2.78(m,2H), 2.37-2.24(m,1H), 2.21(s,3H), 2.01(s,3H), 1.90-1.59(m,6H).

### Example 170

In the same manner as in Example 82, the reaction was carried out using N,N-diethyl-2-(3-methoxybenzyl)benzamide (1.0 g) and 4-cyano-1-methylpiperidine (0.6 g) to give 4-(3-methoxyphenyl)-3-(1-methylpiperidin-4-yl)-2H-isoquinolin-1-one (0.3 g). melting point: 181-183°C. ¹H-NMR(400MHz,CDCl₃) δ: 8.55(brs,1H), 8.40(d,J=7.1Hz,1H), 7.53-7.35(m,3H), 7.03(d,J=8.0Hz,1H), 6.97(dd,J=5.9Hz, 2.4,1H), 6.81(d,J=7.0Hz,1H), 6.76(d,J=2.7Hz,1H), 3.82(s,3H), 2.93-2.80(m,2H), 2.50-2.39(m,1H), 2.23(s,3H), 1.90-1.60(m,6H).

### Example 171

In the same manner as in Example 82, the reaction was carried out using N,N-diethyl-3-methoxymethyloxy-2-methylbenzamide (1.5 g) and 4-cyano-1-methylpiperidine (1.2 g) to give 5-methoxymethyloxy-3-(1-methylpiperidin-4-yl)-2H-isoquinolin-1-one (1.2 g). melting point: 194-196°C. ¹H-NMR(400MHz,CDCl₃) δ: 10.14(brs,1H), 8.00-7.95(m,1H), 7.38-7.25(m,1H), 6.71(s,1H), 5.29(s,2H), 3.50(s,3H), 3.00(d,J=11.4Hz,2H), 2.53-2.41(m,1H), 2.33(s,3H), 2.17-1.95(m,4H), 1.90-1.75(m,2H).MS (EI): 302 (M+)

### Example 173

The reaction was carried out in the same manner as in Example 120 using 2-amino-3-methylbenzamide (3.38 g) and 6-(diethylamino)hexanoic acid hydrochloride (3.24 g) to give 8-methyl-2-[5-(diethylamino)pentyl]-3H-quinazolin-4-one (124 mg, 8%).
¹H-NMR(DMSO-d₆) d: 0.90 (6H,m) , 1.32-1.42 (4H,m) , 1.70-1.78(2H,m), 2.30-2.49(6H,m), 2.58-2.66(2H,m), 3.29(3H,s), 7.29-7.33(1H,m), 7.62(1H,d,J=7.3Hz), 7.90(1H,d,J=7.3Hz), 12.12(1H,m).

### Example 174

The reaction is carried out in the same manner as in Example 120 using 2-amino-3-methylbenzamide and 5-(diethylamino)pentanoic acid hydrochloride to give 8-methyl-2-[4-(diethylamino)butyl]-3H-quinazolin-4-one.

### Example 175

The reaction was carried out in the same manner as in Example 120 using 2-amino-3-methylbenzamide (2.98 g) and 5-(dimethylamino)pentanoic acid hydrochloride (2.45 g) to give 8-methyl-2-[4-(dimethylamino)butyl]-3H-quinazolin-4-one (2.10 g, 77%).
¹H-NMR(DMSO-d₆) d: 1.43-1.47(2H,m), 1.68-1.72(2H,m), 2.10(6H,s), 2.22-2.25(2H,m), 2.57-2.61(2H,m), 3.40(3H,s), 7.27-7.32(1H,m), 7.60(1H,d,J=7.6Hz), 7.88(1H,d,J=7.9Hz).

### Example 176

The reaction was carried out in the same manner as in Example 120 using 2-amino-3-methylbenzamide (1.44 g) and 4-(pyrrolidin-1-yl)butanoic acid hydrochloride (1.56 g) to give 8-methyl-2-[3-(pyrrolidin-1-yl)propyl]-3H-quinazolin-4-one (38 mg).
¹H-NMR(CDCl₃) d: 1.91-2.01(6H,m), 2.59(3H,s), 2.70-2.74(6H,m), 2.92-2.95(2H,m), 7.27-7.31(1H,m), 7.54(1H,d,J=7.1Hz), 8.08(1H,d,J=7.1Hz).

### Example 177

In the same manner as in Example 82, the reaction was carried out using N,N-diethyl-2-methylnicotinamide (1.0 g) and 4-cyano-1-methylpiperidine (0.7 g) to give 7-(1-methylpiperidin-4-yl)-6H-1,6-naphthyridin-5-one 1/10 water adduct (0.4 g). melting point: >240°C/decomposition, ¹H-NMR(400MHz,DMSO-d₆) δ: 1.60-1.70(2H,m), 1.85-1.95(4H,m), 2.17(3H,s), 2.35-2.50(1H,m), 2.86(1H,d,J=8Hz), 6.40(1H,s), 7.41(1H,dd,J=8,5Hz), 8.41(1H,d,J=6Hz), 8.84(1H,dd,J=5,2Hz), 11.49(1H,brs). MS (EI) : 243 (M⁺)

### Example 178

In the same manner as in Example 82, the reaction was carried out using N,N-diethyl-2,3-dimethylbenzamide (1.0 g) and 4-cyano-1-methylpiperidine (0.6 g) to give 5-methyl-3-(1-methylpiperidin-4-yl)-2H-isoquinolin-1-one (0.3 g). melting point: 196-198°C. ¹H-NMR(400MHz,CDCl₃) δ: 10.24(brs,1H), 8.22(d,J=8.1Hz,1H), 7.45(d,J=6.8Hz,1H), 7.31(t,J=8.0Hz,1H), 6.42(s,1H), 3.04(d,J=11.5Hz,2H), 2.58-2.43(m,1H), 2.49(s,3H), 2.37(s,3H), 2.21-2.07(m,2H), 2.07-1.96(m,2H), 1.96-1.80(m,2H). MS (EI):256 (M+)

### Example 179

In the same manner as in Example 82, the reaction was carried out using N,N-diethyl-2-ethylbenzamide (1.0 g) and 4-cyano-1-methylpiperidine (0.8 g) to give 4-methyl-3-(1-methylpiperidin-4-yl)-2H-isoquinolin-1-one (0.9 g). melting point: 202-204°C. ¹H-NMR(400MHz,CDCl₃) δ: 8.40(d,J=7.8Hz,1H), 8.38(brs,1H), 7.70-7.60(m,2H), 7.48-7.41(m,1H), 3.00(d,J=11.7Hz,2H), 2.95-2.83(m,1H), 2.32(s,3H), 2.27(s,3H), 2.15-2.01(m,2H), 1.89-1.70(m,4H).

### Example 180

In the same manner as in Example 82, the reaction was carried out using N,N-diethyl-3-(dimethylamino)-2-methylbenzamide (1.0 g) and 4-cyano-1-methylpiperidine (0.7 g) to give 5-(dimethylamino)-3-(1-methylpiperidin-4-yl)-2H-isoquinolin-1-one (0.6 g). melting point: 216-218°C. ¹H-NMR(400MHz,CDCl₃) δ: 10.51(brs,1H), 8.04(d,J=7.8Hz,1H), 7.34(t,J=7.8Hz,1H), 7.25(d,J=7.8Hz,1H), 6.68(s,1H), 2.99(d,J=11.5Hz,2H), 2.78 (s,6H) , 2.57-2.43(m,1H), 2.33(s,3H), 2.16-1.99(m,4H), 1.88-1.78(m,2H). MS(EI):285(M+)

### Example 181

3-(1-Methylpiperidin-4-yl)-2H-1-oxoisoq-uinoline-5-carboxylic acid hydrochloride (3.7 g) and triethylamine (4.8 mL) were suspended in tetrahydrofuran (60 mL), and diphenylphosphoryl azide (2.9 mL) was added. The mixture was heated under reflux for 4 hr. Methanol (40 mL) was added to the reaction solution, and the mixture was further heated under reflux for 4 hr. After the completion of the reaction, the solvent was concentrated, and an aqueous potassium carbonate solution was added to the obtained residue. The precipitated crystals were collected by filtration to give 1,3-di(3-(1-methylpiperidin-4-yl)-2H-1-oxoisoquinolin-5-yl)urea (1.0 g). melting point: >280°C.

1,3-Di(3-(1-methylpiperidin-4-yl)-2H-1-oxoisoquinolin-5-yl)urea (1.0 g) was dissolved in 20% aqueous potassium hydroxide solution (10 mL), and the mixture was heated under reflux for 10 hr. After the completion of the reaction, the reaction solution was adsorbed on silica gel, and purified by silica gel column chromatography. A chloroform:methanol:triethylamine=3:1:0.08 effluent fraction was concentrated, and the precipitated crystals were collected by filtration and washed with isopropanol to give 5-amino-3-(1-methylpiperidin-4-yl)-2H-isoquinolin-1-one (0.6 g). melting point: 222-224°C. ¹H-NMR(400MHz,DMSO-d₆)δ: 10.90(brs,1H), 7.35(d,J=7.6Hz,1H), 7.06(t,J=7.8Hz,1H), 6.80(d,J=7.6Hz,1H), 6.45(s,1H), 5.48(s,2H), 2.85(d,J=11.2Hz,2H), 2.39-2.28(m,1H), 2.17(s,3H), 1.95-1.78 (m,4H) , 1.78-1.60(m,2H). MS (EI) :257 (M+)

### Example 182

In the same manner as in Example 82, the reaction was carried out using N,N-diethyl-2-(2-fluorobenzyl)benzamide (1.0 g) and 4-cyano-1-methylpiperidine (0.6 g) to give 4-(2-fluorophenyl)-3-(1-methylpiperidin-4-yl)-2H-isoquinolin-1-one (0.4 g). melting point: 247-249°C. ¹H-NMR(400MHz,CDCl₃)δ: 8.79(brs,1H), 8.43(d,J=6.6,1H), 7.56-7.35(m,3H), 7.30-7.12(m,3H), 6.96(d,J=8.0,1H), 2.95-2.80(m,2H), 2.40-2.28(m,1H), 2.23(s,3H), 1.90-1.70(m,4H), 1.65(d,J=13.9,2H).

### Example 183

In the same manner as in Example 82, the reaction was carried out using N,N-diethyl-5-chloro-2-methylbenzamide (1.0 g) and 4-cyano-1-methylpiperidine (0.7 g) to give 7-chloro-3-(1-methylpiperidin-4-yl)-2H-isoquinolin-1-one (0.5 g). melting point: 253-255°C. ¹H-NMR(400MHz,DMSO-d₆)δ: 11.39(brs,1H), 8.03(s,1H), 7.70-7.60(m,2H), 6.40(s,1H), 2.85(d,J=11.5,2H), 2.41-2.25(m,1H), 2.17(s,3H), 1.88(dd,J=11.7,10.2,4H), 1.68-1.55(m,2H). MS (EI) :276 (M+)

### Example 184

In the same manner as in Example 158, the reaction was carried out using 5-hydroxy-3-(1-methylpiperidin-4-yl)-2H-isoquinolin-1-one hydrochloride (0.8 g) to give 5-hydroxy-3-(1-methylpiperidin-4-yl)-3,4-dihydro-2H-isoquinolin-1-one (0.1 g). melting point: 216-218°C. ¹H-NMR(400MHz,DMSO-d₆)δ: 9.66(s,1H), 7.85(s,1H), 7.29(d,J=7.6,1H), 7.10(t,J=7.8,1H), 6.94(d,J=7.8,1H), 2.80-2.60(m,4H), 2.09(s,3H), 1.76-1.45(m,5H), 1.40-1.20 (m,3H). MS(EI):260(M+)

### Example 185

In the same manner as in Example 82, the reaction was carried out using N,N-diethyl-3-methoxymethyloxy-2-methylbenzamide (1.5 g) and 4-(dimethylamino)butyronitrile (1.0 g) to give 5-methoxymethyloxy-3-(3-dimethylaminopropyl)-2H-isoquinolin-1-one (0.4 g). melting point: 110-112°C. ¹H-NMR(400MHz,CDCl₃)δ: 11.55(brs,1H), 7.99(dd,J=4.9,2.2,1H), 7.33-7.24(m,2H), 6.61(s,1H), 5.28(s,2H), 3.50(s,3H), 2.67(t,J=6.4,2H), 2.37(t,J=6.1,2H), 2.32(s,6H), 1.83-1.75(m,2H). MS (EI) :290 (M+)

### Example 186

In the same manner as in Example 108, the reaction was carried out using 5-methoxymethyloxy-3-(3-dimethylaminopropyl)-2H-isoquinolin-1-one (0.3 g) to give 5-hydroxy-3-(3-dimethylaminopropyl)-2H-isoquinolin-1-one hydrochloride (0.25 g). 141-143°C. ¹H-NMR(400MHz,DMSO-d₆)δ: 11.22(brs,1H), 10.78-10.40(m,2H), 7.57(d,J=8.1,1H), 7.19(t,J=7.8,1H), 7.15-7.05(m,1H), 6.51(s,1H), 3.10-2.92(m,2H), 2.72(s,6H), 2.60-2.43(m,2H), 2.10-1.90(m,2H). MS(EI):246(M+)

### Example 187

In the same manner as in Example 82, the reaction was carried out using N,N-diethyl-3-methoxymethyloxy-2-methylbenzamide (1.5 g) and 5-(dimethylamino)pentanenitrile (1.0 g) to give 5-methoxymethyloxy-3-(4-dimethylaminobutyl)-2H-isoquinolin-1-one (0.4 g). melting point: 109-111°C. ¹H-NMR(400MHz,CDCl₃)δ: 10.71(brs,1H), 7.99(dd,J=3.4,2.6,1H), 7.35-7.25(m,2H), 6.65(s,1H), 5.29(s,2H), 3.51(s,3H), 2.61(t,J=7.3,2H), 2.35(t,J=6.8,2H), 2.25(s,6H), 1.83-1.70(m,2H), 1.61-1.50(m,2H). MS(EI):304(M+)

### Example 188

In the same manner as in Example 108, the reaction was carried out using 5-methoxymethyloxy-3-(4-dimethylaminobutyl)-2H-isoquinolin-1-one (0.3 g) to give 5-hydroxy-3-(4-dimethylaminobutyl)-2H-isoquinolin-1-one hydrochloride (0.25 g). melting point: 231-233°C. ¹H-NMR(400MHz,DMSO-d₆)δ: 11.14(brs,1H), 10.01(brs,1H), 7.56(d,J=8.1,1H), 7.17(t,J=8.1,1H), 7.02(d,J=6.8,1H), 6.45(s,1H), 2.52-2.35(m,2H), 2.19(t,J=7.1,2H), 2.08(s,6H), 1.60(t,J=7.6,2H), 1.40(t,J=7.3,2H). MS(EI):260(M+)

### Example 189

In the same manner as in Example 82, the reaction was carried out using N,N-diethyl-3-methoxymethyloxy-2-methylbenzamide (1.5 g) and 3-(piperidin-1-yl)propionitrile (1.1 g) to give 5-methoxymethyloxy-3-(2-(piperidin-1-yl)ethyl)-2H-isoquinolin-1-one (0.5 g). In the same manner as in Example 108, the reaction was carried out using 5-methoxymethyloxy-3-(2-(piperidin-1-yl)ethyl)-2H-isoquinolin-1-one (0.5 g) to give 5-hydroxy-3-(2-(piperidin-1-yl)ethyl)-2H-isoquinolin-1-one (0.4 g). melting point: 261-263°C. ¹H-NMR(400MHz,DMSO-d₆)δ: 11.29(s,1H), 10.51(brs,1H), 10.41-10.00(brs,1H), 7.57(d,J=7.8,1H), 7.22(t,J=7.8,1H), 7.10(d,J=7.8,1H), 6.58(s,1H), 3.44(d,J=11.0,2H), 3.40-3.23(m,2H), 3.01(t,J=8.8,2H), 2.95-2.80(m,2H), 1.85-1. 60 (m, 5H) , 1. 48-1. 30 (m,1H). MS (EI):272(M+)

### Example 190

In the same manner as in Example 82, the reaction was carried out using N,N-diethyl-2-methylbenzamide (5.2 g) and 4-cyanopyridine (3.4 g) to give 3-(4-pyridyl)-2H-isoquinolin-1-one hydrochloride 0.15 hydrate (2.0 g). melting point: >270°C. ¹H-NMR(DMSO-d₆)δ: 7.52(1H,s), 7.61-7.67(1H,m), 7.78-7.83 (2H,m), 8.25(1H,d,J=6Hz), 8.36(2H,d,J=7Hz), 8.97(2H,d,J=7Hz), 11.90(1H,brs).

3-(4-Pyridyl)-2H-isoquinolin-1-one hydrochloride was converted to a free base to give 1.8 g of the compound. This compound was dissolved in dimethylformamide (40 mL), and methyl iodide (0.48 mL) was added at room temperature. After the completion of the reaction, the solvent was concentrated, and the obtained residue was dissolved in methanol (40 mL). Then, sodium borohydride (0.85 g) was added under ice-cooling. After the completion of the reaction, the solvent was concentrated, and an aqueous potassium carbonate solution was added to the residue. The mixture was extracted with chloroform, and the organic layer was dried over magnesium sulfate and concentrated. The obtained residue was purified by silica gel column chromatography. A chloroform:methanol=5:1 effluent fraction was concentrated, and the precipitated crystals were collected by filtration to give 3-(1-methyl-1,2,3,6-tetrahydropyridin-4-yl)-2H-isoquinolin-1-one (0.96 g). melting point: 222-223°C. ¹H-NMR(CDCl₃)δ: 2.42(3H,s), 2.55-2.59(2H,m), 2.66-2.69(2H,m), 3.16-3.20(2H,m), 6.30(1H,s), 6.46(1H,s), 7.41-7.45(1H,m), 7.50(1H,d,J=8Hz), 7.59-7.63(1H,m), 8.33(1H,d,J=8Hz), 9.37(1H,brs).

### Example 191

In the same manner as in Example 82, the reaction was carried out using N,N-diethyl-2-methylbenzamide (1.9 g) and 1-benzyl-3-cyanopiperidine (1.8 g) to give 3-(1-benzylpiperidin-3-yl)-2H-isoquinolin-1-one (1.64 g). melting point: 176-178°C

### Example 192

3-(1-Benzylpiperidin-3-yl)-2H-isoquinolin-1-one (1.5 g) was dissolved in methylene chloride (15 mL), and 1-chloroethyl chlorocarbonate (0.68 mL) was added under ice-cooling. The mixture was stirred at room temperature for 2 hr. The solvent was concentrated, and the residue was dissolved in methanol. The mixture was heated under reflux for 10 min. The solvent was concentrated, and hexane was added to the residue. The mixture was extracted with dilute hydrochloric acid, and an aqueous potassium carbonate solution was added to basify the aqueous layer. The mixture was extracted with chloroform. The solvent was concentrated, and the obtained residue was dissolved in acetonitrile (20 mL) and 37% aqueous formalin solution (1.4 mL). Then, sodium cyanoborohydride (0.33 g) and acetic acid (0.18 mL) were added under ice-cooling. After the completion of the reaction, the reaction solution was concentrated, and an aqueous potassium carbonate solution was added to the obtained residue. The mixture was extracted with chloroform. The organic layer was dried over magnesium sulfate, concentrated and purified by silica gel column chromatography. A chloroform:methanol=4:1 effluent fraction was concentrated, and the precipitated crystals were washed with diisopropyl ether to give 3-(1-methylpiperidin-3-yl)-2H-isoquinolin-1-one (0.18 g), melting point: 155-156°C. ¹H-NMR(CDCl₃)δ: 1.52-1.78(5H,m), 2.18-2.25(1H,m), 2.31(3H,s), 2.45-2.55(1H,s), 2.65-2.86(2H,m), 6.23(1H,s),7.36-7.44(2H,m), 7.55-7.60(1H,m), 8.35(1H,d,J=8Hz), 11.40(1H,brs).

### Example 193

In the same manner as in Example 82, the reaction was carried out using N,N-diethyl-2-methylbenzamide (1.7 g) and 1-methyl-5-cyano-1,2,3,6-tetrahydropyridine (1.8 g) to give 3-(1-methyl-1,2,3,6-tetrahydropyridin-5-yl)-2H-isoquinolin-1-one (0.1 g). melting point: 214-216°C. ¹H-NMR(CDCl₃) δ: 2.39-2.47(2H,m), 2.47(3H,s), 2.57-2.64(2H,m), 3.27-3.29(2H,m), 6.32-6.34(1H,m), 6.38(1H,s), 7.40-7.45(1H,m), 7.49(1H,d,J=8Hz), 7.59-7.63(1H,m), 8.33(1H,d,J=8Hz), 9.04(1H,brs).

### Example 194

In the same manner as in Example 1, the reaction was carried out using 3-chloro-2H-isoquinolin-1-one (1.0 g) and 2-hydroxymethylpiperazine (1.5 g) to give 3-(3-hydroxymethylpiperazin-1-yl)-2H-isoquinolin-1-one (0.8 g). melting point: >120°C/decomposition. ¹H-NMR(400MHz,DMSO-d₆)δ: 2.32(1H,t,J=10Hz), 2.50-2.65(1H,m), 2.70-2.80(2H,m), 2.93(1H,d,J=12Hz), 3.30-3.45(4H,m), 3.49(1H,d,J=11Hz), 4.65(1H,t,J=5Hz), 5.72(1H,s), 7.18(1H,t,J=8Hz), 7.42(1H,d,J=8Hz), 7.51(1H,t,J=8Hz), 7.98(1H,d,J=8Hz), 11.10(1H,br.s). MS (EI) :259 (M⁺)

### Example 195

In the same manner as in Example 1, the reaction was carried out using 3-chloro-2H-isoquinolin-1-one (1.0 g) and 1-ethyl-2-hydroxymethylpiperazine (1.5 g) to give 3-(4-ethyl-3-hydroxymethylpiperazin-1-yl)-2H-isoquinolin-1-one (0.6 g). ¹H-NMR(400MHz,DMSO-d₆) δ: 0.96(3H,t,J=7Hz), 2.30-2.50(3H,m), 2.65-2.90(2H,m), 3.25-3.65(6H,m), 4.55(1H,t,J=5Hz), 5.73(1H,s), 7.16(1H,t,J=8Hz), 7.40(1H,d,J=8Hz), 7.49(1H,t,J=8Hz), 7.97(1H,d,J=8Hz), 11.05(1H,br.s). MS (EI):287(M⁺)

### Example 196

In the same manner as in Example 1, the reaction was carried out using 3-chloro-2H-isoquinolin-1-one (1.0 g) and 2-hydroxymethyl-1-propylpiperazine (1.5 g) to give 3-(3-hydroxymethyl-4-propylpiperazin-1-yl)-2H-isoquinolin-1-one (0.6 g). melting point: 152-154°C. ¹H-NMR(400MHz,DMSO-d₆)δ: 0.84(3H,t,J=7Hz), 1.35-1.50(2H,m), 2.25-2.50(2H,m), 2.60-2.90(4H,m), 3.30-3.40(3H,m), 3.40-3.50(1H,m), 3.55-3.65(1H,m), 4.57(1H,t,J=5Hz), 5.75(1H,s), 7.19(1H,t,J=8Hz), 7.42(1H,d,J=8Hz), 7.52(1H,t,J=8Hz), 7.99(1H,d,J=8Hz), 11.06(1H,s). MS (EI) :301(M⁺)

### Example 197

In the same manner as in Example 1, the reaction was carried out using 3-chloro-2H-isoquinolin-1-one (1.0 g) and 1-benzyl-2-hydroxymethylpiperazine (1.7 g) to give 3-(4-benzyl-3-hydroxymethylpiperazin-1-yl)-2H-isoquinolin-1-one (0.7 g). melting point: 176-177°C. ¹H-NMR(400MHz,DMSO-d₆)δ: 2.27(1H,t,J=8Hz), 2.54(1H,br.s), 2.69(1H,d,J=14Hz), 2.84(1H,t,J=12Hz), 3.30-3.40(3H,m), 3.50-3.60(1H,m), 3.70-3.80(1H,m), 4.06(1H,d,J=14Hz), 4.69(1H,t,J=5Hz), 5.76(1H,s), 7.15-7.40(6H,m), 7.42(1H,d,J=8Hz), 7.50(1H,t,J=8Hz), 7.99(1H,d,J=8Hz), 11.07(1H,s). MS(EI):349(M⁺)

### Example 198

In the same manner as in Example 1, the reaction was carried out using 5-bromo-3-chloro-2H-isoquinolin-1-one (1.0 g) and 2-hydroxymethylpiperazine (1.4 g) to give 5-bromo-3-(3-hydroxymethylpiperazin-1-yl)-2H-isoquinolin-1-one (0.8 g). melting point: >230°C/decomposition.

### Example 199

5-Bromo-3-(3-hydroxymethylpiperazin-1-yl)-2H-isoquinolin-1-one (0.7 g) was dissolved in acetonitrile (20 mL) and 37% aqueous formalin solution (1.4 mL), and sodium cyanoborohydride (0.33 g) and acetic acid (0.18 mL) were added under ice-cooling. After the completion of the reaction, the reaction solution was concentrated, and an aqueous potassium carbonate solution was added to the obtained residue. The mixture was extracted with chloroform. The organic layer was dried over magnesium sulfate, concentrated and purified by silica gel column chromatography. A chloroform:methanol=4:1 effluent fraction was concentrated, and the precipitated crystals were washed with diisopropyl ether to give 5-bromo-3-(3-hydroxymethyl-4-methylpiperazin-1-yl)-2H-isoquinolin-1-one (0.5 g). melting point: 200-202°C. ¹H-NMR(400MHz,DMSO-d₆)δ: 2.12(1H,br.s), 2.20-2.30(1H,m), 2.24(3H,s), 2.63(1H,t,J=11Hz), 2.75-2.85(2H,m), 3.30-3.40(1H,m), 3.55-3.65(3H,m), 4.62(1H,t,J=5Hz), 5.77(1H,s), 7.09(1H,t,J=7Hz), 7.85(1H,d,J=7Hz), 8.02(1H,d,J=7Hz), 11.38(1H,s). MS(EI):351,353(M⁺)

### Example 200

In the same manner as in Example 1, the reaction was carried out using 3-chloro-2H-isoquinolin-1-one (1.0 g) and 4-piperidinopiperidine (1.4 g) to give 3-(4-piperidinopiperidin-1-yl)-2H-isoquinolin-1-one (0.8 g). melting point: >200°C/decomposition. ¹H-NMR(400MHz,DMSO-dₛ) δ: 1.30-1.60(8H,m), 1.76(2H,d,J=11Hz), 2.25-2.55(5H,m), 2.60(2H,t,J=11Hz), 3.68(2H,d,J=12Hz), 5.76(1H,s), 7.18(1H,t,J=8Hz), 7.41(1H,d,J=8Hz), 7.52(1H,t,J=8Hz), 7.98(1H,d,J=8Hz), 11.07(1H,brs). MS(EI):311(M⁺)

### Example 201

In the same manner as in Example 18, the reaction was carried out using methyl 2-methoxycarbonylphenylacetimidate hydrochloride (1.0 g) and 3-hydroxymethylpiperidine (0.8 g) to give 3-(3-hydroxymethylpiperidin-1-yl)-2H-isoquinolin-1-one (0.3 g). melting point: 143-144°C.

### Example 202

In the same manner as in Example 18, the reaction was carried out using methyl 2-methoxycarbonylphenylacetimidate hydrochloride (1.0 g) and 3-(dimethylcarbamoyl)piperidine (0.8 g) to give 3-(3-(dimethylcarbamoyl)piperidin-1-yl)-2H-isoquinolin-1-one (0.3 g). melting point: 187-188°C.

### Example 203

In the same manner as in Example 1, the reaction was carried out using 3-chloro-2H-isoquinolin-1-one (1.0 g) and 2-hydroxymethyl-1-isobutylpiperazine (1.4 g) to give 3-(3-hydroxymethyl-4-isobutylpiperazin-1-yl)-2H-isoquinolin-1-one (0.8 g) . melting point: 130-132°C. ¹H-NMR(400MHz,DMSO-d₆)δ: 0.84(3H,d,J=6Hz), 1.65-1.75(1H,m), 2.05(1H,dd,J=13,6Hz), 2.30(1H,t,J=8Hz), 2.40-2.55(2H,m), 2.80-3.00(3H,m), 3.25-3.40(2H,m), 3.40-3.50(1H,m), 3.50-3.60(1H,m), 4.60(1H,t,J=5Hz), 5.76(1H,s), 7.19(1H,t,J=8Hz), 7.42(1H,d,J=8Hz), 7.52(1H,t,J=8Hz), 7.99(1H,d,J=8Hz), 11.10(1H,s). MS(EI):315(M⁺)

### Example 204

The reaction was carried out in the same manner as in Example 82 using N,N-diethyl-2-methylbenzamide (3.82 g) and 5-(dimethylamino)pentanenitrile (3.03 g) to give 3-[4-(dimethylamino)butyl)-2H-isoquinolin-1-one (107 mg, 2%). ¹H-NMR(CDCl₃) d: 1.53-1.60(2H,m), 1.72-1.79(2H,m), 2.25(6H,s), 2.12-2.14(2H,m), 2.58-2.60(2H,m), 6.26(1H,s), 7.37-7.44(2H,m), 7.56-7.61(1H,m), 8.33(1H,d,J=8.0Hz).

### Example 205

In the same manner as in Example 1, the reaction was carried out using 3-chloro-5-fluoro-2H-isoquinolin-1-one (1.0 g) and 2-hydroxymethyl-1-methylpiperazine (1.4 g) to give 5-fluoro-3-(3-hydroxymethyl-4-methylpiperazin-1-yl)-2H-isoquinolin-1-one (0.4 g). melting point: 216-217°C. ¹H-NMR(400MHz,DMSO-d₆)δ: 2.10(1H,br.s), 2.20-2.30(1H,m), 2.23(3H,s), 2.59(1H,t,J=11Hz), 2.70-2.80(2H,m), 3.30-3.40(1H,m), 3.55-3.65(3H,m), 4.62(1H,t,J=6Hz), 5.67(1H,s), 7.17(1H,dd,J=8,5Hz), 7.41(1H,t,J=8Hz), 7.82(1H,d,J=8Hz), 11.33(1H,s). MS (EI) :291 (M⁺)

### Example 206

In the same manner as in Example 18, the reaction was carried out using methyl 2-methoxycarbonylphenylacetimidate hydrochloride (1.0 g) and 3-(dimethylaminomethyl)piperidine (0.7 g) to give 3-(3-(dimethylaminomethyl)piperidin-1-yl)-2H-isoquinolin-1-one (0.5 g). melting point: 141-143°C. ¹H-NMR(400MHz,DMSO-d₆)δ: 1.02 (1H,q,J=11Hz), 1.75-2.00 (4H,m) , 2.00-2.10(1H,m), 2.13(6H,s), 2.35(1H,t,J=11Hz), 2.66(1H,t,J=11Hz), 3.45-3.60(2H,m), 5.72(1H,s), 7.16(1H,t,J=8Hz), 7.41(1H,d,J=8Hz), 7.50(1H,t,J=8Hz), 7.97(1H,d,J=8Hz), 11.16(1H,s). MS (EI) : 285 (M⁺)

### Example 207

In the same manner as in Example 82, the reaction was carried out using N,N-diethyl-2,4-dimethylbenzamide (1.0 g) and 4-cyano-1-methylpiperidine (0.8 g) to give 6-methyl-3-(1-methylpiperidin-4-yl)-2H-isoquinolin-1-one (0.8 g). melting point: 236-238°C. ¹H-NMR(400MHz,CDCl₃)δ: 10.23(brs,1H), 8.23-8.19(m,1H), 7.25-7.20(m,2H), 6.23(s,1H), 3.03-2.90(m,2H), 2.51-2.40(m,2H), 2.44(s,3H), 2.32(s,3H), 2.20-1.91(m,4H), 1.90-1.70 (m, 2H) . MS (EI):256 (M+)

### Example 208

In the same manner as in Example 82, the reaction was carried out using N,N-diethyl-2,5-dimethylbenzamide (1.0 g) and 4-cyano-1-methylpiperidine (0.8 g) to give 7-methyl-3-(1-methylpiperidin-4-yl)-2H-isoquinolin-1-one (0.3 g). melting point: 239-241°C. ¹H-NMR(400MHz,CDCl₃)δ: 10.50(brs,1H), 8.13(s,1H), 7.45-7.35 (m,2H) , 6.28(s,1H), 3.01(d,J=11.4,2H), 2.57-2.41(m,1H), 2.46(s,3H), 2.35(s,3H), 2.14(t,J=11.7,2H), 2.02(d,J=13.4,2H), 1.86-1.75(m,2H). MS(EI):256(M+)

### Example 209

In the same manner as in Example 82, the reaction was carried out using N,N-diethyl-2,6-dimethylbenzamide (1.0 g) and 4-cyano-1-methylpiperidine (0.8 g) to give 8-methyl-3-(1-methylpiperidin-4-yl)-2H-isoquinolin-1-one (0.2 g). melting point: 200-202°C. ¹H-NMR(400MHz,CDCl₃)δ: 11.33(brs,1H), 7.42(t,J=7.8,1H), 7.29(d,J=7.8,1H), 7.13(d,J=7.0,1H), 6.23(s,1H), 2.98(d,J=12.2,2H), 2.92(s,3H), 2.56-1.92(m,1H), 2.31(s,3H), 2.07(t,J=10.7,4H), 1.83-1.72(m,2H). MS(EI):256(M+)

### Example 210

In the same manner as in Example 82, the reaction was carried out using N,N-diethyl-5-methoxymethyloxy-2-methylbenzamide (1.0 g) and 4-cyano-1-methylpiperidine (0.6 g) to give 7-methoxymethyloxy-3-(1-methylpiperidin-4-yl)-2H-isoquinolin-1-one (0.6 g). melting point: 200-202°C. ¹H-NMR(400MHz,CDCl₃)δ: 9.86(brs,1H), 7.92(d,J=2.7,1H), 7.43(d,J=8.8,1H), 7.32(dd,J=6.1,2.4,1H), 6.27(s,1H), 5.26(s,2H), 3.48(s,3H), 3.02(d,J=11.2,2H), 2.50-2.38(m,1H), 2.36(s,3H), 2.21-2.08(m,2H), 2.06-1.95(m,2H), 1.89-1.75(m,2H). MS(EI):302(M+)

### Example 211

In the same manner as in Example 108, the reaction was carried out using 7-methoxymethyloxy-3-(1-methylpiperidin-4-yl)-2H-isoquinolin-1-one (0.4 g) to give 7-hydroxy-3-(1-methylpiperidin-4-yl)-2H-isoquinolin-1-one hydrochloride (0.3 g). melting point: 284-286°C. ¹H-NMR(400MHz,DMSO-d₆)δ: 11.11(brs,1H), 10.46(brs,1H), 9.87(s,1H), 7.50-7.44(m,2H), 7.14(dd,J=5.9,2.7,1H), 6.23(s,1H), 3.47(d,J=11.7,2H), 3.06-2.91(m,2H), 2.74(s,3H), 2.62(t,J=12.0,1H), 2.14(d,J=13.4,2H), 1.93-1.79(m,2H). MS(EI):258(M+)

### Example 212

In the same manner as in Example 1, the reaction was carried out using 3-chloro-5-methyl-2H-isoquinolin-1-one (3.0 g) and (S)-2-hydroxymethylpiperazine (5.0 g) to give (S)-3-(3-hydroxymethylpiperazin-1-yl)-5-methyl-2H-isoquinolin-1-one (3.4 g). (S)-3-(3-Hydroxymethylpiperazin-1-yl)-5-methyl-2H-isoquinolin-1-one (3.4 g) was dissolved in acetonitrile (60 mL) and 37% aqueous formalin solution (7.4 mL), and sodium cyanoborohydride (2.6 g) and acetic acid (0.74 mL) were added under ice-cooling. After the completion of the reaction, the reaction solution was concentrated, and an aqueous potassium carbonate solution was added to the obtained residue. The mixture was extracted with chloroform, and the organic layer was dried over magnesium sulfate, concentrated and purified by silica gel column chromatography. A chloroform:methanol=4:1 effluent fraction was concentrated, and the precipitated crystals were washed with diisopropyl ether to give (S)-3-(3-hydroxymethyl-4-methylpiperazin-1-yl)-5-methyl-2H-isoquinolin-1-one (2.1 g). melting point: 184-186°C. ¹H-NMR(400MHz,DMSO-d₆)δ: 2.11(1H,br.s), 2.20-2.30(1H,m), 2.23(3H,s), 2.38(3H,s), 2.57(1H,t,J=10Hz), 2.70-2.85(2H,m), 3.30-3.40(1H,m), 3.50-3.65(3H,m), 4.61(1H,t,J=5Hz), 5.62(1H,s), 7.08(1H,t,J=8Hz), 7.38(1H,d,J=8Hz), 7.86(1H,d,J=8Hz), 11.17(1H,s). MS (EI) : 287 (M⁺)

### Example 213

In the same manner as in Example 82, the reaction was carried out using N,N-diethyl-2-methylbenzamide (1.5 g) and 4-(pyrrolidin-1-yl)butyronitrile (1.1 g) to give 3-(3-(pyrrolidin-1-yl)propyl)-2H-isoquinolin-1-one (0.2 g). melting point: 117-119°C.

### Example 214

In the same manner as in Example 1, the reaction was carried out using 3,5-dichloro-2H-isoquinolin-1-one (1.0 g) and 1-methyl-2-hydroxymethylpiperazine (1.4 g) to give 5-chloro-3-(3-hydroxymethyl-4-methylpiperazin-1-yl)-2H-isoquinolin-1-one (0.5 g). melting point: 212-214°C. ¹H-NMR(400MHz,DMSO-d₆)δ: 2.11(1H,br.s), 2.20-2.30(1H,m), 2.24(3H,s), 2.62(1H,t,J=11Hz), 2.75-2.85(2H,m), 3.30-3.40(1H,m), 3.55-3.70(3H,m), 4.64(1H,t,J=5Hz), 5.79(1H,s), 7.16(1H,t,J=8Hz), 7.69(1H,d,J=8Hz), 7.98(1H,d,J=8Hz), 11.40(1H,s). MS(EI):307,309(M⁺)

### Example 216

The reaction was carried out in the same manner as in Example 82 using N,N-diethyl-2-methylbenzamide (3.82 g) and 4-(piperidin-1-yl)butyronitrile (3.65 g) to give 3-[3-(piperidin-1-yl)propyl]-2H-isoquinolin-1-one (326 mg, 6%). ¹H-NMR(CDCl₃) d: 1.52(2H,m), 1.87(6H,m), 2.51(6H,m), 2.69-2.72(2H,m), 6.27(1H,s), 7.39-7.46(2H,m), 7.58-7.62(1H,m), 8.35(1H,d,J=8.0Hz), 11.98(1H,m).

### Example 217

In the same manner as in Example 189, the reaction was carried out using N,N-diethyl-3-methoxymethyloxy-2-methylbenzamide (1.5 g) and 4-(pyrrolidin-1-yl)butyronitrile (1.0 g) to give 5-hydroxy-3-(3-(pyrrolidin-1-yl)propyl)-2H-isoquinolin-1-one (0.2 g). melting point: 222-225°C.

### Example 218

In the same manner as in Example 82, the reaction was carried out using N,N-diethyl-2,3-dimethylbenzamide (8.20 g) and 3-(piperidin-1-yl)propionitrile (6.63 g) to give 5-methyl-3-[2-(piperidin-1-yl)ethyl)-2H-isoquinolin-1-one (410 mg, 4%). ¹H-NMR(CDCl₃) d: 1.52-1.57(2H,m), 1.69-1.77(4H,m), 2.48(3H,s), 2.50-2.53(4H,m), 2.67-2.69(4H,m), 6.30(1H,s), 7.28-7.31(1H,m), 7.41-7.44(1H,m), 8.23(1H,d,J=8.1Hz), 11.47(1H,m).

### Example 219

In the same manner as in Example 82, the reaction was carried out using N,N-diethyl-2-methylbenzamide (3.82 g) and 3-(piperidin-1-yl)propionitrile (3.32 g) to give 3-[2-(piperidin-1-yl)ethyl]-2H-isoquinolin-1-one (151 mg, 3%). ¹H-NMR(CDCl₃) d: 1.51-1.56(2H,m), 1.69-1.77(4H,m), 2.53(4H,m), 2.67-2.71 (4H,m), 6.19(1H,s), 7.36-7.43 (2H,m) , 7.55-7.61(1H,m), 8.34-8.37(1H,m), 11.51(1H,m).

### Example 220

The reaction was carried out in the same manner as in Example 82 using N,N-diethyl-2-methylbenzamide (3.82 g) and 3-(pyrrolidin-1-yl)propionitrile (2.98 g) to give 3-[2-(pyrrolidin-1-yl)ethyl]-2H-isoquinolin-1-one (186 mg, 4%). ¹H-NMR(CDCl₃) d: 1. 95 (4H,m) , 2.85-3.01(8H,m), 6.29(1H,s), 7.38-7.46(2H,m), 7.57-7.64(1H,m), 8.34(1H,d,J=8.1Hz), 11.44(1H,m).

### Example 221

The reaction was carried out in the same manner as in Example 82 using N,N-diethyl-2,3-dimethylbenzamide (4.10 g) and 3-(pyrrolidin-1-yl)propionitrile (2.98 g) to give 5-methyl-3-[2-(pyrrolidin-1-yl)ethyl)-2H-isoquinolin-1-one (52 mg, 0.4%).
¹H-NMR(CDCl₃) d: 2.19(4H,m), 2.52(3H,s), 3.29(4H,m), 3.50(4H,m), 6.63(1H,s), 7.33-7.39(1H,m), 7.49(1H,d,J=6.8Hz), 8.17(1H,d,J=7.8Hz).

### Example 222

The reaction was carried out in the same manner as in Example 82 using N,N-diethyl-2,3-dimethylbenzamide (5.64 g) and 4-(pyrrolidin-1-yl)butyronitrile (4.62 g) to give 5-methyl-3-[3-(pyrrolidin-1-yl)propyl-1-yl]-2H-isoquinolin-1-one (453 mg, 6%).
¹H-NMR(CDCl₃) d: 1.85-1.99 (6H,m) , 2.50(3H,s), 2.60-2.76(8H,m), 6.35(1H,s), 7.27-7.32(1H,m), 7.43(1H,d,J=6.8Hz), 8.22(1H,d,J=7.8Hz), 12.06(1H,m).

### Example 223

3-Amino-1,5-dihydro-6-(4-pyridyl)pyrazolo[4,3-c]pyridin-4-one (2.2 g) and phosphoric acid (3.3 g) were suspended in water (40 mL), and an aqueous sodium nitrite (0.73 g) solution (2 mL) was added dropwise under ice-cooling. After the completion of the dropwise addition, the mixture was stirred at room temperature for 3 hr. After the completion of the reaction, the reaction solution was alkalified by addition of an aqueous potassium carbonate solution, adsorbed to silica gel and purified by silica gel column chromatography. A chloroform:methanol=4:1 effluent fraction was concentrated to give 1,5-dihydro-6-(4-pyridyl)pyrazolo[4,3-c]pyridin-4-one (0.42 g). ¹H-NMR(DMSO-d₆) δ: 6.95(1H,s), 7.78(2H,d,J=6Hz), 8.16(1H,s), 8.67(2H,d,J=6Hz).

The reaction was carried out in the same manner as in Example 190 using 1,5-dihydro-6-(4-pyridyl)pyrazolo[4,3-c]pyridin-4-one (0.42 g) to give 1,5-dihydro-6-(1-methyl-1,2,3,6-tetrahydropyridin-4-yl)pyrazolo[4,3-c]pyridin-4-one (0.13 g). ¹H-NMR(DMSO-d₆)δ: 2.27(3H,s), 2.40-2.54(4H,m), 6.36(1H,s), 6.44-6.49(1H,m), 8.01(1H,s), 10.48(1H,brs).

### Example 224

In the same manner as in Example 82, the reaction was carried out using N,N,N,N-tetramethyl-2-methylisophthalamide (9.6 g) and 4-cyano-1-methylpiperidine (5.1 g) to give N,N-dimethyl-3-(1-methylpiperidin-1-yl)-2H-1-oxoisoquinoline-5-carboxamide 1/4 water adduct (7.4 g). ¹H-NMR(CDCl₃)δ: 1.70-1.92(2H,m), 1.95-2.12(4H,m), 2.35(3H,s), 2.44-2.56(1H,m), 2.83(3H,s), 2.95-3.06(2H,m), 3.22(3H,s), 6.27(1H,s), 7.46(1H,t,J=8Hz), 7.57(1H,dd,J=1Hz,7Hz), 8.41(1H,ddd,J=1Hz,2Hz,8Hz), 10.37(1H,brs). MS(EI):313(M+).

### Example 225

Octahydroindolizin-7-one (11.5 g) and p-toluenesulfonylmethyl isocyanide (15.0 g) were dissolved in dimethoxyethane (200 mL) and ethanol (5.4 mL), and potassium t-butoxide (16.6 g) was added under ice-cooling. After the completion of the reaction, the solvent was concentrated, and an aqueous potassium carbonate solution was added to the obtained residue. The mixture was extracted with chloroform, and the extract was dried over magnesium sulfate. The solvent was concentrated to give 7-cyanooctahydroindolizine as a diastereomer mixture. This mixture was purified by silica gel column chromatography. From ethyl acetate:methanol 5:1 and 4:1 effluent fractions, low polarity isomer (A) (2.0 g) and high polarity isomer (B) (4.1 g), respectively, were obtained as an oil.
Low polarity isomer (A) ¹H-NMR(CDCl₃)δ: 1.43-2.24(13H,m), 2.40-2.48(1H,m), 3.02-3.18(2H,m).
High polarity isomer (B) ¹H-NMR(CDCl₃)δ: 1.35-1.53 (2H,m), 1.68-2.25(8H,m), 2.32-2.44(1H,m), 3.03-3.14(3H,m).

The reaction was carried out in the same manner as in Example 82 using N,N-dimethyl-2,3-dimethylbenzamide (1.4 g) and low polarity isomer (A) (0.75 g) of 7-cyanooctahydroindolizine to give 5-methyl-3-(octahydroindolizin-7-yl)-2H-isoquinolin-1-one 3/4 water adduct (0.2 g). ¹H-NMR(DMSO-d₆)δ: 1.32-2.25(11H,m), 2.47(3H,s), 2.49-2.61(1H,m), 2.97-3.25(2H,m), 6.35(1H,s), 7.28(1H,t,J=8Hz), 7.49(1H,d,J=7Hz), 8.00(1H,d,J=8Hz), 11.14(1H,brs). MS (EI) : 282 (M+).

### Example 226

The reaction was carried out in the same manner as in Example 82 using N,N-dimethyl-2,3-dimethylbenzamide (1.4 g) and high polarity isomer (B) (0.75 g) of 7-cyanooctahydroindolizine to give 5-methyl-3-(octahydroindolizin-7-yl)-2H-isoquinolin-1-one 1/2 water adduct (0.3 g). ¹H-NMR(DMSO-d₆)δ: 1.30-2.25(11H,m), 2.47(3H,s), 2.49-2.65(1H,m), 2.92-3.21(2H,m), 6.35(1H,s), 7.28(1H,t,J=8Hz), 7.49(1H,d,J=7Hz), 8.00(1H,d,J=8Hz), 11.14(1H,brs). MS (EI) : 282 (M+).

### Example 227

N,N-Dimethyl-3-(1-methylpiperidin-1-yl)-2H-1-oxoisoquinoline-5-carboxamide (5.4 g) was dissolved in conc. hydrochloric acid (100 mL), and the mixture was heated under reflux. After the completion of the reaction, the solvent was concentrated, and acetone was added to the obtained residue. The precipitated crystals were collected by filtration to give 3-(1-methylpiperidin-1-yl)-2H-1-oxoisoquinoline-5-carboxylic acid hydrochloride. ¹H-NMR(DMSO-d₆)δ: 1.80-2.01 (2H,m) , 2.10-2.24(2H,m), 2.76(3H,s), 2.95-3.11(2H,m), 3.20-3.31(1H,m), 3.24-3.57(2H,m), 7.36(1H,s), 7.52(1H,t,J=8Hz), 8.28(1H,dd,J=2Hz,8Hz), 8.40(1H,d,J=8Hz), 10.51(1H,brs), 11.58(1H,brs), 13.25(1H,brs).

### Example 228

The reaction was carried out in the same manner as in Example 82 using N,N-diethyl-2,3-dimethylbenzamide (4.10 g) and 4-(piperidin-1-yl)butyronitrile (3.65 g) to give 5-methyl-3-[3-(piperidin-1-yl)propyl]-2H-isoquinolin-1-one (196 mg, 4%).
¹H-NMR(CDCl₃) d: 1.55-1.56(2H,m), 1.80-1.90(6H,m), 2.39-2.43(6H,m), 2.49(3H,s), 2.68-2.73(2H,m), 6.33(1H,s), 7.26-7.28(1H,m), 7.42(1H,d,J=7.3Hz), 8.23(1H,d,J=8.4Hz).

### Example 229

The reaction was carried out in the same manner as in Example 82 using N,N-diethyl-2-methylbenzamide (3.82 g) and 2-dimethylaminoacetonitrile (2.02 g) to give 3-(dimethylamino)methyl-2H-isoquinolin-1-one (1.20 g, 32%). ¹H-NMR(CDCl₃) d: 2.29(6H,s), 3.36(2H,m), 6.32(1H,s), 7.42-7.49(2H,m), 7.60-7.66(1H,m), 8.38(1H,d,J=8.4Hz), 9.22(1H,m).

### Example 230

The reaction was carried out in the same manner as in Example 82 using N,N-diethyl-2-methylbenzamide (3.82 g) and (4-methylpiperazin-1-yl)acetonitrile (3.34 g) to give 3-[(4-methylpiperazin-1-yl)methyl]-2H-isoquinolin-1-one (1.83 g, 36%).
¹H-NMR(CDCl₃) d: 2.31(3H,s), 2.51-2.54(4H,m), 3.44(2H,m), 6.35(1H,s), 7.42-7.49(2H,m), 7.60-7.66(1H,m), 8.37(1H,d,J=8.1Hz), 9.13(1H,m).

### Example 231

The reaction was carried out in the same manner as in Example 82 using N,N-diethyl-2-methylbenzamide (3.82 g) and N-cyanomethylpiperidine (2.98 g) to give 3-(piperidinomethyl)-2H-isoquinolin-1-one (2.03 g, 43%).
¹H-NMR(CDCl₃) d: 1.46-1.64 (6H,m) , 2.40-2.43 (4H,m) , 3.37(2H,s), 6.31(1H,s), 7.41-7.48(2H,m), 7.59-7.65(1H,m), 8.37(1H,d,J=7.6Hz), 9.24(1H,m).

### Example 232

The reaction was carried out in the same manner as in Example 82 using N,N-diethyl-2-methylbenzamide (3.82 g) and N-morpholinoacetonitrile (3.03 g) to give 3-[(morpholin-1-yl)methyl]-2H-isoquinolin-1-one (1.38 g, 29%).
¹H-NMR(CDCl₃) d: 2.49-2.53(4H,m), 3.43-3.47(2H,m), 3.72-3.76(4H,m), 6.35(1H,s), 7.43-7.49(2H,m), 7.61-7.67(1H,m), 8.36(1H,d,J=9.9Hz), 9.12(1H,m).

### Example 233

The reaction was carried out in the same manner as in Example 82 using N,N-diethyl-2-methylbenzamide (3.82 g) and (homopiperidin-2-yl)acetonitrile (3.32 g) to give 3-[(homopiperidin-1-yl)methyl]-2H-isoquinolin-1-one (2.54 g, 50%).
¹H-NMR(CDCl₃) d: 1.62-1.65(8H,m), 2.63-2.65(4H,m), 3.52-3.54(2H,m), 7.24(1H,s), 7.41-7.47(2H,m), 7.59-7.65(1H,m), 8.38(1H,d,J=7.9Hz), 9.25(1H,m).

### Example 234

The reaction was carried out in the same manner as in Example 82 using N,N-diethyl-2-methylbenzamide (3.82 g) and 4-(homopiperidin-1-yl)butyronitrile (3.99 g) to give 3-[3-(homopiperidin-1-yl)propyl]-2H-isoquinolin-1-one (2.12 g, 38%).
¹H-NMR(CDCl₃) d: 1.56-1.84(11H,m), 2.54-2.58(2H,m), 2.65-2.76(5H,m), 6.23(1H,s), 7.36-7.44(2H,m), 7.55-7.61(1H,m), 8.35(1H,d,J=7.8Hz), 11.84(1H,m).

### Example 235

3-(Piperidin-4-yl)-5-methyl-2H-isoquinolin-1-one (0.73 g) and sulfamide (0.32 g) were dissolved in 1,3-dimethylimidazolin-2-one (1.4 mL), and the mixture was refluxed under heating at 130°C for 6 hr. After the completion of the reaction, the reaction solution was purified by silica gel column chromatography. A chloroform:methanol 10:1 effluent fraction was concentrated, and the precipitated crystals were washed with ethyl acetate to give 3-(1-sulfamoylpiperidin-4-yl)-5-methyl-2H-isoquinolin-1-one 1/4 water adduct (0.57 g).
¹H-NMR(DMSO-d₆)δ: 1.68-1.84(2H,m), 1.94-2.05(2H,m) , 2.47(3H,s), 2.51-2.60(3H,m), 3.56-3.65(2H,m), 6.38(1H,s), 6.70(2H,s), 7.29(1H,t,J=8Hz), 7.49(1H,d,J=7Hz), 8.00(1H,d,J=8Hz), 11.17(1H,brs).

### Example 236

In the same manner as in Example 1, the reaction was carried out using 3-chloro-5-methyl-2H-isoquinolin-1-one (1.0 g) and 1-methylpiperazin-2-one (2.0 g) to give 3-(4-methyl-3-oxopiperazin-1-yl)-5-methyl-2H-isoquinolin-1-one 1/10 water adduct (0.7 g). ¹H-NMR(DMSO-d₆)δ: 2. 41 (3H, s) , 2.90(3H,s), 3.37-3.52(4H,m), 3.81(2H,s), 5.68(1H,s), 7.12(1H,t,J=8Hz), 7.40(1H,d,J=6Hz), 7.90(1H,d,J=8Hz), 11.21(1H,brs).
MS (EI):271(M+).

### Example 237

3-(Piperidin-4-yl)-5-methyl-2H-isoquinolin-1-one (6.3 g), oxalic acid 2 hydrate (6.6 g) and sodium nitrite (3.6 g) were suspended in dimethylformamide, and the mixture was stirred at room temperature for 2 hr. After the completion of the reaction, an aqueous potassium carbonate solution was added to the reaction solution. The mixture was extracted with a mixed solvent of chloroform and methanol and dried over magnesium sulfate. The solvent was concentrated to give 3-(1-nitrosopiperidin-4-yl)-5-methyl-2H-isoquinolin-1-one (7.0 g) as pale yellow crystals. 3-(1-Nitrosopiperidin-4-yl)-5-methyl-2H-isoquinolin-1-one (7.0 g) was dissolved in acetic acid (70 mL), and a zinc powder (6.7 g) was added under ice-cooling. The reaction solution was stirred with heating at 100°C for 3 hr and cooled. The reaction solution was filtered through celite, and the filtrate was concentrated. The obtained residue was alkalified by addition of 1N aqueous sodium hydroxide solution, and the mixture was extracted with a mixed solvent of chloroform-methanol. The extract was dried over magnesium sulfate, and the solvent was concentrated. The obtained residue was purified by silica gel column chromatography, and a chloroform:methanol 2:1 effluent fraction was concentrated. The obtained crystals were washed with ethyl acetate to give 3-(1-aminopiperidin-4-yl)-5-methyl-2H-isoquinolin-1-one (4.6 g). ¹H-NMR (DMSO-d₆)δ: 1.67-1.90(4H,m), 2.05-2.16(2H,m), 2.34-2.43(1H,m), 2.46(3H,s), 3.02-3.10(2H,m), 3.44(2H,brs), 6.34(1H,s), 7.29(1H,t,J=8Hz), 7.49(1H,d,J=7Hz), 7.99(1H,d,J=8Hz), 11.23(1H,brs).
MS(EI):257(M+).

### Example 238

3-(1-Aminopiperidin-4-yl)-5-methyl-2H-isoquinolin-1-one (0.77 g) was dissolved in pyridine (10 mL), and methanesulfonyl chloride (0.28 mL) was added dropwise under ice-cooling. After the completion of the reaction, water was added to the reaction solution, and the mixture was extracted with a mixed solvent of chloroform-methanol. The extract was dried over magnesium sulfate, and the solvent was concentrated. The obtained residue was purified by silica gel column chromatography. A chloroform:methanol 30:1 effluent fraction was concentrated, and the obtained crystals were washed with ethyl acetate to give 3-(1-(methanesulfonylamino)piperidin-4-yl)-5-methyl-2H-isoquinolin-1-one (0.55 g). ¹H-NMR(DMSO-d₆)δ: 1.79-1.91 (4H,m), 2.47(3H,s), 2.51-2.54(1H,m), 2.55-2.69(2H,m), 2.93(3H,s), 3.15-3.23(2H,m), 6.37(1H,s), 7.29(1H,t,J=8Hz), 7.49(1H,d,J=7Hz), 8.00(1H,d,J=8Hz), 8.21(1H,s), 11.11(1H,brs). MS(EI):335(M+).

### Example 239

In the same manner as in Example 238, the reaction was carried out using 3-(1-aminopiperidin-4-yl)-5-methyl-2H-isoquinolin-1-one (0.77 g) and trifluoroacetyl chloride (0.51 mL) to give 3-(1-trifluoroacetoaminopiperidin-4-yl)-5-methyl-2H-isoquinolin-1-one (0.67 g). ¹H-NMR(DMSO-d₆)δ: 1.79-2.00(4H,m), 2.48(3H,s), 2.51-2.58(1H,m), 2.71-2.83(2H,m), 3.02-3.12(2H,m), 6.39(1H,s), 7.29(1H,t,J=8Hz), 7.49(1H,d,J=7Hz), 8.00(1H,d,J=8Hz), 10.44(1H,s), 11.14(1H,brs). MS(EI):353(M+).

### Example 240

The reaction was carried out in the same manner as in Example 82 using N,N-diethyl-2-methylbenzamide (3.82 g) and 3-(homopiperidin-1-yl)propionitrile (3.65 g) to give 3-[2-(homopiperidin-1-yl)ethyl]-2H-isoquinolin-1-one (353 mg, 7%). ¹H-NMR(CDCl₃) d: 1.60-1.61(7H,m), 2.61-2.65(2H,m), 2.74-2.86(7H,m), 6.18(1H,s), 7.36-7.40(2H,m), 7.46-7.58(1H,m), 8.35(1H,d,J=7.8Hz), 11.78(1H,m).

### Example 241

2-Hydroxyimino-4-methyl-1-indanone (16.3 g) and p-toluenesulfonyl chloride (19.6 g) were suspended in aqueous sodium hydroxide solution (8.7 g/127 mL), and the mixture was stirred at 50°C for 3 hr. The reaction solution was acidified by the addition of an aqueous citric acid solution and extracted with ethyl acetate. The organic layer was dried over magnesium sulfate, and the solvent was concentrated. Ethyl ether was added to the obtained residue, and the precipitated crystals were collected by filtration to give 2-cyanomethyl-3-methylbenzoic acid (11.8 g). ¹H-NMR(DMSO-d₆)δ: 2.42(3H,s), 4.20(2H,s), 7.37(1H,t,J=8Hz), 7.50(1H,d,J=8Hz), 7.76(1H,d,J=8Hz), 13.28(1H,brs).

2-Cyanomethyl-3-methylbenzoic acid (15.3 g) was suspended in methylene chloride (150 mL), and dimethylformamide (0.1 mL) was added. Oxalyl chloride (9.2 mL) was added dropwise under ice-cooling. The reaction solution was stirred at room temperature for 2 hr, and the solvent was concentrated. The residue was dissolved in tetrahydrofuran (100 mL) and added dropwise to 28% aqueous ammonia under ice-cooling. The reaction solution was heated under reflux for 30 min. and cooled, and the solvent was concentrated. The precipitated crystals were collected by filtration to give 3-amino-5-methyl-2H-isoquinolin-1-one (9.4 g).
¹H-NMR(DMSO-d₆)δ: 2.28(3H,s), 5.46(1H,s), 5.60(2H,s), 6.88(1H,t,J=8Hz), 7.27(1H,d,J=8Hz), 7.78(1H,d,J=8Hz), 10.63(1H,brs).

3-Amino-5-methyl-2H-isoquinolin-1-one (0.87 g) and N,N-dimethylaminoglycine hydrochloride (0.91 g) were suspended in pyridine (2.0 mL) and methylene chloride (20 mL), and 2-chloro-1,3-dimethylimidazolinium chloride (1.1 g) was added under ice-cooling. After the completion of the reaction, the reaction solution was dissolved in a mixed solvent of chloroform-methanol and washed with an aqueous potassium carbonate solution. The mixture was dried over magnesium sulfate, and the solvent was concentrated. The obtained residue was purified by silica gel column chromatography, and a chloroform:methanol=30:1 effluent fraction was concentrated. The precipitated crystals were collected by filtration to give N-(5-methyl-2H-1-oxoisoquinolin-3-yl)-2-(dimethylamino)acetamide (0. 5 g). ¹H-NMR(DMSO-d₆)δ: 2.31(6H,s), 2.41(3H,s), 3.12(2H,s), 7.04(1H,s), 7.22(1H,t,J=8Hz), 7.48(1H,d,J=8Hz), 7.96(1H,d,J=8Hz), 10.11(1H,brs), 11.40(1H,brs). MS(EI):259(M+).

### Example 243

In the same manner as in Example 241, the reaction was carried out using 3-amino-5-methyl-2H-isoquinolin-1-one (0.87 g) and 3-(dimethylamino)propionic acid hydrochloride (1.0 g) to give N-(5-methyl-2H-1-oxoisoquinolin-3-yl)-3-(dimethylamino)propanamide (0.4 g). ¹H-NMR(CDCl₃)δ: 2.435(6H,s), 2.443 (3H,s) , 2.50-2.55(2H,m), 2.66-2.71(2H,m), 5.79(1H,s), 7.22(1H,t,J=8Hz), 7.38-7.41(1H,m), 8.20(1H,d,J=8Hz), 11.62(1H,brs), 12.62(1H,brs).
MS(EI):273(M+).

### Example 244

In the same manner as in Example 82, 3-(1-aminopiperidin-4-yl)-5-methyl-2H-isoquinolin-1-one (1.0 g) was subjected to reductive methylation to give 3-(1-dimethylaminopiperidin-4-yl)-5-methyl-2H-isoquinolin-1-one (0.3 g). ¹H-NMR(DMSO-d₆)δ: 1.61-1.87(2H,m), 1.90-2.00(2H,m), 2.29(6H,m), 2.30-2.40(3H,m), 2.46(3H,s), 2.93-3.02(2H,m), 6.34(1H,s), 7.28(1H,t,J=8Hz), 7.49(1H,d,J=7Hz), 7.99(1H,d,J=8Hz), 11.23(1H,brs).
MS(EI):285(M+).

### Example 245

In the same manner as in Example 241, the reaction was carried out using 3-amino-5-methyl-2H-isoquinolin-1-one (0.87 g) and 4-(dimethylamino)butanoic acid hydrochloride (1.1 g) to give N-(5-methyl-2H-1-oxoisoquinolin-3-yl)-4-(dimethylamino) butanamide (0.5 g). ¹H-NMR(CDCl₃)δ: 1.83-1.92(2H,m), 2.42(3H,s), 2.43(6H,s), 2.54-2.63(4H,m), 5.76(1H,s), 7.20(1H,t,J=8Hz), 7.39(1H,d,J=7Hz), 8.20(1H,d,J=8Hz), 11.81(1H,brs), 12.79(1H,brs).
MS(EI):287(M+).

### Example 246

In the same manner as in Example 241, the reaction was carried out using 3-amino-2H-isoquinolin-1-one (0.8 g) and 4-(dimethylamino)butanoic acid hydrochloride (1.1 g) to give N-(2H-1-oxoisoquinolin-3-yl)-4-(dimethylamino)butanamide (0.6 g). ¹H-NMR(CDCl₃)δ: 1.82-1.90 (2H,m) , 2.41(6H,s), 2.53-2.66(4H,m), 5.68(1H,s), 7.25-7.37(2H,m), 7.54(1H,t,J=7Hz), 8.32(1H,d,J=8Hz), 11.86(1H,brs), 12.68(1H,brs).
MS(EI):273(M+).

### Example 247

N-(t-Butoxycarbonylmethyl)-N-methylethylenediamine (14.2 g) and sodium bicarbonate (6.3 g) were dissolved in methanol (200 mL), and methyl 2-methoxycarbonylphenylacetimidate hydrochloride (16 g) was added under ice-cooling. After the completion of the reaction, the solvent was concentrated, and the obtained residue was dissolved in chloroform. The solution was washed with an aqueous potassium carbonate solution. The organic layer was dried over potassium carbonate, and the solvent was concentrated. The residue was purified by silica gel column chromatography, and a chloroform:methanol 40:1 effluent fraction was concentrated. The precipitated crystals were collected by filtration to give 3-(2-(N-(t-butoxycarbonylmethyl)-N-methylamino)ethylamino)-2H-isoquinolin-1-one (6. 6 g). ¹H-NMR(CDCl₃)δ: 1.48(9H,s), 2.45(3H,s), 2.77-2.86(2H,m), 3.15-3.23(2H,m), 3.27(2H,s), 5.20-5.28(1H,m), 5.46(1H,s), 7.11(1H,t,J=8Hz), 7.28(1H,d,J=8Hz), 7.46(1H,t,J=8Hz), 8.21(1H,dd,J=1Hz,8Hz).

3-(2-(N-(t-Butoxycarbonylmethyl)-N-methylamino)ethylamino)-2H-isoquinolin-1-one (2.0 g) was dissolved in methanol (20 mL), and potassium carbonate (1.0 g) was added. The mixture was heated under reflux for 4 hr. After the completion of the reaction, the solvent was concentrated, and the obtained residue was dissolved in chloroform and washed with an aqueous potassium carbonate solution. The organic layer was dried over potassium carbonate, and the solvent was concentrated. The residue was purified by silica gel column chromatography, and a chloroform:methanol 20:1 effluent fraction was concentrated. The precipitated crystals were collected by filtration to give 3-(4-methyl-2-oxopiperazin-1-yl)-2H-isoquinolin-1-one (0.38 g). ¹H-NMR(DMSO-d₆)δ: 2.30 (3H,s) , 2.72 (2H,t,J=5Hz) , 3.11(2H,s), 3.65(2H,t,J=5Hz), 6.51(1H,s), 7.45-7.51(1H,m), 7.61-7.73(2H,m), 8.15-8.18(1H,m), 11.51(1H,brs).

### Example 248

In the same manner as in Example 82, the reaction was carried out using N,N-dimethyl-2,3-dimethylbenzamide (2.0 g) and 1-(t-butyloxycarbonyl)-3-cyanopyrrolidine (1.0 g) to give 5-methyl-3-[1-(t-butyloxycarbonyl)pyrrolidin-3-yl]-2H-isoquinolin-1-one (0.2 g). 5-Methyl-3-[1-(t-butyloxycarbonyl)pyrrolidin-3-yl]-2H-isoquinolin-1-one (0.2 g) was dissolved in chloroform (2 ml). 4 mol/L Hydrochloric acid-dioxane (1 mL) was added, and the mixture was stirred. After the completion of the reaction, the solvent was concentrated, and the precipitated crystals were collected by filtration to give 5-methyl-3-(pyrrolidin-3-yl)-2H-isoquinolin-1-one hydrochloride (0.2 g). The reaction was carried out in the same manner as in Example 82 using 5-methyl-3-(pyrrolidin-3-yl)-2H-isoquinolin-1-one hydrochloride (0.2 g) to give 5-methyl-3-(1-methylpyrrolidin-3-yl)-2H-isoquinolin-1-one (0.1 g). ¹H-NMR(300MHz,CDCl₃)δ: 1.82-1.95(1H,m), 2.10-2.22(1H,m), 2.30-2.43(2H,m), 2.43(3H,s), 2.49(3H,s), 2.99-3.02(1H,m), 3.13-3.25(2H,m), 6.32(1H,s), 7.29(1H,t,J=7.8Hz), 7.44(1H,d,J=7.8Hz), 8.23(1H,d,J=7.8Hz), 10.4(1H,brs).

### Example 249

In the same manner as in Example 82, the reaction was carried out using N,N-dimethyl-2-methylbenzamide (2.1 g) and 1-(t-butyloxycarbonyl)-3-cyanopyrrolidine (1.0 g) to give 3-[1-(t-butyloxycarbonyl)pyrrolidin-3-yl]-2H-isoquinolin-1-one (0.25 g). 3-[1-(t-Butyloxycarbonyl)pyrrolidin-3-yl]-2H-isoquinolin-1-one (0.2 g) was dissolved in chloroform (2 ml), 4 mol/L hydrochloric acid-dioxane (1 mL) was added, and the mixture was stirred. After the completion of the reaction, the solvent was concentrated, and the precipitated crystals were collected by filtration to give 3-(pyrrolidin-3-yl)-2H-isoquinolin-1-one hydrochloride (0.23 g). The reaction was carried out in the same manner as in Example 82 using 3-(pyrrolidin-3-yl)-2H-isoquinolin-1-one hydrochloride (0.2 g) to give 3-(1-methylpyrrolidin-3-yl)-2H-isoquinolin-1-one (0.11 g). ¹H-NMR(300MHz,CDCl₃)δ: 1.80-1.95(1H,m), 2.05-2.22(1H,m), 2.22-2.43(2H,m), 2.43(3H,s), 2.98-3.02(1H,m), 3.13-3.19(2H,m), 6.21(1H,s), 7.38-7.45(2H,m), 7.57-7.62(1H,m), 8.36(1H,d,J=7.8Hz), 10.4(1H,brs).
MS (ESI) 229(M⁺+1)

The structural formulas of respective Example compounds are shown in the following.

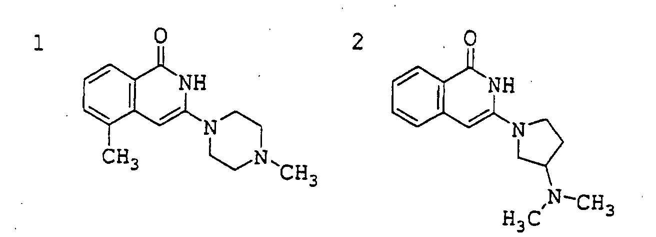

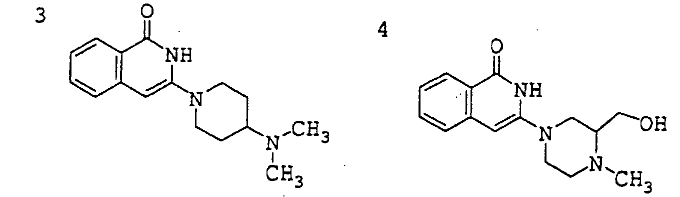

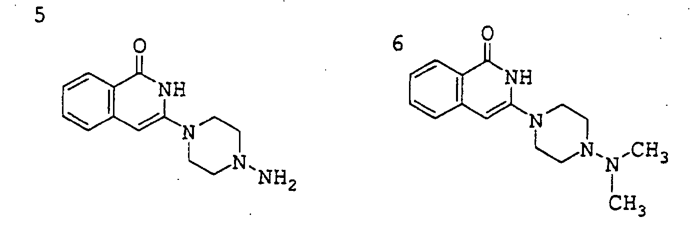

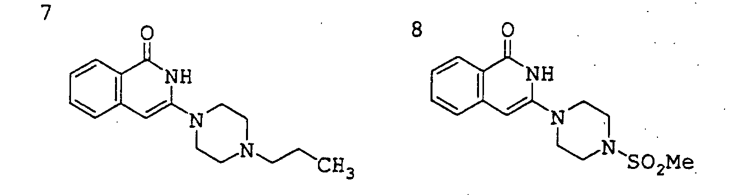

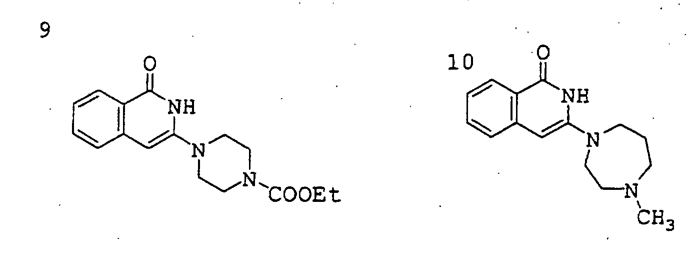

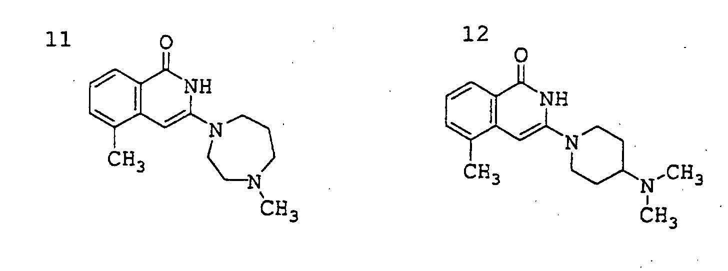

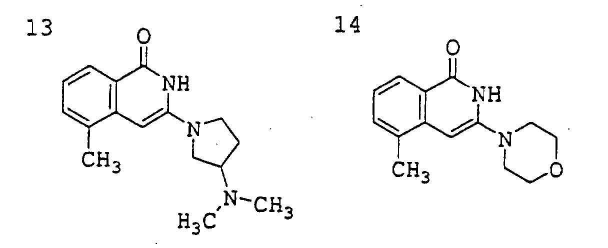

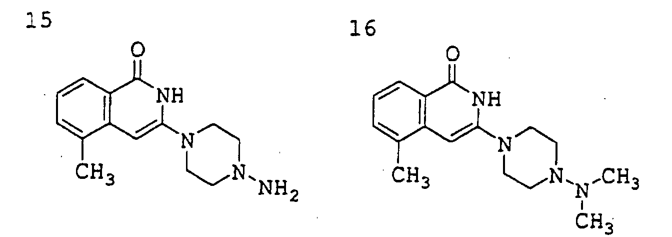

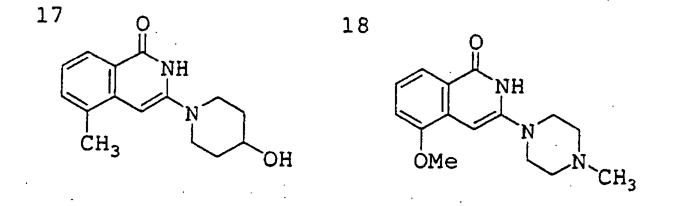

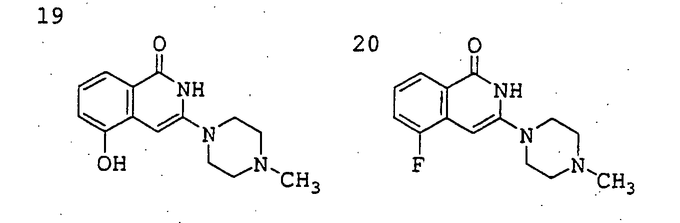

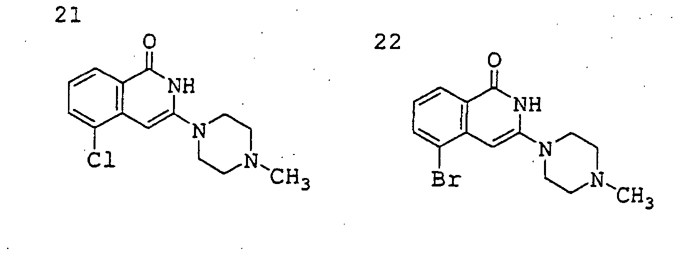

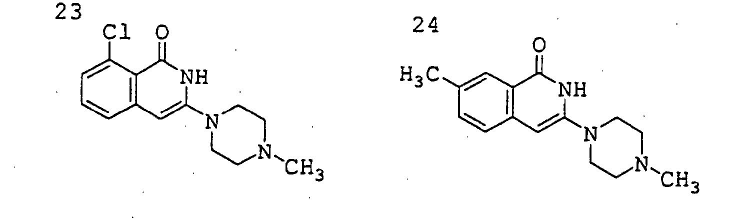

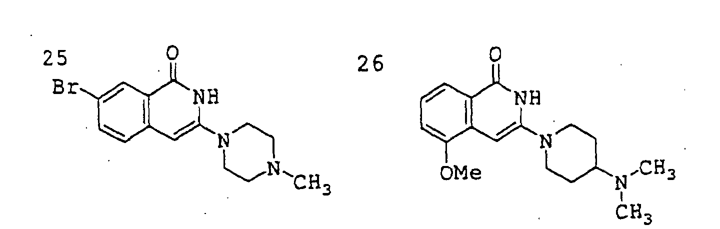

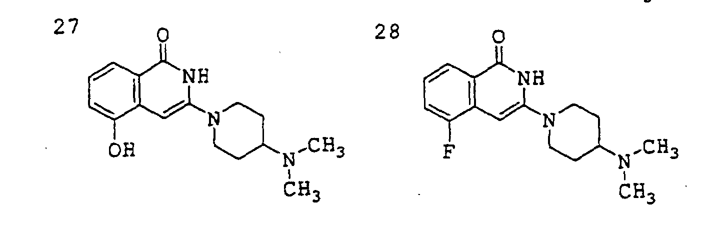

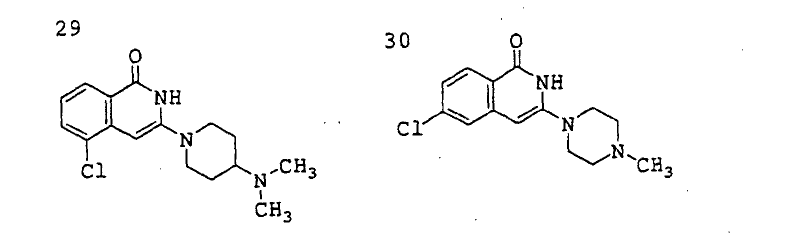

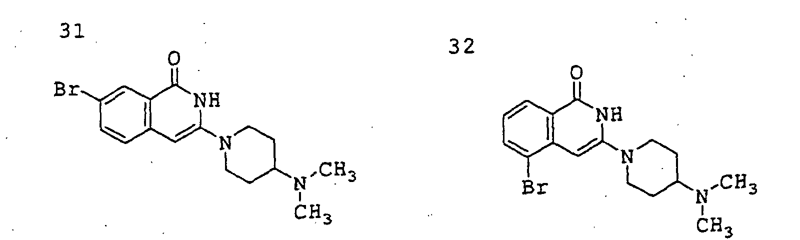

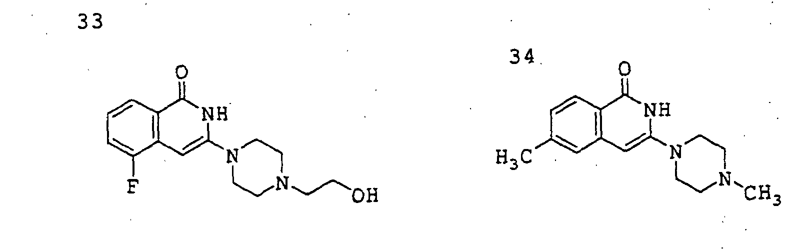

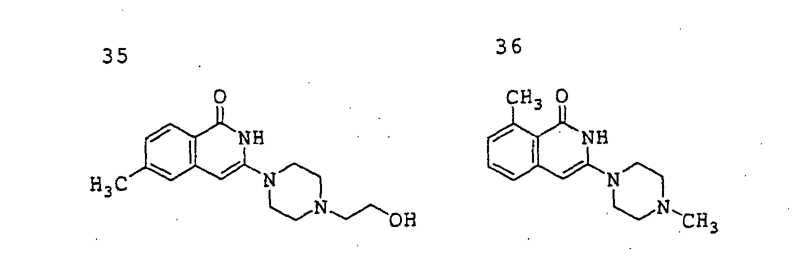

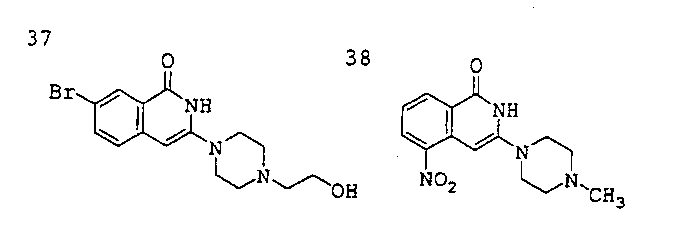

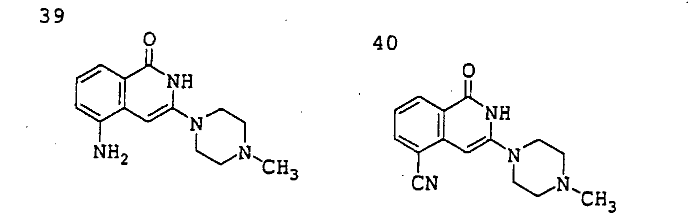

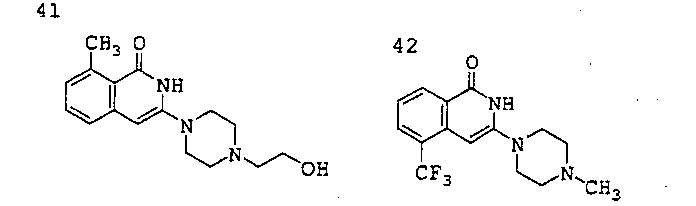

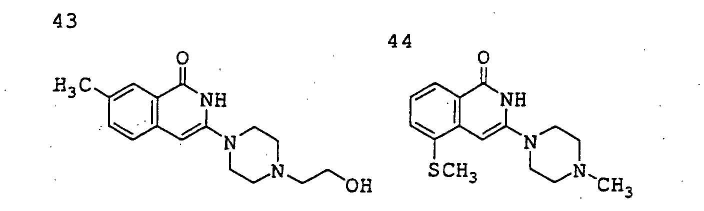

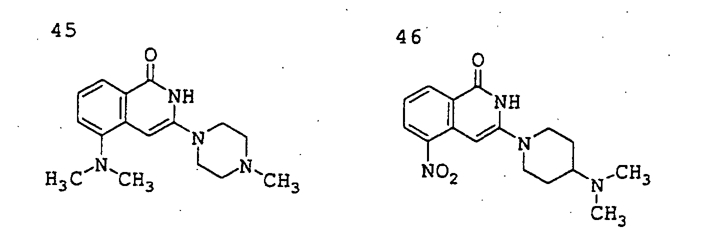

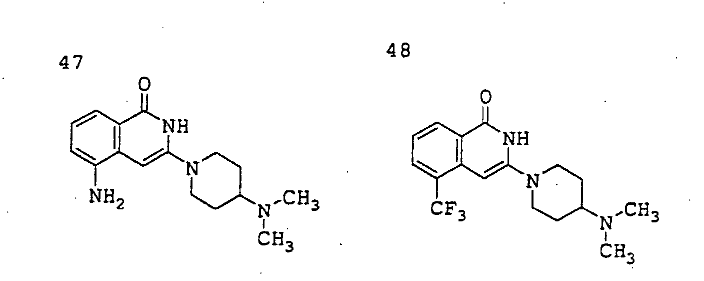

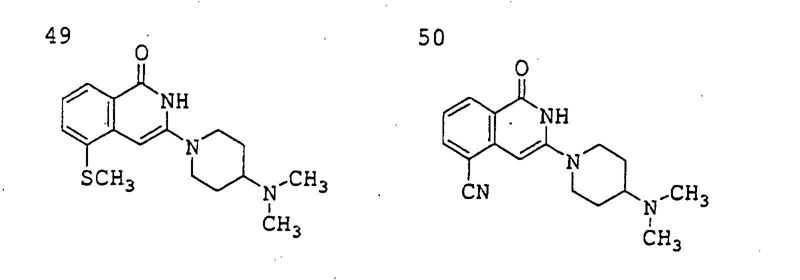

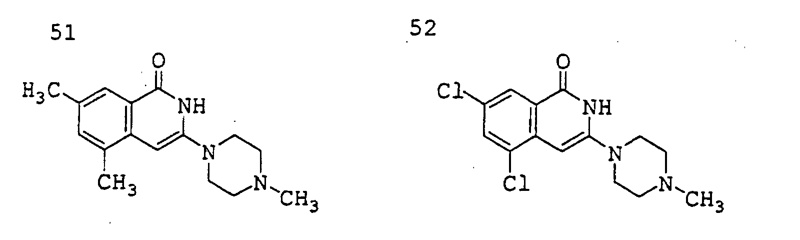

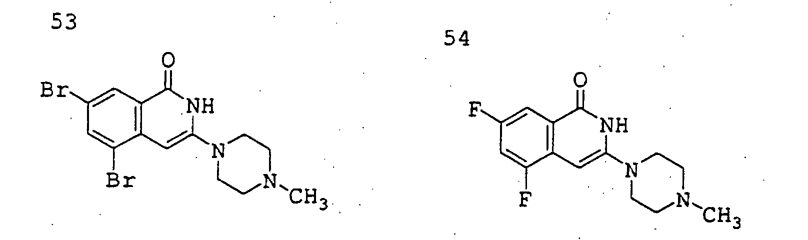

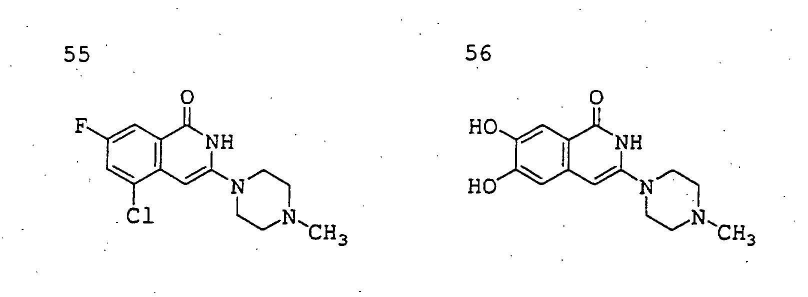

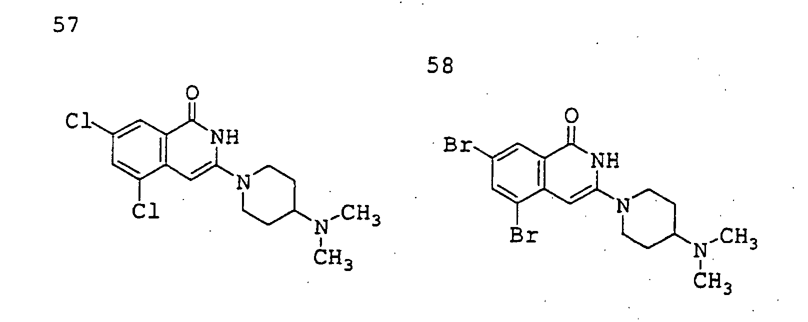

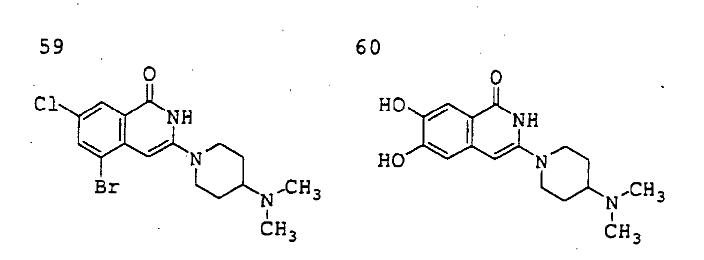

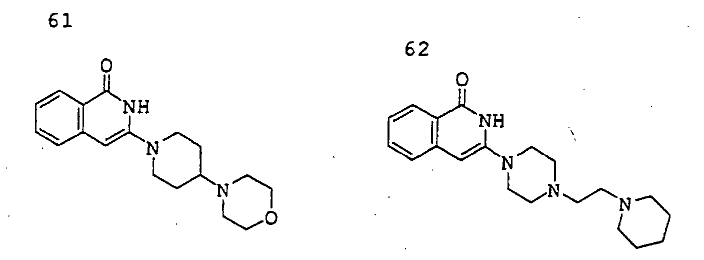

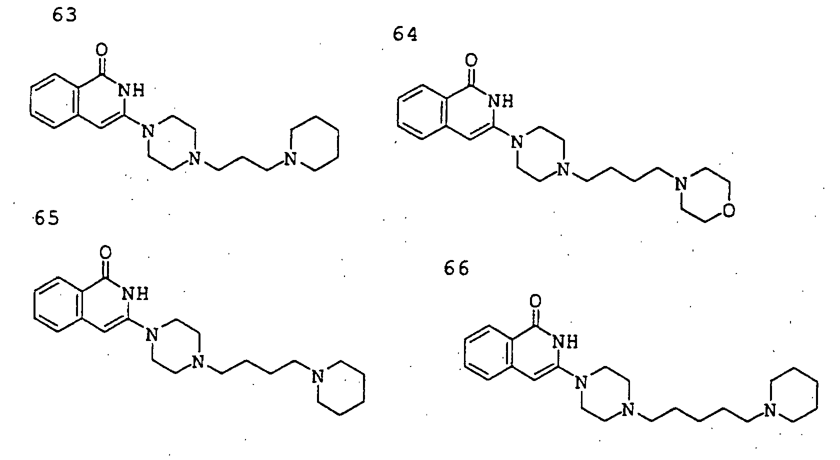

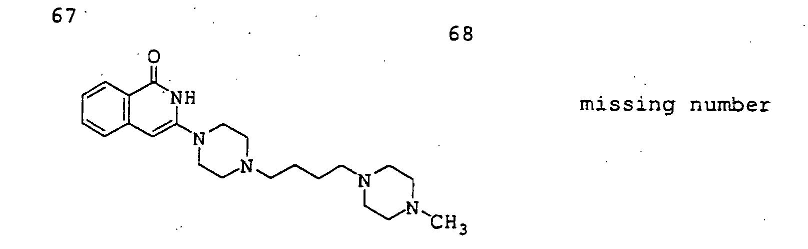

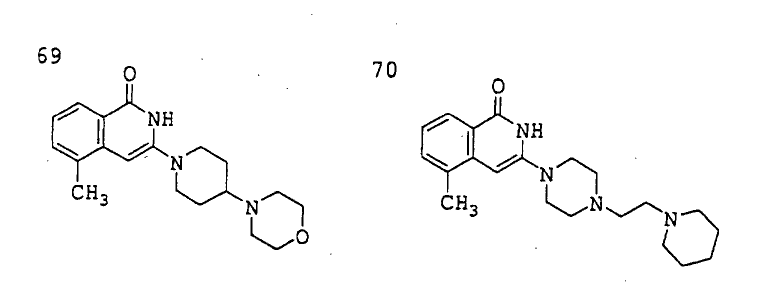

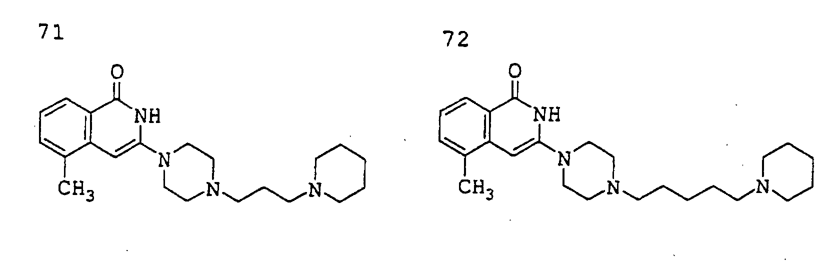

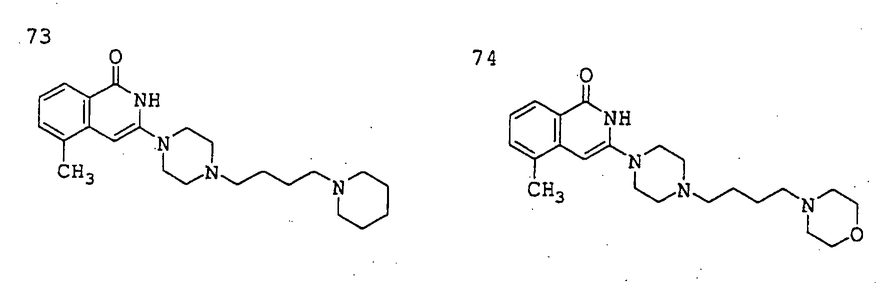

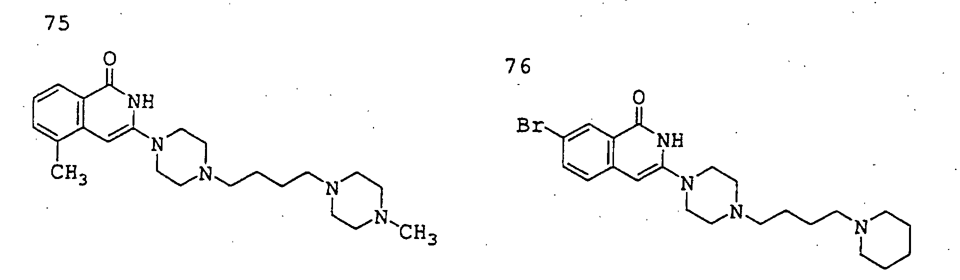

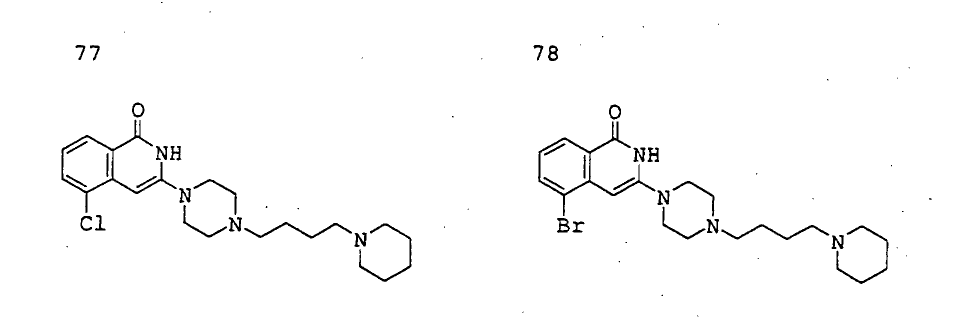

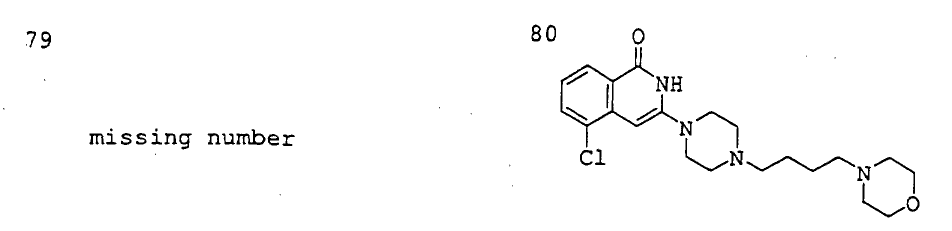

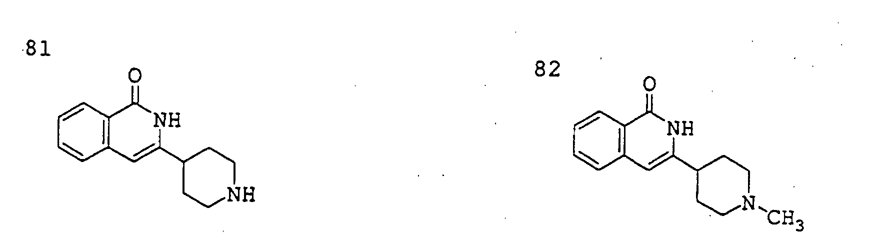

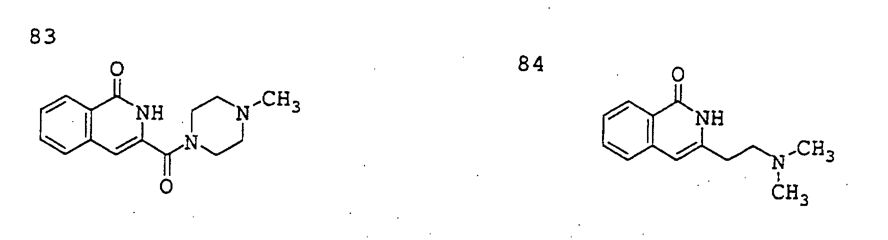

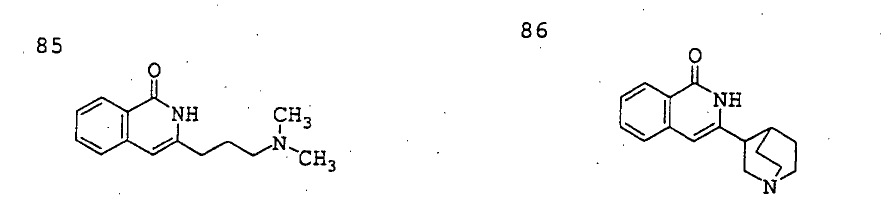

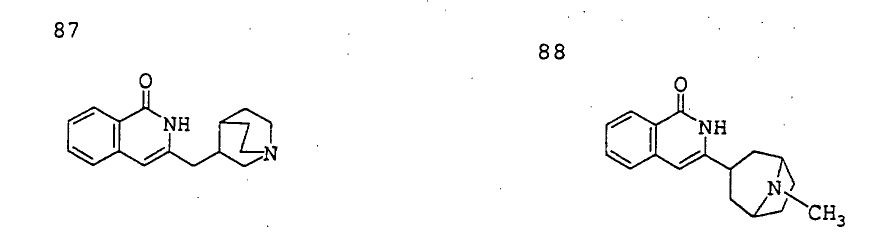

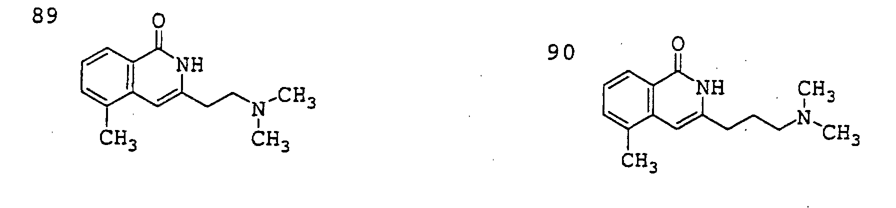

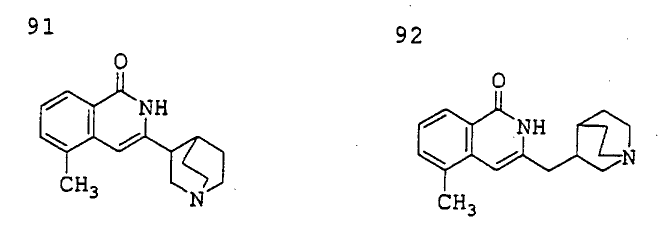

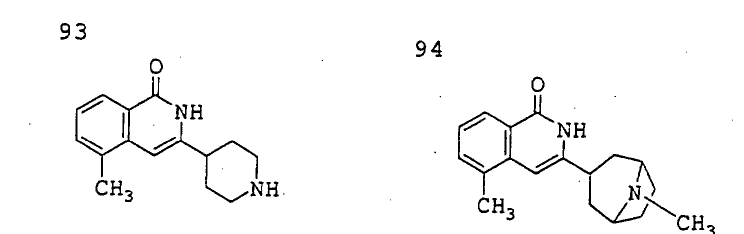

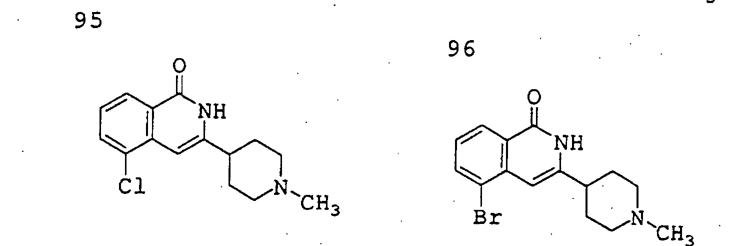

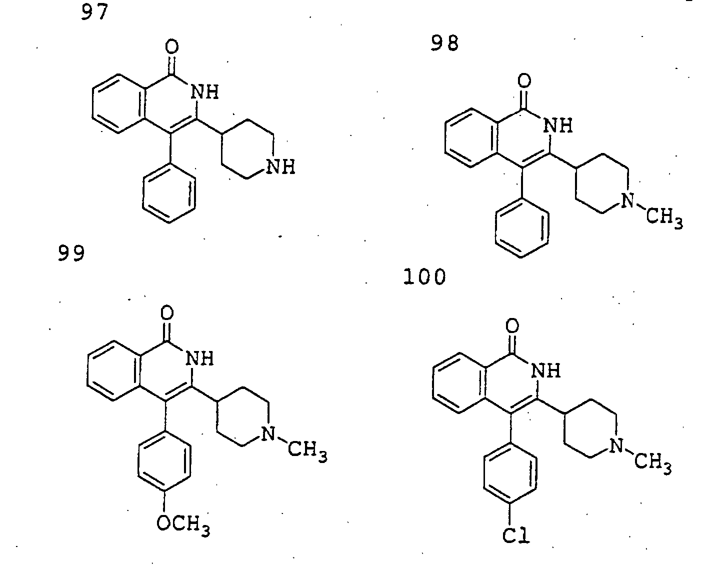

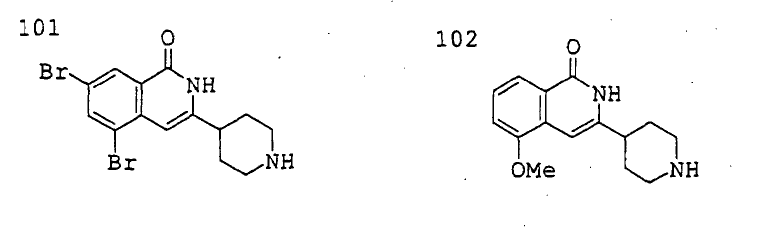

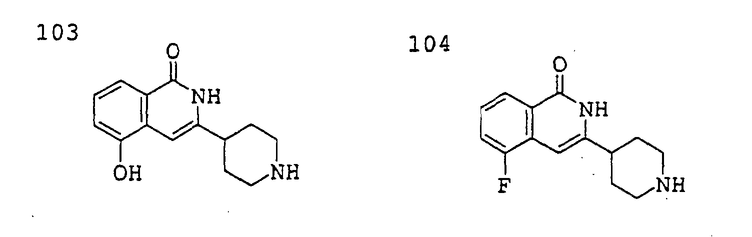

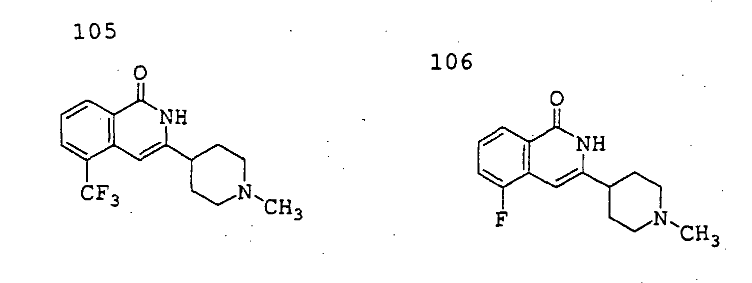

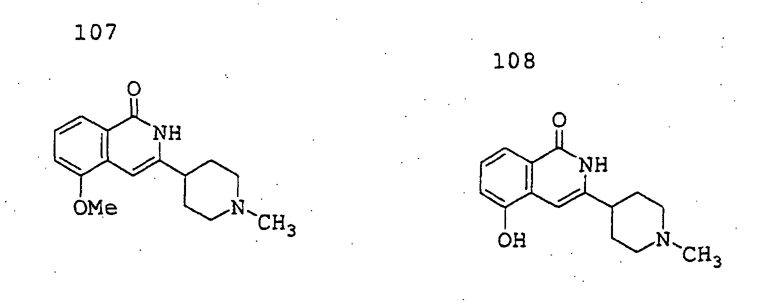

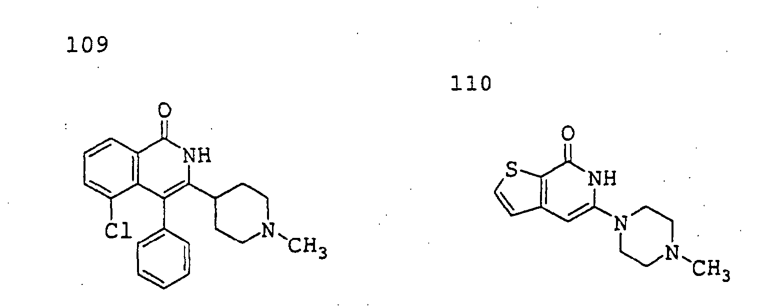

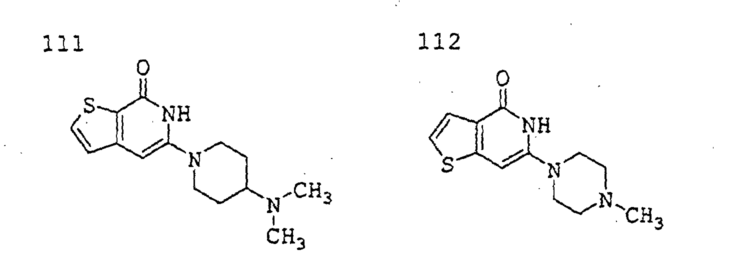

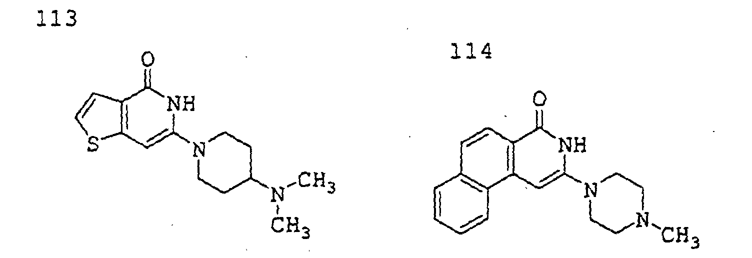

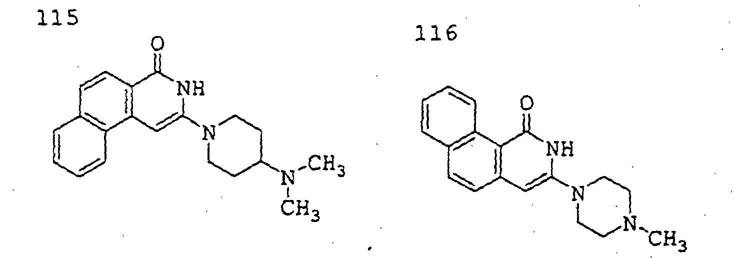

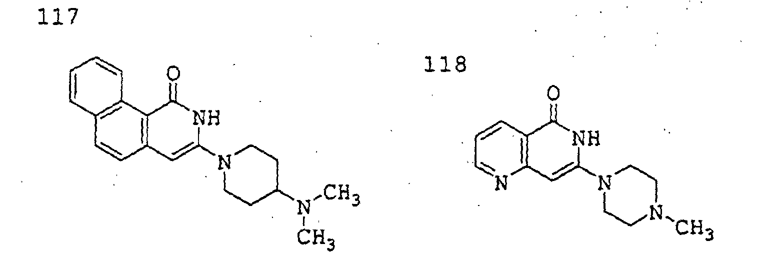

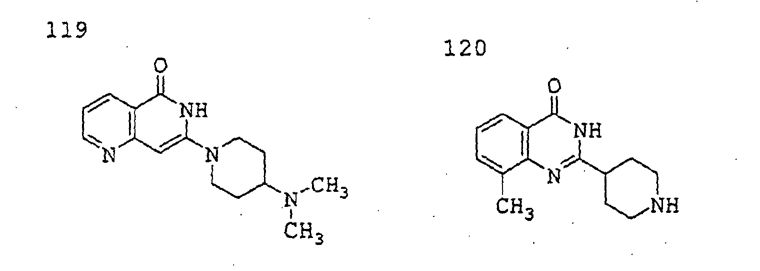

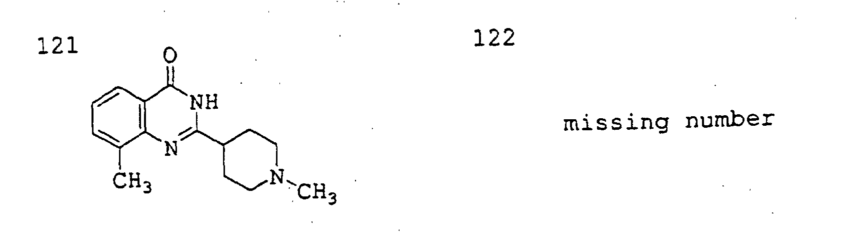

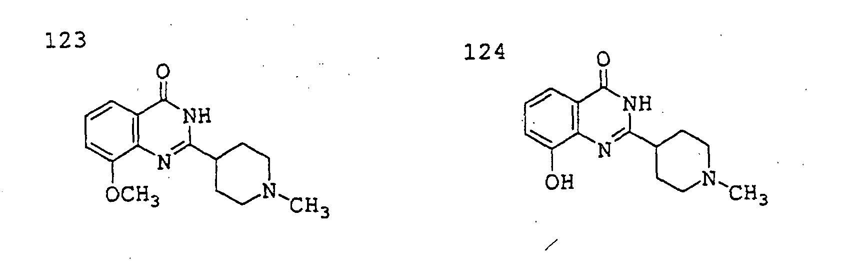

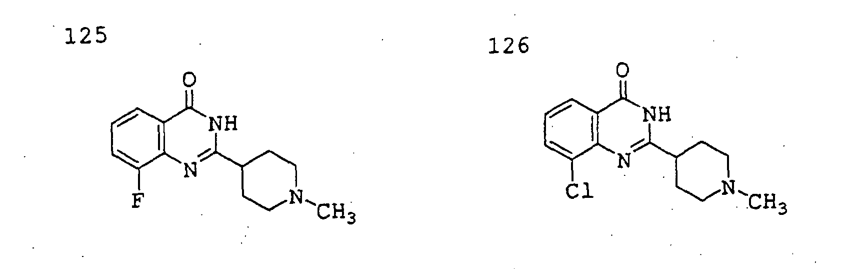

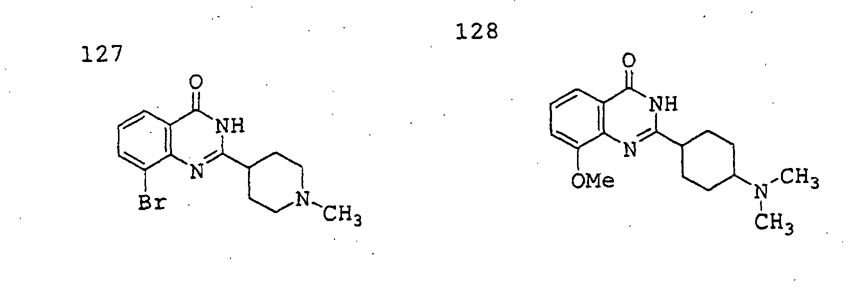

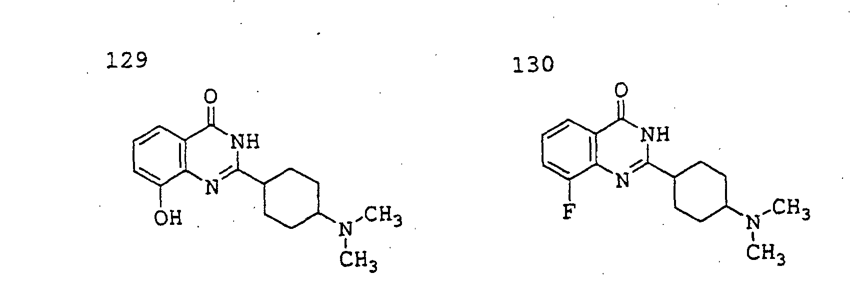

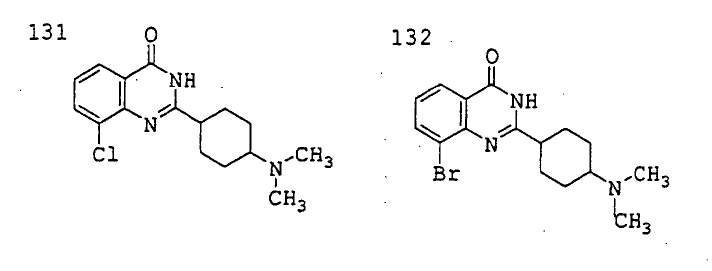

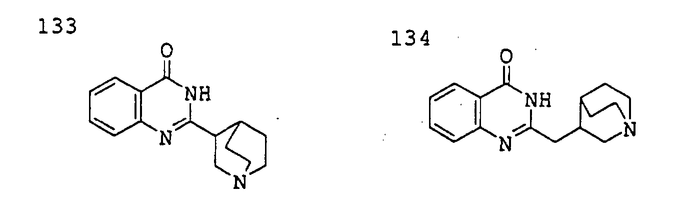

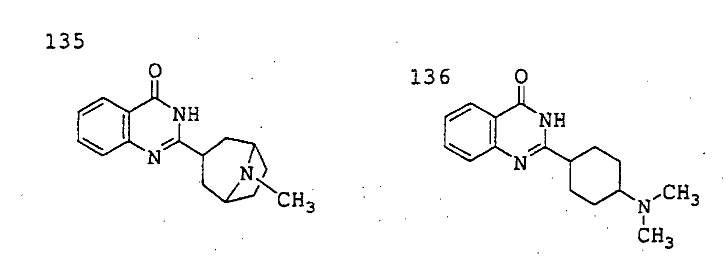

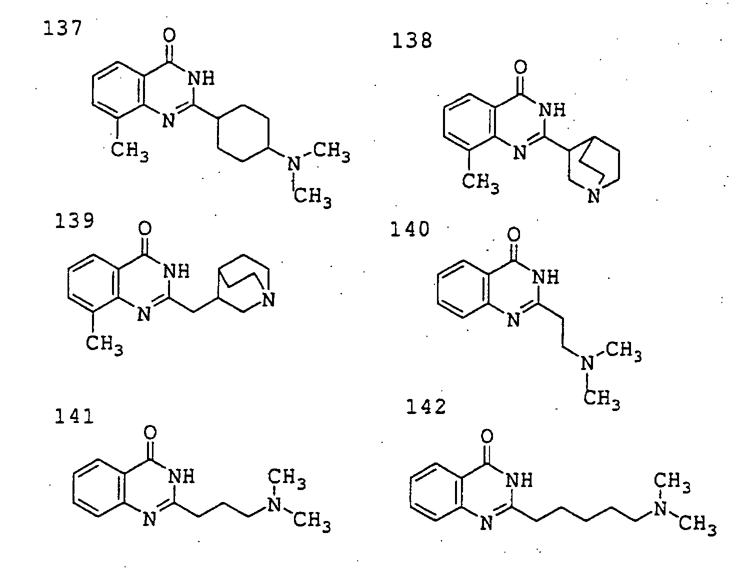

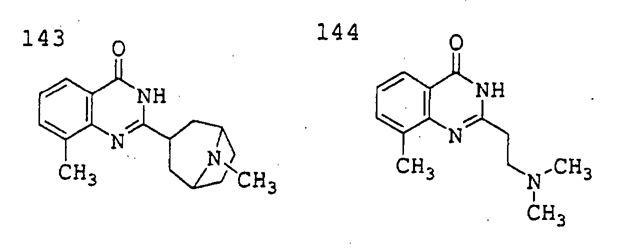

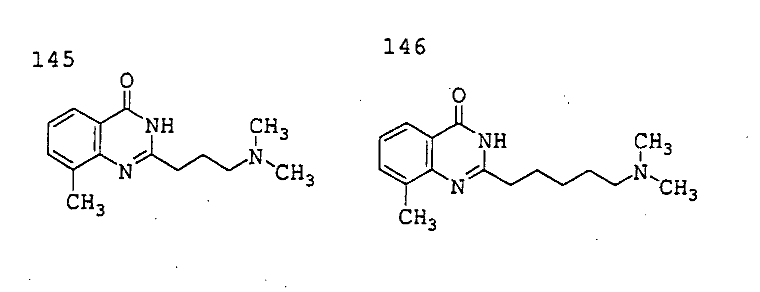

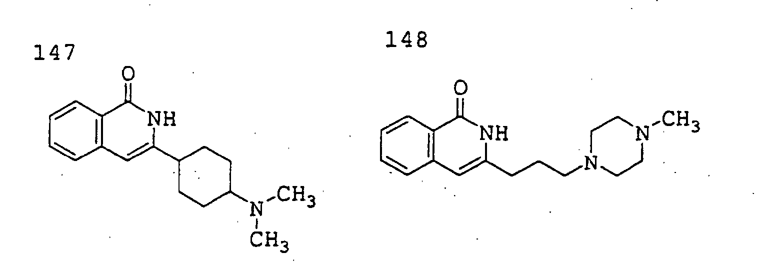

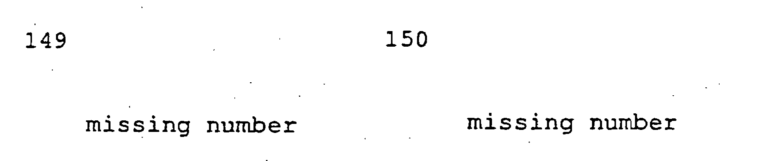

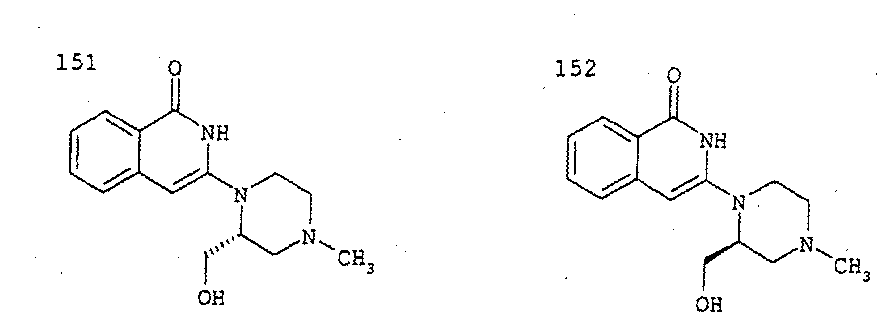

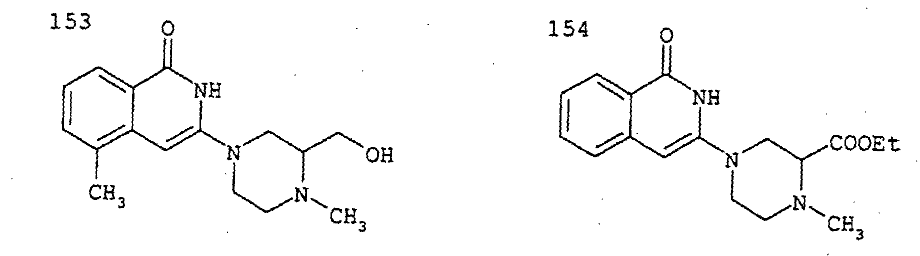

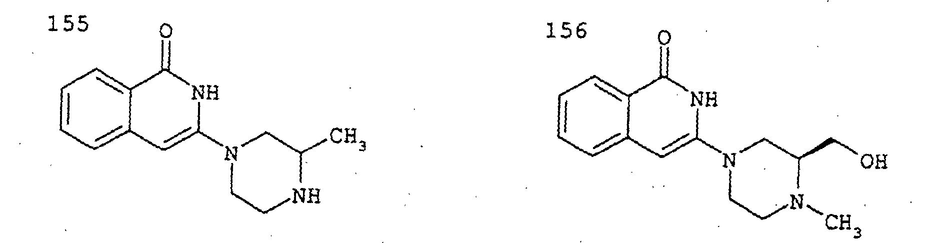

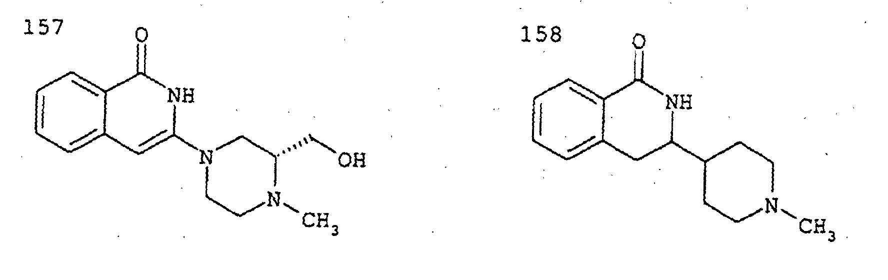

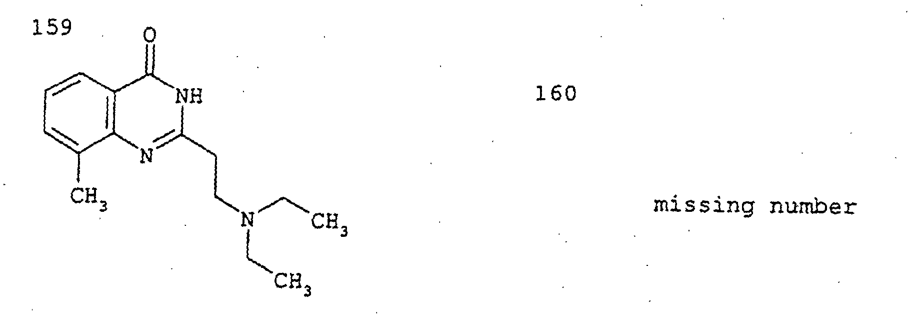

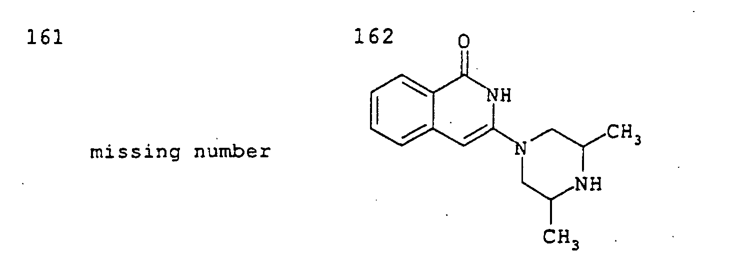

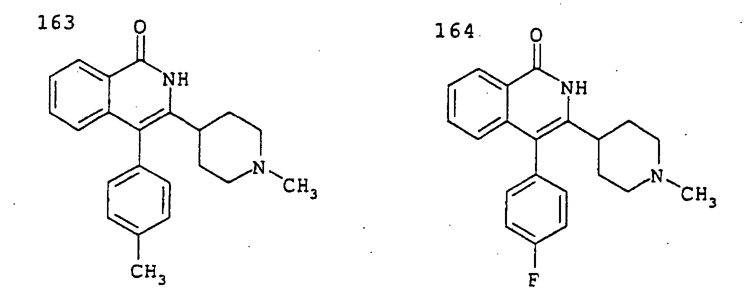

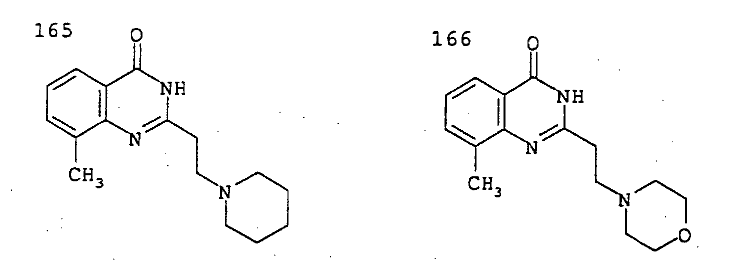

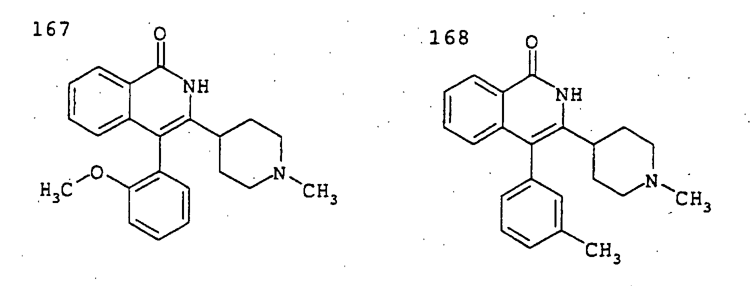

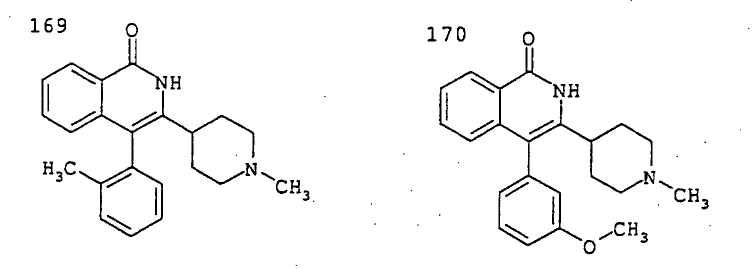

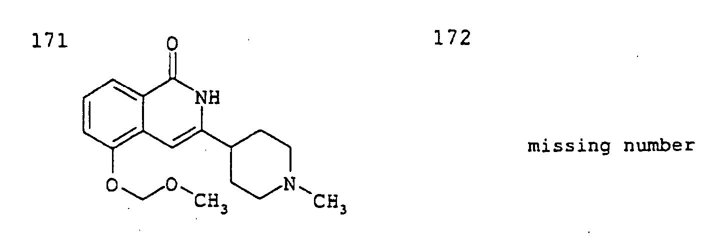

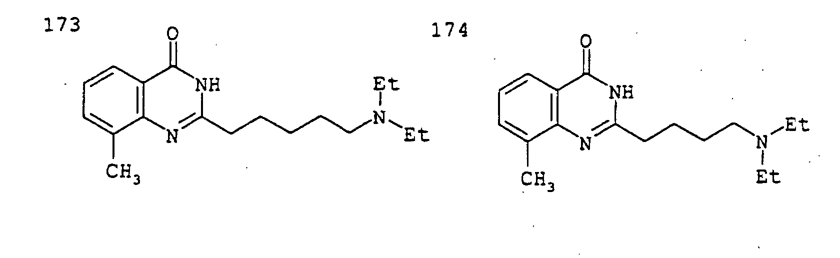

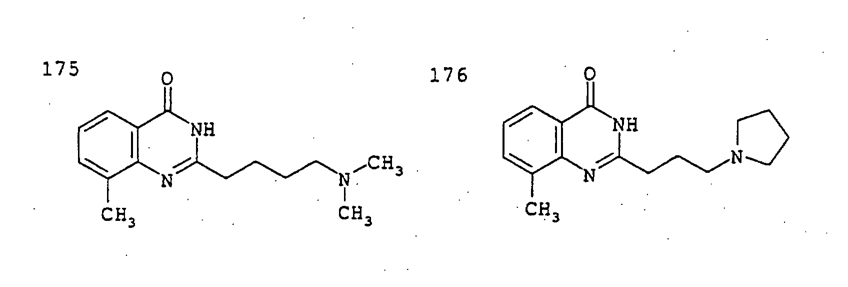

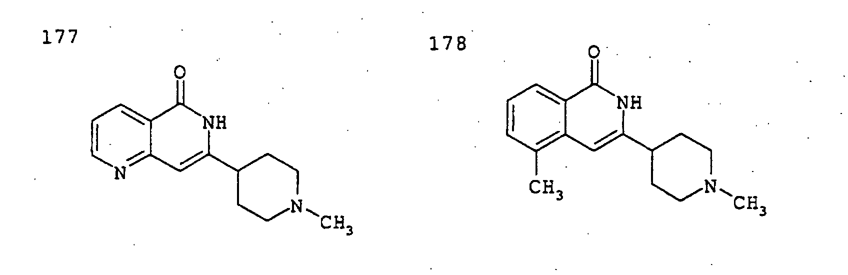

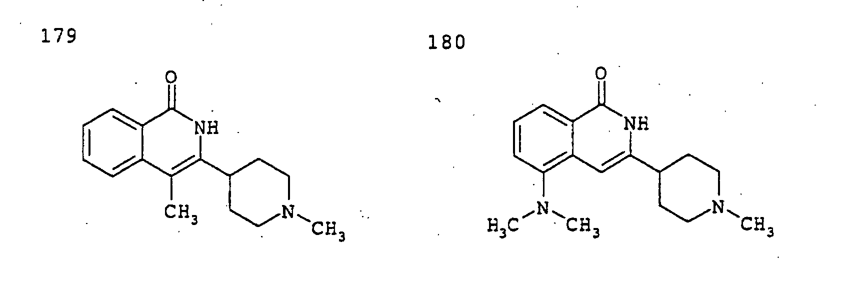

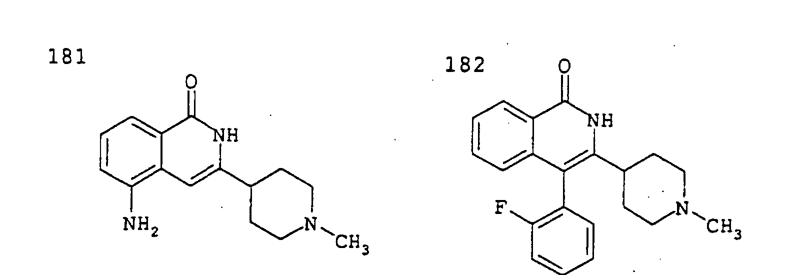

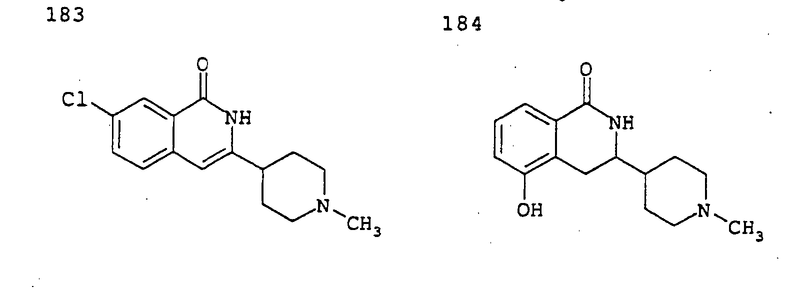

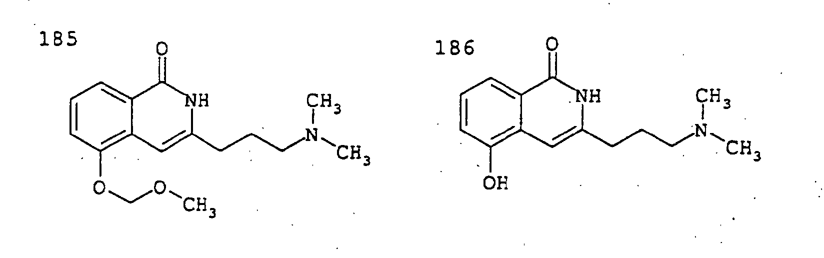

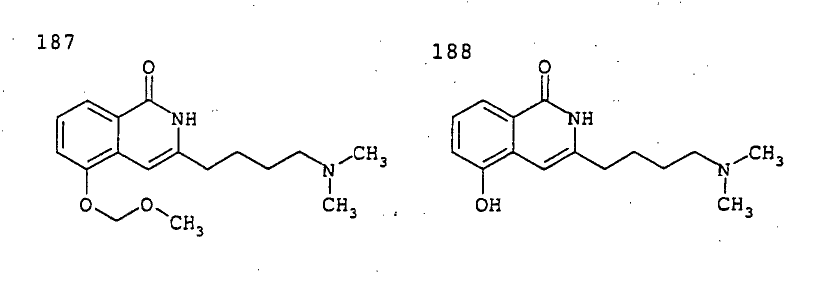

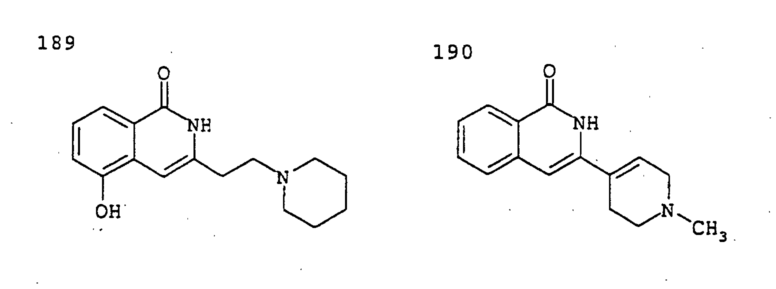

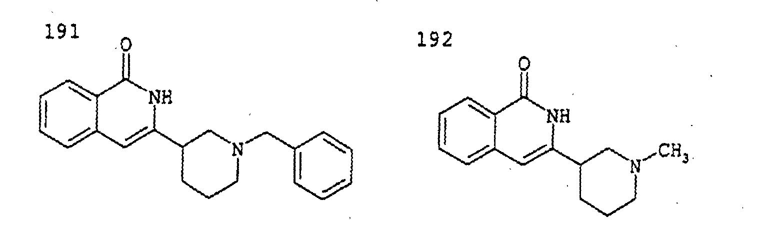

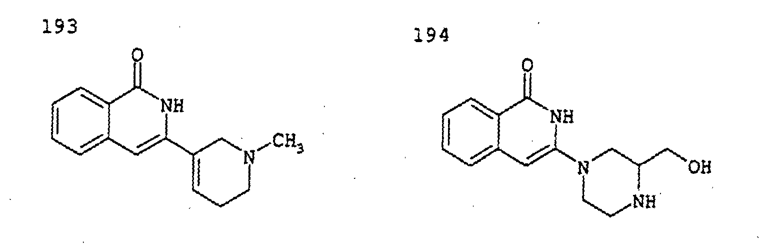

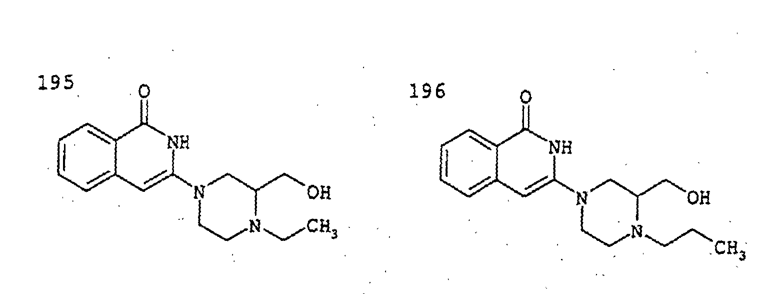

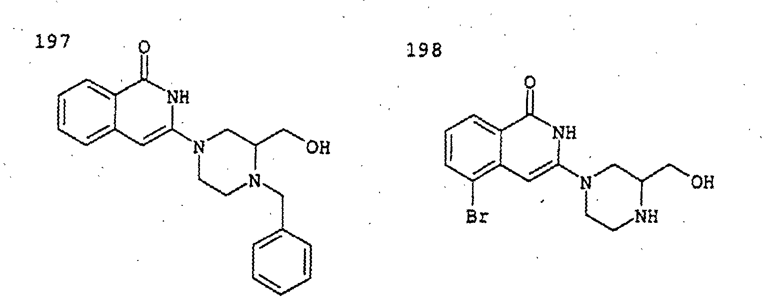

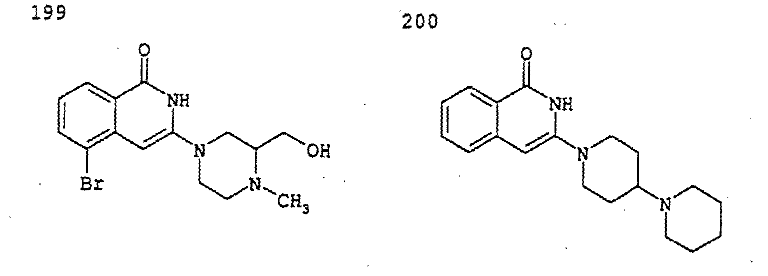

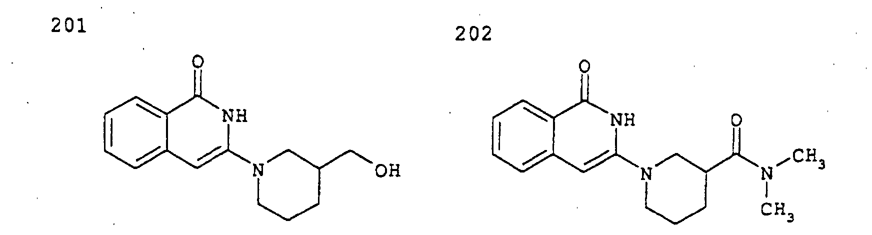

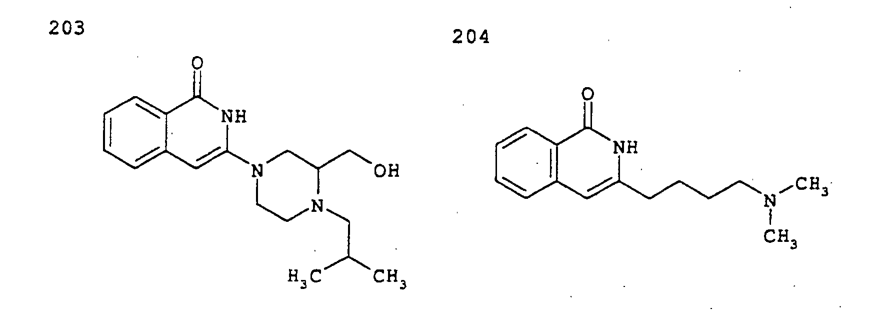

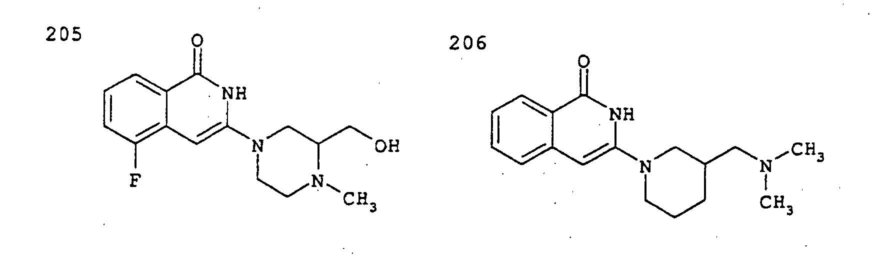

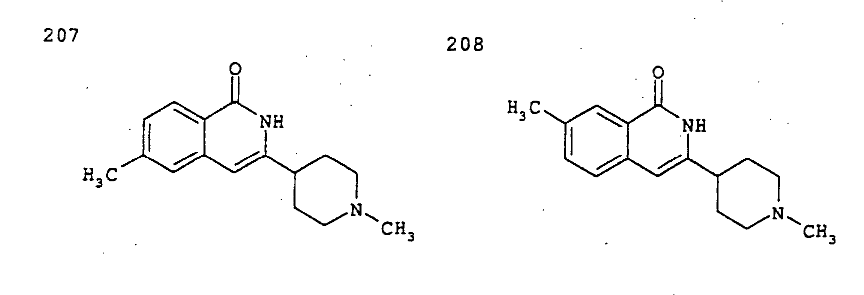

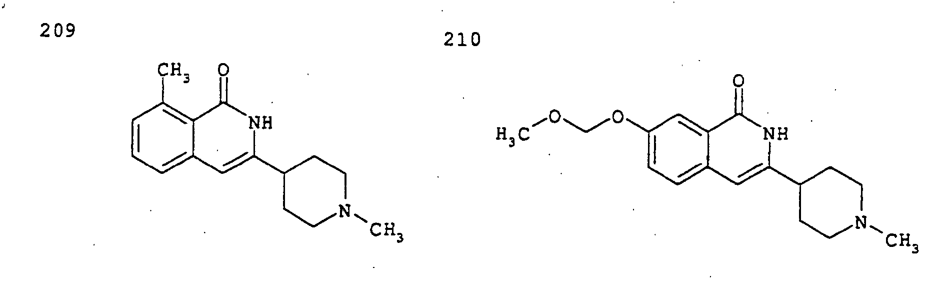

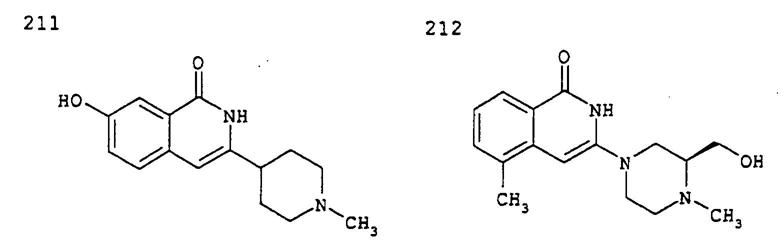

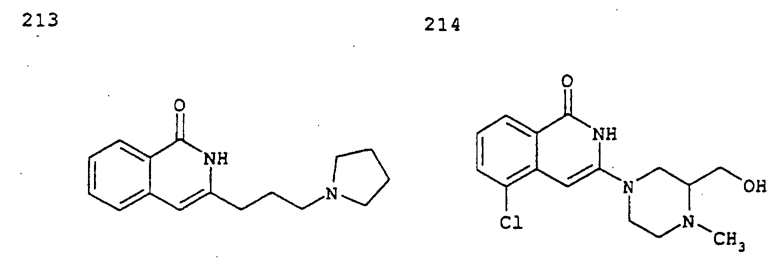

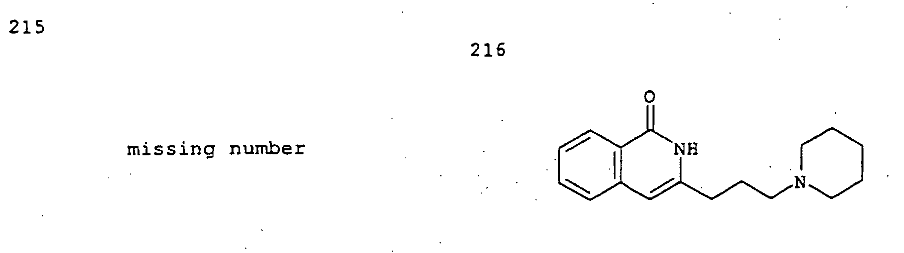

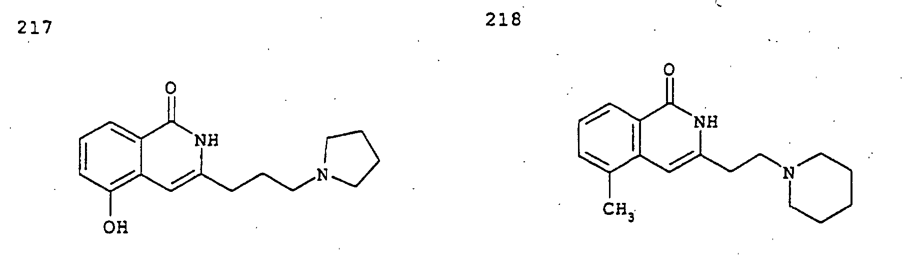

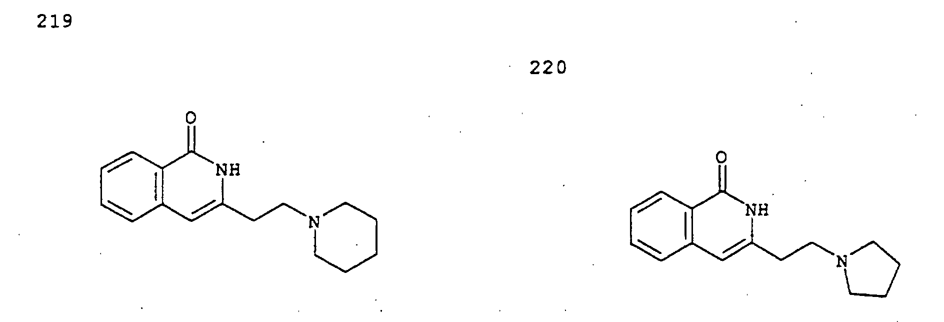

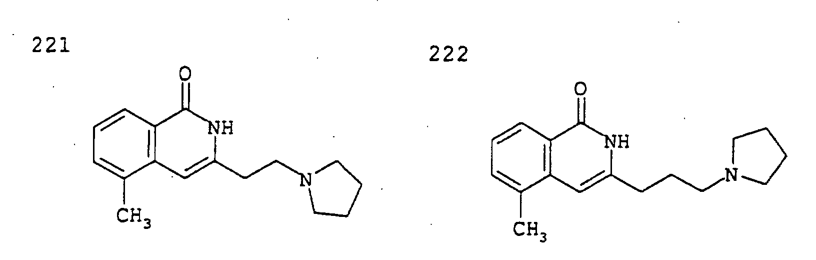

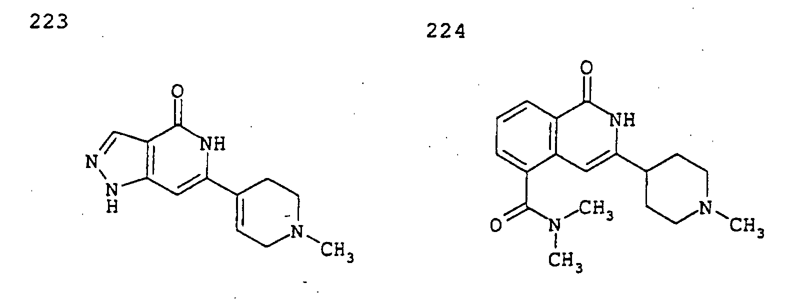

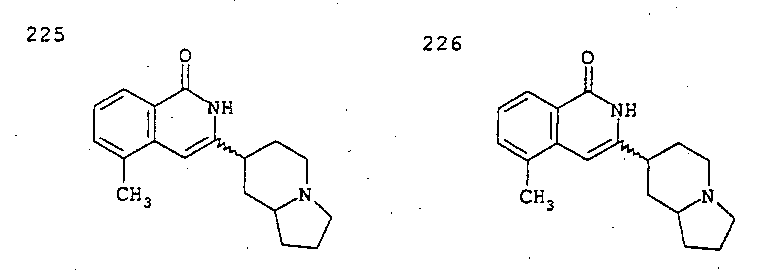

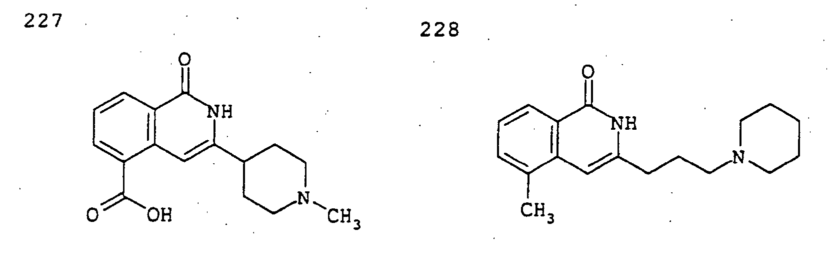

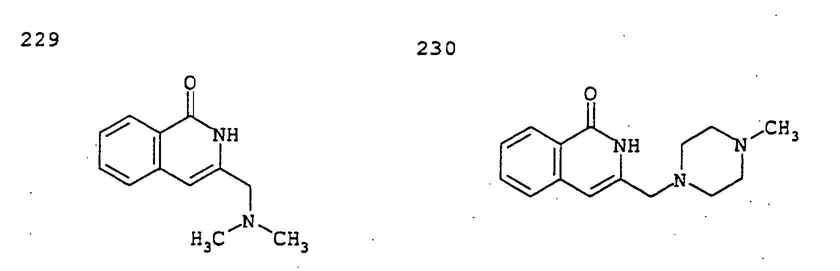

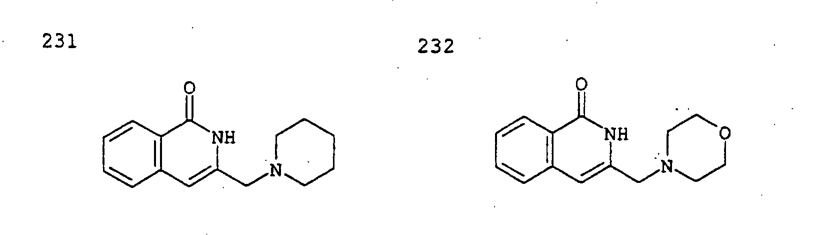

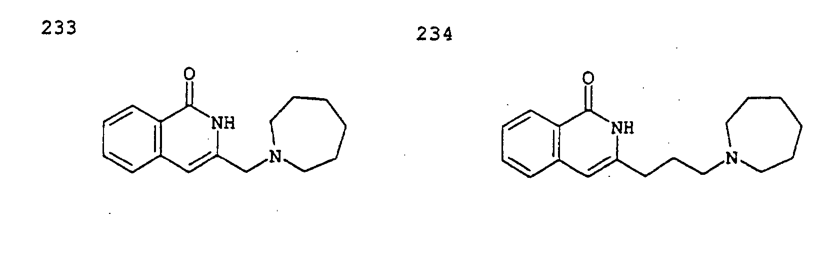

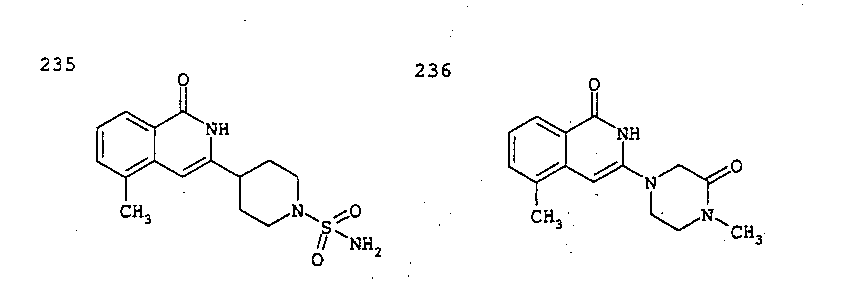

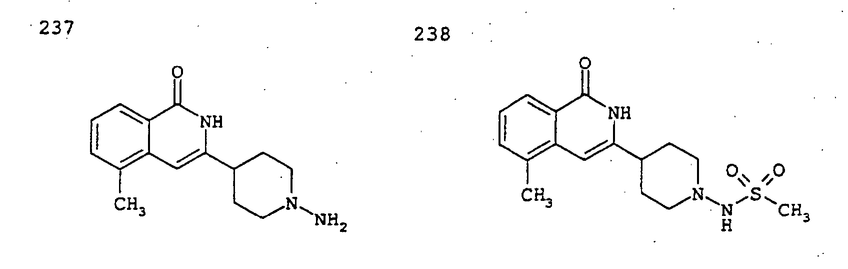

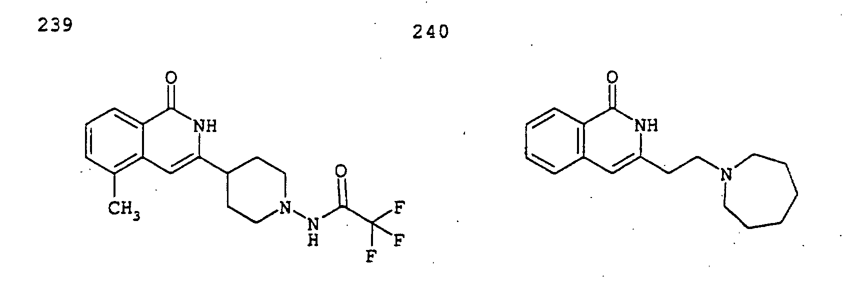

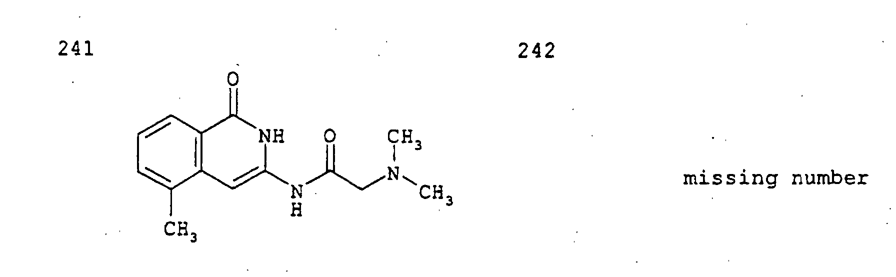

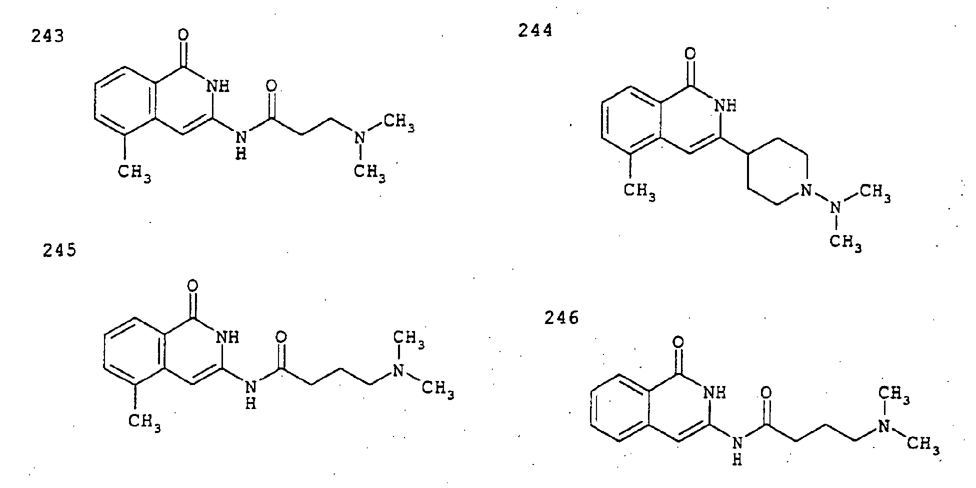

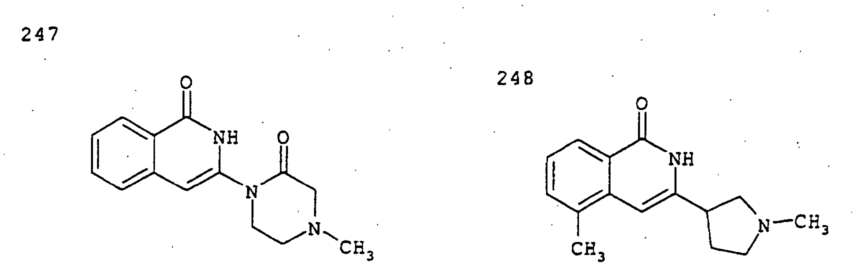

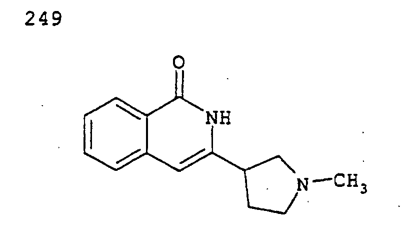

### Formulation Example 1

The compound of Example 1 (0.5 part), lactose (25 parts), crystalline cellulose (35 parts) and cornstarch (3 parts) were thoroughly admixed and kneaded well with a binder prepared from cornstarch (2 parts). The kneaded product was passed through a 16 mesh sieve, dried in an oven at 50°C and passed through a 24 mesh sieve. The kneaded powder obtained here, cornstarch (8 parts), crystalline cellulose (11 parts) and talc (9 parts) were admixed well and compression tableted to give tablets containing 0.5 mg of the active ingredient per tablet.

The superior pharmacological activity of the compound of the formula (I) is verified by a series of tests shown below.

### Experimental Example 1: PARP enzyme activity inhibitory action

Recombinant human PARP (4667-02X, Trevigen) was used as an enzyme source. ³H-NAD (1.85 kBq, NAD[adenine-2,8-³H], Daiichi Pure Chemicals) and activated DNA (0.02 mg/mL, 4667-03X, Trevigen) were added to an enzyme reaction buffer (10 mM Tris/HCl (pH 8.0), 1 mM MgCl₂, 28 mM KCl, 28 mM NaCl), and the enzyme source was added thereto to initiate Poly ADP ribosylation. After incubation at 25°C for 15 min., the reaction was quenched with 20% trichloroacetic acid, and the resulting acid insoluble fraction was adsorbed on a GF/B filter. The filter was washed several times with 5% trichloroacetic acid, and the radiation dose on the filter was measured by liquid scintillation counter. The PARP activity was measured by subtracting the radiation dose of an enzyme source non-addition sample as a blank value, and 50% enzyme inhibition value (IC₅₀ value) of each test compound was calculated based on the radiation dose of a compound non-addition sample as 100%. The average values of 3 measurements are shown in Table 1.

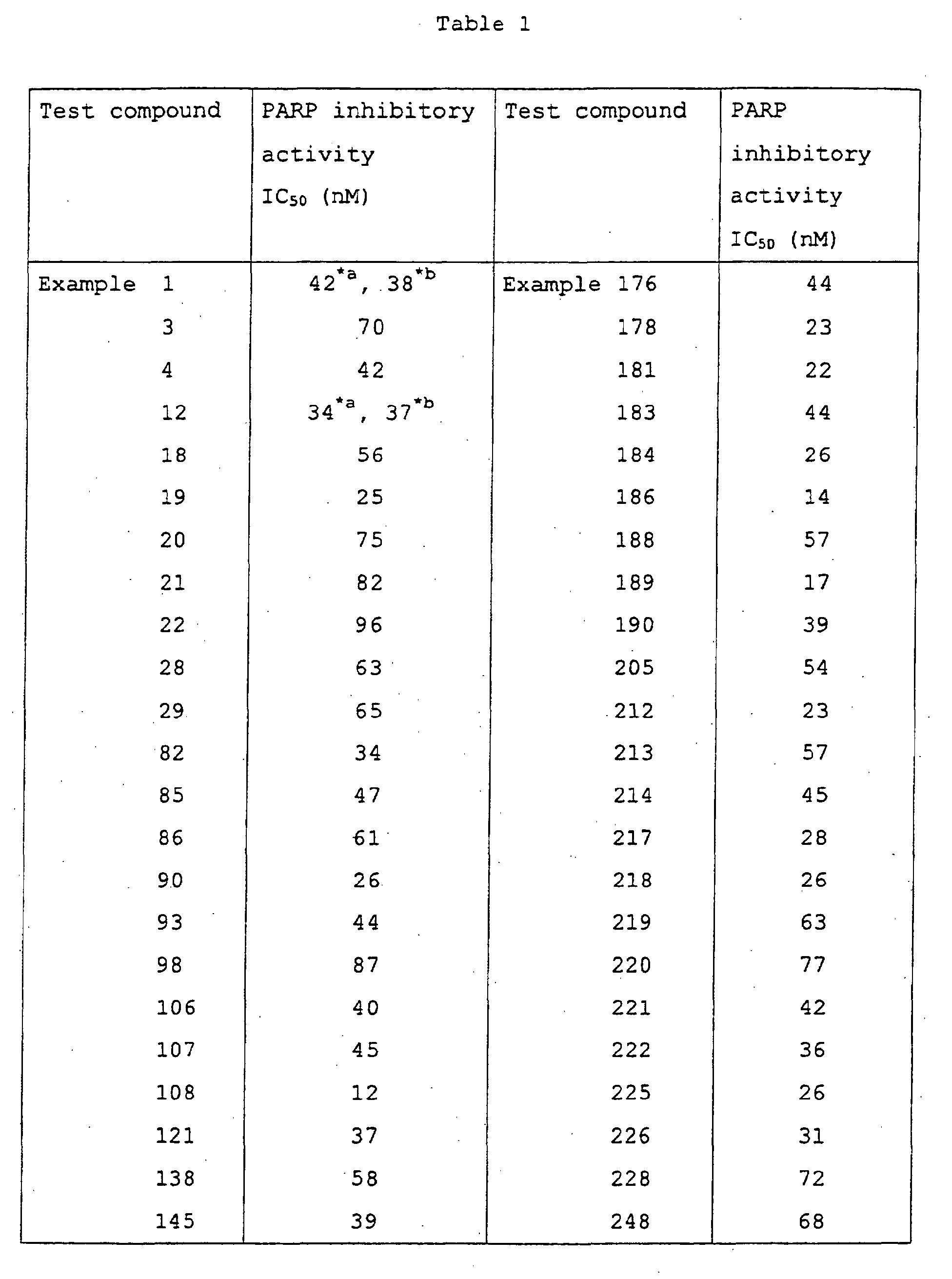

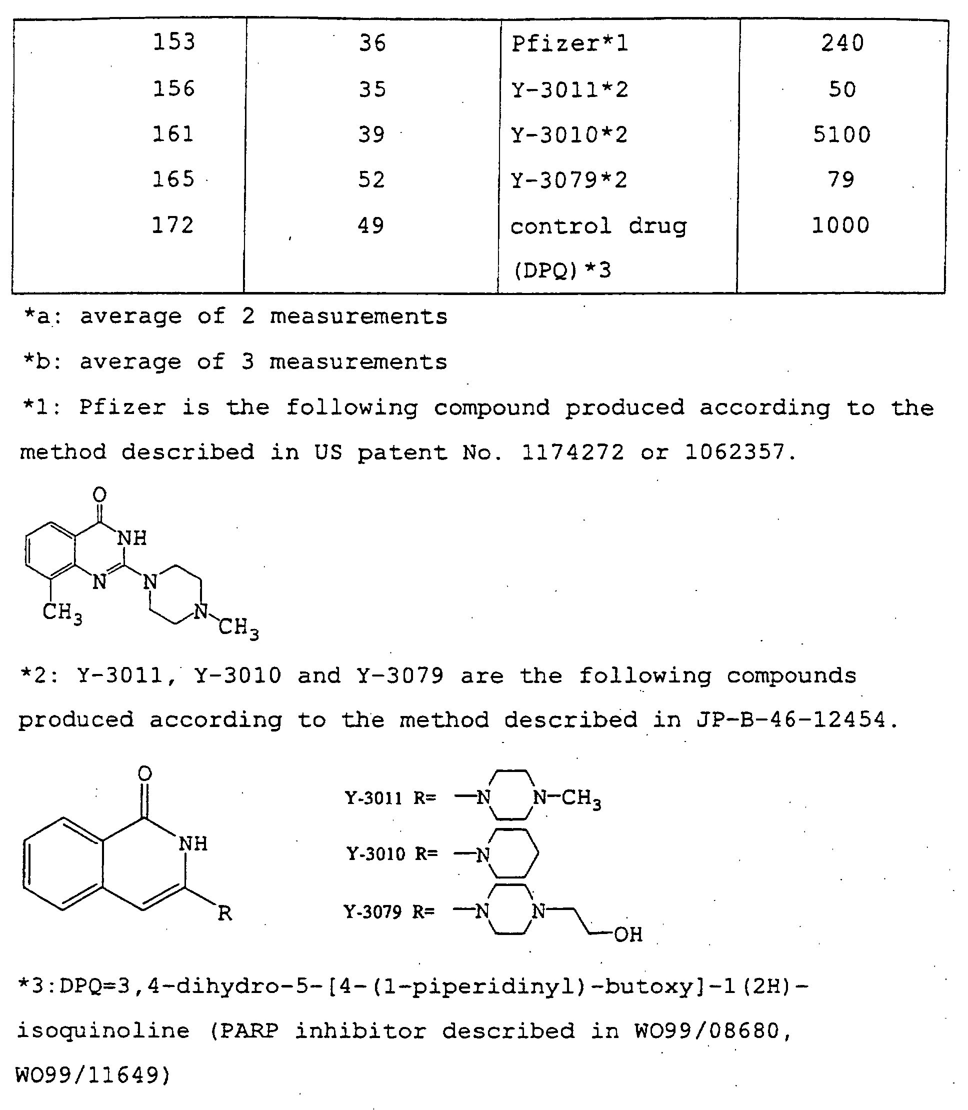

From the above, it is clear that the compounds shown in Examples of the present invention all have superior PARP inhibitory activity as compared to known compounds.

From the above-mentioned results, Y-3011, from among Y-3011, Y-3010 and Y-3079, has the most superior PARP inhibitory activity. Therefore, Y-3011 is used as a representative compound of JP-B-46-12454 in the following Experimental Examples 3 and 4.

### Experimental Example 2: hydrogen peroxide (H₂O₂) induced cytotoxicity suppressive action

P388D1 cells cultured to confluence in a 25 cm² flask were sown in a 96-well plate at a density of 4×10⁶ cells/well. The test compound was added, and the cells were incubated for 15 min (37°C), after which H₂O₂ (2 mM) was added, and the cells were incubated for 4 hr. After the completion of the incubation, the culture supernatant was recovered, and the LDH activity, which is an index of cytotoxicity, was measured using an LDH-cytotoxicity test kit (Wako Pure Chemical Industries, Ltd.). The 50% suppression value (IC₅₀ value) of each test compound was calculated based on the LDH activity (absorbance) of a compound non-addition sample as 100%. The results are shown in Table 2.

**Table 2**

| Test compound | | Cell death suppressive action IC₅₀ (µM) |
|---|---|---|
| Example | 1 | 0.12 |
| | 12 | 0.11 |
| | 82 | 0.19 |
| | 90 | 0.14 |
| | 108 | 0.124 |
| | 121 | 0.24 |
| | 156 | 0.23 |
| | 178 | 0.09 |
| | 181 | 0.22 |
| | 186 | 0.13 |
| | 189 | 0.085 |
| | 212 | 0.070 |
| control drug (DPQ) | | 3.30 |

### Experimental Example 3: stability test

Each test compound (5 mg) was dissolved in 0.1 mol/L aqueous citric acid solution (1 ml), and the residual ratio of each compound was measured by HPLC under shading or no shading at room temperature one day and 8 days later. The residual ratio was calculated by comparing the area of HPLC immediately after dissolution of each compound with the area of HPLC one day or 8 days later. The results are shown in Table 3. column CAPCELPAK UG120(C18)(Shiseido Co., Ltd.) mobile phase 50 mmol/L aqueous sodium perchlorate solution (pH 2.5):acetonitrile=80:20
detection wavelength 254 nm
flow rate 1.0 ml/min

**Table 3**

| test compound | | residual ratio(%) under shading | | residual ratio(%) without shading | |
|---|---|---|---|---|---|
| | | 1 day later | 8 days later | 1 day later | 8 days later |
| Example | 82 | ≒100 | ≒100 | ≒100 | ≒100 |
| | 108 | ≒100 | ≒100 | ≒100 | ≒100 |
| | 121 | ≒100 | ≒100 | - | - |
| Y-3011 | | 76 | 32 | - | - |
| - : not measured | | | | | |

From the above, it is clear that the compound of the present invention is more stable in aqueous solutions.

### Experimental Example 4: affinity for adrenalin α1 receptor; 3H-prazosin binding

Preparation of crude synapse membrane and binding experiment were performed according to European Journal of Pharmacology, vol. 55, page 323 (1979). Crude synapse membranes were prepared from cryopreserved rat brain tissues, and a membrane sample and 3H-prazosin were incubated in the presence of a test compound at 25°C for 30 min. After the completion of the reaction, incubation product was filtered by suction through a Whatman GF/B filter (product name), and the radioactivity on the filter was measured by liquid scintillation counter. The amount of non-specific binding was determined in the presence of 1 µM prazosin. The 50% suppression concentration (IC₅₀) of the test compound was calculated from nonlinear regression, and the inhibition constant (Ki value) was determined. The results are shown in Table 4.

**Table 4**

| test compound | | affinity for adrenalin α1 receptor Ki (µM) |
|---|---|---|
| Example | 156 | >100 |
| | 212 | >100 |
| Y-3011 | | 4.0 |

From the above, since hypotensive action is considered to be a contraindication for cerebral infarction patients, the compound of the present invention free of affinity for adrenalin α1 receptor is preferable as a therapeutic drug for cerebral infarction.

### Industrial Applicability

The compound of the formula (I), an optical isomer thereof, a pharmaceutically acceptable salt thereof, a hydrate thereof and a water adduct thereof have potent PARP inhibitory action and are useful as a therapeutic drug for cerebral infarction (particularly k).

This application is based on a patent application No. 2001-154571 filed in Japan, the contents of which are hereby incorporated by reference.

## Claims

1. A fused heterocyclic compound represented by the formula (I) : 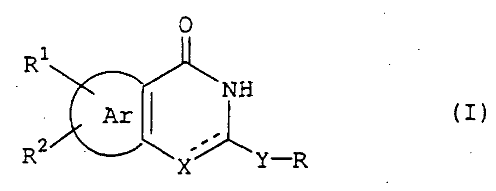 wherein
a dotted line part is a single bond or a double bond;
ring Ar is a benzene ring, a naphthalene ring or an aromatic heterocycle;
X is a carbon atom optionally substituted by an alkyl, an aromatic heterocyclic group optionally having substituent(s) or a phenyl optionally having substituent(s), or a nitrogen atom;
Y is
- (CH₂)ₘ-,
- (CH₂)ₘ-N(R⁴)-CO- (CH₂)ₙ-,
- (CH₂)ₘ-CO-N(R⁴)-(CH₂)ₙ-,
- (CH₂)ₘ-CO-O-(CH₂)ₙ-,
- (CH₂)ₘ-O-CO-(CH₂)ₙ-,
- (CH₂)ₘ-O-(CH₂)ₙ- or
- (CH₂)ₘ-CO-(CH₂)ₙ-
wherein m and n are the same or different and each is 0 or an integer of 1-10, R⁴ is a hydrogen or an alkyl, and -(CH₂)ₘ- is linked with a parent-nucleus;
R¹ and R² are the same or different and each is a hydrogen, a halogen, an alkyl, an alkoxy, a haloalkyl, a hydroxy, an amino, a dialkylamino, a nitro, a cyano, an acyl, a carboxy, an ester, a carbamoyl, an N-alkylcarbamoyl, an N,N-dialkylcarbamoyl, an acylamino, a diacylamino, a thiol, an alkylthio, an alkoxycarbonylamino, a sulfamoyl, an N-alkylsulfamoyl, an N,N-dialkylsulfamoyl or an alkoxyalkyloxy; and
R is an amino, a monoalkylamino, a dialkylamino, a morpholino or a thiomorpholino, or represented by the following formula (a)-(e): 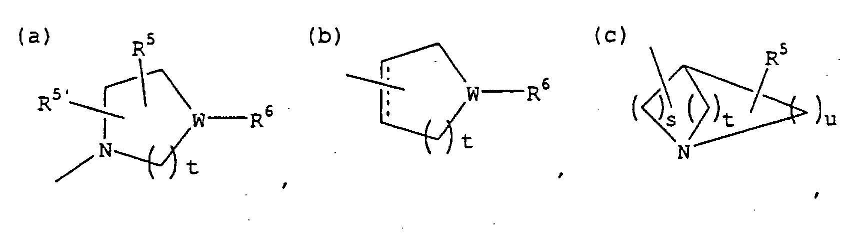 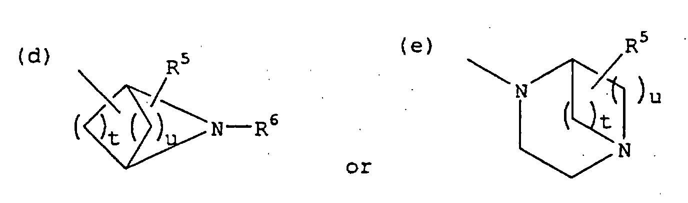 wherein a dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, monoalkylamino, dialkylamino, alkyl, alkoxycarbonyl, alkylsulfonyl, acyl, acylamino, benzoylamino optionally having substituent(s), hydroxy, arylalkyl, sulfamoyl or alkylsulfonylamino, or represented by the following formula (f)-(i): 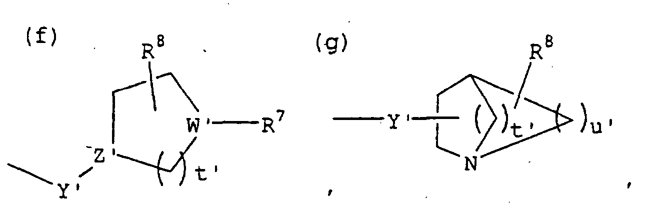 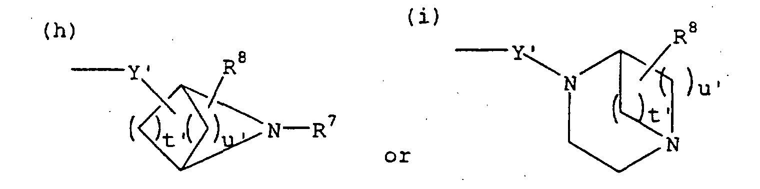
wherein Y' is as defined for Y above, Z' is CH or a nitrogen atom, W' is CH, a nitrogen atom or an oxygen atom, t' is an integer of 1-3, u' is an integer of 1-3, provided that when the above-mentioned formula (a) is piperazine, then R⁶ may be hydroxyalkyl, R⁷ is hydrogen, amino, monoalkylamino, dialkylamino, alkyl, alkoxycarbonyl, alkylsulfonyl, acyl, hydroxyalkyl, acylamino or benzoylamino optionally having substituent(s), provided that when W' is an oxygen atom, then R⁷ should be absent, and R⁸ is hydrogen, alkyl or hydroxyalkyl,
provided that (1) when X is the unsubstituted carbon atom, the ring Ar is a benzene ring, Y is -(CH₂)ₘ- (m=0) and R is monoalkylamino, dialkylamino, piperidinyl, 3-methyl-1-piperidino, piperazin-1-yl, 4-methylpiperazin-1-yl, 1-piperidino, 4-morpholino or 4-(2-hydroxyethyl)piperazin-1-yl, then R¹ should be halogen, alkyl, alkoxy, haloalkyl, hydroxy, amino, dialkylamino, nitro, cyano, acyl, carboxy, ester, carbamoyl, N-alkylcarbamoyl, N,N-dialkylcarbamoyl, acylamino, diacylamino, thiol, alkylthio, alkoxycarbonylamino, sulfamoyl, N-alkylsulfamoyl or N,N-dialkylsulfamoyl, and (2) when X is a nitrogen atom and Y is -(CH₂)ₘ- (m=0), then R should be represented by any of the above-mentioned formulas (b)-(d), an optically active form thereof, a pharmaceutically acceptable salt thereof, a hydrate thereof or a water adduct thereof.

2. The fused heterocyclic compound of claim 1, wherein, in the formula (I),
X is a carbon atom optionally substituted by alkyl, aromatic heterocyclic group optionally having substituent(s) or phenyl optionally having substituent(s),
an optically active form thereof, a pharmaceutically acceptable salt thereof, a hydrate thereof or a water adduct thereof.

3. The fused heterocyclic compound of claim 1, wherein, in the formula (I),
R¹ is halogen, alkyl, alkoxy, haloalkyl, hydroxy, amino, dialkylamino, nitro, cyano, acyl, carboxy, ester, carbamoyl, N-alkylcarbamoyl, N,N-dialkylcarbamoyl, acylamino, diacylamino, thiol, alkylthio, alkoxycarbonylamino, sulfamoyl, N-alkylsulfamoyl, N,N-dialkylsulfamoyl or alkoxyalkyloxy, and
R² is hydrogen,
an optically active form thereof, a pharmaceutically acceptable salt thereof, a hydrate thereof or a water adduct thereof.

4. The fused heterocyclic compound of claim 1, wherein, in the formula (I),
the dotted line part is a single bond or a double bond,
ring Ar is a benzene ring, a naphthalene ring or an aromatic heterocycle,
X is a carbon atom optionally substituted by alkyl or phenyl optionally having substituent(s), or a nitrogen atom,
Y is
- (CH₂)ₘ-,
-(CH₂)ₘ-N(R⁴)-CO-(CH₂)ₙ- or
-(CH₂)ₘ-CO-(CH₂)ₙ-
wherein m and n are the same or different and each is 0 or an integer of 1-10, R⁴ is hydrogen, and -(CH₂)ₘ- is linked with a parent-nucleus,
R¹ and R² are the same or different and each is hydrogen, halogen, alkyl, alkoxy, haloalkyl, hydroxy, amino, dialkylamino, nitro, cyano, carboxy, N,N-dialkylcarbamoyl, alkylthio or alkoxyalkyloxy, and
R is dialkylamino or morpholino, or represented by the following formula (a)-(d): 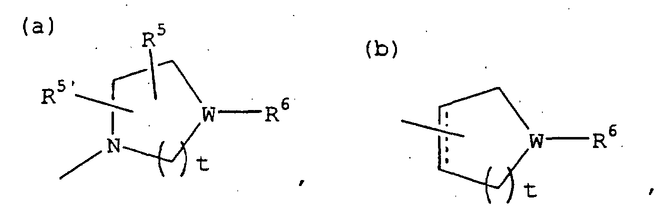 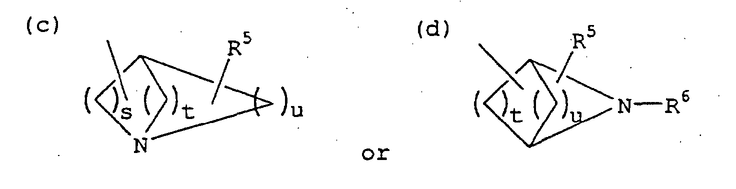 wherein a dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, dialkylamino, alkyl, alkoxycarbonyl, alkylsulfonyl, acylamino, hydroxy, arylalkyl, sulfamoyl or alkylsulfonylamino, or represented by the following formula (f): 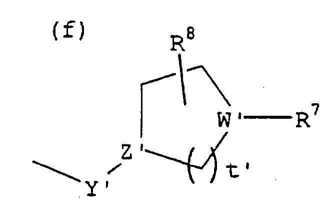 wherein Y' is as defined for Y above, Z' is CH or a nitrogen atom, W' is CH, a nitrogen atom or an oxygen atom, t' is an integer of 1-3, provided that when the above-mentioned formula (a) is piperazine, then R⁶ may be hydroxyalkyl, R⁷ is hydrogen or alkyl, provided that when W' is an oxygen atom, then R⁷ should be absent, and R⁸ is hydrogen,
an optically active form thereof, a pharmaceutically acceptable salt thereof, a hydrate thereof or a water adduct thereof.

5. The fused heterocyclic compound of claim 1, wherein, in the formula (I),
the dotted line part is a single bond or a double bond,
ring Ar is a benzene ring, a naphthalene ring, or an aromatic heterocycle selected from pyridine, pyrazole and thiophene,
X is a carbon atom optionally substituted by alkyl or phenyl optionally having substituent(s) selected from the group consisting of halogen, alkyl and alkoxy, or a nitrogen atom,
Y is
- (CH₂)ₘ-,
-(CH₂)ₘ-N(R⁴)-CO-(CH₂)ₙ- or
- (CH₂)ₘ-CO-(CH₂)ₙ-
wherein m and n are the same or different and each is 0 or an integer of 1-5, R⁴ is hydrogen, and -(CH₂)ₘ- is linked with a parent-nucleus,
R¹ and R² are the same or different and each is hydrogen, halogen, alkyl, alkoxy, haloalkyl, hydroxy, amino, dialkylamino, nitro, cyano, carboxy, N,N-dialkylcarbamoyl, alkylthio or alkoxyalkyloxy, and
R is dialkylamino or morpholino, or represented by the following formula (a)-(d): 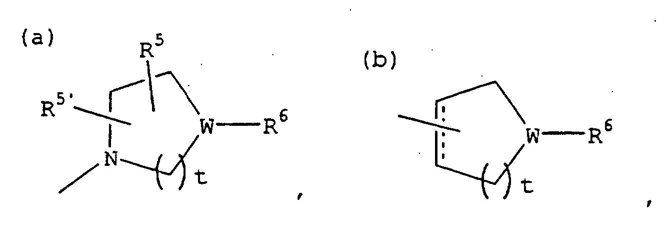 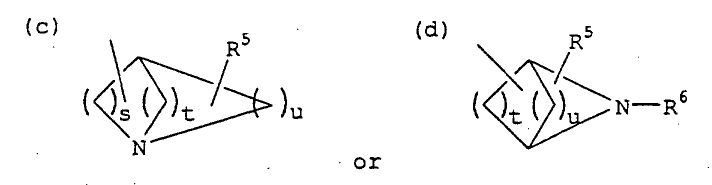 wherein the dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, dialkylamino, alkyl, alkoxycarbonyl, alkylsulfonyl, acylamino, hydroxy, arylalkyl, sulfamoyl or alkylsulfonylamino, or represented by the following formula (f): 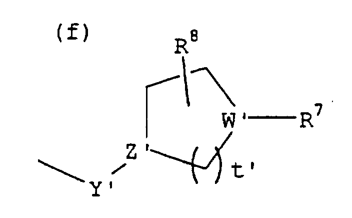 wherein Y' is as defined for Y above, Z' is nitrogen atom, W' is CH, a nitrogen atom or an oxygen atom, t' is an integer of 1-3, provided that when the above-mentioned formula (a) is piperazine, then R⁶ may be hydroxyalkyl, R⁷ is hydrogen or alkyl, provided that when W' is an oxygen atom, then R⁷ should be absent, and R⁸ is a hydrogen;
provided that when X is a nitrogen atom, then R should be represented by the above-mentioned formula (b), an optically active form thereof, a pharmaceutically acceptable salt thereof, a hydrate thereof or a water adduct thereof.

6. The fused heterocyclic compound of claim 1, which is selected from
(1) 5-methyl-3-(4-methylpiperazin-1-yl)-2H-isoquinolin-1-one,
(2) 3-(3-dimethylaminopyrrolidin-1-yl)-2H-isoquinolin-1-one,
(3) 3-(4-dimethylaminopiperidin-1-yl)-2H-isoquinolin-1-one,
(4) 3-(3-hydroxymethyl-4-methylpiperazin-1-yl)-2H-isoquinolin-1-one,
(5) 3-(4-aminopiperazin-1-yl)-2H-isoquinolin-1-one,
(6) 3-(4-dimethylaminopiperazin-1-yl)-2H-isoquinolin-1-one,
(7) 3-(4-propylpiperazin-1-yl)-2H-isoquinolin-1-one,
(8) 3-(4-methanesulfonylpiperazin-1-yl)-2H-isoquinolin-1-one,
(9) 3-(4-ethoxycarbonylpiperazin-1-yl)-2H-isoquinolin-1-one,
(10) 3-(4-methylhomopiperazin-1-yl)-2H-isoquinolin-1-one,
(11) 5-methyl-3-(4-methylhomopiperazin-1-yl)-2H-isoquinolin-1-one,
(12) 5-methyl-3-(4-dimethylaminopiperidin-1-yl)-2H-isoquinolin-1-one,
(13) 3-(3-dimethylaminopyrrolidin-1-yl)-5-methyl-2H-isoquinolin-1-one,
(14) 5-methyl-3-(4-morpholino)-2H-isoquinolin-1-one,
(15) 3-(4-aminopiperazin-1-yl)-5-methyl-2H-isoquinolin-1-one,
(16) 3-(4-dimethylaminopiperazin-1-yl)-5-methyl-2H-isoquinolin-1-one,
(17) 3-(4-hydroxypiperidin-1-yl)-5-methyl-2H-isoquinolin-1-one,
(18) 5-methoxy-3-(4-methylpiperazin-1-yl)-2H-isoquinolin-1-one,
(19) 5-hydroxy-3-(4-methylpiperazin-1-yl)-2H-isoquinolin-1-one,
(20) 5-fluoro-3-(4-methylpiperazin-1-yl)-2H-isoquinolin-1-one,
(21) 5-chloro-3-(4-methylpiperazin-1-yl)-2H-isoquinolin-1-one,
(22) 5-bromo-3-(4-methylpiperazin-1-yl)-2H-isoquinolin-1-one,
(23) 8-chloro-3-(4-methylpiperazin-1-yl)-2H-isoquinolin-1-one,
(24) 7-methyl-3-(4-methylpiperazin-1-yl)-2H-isoquinolin-1-one,
(25) 7-bromo-3-(4-methylpiperazin-1-yl)-2H-isoquinolin-1-one,
(26) 3-(4-dimethylaminopiperidin-1-yl)-5-methoxy-2H-isoquinolin-1-one,
(27) 5-hydroxy-3-(4-dimethylaminopiperidin-1-yl)-2H-isoquinolin-1-one,
(28) 5-fluoro-3-(4-dimethylaminopiperidin-1-yl)-2H-isoquinolin-1-one,
(29) 5-chloro-3-(4-dimethylaminopiperidin-1-yl)-2H-isoquinolin-1-one,
(30) 6-chloro-3-(4-methylpiperazin-1-yl)-2H-isoquinolin-1-one,
(31) 7-bromo-3-(4-dimethylaminopiperidin-1-yl)-2H-isoquinolin-1-one,
(32) 5-bromo-3-(4-dimethylaminopiperidin-1-yl)-2H-isoquinolin-1-one,
(33) 5-fluoro-3-(4-(2-hydroxyethyl)piperazin-1-yl)-2H-isoquinolin-1-one,
(34) 6-methyl-3-(4-methylpiperazin-1-yl)-2H-isoquinolin-1-one,
(35) 3-(4-(2-hydroxyethyl)piperazin-1-yl)-6-methyl-2H-isoquinolin-1-one,
(36) 8-methyl-3-(4-methylpiperazin-1-yl)-2H-isoquinolin-1-one,
(37) 7-bromo-3-(4-(2-hydroxyethyl)piperazin-1-yl)-2H-isoquinolin-1-one,
(38) 3-(4-methylpiperazin-1-yl)-5-nitro-2H-isoquinolin-1-one,
(39) 5-amino-3-(4-methylpiperazin-1-yl)-2H-isoquinolin-1-one 1 water adduct,
(40) 5-cyano-3-(4-methylpiperazin-1-yl)-2H-isoquinolin-1-one,
(41) 3-[4-(2-hydroxyethyl)piperazin-1-yl]-8-methyl-2H-isoquinolin-1-one,
(42) 3-(4-methylpiperazin-1-yl)-5-trifluoromethyl-2H-isoquinolin-1-one,
(43) 3-[4-(2-hydroxyethyl)piperazin-1-yl]-7-methyl-1H-isoquinolin-1-one,
(44) 3-(4-methylpiperazin-1-yl)-5-methylthio-2H-isoquinolin-1-one,
(45) 5-dimethylamino-3-(4-methylpiperazin-1-yl)-2H-isoquinolin-1-one,
(46) 3-(4-dimethylaminopiperidin-1-yl)-5-nitro-2H-isoquinolin-1-one,
(47) 5-amino-3-(4-dimethylaminopiperidin-1-yl)-2H-isoquinolin-1-one,
(48) 3-(4-dimethylaminopiperidin-1-yl)-5-trifluoromethyl-2H-isoquinolin-1-one,
(49) 3-(4-dimethylaminopiperidin-1-yl)-5-methylthio-2H-isoquinolin-1-one,
(50) 5-cyano-3-(4-dimethylaminopiperidin-1-yl)-2H-isoquinolin-1-one,
(51) 5,7-dimethyl-3-(4-methylpiperazin-1-yl)-2H-isoquinolin-1-one,
(52) 5,7-dichloro-3-(4-methylpiperazin-1-yl)-2H-isoquinolin-1-one,
(53) 5,7-dibromo-3-(4-methylpiperazin-1-yl)-2H-isoquinolin-1-one,
(54) 5,7-difluoro-3-(4-methylpiperazin-1-yl)-2H-isoquinolin-1-one,
(55) 5-chloro-7-fluoro-3-(4-methylpiperazin-1-yl)-2H-isoquinolin-1-one,
(56) 6,7-dihydroxy-3-(4-methylpiperazin-1-yl)-2H-isoquinolin-1-one,
(57) 5,7-dichloro-3-(4-dimethylaminopiperidin-1-yl)-2H-isoquinolin-1-one,
(58) 5,7-dibromo-3-(4-dimethylaminopiperidin-1-yl)-2H-isoquinolin-1-one,
(59) 5-bromo-7-chloro-3-(4-dimethylaminopiperidin-1-yl)-2H-isoquinolin-1-one,
(60) 6,7-dihydroxy-3-(4-dimethylaminopiperidin-1-yl)-2H-isoquinolin-1-one,
(61) 3-[4-(4-morpholino)piperidin-1-yl]-2H-isoquinolin-1-one,
(62) 3-{4-[2-(piperidin-1-yl)ethyl)piperazin-1-yl}-2H-isoquinolin-1-one,
(63) 3-{4-[3-(piperidin-1-yl)propyl)piperazin-1-yl}-2H-isoquinolin-1-one,
(64) 3-{4-[4-(4-morpholino)butyl]piperazin-1-yl}-2H-isoquinolin-1-one,
(65) 3-{4-[4-(piperidin-1-yl)butyl]piperazin-1-yl}-2H-isoquinolin-1-one,
(66) 3-{4-[5-(piperidin-1-yl)pentyl)piperazin-1-yl}-2H-isoquinolin-1-one,
(67) 3-(4-(4-(4-methylpiperazin-1-yl)butyl)piperazin-1-yl)-2H-isoquinolin-1-one,
(69) 5-methyl-3-[4-(4-morpholino)piperidin-1-yl]-2H-isoquinolin-1-one,
(70) 5-methyl-3-{4-[2-(piperidin-1-yl)ethyl]piperazin-1-yl}-2H-isoquinolin-1-one,
(71) 5-methyl-3-{4-[3-(piperidin-1-yl)propyl]piperazin-1-yl}-2H-isoquinolin-1-one,
(72) 5-methyl-3-{4-[5-(piperidin-1-yl)pentyl]piperazin-1-yl}-2H-isoquinolin-1-one,
(73) 5-methyl-3-{4-[4-(piperidin-1-yl)butyl]piperazin-1-yl}-2H-isoquinolin-1-one,
(74) 5-methyl-3-{4-[4-(4-morpholino)butyl]piperazin-1-yl}-2H-isoquinolin-1-one,
(75) 5-methyl-3-(4-(4-(4-methylpiperazin-1-yl)butyl)piperazin-1-yl)-2H-isoquinolin-1-one,
(76) 7-bromo-3-{4-[4-(piperidin-1-yl)butyl]piperazin-1-yl}-2H-isoquinolin-1-one,
(77) 5-chloro-3-{4-[4-(piperidin-1-yl)butyl]piperazin-1-yl}-2H-isoquinolin-1-one,
(78) 5-bromo-3-{4-[4-(piperidin-1-yl)butyl]piperazin-1-yl}-2H-isoquinolin-1-one,
(80) 5-chloro-3-{4-[4-(4-morpholino)butyl]piperazin-1-yl}-2H-isoquinolin-1-one,
(81) 3-(piperidin-4-yl)-2H-isoquinolin-1-one hydrobromide,
(82) 3-(1-methylpiperidin-4-yl)-2H-isoquinolin-1-one 1/5 water adduct,
(83) 3-((4-methylpiperazin-1-yl)carbonyl)-2H-isoquinolin-1-one,
(84) 3-(2-(dimethylamino)ethyl)-2H-isoquinolin-1-one,
(85) 3-(3-(dimethylamino)propyl)-2H-isoquinolin-1-one,
(86) 3-(1-azabicyclo[2.2.2]octan-3-yl)-2H-isoquinolin-1-one,
(87) 3-((1-azabicyclo[2.2.2]octan-3-yl)methyl)-2H-isoquinolin-1-one,
(88) 3-(8-methyl-8-azabicyclo[3.2.1]octan-3-yl)-2H-isoquinolin-1-one,
(89) 5-methyl-3-(2-(dimethylamino)ethyl)-2H-isoquinolin-1-one,
(90) 3-(3-(dimethylamino)propyl)-5-methyl-2H-isoquinolin-1-one,
(91) 3-(1-azabicyclo[2.2.2]octan-3-yl)-5-methyl-2H-isoquinolin-1-one,
(92) 3-((1-azabicyclo[2.2.2]octan-3-yl)methyl)-5-methyl-2H-isoquinolin-1-one,
(93) 3-(piperidin-4-yl)-5-methyl-2H-isoquinolin-1-one hydrochloride,
(94) 5-methyl-3-(8-methyl-8-azabicyclo[3.2.1]octan-3-yl)-2H-isoquinolin-1-one,
(95) 5-chloro-3-(1-methylpiperidin-4-yl)-2H-isoquinolin-2-one,
(96) 5-bromo-3-(1-methylpiperidin-4-yl)-2H-isoquinolin-1-one,
(97) 4-phenyl-3-(piperidin-4-yl)-2H-isoquinolin-1-one hydrobromide,
(98) 3-(1-methylpiperidin-4-yl)-4-phenyl-2H-isoquinolin-1-one 1/5 water adduct,
(99) 4-(4-methoxyphenyl)-3-(1-methylpiperidin-4-yl)-2H-isoquinolin-1-one,
(100) 4-(4-chlorophenyl)-3-(1-methylpiperidin-4-yl)-2H-isoquinolin-1-one,
(101) 5,7-dibromo-3-(piperidin-4-yl)-2H-isoquinolin-1-one,
(102) 5-methoxy-3-(piperidin-4-yl)-2H-isoquinolin-1-one,
(103) 5-hydroxy-3-(piperidin-4-yl)-2H-isoquinolin-1-one,
(104) 5-fluoro-3-(piperidin-4-yl)-2H-isoquinolin-1-one,
(105) 3-(1-methylpiperidin-4-yl)-5-trifluoromethyl-2H-isoquinolin-1-one,
(106) 5-fluoro-3-(1-methylpiperidin-4-yl)-2H-isoquinolin-1-one,
(107) 5-methoxy-3-(1-methylpiperidin-4-yl)-2H-isoquinolin-1-one,
(108) 5-hydroxy-3-(1-methylpiperidin-4-yl)-2H-isoquinolin-1-one,
(109) 5-chloro-3-(1-methylpiperidin-4-yl)-4-phenyl-2H-isoquinolin-1-one,
(110) 5-(4-methylpiperazin-1-yl)-6H-thieno[2,3-c]pyridin-7-one,
(111) 5-(4-dimethylaminopiperidin-1-yl)-6H-thieno[2,3-c]pyridin-7-one,
(112) 6-(4-methylpiperazin-1-yl)-5H-thieno[3,2-c]pyridin-4-one,
(113) 6-(4-dimethylaminopiperidin-1-yl)-5H-thieno[3,2-c]pyridin-4-one,
(114) 3-(4-methylpiperazin-1-yl)-2H-benz[f]isoquinolin-1-one,
(115) 3-(4-dimethylaminopiperidin-1-yl)-2H-benz[f]isoquinolin-1-one,
(116) 3-(4-methylpiperazin-1-yl)-2H-benz[h]isoquinolin-1-one;
(117) 3-(4-dimethylaminopiperidin-1-yl)-2H-benz[h]isoquinolin-1-one,
(118) 7-(4-methylpiperazin-1-yl)-6H-1,6-naphthyridin-5-one,
(119) 7-(4-dimethylaminopiperidin-1-yl)-6H-1,6-naphthyridin-5-one,
(120) 8-methyl-2-(piperidin-4-yl)-3H-quinazolin-4-one,
(121) 2-(1-methylpiperidin-4-yl)-8-methyl-3H-quinazolin-4-one,
(123) 8-methoxy-2-(1-methylpiperidin-4-yl)-3H-quinazolin-4-one,
(124) 8-hydroxy-2-(1-methylpiperidin-4-yl)-3H-quinazolin-4-one,
(125) 8-fluoro-2-(1-methylpiperidin-4-yl)-3H-quinazolin-4-one,
(126) 8-chloro-2-(1-methylpiperidin-4-yl)-3H-quinazolin-4-one,
(127) 8-bromo-2-(1-methylpiperidin-4-yl)-3H-quinazolin-4-one,
(128) 8-methoxy-2-(4-dimethylaminocyclohexan-1-yl)-3H-quinazolin-4-one,
(129) 8-hydroxy-2-(4-dimethylaminocyclohexan-1-yl)-3H-quinazolin-4-one,
(130) 8-fluoro-2-(4-dimethylaminocyclohexan-1-yl)-3H-quinazolin-4-one,
(131) 8-chloro-2-(4-dimethylaminocyclohexan-1-yl)-3H-quinazolin-4-one,
(132) 8-bromo-2-(4-dimethylaminocyclohexan-1-yl)-3H-quinazolin-4-one,
(133) 2-(1-azabicyclo[2.2.2]octan-3-yl)-3H-quinazolin-4-one,
(134) 2-((1-azabicyclo[2.2.2]octan-3-yl)methyl)-3H-quinazolin-4-one,
(135) 2-(8-methyl-8-azabicyclo[3.2.1]octan-3-yl)-3H-quinazolin-4-one,
(136) 2-(4-dimethylaminocyclohexan-1-yl)-3H-quinazolin-4-one,
(137) 8-methyl-2-(4-dimethylaminocyclohexan-1-yl)-3H-quinazolin-4-one,
(138) 2-(1-azabicyclo[2.2.2]octan-3-yl)-8-methyl-3H-quinazolin-4-one,
(139) 2-((1-azabicyclo[2.2.2]octan-3-yl)methyl)-8-methyl-3H-quinazolin-4-one,
(140) 2-(2-(dimethylamino)ethyl)-3H-quinazolin-4-one,
(141) 2-(3-(dimethylamino)propyl)-3H-quinazolin-4-one,
(142) 2-(5-(dimethylamino)pentyl)-3H-quinazolin-4-one,
(143) 8-methyl-2-(8-methyl-8-azabicyclo[3.2.1]octan-3-yl)-3H-quinazolin-4-one,
(144) 8-methyl-2-(2-(dimethylamino)ethyl)-3H-quinazolin-4-one,
(145) 8-methyl-2-(3-(dimethylamino)propyl)-3H-quinazolin-4-one,
(146) 8-methyl-2-(5-(dimethylamino)pentyl)-3H-quinazolin-4-one,
(147) 3-(4-(dimethylamino)cyclohexan-1-yl)-2H-isoquinolin-1-one, and
(148) 3-(3-(4-methylpiperazin-1-yl)propyl)-2H-isoquinolin-1-one,
an optically active form thereof, a pharmaceutically
acceptable salt thereof, a hydrate thereof or a water adduct thereof.

7. The fused heterocyclic compound of claim 1, which is selected from
(151) (R)-3-(2-hydroxymethyl-4-methylpiperazin-1-yl)-2H-isoquinolin-1-one,
(152) (S)-3-(2-hydroxymethyl-4-methylpiperazin-1-yl)-2H-isoquinolin-1-one,
(153) 3-(3-hydroxymethyl-4-methylpiperazin-1-yl)-5-methyl-2H-isoquinolin-1-one,
(154) 3-(3-ethoxycarbonyl-4-methylpiperazin-1-yl)-2H-isoquinolin-1-one,
(155) 3-(3-methylpiperazin-1-yl)-2H-isoquinolin-1-one,
(156) (S)-3-(3-hydroxymethyl-4-methylpiperazin-1-yl)-2H-isoquinolin-1-one,
(157) (R)-3-(3-hydroxymethyl-4-methylpiperazin-1-yl)-2H-isoquinolin-1-one,
(158) 3-(1-methylpiperidin-4-yl)-3,4-dihydro-2H-isoquinolin-1-one,
(159) 8-methyl-2-[2-(diethylamino)ethyl]-3H-quinazolin-4-one,
(162) 3-(3,5-dimethylpiperazin-1-yl)-2H-isoquinolin-1-one,
(163) 4-(4-methylphenyl)-3-(1-methylpiperidin-4-yl)-2H-isoquinolin-1-one,
(164) 4-(4-fluorophenyl)-3-(1-methylpiperidin-4-yl)-2H-isoquinolin-1-one,
(165) 8-methyl-2-(2-piperidinoethyl)-3H-quinazolin-4-one,
(166) 8-methyl-2-[2-(morpholin-4-yl)ethyl]-3H-quinazolin-4-one,
(167) 4-(2-methoxyphenyl)-3-(1-methylpiperidin-4-yl)-2H-isoquinolin-1-one,
(168) 4-(3-methylphenyl)-3-(1-methylpiperidin-4-yl)-2H-isoquinolin-1-one,
(169) 4-(2-methylphenyl)-3-(1-methylpiperidin-4-yl)-2H-isoquinolin-1-one,
(170) 4-(3-methoxyphenyl)-3-(1-methylpiperidin-4-yl)-2H-isoquinolin-1-one,
(171) 5-methoxymethyloxy-3-(1-methylpiperidin-4-yl)-2H-isoquinolin-1-one,
(173) 8-methyl-2-[5-(diethylamino)pentyl]-3H-quinazolin-4-one,
(174) 8-methyl-2-[4-(diethylamino)butyl]-3H-quinazolin-4-one,
(175) 8-methyl-2-[4-(dimethylamino)butyl]-3H-quinazolin-4-one,
(176) 8-methyl-2-[3-(pyrrolidin-1-yl)propyl]-3H-quinazolin-4-one,
(177) 7-(1-methylpiperidin-4-yl)-6H-1,6-naphthyridin-5-one 1/10 water adduct,
(178) 5-methyl-3-(1-methylpiperidin-4-yl)-2H-isoquinolin-1-one,
(179) 4-methyl-3-(1-methylpiperidin-4-yl)-2H-isoquinolin-1-one,
(180) 5-(dimethylamino)-3-(1-methylpiperidin-4-yl)-2H-isoquinolin-1-one,
(181) 5-amino-3-(1-methylpiperidin-4-yl)-2H-isoquinolin-1-one,
(182) 4-(2-fluorophenyl)-3-(1-methylpiperidin-4-yl)-2H-isoquinolin-1-one,
(183) 7-chloro-3-(1-methylpiperidin-4-yl)-2H-isoquinolin-1-one,
(184) 5-hydroxy-3-(1-methylpiperidin-4-yl)-3,4-dihydro-2H-isoquinolin-1-one,
(185) 5-methoxymethyloxy-3-(3-dimethylaminopropyl)-2H-isoquinolin-1-one,
(186) 5-hydroxy-3-(3-dimethylaminopropyl)-2H-isoquinolin-1-one hydrochloride,
(187) 5-methoxymethyloxy-3-(4-dimethylaminobutyl)-2H-isoquinolin-1-one,
(188) 5-hydroxy-3-(4-dimethylaminobutyl)-2H-isoquinolin-1-one hydrochloride,
(189) 5-hydroxy-3-(2-(piperidin-1-yl)ethyl)-2H-isoquinolin-1-one,
(190) 3-(1-methyl-1,2,3,6-tetrahydropyridin-4-yl)-2H-isoquinolin-1-one,
(191) 3-(1-benzylpiperidin-3-yl)-2H-isoquinolin-1-one,
(192) 3-(1-methylpiperidin-3-yl)-2H-isoquinolin-1-one,
(193) 3-(1-methyl-1,2,3,6-tetrahydropyridin-5-yl)-2H-isoquinolin-1-one,
(194) 3-(3-hydroxymethylpiperazin-1-yl)-2H-isoquinolin-1-one,
(195) 3-(4-ethyl-3-hydroxymethylpiperazin-1-yl)-2H-isoquinolin-1-one,
(196) 3-(3-hydroxymethyl-4-propylpiperazin-1-yl)-2H-isoquinolin-1-one,
(197) 3-(4-benzyl-3-hydroxymethylpiperazin-1-yl)-2H-isoquinolin-1-one,
(198) 5-bromo-3-(3-hydroxymethylpiperazin-1-yl)-2H-isoquinolin-1-one,
(199) 5-bromo-3-(3-hydroxymethyl-4-methylpiperazin-1-yl)-2H-isoquinolin-1-one,
(200) 3-(4-piperidinopiperidin-1-yl)-2H-isoquinolin-1-one,
(201) 3-(3-hydroxymethylpiperidin-1-yl)-2H-isoquinolin-1-one,
(202) 3-(3-(dimethylcarbamoyl)piperidin-1-yl)-2H-isoquinolin-1-one,
(203) 3-(3-hydroxymethyl-4-isobutylpiperazin-1-yl)-2H-isoquinolin-1-one,
(204) 3-[4-(dimethylamino)butyl]-2H-isoquinolin-1-one,
(205) 5-fluoro-3-(3-hydroxymethyl-4-methylpiperazin-1-yl)-2H-isoquinolin-1-one,
(206) 3-(3-(dimethylaminomethyl)piperidin-1-yl)-2H-isoquinolin-1-one,
(207) 6-methyl-3-(1-methylpiperidin-4-yl)-2H-isoquinolin-1-one,
(208) 7-methyl-3-(1-methylpiperidin-4-yl)-2H-isoquinolin-1-one,
(209) 8-methyl-3-(1-methylpiperidin-4-yl)-2H-isoquinolin-1-one,
(210) 7-methoxymethyloxy-3-(1-methylpiperidin-4-yl)-2H-isoquinolin-1-one,
(211) 7-hydroxy-3-(1-methylpiperidin-4-yl)-2H-isoquinolin-1-one hydrochloride,
(212) (S)-3-(3-hydroxymethyl-4-methylpiperazin-1-yl)-5-methyl-2H-isoquinolin-1-one,
(213) 3-(3-(pyrrolidin-1-yl)propyl)-2H-isoquinolin-1-one,
(214) 5-chloro-3-(3-hydroxymethyl-4-methylpiperazin-1-yl)-2H-isoquinolin-1-one,
(216) 3-[3-(piperidin-1-yl)propyl]-2H-isoquinolin-1-one,
(217) 5-hydroxy-3-(3-(pyrrolidin-1-yl)propyl)-2H-isoquinolin-1-one,
(218) 5-methyl-3-[2-(piperidin-1-yl)ethyl]-2H-isoquinolin-1-one,
(219) 3-[2-(piperidin-1-yl)ethyl]-2H-isoquinolin-1-one,
(220) 3-[2-(pyrrolidin-1-yl)ethyl]-2H-isoquinolin-1-one,
(221) 5-methyl-3-[2-(pyrrolidin-1-yl)ethyl]-2H-isoquinolin-1-one,
(222) 5-methyl-3-[3-(pyrrolidin-1-yl)propyl-1-yl]-2H-isoquinolin-1-one,
(223) 1,5-dihydro-6-(1-methyl-1,2,3,6-tetrahydropyridin-4-yl)pyrazolo[4,3-c]pyridin-4-one,
(224) N,N-dimethyl-3-(1-methylpiperidin-1-yl)-2H-1-oxoisoquinoline-5-carboxamide 1/4 water adduct,
(225) 5-methyl-3-(octahydroindolizin-7-yl)-2H-isoquinolin-1-one 3/4 water adduct,
(226) 5-methyl-3-(octahydroindolizin-7-yl)-2H-isoquinolin-1-one 1/2 water adduct,
(227) 3-(2-methylpiperidin-1-yl)-2H-1-oxoisoquinoline-5-carboxylic acid hydrochloride,
(228) 5-methyl-3-[3-(piperidin-1-yl)propyl]-2H-isoquinolin-1-one,
(229) 3-(dimethylamino)methyl-2H-isoquinolin-1-one,
(230) 3-[(4-methylpiperazin-1-yl)methyl)-2H-isoquinolin-1-one,
(231) 3-(piperidinomethyl)-2H-isoquinolin-1-one,
(232) 3-[(morpholin-1-yl)methyl)-2H-isoquinolin-1-one,
(233) 3-[(homopiperidin-1-yl)methyl]-2H-isoquinolin-1-one,
(234) 3-[3-(homopiperidin-1-yl)propyl]-2H-isoquinolin-1-one,
(235) 3-(1-sulfamoylpiperidin-4-yl)-5-methyl-2H-isoquinolin-1-one 1/4 water adduct,
(236) 3-(4-methyl-3-oxopiperazin-1-yl)-5-methyl-2H-isoquinolin-1-one 1/10 water adduct,
(237) 3-(1-aminopiperidin-4-yl)-5-methyl-2H-isoquinolin-1-one,
(238) 3-(1-(methanesulfonylamino)piperidin-4-yl)-5-methyl-2H-isoquinolin-1-one,
(239) 3-(1-trifluoroacetaminopiperidin-4-yl)-5-methyl-2H-isoquinolin-1-one,
(240) 3-[2-(homopiperidin-1-yl)ethyl]-2H-isoquinolin-1-one,
(241) N-(5-methyl-2H-1-oxoisoquinolin-3-yl)-2-(dimethylamino)acetamide,
(243) N-(5-methyl-2H-1-oxoisoquinolin-3-yl)-3-(dimethylamino)propanamide,
(244) 3-(1-dimethylaminopiperidin-4-yl)-5-methyl-2H-isoquinolin-1-one,
(245) N-(5-methyl-2H-1-oxoisoquinolin-3-yl)-4-(dimethylamino)butanamide,
(246) N-(2H-1-oxoisoquinolin-3-yl)-4-(dimethylamino)butanamide,
(247) 3-(4-methyl-2-oxopiperazin-1-yl)-2H-isoquinolin-1-one,
(248) 5-methyl-3-(1-methylpyrrolidin-3-yl)-2H-isoquinolin-1-one, and
(249) 3-(1-methylpyrrolidin-3-yl)-2H-isoquinolin-1-one,
an optically active form thereof, a pharmaceutically acceptable salt thereof, a hydrate thereof or a water adduct thereof.

8. The fused heterocyclic compound of claim 1, wherein, in the formula (I),
the dotted line part is a single bond or a double bond,
ring Ar is a benzene ring, a naphthalene ring or an aromatic heterocycle,
X is a carbon atom optionally substituted by alkyl or phenyl optionally having substituent(s), or a nitrogen atom,
Y is
- (CH₂)ₘ-,
- (CH₂)ₘ-N(R⁴)-CO-(CH₂)ₙ- or
- (CH₂)ₘ-CO-(CH₂)ₙ-
wherein m and n are the same or different and each is 0 or an integer of 1-10, R⁴ is hydrogen, and -(CH₂)ₘ- is linked with a parent-nucleus,
R¹ and R² are the same or different and each is hydrogen, halogen, alkyl, alkoxy, hydroxy, amino, dialkylamino, nitro, carboxy, N,N-dialkylcarbamoyl or alkoxyalkyloxy, and R is dialkylamino or morpholino, or represented by the following formula (a)-(c): 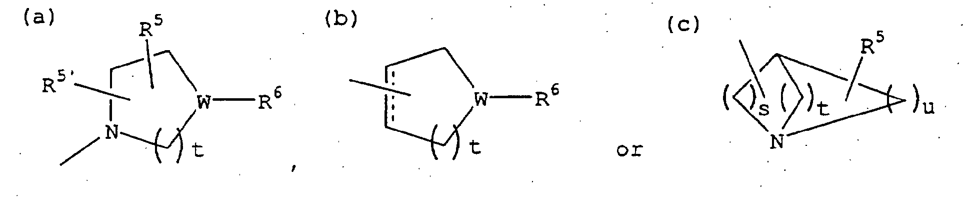 wherein the dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, dialkylamino, alkyl, alkoxycarbonyl, alkylsulfonyl, acylamino, hydroxy, arylalkyl, sulfamoyl or alkylsulfonylamino, or represented by the following formula (f): 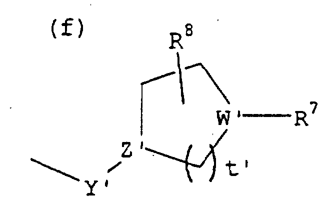 wherein Y' is as defined for Y above, Z' is a nitrogen atom, W' is CH, a nitrogen atom or an oxygen atom, t' is an integer of 1-3, provided that when the above-mentioned formula (a) is piperazine, then R⁶ may be hydroxyalkyl, R⁷ is hydrogen or alkyl, provided that when W' is an oxygen atom, then R⁷ should be absent, and R⁸ is hydrogen,
an optically active form thereof, a pharmaceutically acceptable salt thereof, a hydrate thereof or a water adduct thereof.

9. The fused heterocyclic compound of claim 1, wherein, in the formula (I),
the dotted line part is a single bond or a double bond,
ring Ar is a benzene ring or a naphthalene ring, or an aromatic heterocycle selected from the group consisting of pyridine, pyrazole and thiophene,
X is a carbon atom optionally substituted by phenyl optionally having substituent(s) selected from the group consisting of halogen, alkyl and alkoxy, or a nitrogen atom,
Y is
- (CH₂)ₘ-,
- (CH₂)ₘ-N(R⁴)-CO-(CH₂)ₙ- or
- (CH₂)ₘ-CO-(CH₂)ₙ-
wherein m and n are the same or different and each is 0 or an integer of 1-5, R⁴ is hydrogen, and -(CH₂)ₘ- is linked with a parent-nucleus,
R¹ and R² are the same or different and each is hydrogen, halogen, alkyl, alkoxy, hydroxy, amino, dialkylamino, carboxy, N,N-dialkylcarbamoyl or alkoxyalkyloxy, and
R is dialkylamino or morpholino, or represented by the following formula (a)-(c): 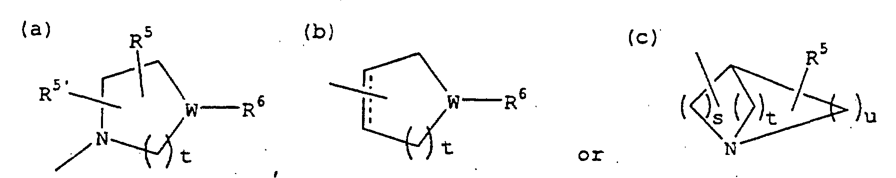 wherein the dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, dialkylamino, alkyl, alkoxycarbonyl, alkylsulfonyl, acylamino, hydroxy, arylalkyl or alkylsulfonylamino, or represented by the following formula (f) : 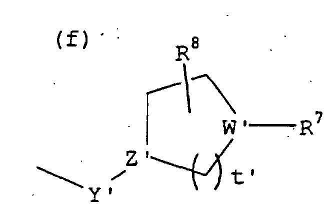 wherein Y' is as defined for Y above, Z' is a nitrogen atom, W' is CH, a nitrogen atom or an oxygen atom, t' is an integer of 1-3, provided that when the above-mentioned formula (a) is piperazine, then R⁶ may be hydroxyalkyl, R⁷ is hydrogen or alkyl, provided that when W' is an oxygen atom, then R⁷ should be absent, and R⁸ is hydrogen,
provided that when X is a nitrogen atom, then R should be represented by the above-mentioned formula (b), an optically active form thereof, a pharmaceutically acceptable salt thereof, a hydrate thereof or a water adduct thereof.

10. The fused heterocyclic compound of claim 1, which is selected from
(1) 5-methyl-3-(4-methylpiperazin-1-yl)-2H-isoquinolin-1-one,
(2) 3-(3-dimethylaminopyrrolidin-1-yl)-2H-isoquinolin-1-one,
(3) 3-(4-dimethylaminopiperidin-1-yl)-2H-isoquinolin-1-one,
(4) 3-(3-hydroxymethyl-4-methylpiperazin-1-yl)-2H-isoquinolin-1-one,
(7) 3-(4-propylpiperazin-1-yl)-2H-isoquinolin-1-one,
(8) 3-(4-methanesulfonylpiperazin-1-yl)-2H-isoquinolin-1-one,
(9) 3-(4-ethoxycarbonylpiperazin-1-yl)-2H-isoquinolin-1-one,
(10) 3-(4-methylhomopiperazin-1-yl)-2H-isoquinolin-1-one,
(11) 5-methyl-3-(4-methylhomopiperazin-1-yl)-2H-isoquinolin-1-one,
(12) 5-methyl-3-(4-dimethylaminopiperidin-1-yl)-2H-isoquinolin-1-one,
(13) 3-(3-dimethylaminopyrrolidin-1-yl)-5-methyl-2H-isoquinolin-1-one,
(14) 5-methyl-3-(4-morpholino)-2H-isoquinolin-1-one,
(17) 3-(4-hydroxypiperidin-1-yl)-5-methyl-2H-isoquinolin-1-one,
(18) 5-methoxy-3-(4-methylpiperazin-1-yl)-2H-isoquinolin-1-one,
(19) 5-hydroxy-3-(4-methylpiperazin-1-yl)-2H-isoquinolin-1-one,
(20) 5-fluoro-3-(4-methylpiperazin-1-yl)-2H-isoquinolin-1-one,
(21) 5-chloro-3-(4-methylpiperazin-1-yl)-2H-isoquinolin-1-one,
(22) 5-bromo-3-(4-methylpiperazin-1-yl)-2H-isoquinolin-1-one,
(23) 8-chloro-3-(4-methylpiperazin-1-yl)-2H-isoquinolin-1-one,
(24) 7-methyl-3-(4-methylpiperazin-1-yl)-2H-isoquinolin-1-one,
(25) 7-bromo-3-(4-methylpiperazin-1-yl)-2H-isoquinolin-1-one,
(28) 5-fluoro-3-(4-dimethylaminopiperidin-1-yl)-2H-isoquinolin-1-one,
(29) 5-chloro-3-(4-dimethylaminopiperidin-1-yl)-2H-isoquinolin-1-one,
(30) 6-chloro-3-(4-methylpiperazin-1-yl)-2H-isoquinolin-1-one,
(31) 7-bromo-3-(4-dimethylaminopiperidin-1-yl)-2H-isoquinolin-1-one,
(32) 5-bromo-3-(4-dimethylaminopiperidin-1-yl)-2H-isoquinolin-1-one,
(33) 5-fluoro-3-(4-(2-hydroxyethyl)piperazin-1-yl)-2H-isoquinolin-1-one,
(34) 6-methyl-3-(4-methylpiperazin-1-yl)-2H-isoquinolin-1-one,
(35) 3-(4-(2-hydroxyethyl)piperazin-1-yl)-6-methyl-2H-isoquinolin-1-one,
(36) 8-methyl-3-(4-methylpiperazin-1-yl)-2H-isoquinolin-1-one,
(37) 7-bromo-3-(4-(2-hydroxyethyl)piperazin-1-yl)-2H-isoquinolin-1-one,
(38) 3-(4-methylpiperazin-1-yl)-5-nitro-2H-isoquinolin-1-one,
(39) 5-amino-3-(4-methylpiperazin-1-yl)-2H-isoquinolin-1-one 1 water adduct,
(41) 3-[4-(2-hydroxyethyl)piperazin-1-yl]-8-methyl-2H-isoquinolin-1-one,
(43) 3-[4-(2-hydroxyethyl)piperazin-1-yl]-7-methyl-1H-isoquinolin-1-one,
(62) 3-{4-[2-(piperidin-1-yl)ethyl]piperazin-1-yl}-2H-isoquinolin-1-one,
(63) 3-{4-[3-(piperidin-1-yl)propyl]piperazin-1-yl}-2H-isoquinolin-1-one,
(65) 3-{4-[4-(piperidin-1-yl)butyl]piperazin-1-yl}-2H-isoquinolin-1-one,
(66) 3-{4-[5-(piperidin-1-yl)pentyl]piperazin-1-yl}-2H-isoquinolin-1-one,
(69) 5-methyl-3-[4-(4-morpholino)piperidin-1-yl]-2H-isoquinolin-1-one,
(70) 5-methyl-3-{4-[2-(piperidin-1-yl)ethyl]piperazin-1-yl}-2H-isoquinolin-1-one,
(71) 5-methyl-3-{4-[3-(piperidin-1-yl)propyl]piperazin-1-yl}-2H-isoquinolin-1-one,
(72) 5-methyl-3-{4-[5-(piperidin-1-yl)pentyl]piperazin-1-yl}-2H-isoquinolin-1-one,
(73) 5-methyl-3-{4-[4-(piperidin-1-yl)butyl]piperazin-1-yl}-2H-isoquinolin-1-one,
(74) 5-methyl-3-{4-[4-(4-morpholino)butyl]piperazin-1-yl}-2H-isoquinolin-1-one,
(75) 5-methyl-3-(4-(4-(4-methylpiperazin-1-yl)butyl)piperazin-1-yl)-2H-isoquinolin-1-one,
(76) 7-bromo-3-{4-[4-(piperidin-1-yl)butyl]piperazin-1-yl}-2H-isoquinolin-1-one,
(77) 5-chloro-3-{4-[4-(piperidin-1-yl)butyl]piperazin-1-yl}-2H-isoquinolin-1-one,
(78) 5-bromo-3-{4-[4-(piperidin-1-yl)butyl]piperazin-1-yl}-2H-isoquinolin-1-one,
(80) 5-chloro-3-{4-[4-(4-morpholino)butyl]piperazin-1-yl}-2H-isoquinolin-1-one,
(81) 3-(piperidin-4-yl)-2H-isoquinolin-1-one hydrobromide,
(82) 3-(1-methylpiperidin-4-yl)-2H-isoquinolin-1-one 1/5 water adduct,
(83) 3-((4-methylpiperazin-1-yl)carbonyl)-2H-isoquinolin-1-one,
(90) 3-(3-(dimethylamino)propyl)-5-methyl-2H-isoquinolin-1-one,
(93) 3-(piperidin-4-yl)-5-methyl-2H-isoquinolin-1-one hydrochloride,
(95) 5-chloro-3-(1-methylpiperidin-4-yl)-2H-isoquinolin-1-one,
(97) 4-phenyl-3-(piperidin-4-yl)-2H-isoquinolin-1-one hydrobromide,
(98) 3-(1-methylpiperidin-4-yl)-4-phenyl-2H-isoquinolin-1-one 1/5 water adduct,
(99) 4-(4-methoxyphenyl)-3-(1-methylpiperidin-4-yl)-2H-isoquinolin-1-one,
(100) 4-(4-chlorophenyl)-3-(1-methylpiperidin-4-yl)-2H-isoquinolin-1-one,
(106) 5-fluoro-3-(1-methylpiperidin-4-yl)-2H-isoquinolin-1-one,
(107) 5-methoxy-3-(1-methylpiperidin-4-yl)-2H-isoquinolin-1-one,
(108) 5-hydroxy-3-(1-methylpiperidin-4-yl)-2H-isoquinolin-1-one,
(110) 5-(4-methylpiperazin-1-yl)-6H-thieno[2,3-c]pyridin-7-one,
(112) 6-(4-methylpiperazin-1-yl)-5H-thieno[3,2-c]pyridin-4-one,
(114) 3-(4-methylpiperazin-1-yl)-2H-benz[f]isoquinolin-1-one,
(115) 3-(4-dimethylaminopiperidin-1-yl)-2H-benz[f]isoquinolin-1-one,
(116) 3-(4-methylpiperazin-1-yl)-2H-benz[h]isoquinolin-1-one,
(117) 3-(4-dimethylaminopiperidin-1-yl)-2H-benz[h]isoquinolin-1-one,
(120) 8-methyl-2-(piperidin-4-yl)-3H-quinazolin-4-one,
(121) 2-(1-methylpiperidin-4-yl)-8-methyl-3H-quinazolin-4-one,
(123) 8-methoxy-2-(1-methylpiperidin-4-yl)-3H-quinazolin-4-one,
(137) 8-methyl-2-(4-dimethylaminocyclohexan-1-yl)-3H-quinazolin-4-one,
(138) 2-(1-azabicyclo[2.2.2]octan-3-yl)-8-methyl-3H-quinazolin-4-one,
(139) 2-((1-azabicyclo[2.2.2]octan-3-yl)methyl)-8-methyl-3H-quinazolin-4-one,
(144) 8-methyl-2-(2-(dimethylamino)ethyl)-3H-quinazolin-4-one,
(145) 8-methyl-2-(3-(dimethylamino)propyl)-3H-quinazolin-4-one,
(146) 8-methyl-2-(5-(dimethylamino)pentyl)-3H-quinazolin-4-one,
(147) 3-(4-(dimethylamino)cyclohexan-1-yl)-2H-isoquinolin-1-one, and
(148) 3-(3-(4-methylpiperazin-1-yl)propyl)-2H-isoquinolin-1-one,
an optically active form thereof, a pharmaceutically acceptable salt thereof, a hydrate thereof or a water adduct thereof.

11. The fused heterocyclic compound of claim 1, which is selected from
(151) (R)-3-(2-hydroxymethyl-4-methylpiperazin-1-yl)-2H-isoquinolin-1-one,
(152) (S)-3-(2-hydroxymethyl-4-methylpiperazin-1-yl)-2H-isoquinolin-1-one,
(153) 3-(3-hydroxymethyl-4-methylpiperazin-1-yl)-5-methyl-2H-isoquinolin-1-one,
(154) 3-(3-ethoxycarbonyl-4-methylpiperazin-1-yl)-2H-isoquinolin-1-one,
(155) 3-(3-methylpiperazin-1-yl)-2H-isoquinolin-1-one,
(156) (S)-3-(3-hydroxymethyl-4-methylpiperazin-1-yl)-2H-isoquinolin-1-one,
(157) (R)-3-(3-hydroxymethyl-4-methylpiperazin-1-yl)-2H-isoquinolin-1-one,
(158) 3-(1-methylpiperidin-4-yl)-3,4-dihydro-2H-isoquinolin-1-one,
(159) 8-methyl-2-[2-(diethylamino)ethyl]-3H-quinazolin-4-one,
(162) 3-(3,5-dimethylpiperazin-1-yl)-2H-isoquinolin-1-one,
(163) 4-(4-methylphenyl)-3-(1-methylpiperidin-4-yl)-2H-isoquinolin-1-one,
(164) 4-(4-fluorophenyl)-3-(1-methylpiperidin-4-yl)-2H-isoquinolin-1-one,
(165) 8-methyl-2-(2-piperidinoethyl)-3H-quinazolin-4-one,
(166) 8-methyl-2-[2-(morpholin-4-yl)ethyl]-3H-quinazolin-4-one,
(167) 4-(2-methoxyphenyl)-3-(1-methylpiperidin-4-yl)-2H-isoquinolin-1-one,
(168) 4-(3-methylphenyl)-3-(1-methylpiperidin-4-yl)-2H-isoquinolin-1-one,
(169) 4-(2-methylphenyl)-3-(1-methylpiperidin-4-yl)-2H-isoquinolin-1-one,
(170) 4-(3-methoxyphenyl)-3-(1-methylpiperidin-4-yl)-2H-isoquinolin-1-one,
(171) 5-methoxymethyloxy-3-(1-methylpiperidin-4-yl)-2H-isoquinolin-1-one,
(173) 8-methyl-2-[5-(diethylamino)pentyl]-3H-quinazolin-4-one,
(175) 8-methyl-2-[4-(dimethylamino)butyl]-3H-quinazolin-4-one,
(176) 8-methyl-2-[3-(pyrrolidin-1-yl)propyl]-3H-quinazolin-4-one,
(177) 7-(1-methylpiperidin-4-yl)-6H-1,6-naphthyridin-5-one 1/10 water adduct,
(178) 5-methyl-3-(1-methylpiperidin-4-yl)-2H-isoquinolin-1-one,
(179) 4-methyl-3-(1-methylpiperidin-4-yl)-2H-isoquinolin-1-one,
(180) 5-(dimethylamino)-3-(1-methylpiperidin-4-yl)-2H-isoquinolin-1-one,
(181) 5-amino-3-(1-methylpiperidin-4-yl)-2H-isoquinolin-1-one,
(182) 4-(2-fluorophenyl)-3-(1-methylpiperidin-4-yl)-2H-isoquinolin-1-one,
(183) 7-chloro-3-(1-methylpiperidin-4-yl)-2H-isoquinolin-1-one,
(184) 5-hydroxy-3-(1-methylpiperidin-4-yl)-3,4-dihydro-2H-isoquinolin-1-one,
(185) 5-methoxymethyloxy-3-(3-dimethylaminopropyl)-2H-isoquinolin-1-one,
(186) 5-hydroxy-3-(3-dimethylaminopropyl)-2H-isoquinolin-1-one hydrochloride,
(187) 5-methoxymethyloxy-3-(4-dimethylaminobutyl)-2H-isoquinolin-1-one,
(188) 5-hydroxy-3-(4-dimethylaminobutyl)-2H-isoquinolin-1-one hydrochloride,
(189) 5-hydroxy-3-(2-(piperidin-1-yl)ethyl)-2H-isoquinolin-1-one,
(190) 3-(1-methyl-1,2,3,6-tetrahydropyridin-4-yl)-2H-isoquinolin-1-one,
(191) 3-(1-benzylpiperidin-3-yl)-2H-isoquinolin-1-one,
(192) 3-(1-methylpiperidin-3-yl)-2H-isoquinolin-1-one,
(193) 3-(1-methyl-1,2,3,6-tetrahydropyridin-5-yl)-2H-isoquinolin-1-one,
(194) 3-(3-hydroxymethylpiperazin-1-yl)-2H-isoquinolin-1-one,
(195) 3-(4-ethyl-3-hydroxymethylpiperazin-1-yl)-2H-isoquinolin-1-one,
(196) 3-(3-hydroxymethyl-4-propylpiperazin-1-yl)-2H-isoquinolin-1-one,
(197) 3-(4-benzyl-3-hydroxymethylpiperazin-1-yl)-2H-isoquinolin-1-one,
(198) 5-bromo-3-(3-hydroxymethylpiperazin-1-yl)-2H-isoquinolin-1-one,
(199) 5-bromo-3-(3-hydroxymethyl-4-methylpiperazin-1-yl)-2H-isoquinolin-1-one,
(200) 3-(4-piperidinopiperidin-1-yl)-2H-isoquinolin-1-one,
(201) 3-(3-hydroxymethylpiperidin-1-yl)-2H-isoquinolin-1-one,
(202) 3-(3-(dimethylcarbamoyl)piperidin-1-yl)-2H-isoquinolin-1-one,
(203) 3-(3-hydroxymethyl-4-isobutylpiperazin-1-yl)-2H-isoquinolin-1-one,
(204) 3-[4-(dimethylamino)butyl]-2H-isoquinolin-1-one,
(205) 5-fluoro-3-(3-hydroxymethyl-4-methylpiperazin-1-yl)-2H-isoquinolin-1-one,
(206) 3-(3-(dimethylaminomethyl)piperidin-1-yl)-2H-isoquinolin-1-one,
(207) 6-methyl-3-(1-methylpiperidin-4-yl)-2H-isoquinolin-1-one,
(208) 7-methyl-3-(1-methylpiperidin-4-yl)-2H-isoquinolin-1-one,
(209) 8-methyl-3-(1-methylpiperidin-4-yl)-2H-isoquinolin-1-one,
(210) 7-methoxymethyloxy-3-(1-methylpiperidin-4-yl)-2H-isoquinolin-1-one,
(211) 7-hydroxy-3-(1-methylpiperidin-4-yl)-2H-isoquinolin-1-one hydrochloride,
(212) (S)-3-(3-hydroxymethyl-4-methylpiperazin-1-yl)-5-methyl-2H-isoquinolin-1-one,
(213) 3-(3-(pyrrolidin-1-yl)propyl)-2H-isoquinolin-1-one,
(214) 5-chloro-3-(3-hydroxymethyl-4-methylpiperazin-1-yl)-2H-isoquinolin-1-one,
(216) 3-[3-(piperidin-1-yl)propyl]-2H-isoquinolin-1-one,
(217) 5-hydroxy-3-(3-(pyrrolidin-1-yl)propyl)-2H-isoquinolin-1-one,
(218) 5-methyl-3-[2-(piperidin-1-yl)ethyl]-2H-isoquinolin-1-one,
(219) 3-[2-(piperidin-1-yl)ethyl]-2H-isoquinolin-1-one,
(220) 3-[2-(pyrrolidin-1-yl)ethyl]-2H-isoquinolin-1-one,
(221) 5-methyl-3-[2-(pyrrolidin-1-yl)ethyl]-2H-isoquinolin-1-one,
(222) 5-methyl-3-[3-(pyrrolidin-1-yl)propyl-1-yl]-2H-isoquinolin-1-one,
(223) 1,5-dihydro-6-(1-methyl-1,2,3,6-tetrahydropyridin-4-yl)pyrazolo[4,3-c]pyridin-4-one,
(224) N,N-dimethyl-3-(1-methylpiperidin-1-yl)-2H-1-oxoisoquinoline-5-carboxamide 1/4 water adduct,
(225) 5-methyl-3-(octahydroindolizin-7-yl)-2H-isoquinolin-1-one 3/4 water adduct,
(226) 5-methyl-3-(octahydroindolizin-7-yl)-2H-isoquinolin-1-one 1/2 water adduct,
(227) 3-(1-methylpiperidin-1-yl)-2H-1-oxoisoquinoline-5-carboxylic acid hydrochloride,
(228) 5-methyl-3-[3-(piperidin-1-yl)propyl]-2H-isoquinolin-1-one,
(229) 3-(dimethylamino)methyl-2H-isoquinolin-1-one,
(230) 3-[(4-methylpiperazin-1-yl)methyl]-2H-isoquinolin-1-one,
(231) 3-(piperidinomethyl)-2H-isoquinolin-1-one,
(232) 3-[(morpholin-1-yl)methyl]-2H-isoquinolin-1-one,
(233) 3-[(homopiperidin-1-yl)methyl]-2H-isoquinolin-1-one,
(234) 3-[3-(homopiperidin-1-yl)propyl]-2H-isoquinolin-1-one,
(235) 3-(1-sulfamoylpiperidin-4-yl)-5-methyl-2H-isoquinolin-1-one 1/4 water adduct,
(236) 3-(4-methyl-3-oxopiperazin-1-yl)-5-methyl-2H-isoquinolin-1-one 1/10 water adduct,
(237) 3-(1-aminopiperidin-4-yl)-5-methyl-2H-isoquinolin-1-one,
(238) 3-(1-(methanesulfonylamino)piperidin-4-yl)-5-methyl-2H-isoquinolin-1-one,
(239) 3-(1-trifluoroacetaminopiperidin-4-yl)-5-methyl-2H-isoquinolin-1-one,
(240) 3-[2-(homopiperidin-1-yl)ethyl]-2H-isoquinolin-1-one,
(241) N-(5-methyl-2H-1-oxoisoquinolin-3-yl)-2-(dimethylamino)acetamide,
(243) N-(5-methyl-2H-1-oxoisoquinolin-3-yl)-3-(dimethylamino)propanamide,
(244) 3-(1-dimethylaminopiperidin-4-yl)-5-methyl-2H-isoquinolin-1-one,
(245) N-(5-methyl-2H-1-oxoisoquinolin-3-yl)-4-(dimethylamino)butanamide,
(246) N-(2H-1-oxoisoquinolin-3-yl)-4-(dimethylamino)butanamide,
(247) 3-(4-methyl-2-oxopiperazin-1-yl)-2H-isoquinolin-1-one,
(248) 5-methyl-3-(1-methylpyrrolidin-3-yl)-2H-isoquinolin-1-one, and
(249) 3-(1-methylpyrrolidin-3-yl)-2H-isoquinolin-1-one, an optically active form thereof, a pharmaceutically acceptable salt thereof, a hydrate thereof or a water adduct thereof.

12. The fused heterocyclic compound of claim 1, wherein, in the formula (I),
the dotted line part is a double bond,
ring Ar is a benzene ring,
X is a carbon atom optionally substituted by phenyl optionally having substituent(s), or a nitrogen atom,
Y is -(CH₂)ₘ- wherein m is 0 or an integer of 1-10,
R¹ and R² are the same or different and each is hydrogen, alkyl, hydroxy or amino, and
R is dialkylamino, or represented by the following formula (a) or (b): 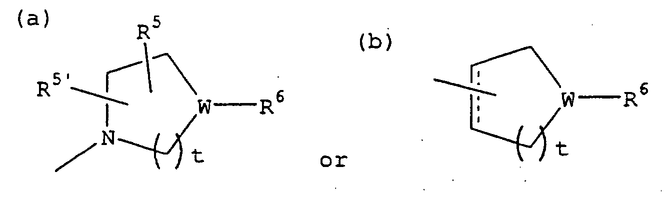 wherein a dotted line part is a single bond, W is CH or a nitrogen atom, t is an integer of 0-3, R⁵ and R^{5'} are the same or different and each is hydroxyalkyl, and R⁶ is hydrogen, alkyl or dialkylamino,
an optically active form thereof, a pharmaceutically acceptable salt thereof, a hydrate thereof or a water adduct thereof.

13. The fused heterocyclic compound of claim 1, wherein, in the formula (I),
the dotted line part is a double bond,
ring Ar is a benzene ring,
X is a carbon atom optionally substituted by phenyl optionally having substituent(s) selected from the group consisting of halogen, alkyl and alkoxy, or a nitrogen atom,
Y is -(CH₂)ₘ- wherein m is 0 or an integer of 1-3,
R¹ is alkyl, hydroxy or amino,
R² is hydrogen, and
R is dialkylamino, or represented by the following formula (a): 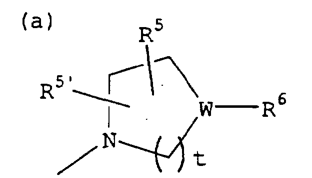 wherein W is CH or a nitrogen atom, t is an integer of 1 or 2, R⁵ is hydroxyalkyl, R^{5'} is hydrogen and R⁶ is hydrogen, alkyl or dialkylamino, or
the following formula (b): 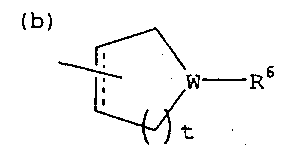 wherein a dotted line part is a single bond, W is a nitrogen atom, t is an integer of 2, and R⁶ is alkyl,
an optically active form thereof, a pharmaceutically acceptable salt thereof, a hydrate thereof or a water adduct thereof.

14. The fused heterocyclic compound of claim 1, which is selected from
(1) 5-methyl-3-(4-methylpiperazin-1-yl)-2H-isoquinolin-1-one,
(3) 3-(4-dimethylaminopiperidin-1-yl)-2H-isoquinolin-1-one,
(4) 3-(3-hydroxymethyl-4-methylpiperazin-1-yl)-2H-isoquinolin-1-one,
(12) 5-methyl-3-(4-dimethylaminopiperidin-1-yl)-2H-isoquinolin-1-one,
(19) 5-hydroxy-3-(4-methylpiperazin-1-yl)-2H-isoquinolin-1-one,
(82) 3-(1-methylpiperidin-4-yl)-2H-isoquinolin-1-one 1/5 water adduct,
(90) 3-(3-(dimethylamino)propyl)-5-methyl-2H-isoquinolin-1-one,
(98) 3-(1-methylpiperidin-4-yl)-4-phenyl-2H-isoquinolin-1-one 1/5 water adduct,
(108) 5-hydroxy-3-(1-methylpiperidin-4-yl)-2H-isoquinolin-1-one, and
(121) 2-(1-methylpiperidin-4-yl)-8-methyl-3H-quinazolin-4-one, an optically active form thereof, a pharmaceutically
acceptable salt thereof, a hydrate thereof or a water adduct thereof.

15. The fused heterocyclic compound of claim 1, which is selected from
(156) (S)-3-(3-hydroxymethyl-4-methylpiperazin-1-yl)-2H-isoquinolin-1-one,
(178) 5-methyl-3-(1-methylpiperidin-4-yl)-2H-isoquinolin-1-one,
(181) 5-amino-3-(1-methylpiperidin-4-yl)-2H-isoquinolin-1-one,
(186) 5-hydroxy-3-(3-dimethylaminopropyl)-2H-isoquinolin-1-one hydrochloride,
(189) 5-hydroxy-3-(2-(piperidin-1-yl)ethyl)-2H-isoquinolin-1-one, and
(212) (S)-3-(3-hydroxymethyl-4-methylpiperazin-1-yl)-5-methyl-2H-isoquinolin-1-one,
an optically active form thereof, a pharmaceutically acceptable salt thereof, a hydrate thereof or a water adduct thereof.

16. The fused heterocyclic compound of claim 1, which is selected from
(82) 3-(1-methylpiperidin-4-yl)-2H-isoquinolin-1-one 1/5 water adduct,
(90) 3-(3-(dimethylamino)propyl)-5-methyl-2H-isoquinolin-1-one, and
(108) 5-hydroxy-3-(1-methylpiperidin-4-yl)-2H-isoquinolin-1-one,
an optically active form thereof, a pharmaceutically acceptable salt thereof, a hydrate thereof or a water adduct thereof.

17. The fused heterocyclic compound of claim 1, wherein, in the formula (I) ,
the dotted line part is a double bond,
ring Ar is a benzene ring,
X is a carbon atom optionally substituted by phenyl optionally having substituent(s), or a nitrogen atom,
Y is -(CH₂)ₘ- wherein m is 0 or an integer of 1-10,
R¹ and R² are the same or different and each is hydrogen, alkyl, hydroxy or amino, and
R is dialkylamino, or represented by the following formula (b) : 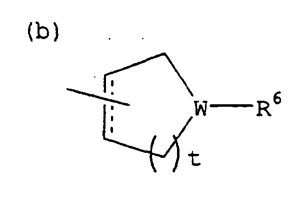 wherein a dotted line part is a single bond, W is CH or a nitrogen atom, t is an integer of 0-3, and R⁶ is hydrogen, dialkylamino or alkyl,
an optically active form thereof, a pharmaceutically acceptable salt thereof, a hydrate thereof or a water adduct thereof.

18. The fused heterocyclic compound of claim 1, wherein, in the formula (I),
the dotted line part is a double bond,
ring Ar is a benzene ring,
X is a carbon atom optionally substituted by phenyl optionally having substituent(s), or a nitrogen atom,
Y is -(CH₂)ₘ- wherein m is 0 or an integer of 1-10,
R¹ and R² are the same or different and each is hydrogen, alkyl, hydroxy or amino, and
R is dialkylamino, or represented by the following formula (a): 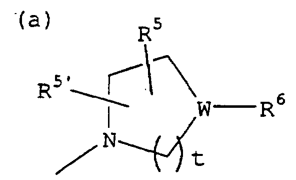 wherein W is CH or a nitrogen atom, t is an integer of 0-3, R⁵ and R^{5'} are the same or different and each is hydroxyalkyl, and R⁶ is hydrogen, dialkylamino or alkyl,
an optically active form thereof, a pharmaceutically acceptable salt thereof, a hydrate thereof or a water adduct thereof.

19. The fused heterocyclic compound of claim 1, which is selected from
(82) 3-(1-methylpiperidin-4-yl)-2H-isoquinolin-1-one 1/5 water adduct,
(108) 5-hydroxy-3-(1-methylpiperidin-4-yl)-2H-isoquinolin-1-one, and
(121) 2-(1-methylpiperidin-4-yl)-8-methyl-3H-quinazolin-4-one,
an optically active form thereof, a pharmaceutically acceptable salt thereof, a hydrate thereof or a water adduct thereof.

20. The fused heterocyclic compound of claim 1, which is selected from
(156) (S)-3-(3-hydroxymethyl-4-methylpiperazin-1-yl)-2H-isoquinolin-1-one and
(212) (S)-3-(3-hydroxymethyl-4-methylpiperazin-1-yl)-5-methyl-2H-isoquinolin-1-one,
an optically active form thereof, a pharmaceutically acceptable salt thereof, a hydrate thereof or a water adduct thereof.

21. The fused heterocyclic compound of claim 1, wherein, in the formula (I),
the dotted line part is a double bond,
ring Ar is a benzene ring,
X is a carbon atom optionally substituted by phenyl optionally having substituent(s), or a nitrogen atom,
Y is -(CH₂)ₘ- wherein m is 0 or an integer of 1-10,
R¹ is alkyl, hydroxy or amino,
R² is hydrogen, and
R is dialkylamino, or represented by the following formula (a) : 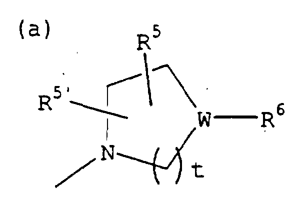 wherein W is CH or a nitrogen atom, t is an integer of 0-3, R⁵ and R^{5'} are the same or different and each is hydroxyalkyl, and R⁶ is hydrogen, dialkylamino or alkyl,
an optically active form thereof, a pharmaceutically acceptable salt thereof, a hydrate thereof or a water adduct thereof.

22. The fused heterocyclic compound of claim 1, which is (1) 5-methyl-3-(4-methylpiperazin-1-yl)-2H-isoquinolin-1-one, an optically active form thereof, a pharmaceutically acceptable salt thereof, a hydrate thereof or a water adduct thereof.

23. A pharmaceutical agent comprising the fused heterocyclic compound of any of claims 1 to 22, an optically active form thereof, a pharmaceutically acceptable salt thereof, a hydrate thereof or a water adduct thereof.

24. A prophylactic and/or therapeutic drug for a disease caused by functional promotion of poly(ADP-ribose) synthase, which comprises a fused heterocyclic compound represented by the formula (I): 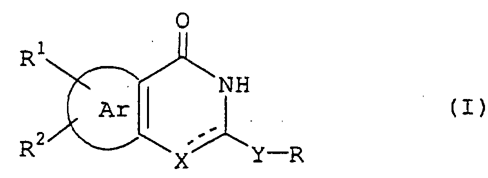 wherein
a dotted line part is a single bond or a double bond;
ring Ar is a benzene ring, a naphthalene ring or an aromatic heterocycle;
X is a carbon atom optionally substituted by an alkyl, an aromatic heterocyclic group optionally having substituent(s) or a phenyl optionally having substituent(s), or a nitrogen atom;
Y is
- (CH₂)ₘ-,
- (CH₂)ₘ-N(R⁴)-CO-(CH₂)ₙ-,
-(CH₂)ₘ-CO-N(R⁴)-(CH₂)ₙ-,
-(CH₂)ₘ-CO-O-(CH₂)ₙ-,
- (CH₂)ₘ-O-CO-(CH₂)ₙ-,
- (CH₂)ₘ-O-(CH₂)ₙ- or
- (CH₂)ₘ-CO-(CH₂)ₙ-
wherein m and n are the same or different and each is 0 or an integer of 1-10, R⁴ is hydrogen or alkyl, and -(CH₂)ₘ- is linked with a parent-nucleus;
R¹ and R² are the same or different and each is a hydrogen, a halogen, an alkyl, an alkoxy, a haloalkyl, a hydroxy, an amino, a dialkylamino, a nitro, a cyano, an acyl, a carboxy, an ester, a carbamoyl, an N-alkylcarbamoyl, an N,N-dialkylcarbamoyl, an acylamino, a diacylamino, a thiol, an alkylthio, an alkoxycarbonylamino, a sulfamoyl, an N-alkylsulfamoyl, an N,N-dialkylsulfamoyl or an alkoxyalkyloxy; and
R is an amino, a monoalkylamino, a dialkylamino, a morpholino or a thiomorpholino, or represented by the following formula (a)-(e): 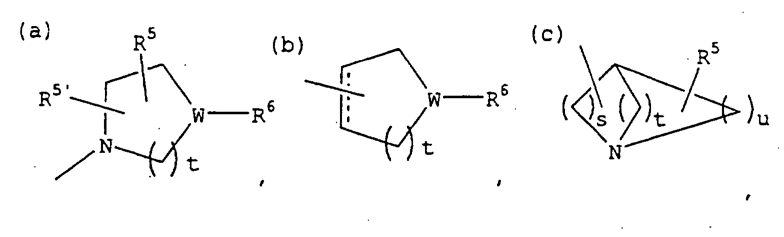 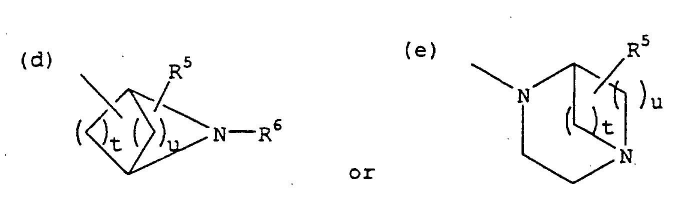 wherein a dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, monoalkylamino, dialkylamino, alkyl, alkoxycarbonyl, alkylsulfonyl, acyl, acylamino, benzoylamino optionally having substituent(s), hydroxy, arylalkyl, sulfamoyl or alkylsulfonylamino, or represented by the following formula (f)-(i): 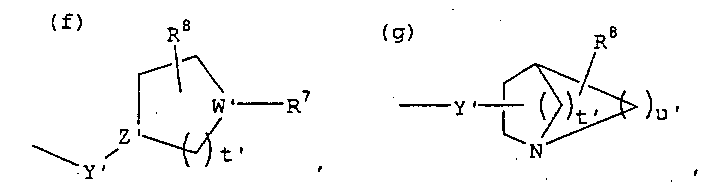 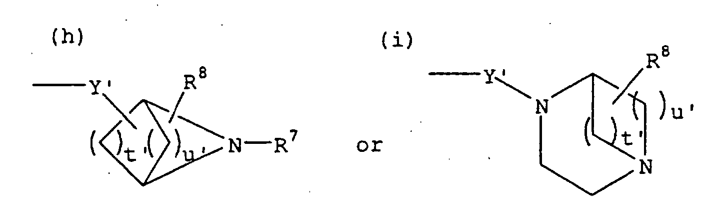
wherein Y' is as defined for Y above, Z' is CH or a nitrogen atom, W' is CH, a nitrogen atom or an oxygen atom, t' is an integer of 1-3, u' is an integer of 1-3, provided that when the above-mentioned formula (a) is piperazine, then R⁶ may be hydroxyalkyl, R⁷ is hydrogen, amino, monoalkylamino, dialkylamino, alkyl, alkoxycarbonyl, alkylsulfonyl, acyl, hydroxyalkyl, acylamino or benzoylamino optionally having substituent(s), provided that when W' is an oxygen atom, then R⁷ should be absent, and R⁸ is hydrogen, alkyl or hydroxyalkyl,
an optically active form thereof, a pharmaceutically acceptable salt thereof, a hydrate thereof or a water adduct thereof.

25. A prophylactic and/or therapeutic drug for a disease caused by functional promotion of poly(ADP-ribose) synthase, which comprises a fused heterocyclic compound represented by the formula (I): 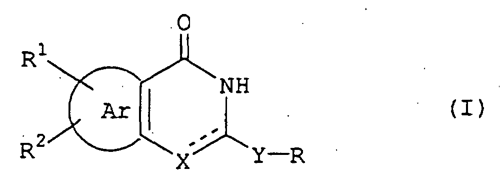 wherein
a dotted line part is a single bond or a double bond;
ring Ar is a benzene ring, a naphthalene ring or an aromatic heterocycle;
X is a carbon atom optionally substituted by an alkyl, an aromatic heterocyclic group optionally having substituent(s) or a phenyl optionally having substituent(s), or a nitrogen atom;
Y is
- (CH₂)ₘ-,
- (CH₂)ₘ-N(R⁴)-CO-(CH₂)ₙ-,
- (CH₂)ₘ-CO-N(R⁴)-(CH₂)ₙ-,
- (CH₂)ₘ-CO-O-(CH₂)ₙ- or
-(CH₂)ₘ-O-(CH₂)ₙ-
wherein m and n are the same or different and each is 0 or an integer of 1-10, R⁴ is hydrogen or alkyl, and - (CH₂)ₘ- is linked with a parent-nucleus;
R¹ and R² are the same or different and each is a hydrogen, a halogen, an alkyl, an alkoxy, a haloalkyl, a hydroxy, an amino, a dialkylamino, a nitro, a cyano, an acyl, a carboxy, an ester, a carbamoyl, an N-alkylcarbamoyl, an N,N-dialkylcarbarnoyl, an acylamino, a diacylamino, a thiol, an alkylthio, an alkoxycarbonylamino, a sulfamoyl, an N-alkylsulfamoyl or an N,N-dialkylsulfamoyl; and
R is an amino, a monoalkylamino, a dialkylamino, a morpholino or a thiomorpholino, or represented by the following formula (a) - (e) 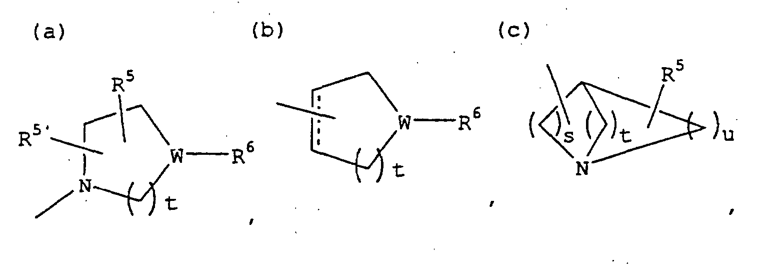 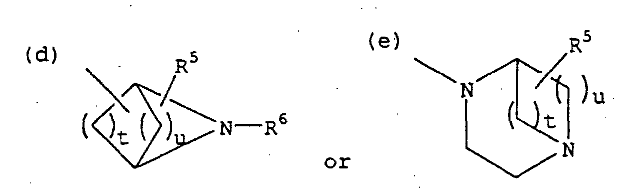
wherein a dotted line part is a single bond or a double bond, W is CH or a nitrogen atom, s is an integer of 1-3, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, monoalkylamino, dialkylamino, alkyl, alkoxycarbonyl, alkylsulfonyl, acyl, acylamino, benzoylamino optionally having substituent(s), hydroxy, arylalkyl, sulfamoyl or alkylsulfonylamino, or represented by the following formula (f)-(i): 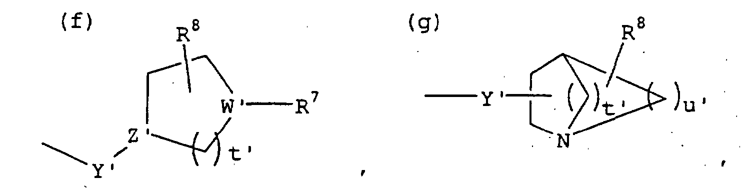 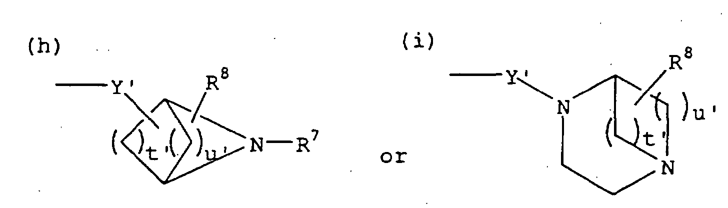 wherein Y' is as defined for Y above, Z' is CH or a nitrogen atom, W' is CH or a nitrogen atom, t' is an integer of 1-3, u' is an integer of 1-3, R⁷ is hydrogen, amino, monoalkylamino, dialkylamino, alkyl, alkoxycarbonyl, alkylsulfonyl, acyl, hydroxyalkyl, acylamino or benzoylamino optionally having substituent(s), and R⁸ is hydrogen, alkyl or hydroxyalkyl,
provided that (1) when Y is -(CH₂)ₘ- (m=0) and R is 4-methylpiperazin-1-yl, 1-piperidino, 4-morpholino or 4-(2-hydroxyethyl)piperazin-1-yl, then R¹ should be halogen, alkyl, alkoxy, haloalkyl, hydroxy, amino, dialkylamino, nitro, cyano, acyl, carboxy, ester, carbamoyl, N-alkylcarbamoyl, N,N-dialkylcarbamoyl, acylamino, diacylamino, thiol, alkylthio, alkoxycarbonylamino, sulfamoyl, N-alkylsulfamoyl or N,N-dialkylsulfamoyl, and (2) when X is a nitrogen atom and Y is -(CH₂)ₘ- (m=0), then R should be represented by any of the formulas (b)-(d) and Z should be CH,
an optically active form thereof, a pharmaceutically acceptable salt thereof, a hydrate thereof or a water adduct thereof.

26. The prophylactic and/or therapeutic drug of claim 24 or 25, which is used for cerebral infarction.

27. The prophylactic and/or therapeutic drug of any of claims 24 to 26, which is used for an acute phase of cerebral infarction.

28. A poly(ADP-ribose) synthase inhibitor comprising a fused heterocyclic compound represented by the formula (I): 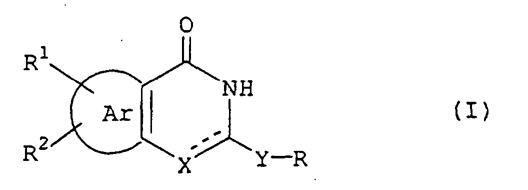 wherein
a dotted line part is a single bond or a double bond;
ring Ar is a benzene ring, a naphthalene ring or an aromatic heterocycle;
X is a carbon atom optionally substituted by an alkyl, an aromatic heterocyclic group optionally having substituent(s) or a phenyl optionally having substituent(s), or a nitrogen atom;
Y is
- (CH₂)ₘ-,
- (CH₂)ₘ-N(R⁴)-CO- (CH₂)ₙ-,
- (CH₂)ₘ-CO-N(R⁴)-(CH₂)ₙ-,
-(CH₂)ₘ-CO-O-(CH₂)ₙ-,
-(CH₂)ₘ-O-CO-(CH₂)ₙ-,
- (CH₂)ₘ-O-(CH₂)ₙ- or
- (CH₂)ₘ-CO-(CH₂)ₙ-
wherein m and n are the same or different and each is 0 or an integer of 1-10, R⁴ is hydrogen or alkyl, and -(CH₂)ₘ- is linked with a parent-nucleus;
R¹ and R² are the same or different and each is a hydrogen, a halogen, an alkyl, an alkoxy, a haloalkyl, a hydroxy, an amino, a dialkylamino, a nitro, a cyano, an acyl, a carboxy, an ester, a carbamoyl, an N-alkylcarbamoyl, an N,N-dialkylcarbamoyl, an acylamino, a diacylamino, a thiol, an alkylthio, an alkoxycarbonylamino, a sulfamoyl, an N-alkylsulfamoyl, an N,N-dialkylsulfamoyl or an alkoxyalkyloxy; and
R is an amino, a monoalkylamino, a dialkylamino, a morpholino or a thiomorpholino, or represented by the following formula (a)-(e): 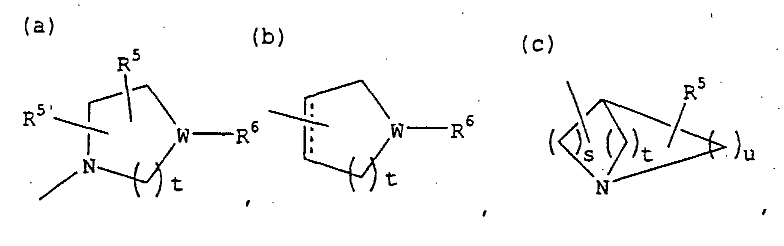 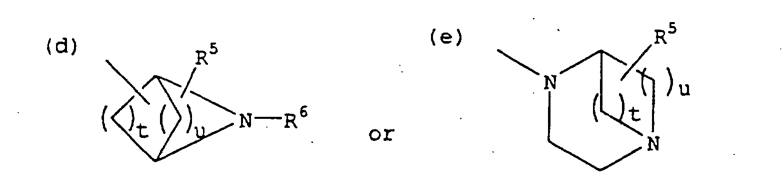 wherein a dotted line part is a single bond or a double bond, W is CH or a nitrogen atom, s is an integer of 1-4, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, monoalkylamino, dialkylamino, alkyl, alkoxycarbonyl, alkylsulfonyl, acyl, acylamino, benzoylamino optionally having substituent(s), hydroxy, arylalkyl, sulfamoyl or alkylsulfonylamino, or represented by the following formula (f)-(i): 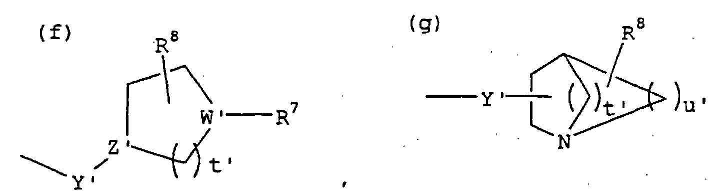 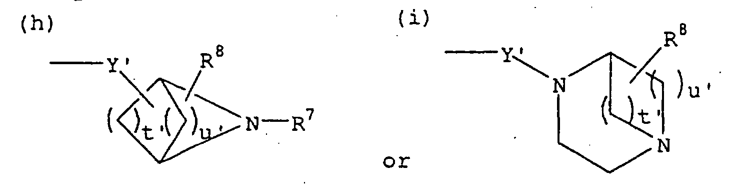
wherein Y' is as defined for Y above, Z' is CH or a nitrogen atom, W' is CH, a nitrogen atom or an oxygen atom, t' is an integer of 1-3, u' is an integer of 1-3, provided that when the above-mentioned formula (a) is piperazine, then R⁶ may be hydroxyalkyl, R⁷ is hydrogen, amino, monoalkylamino, dialkylamino, alkyl, alkoxycarbonyl, alkylsulfonyl, acyl, hydroxyalkyl, acylamino or benzoylamino optionally having substituent(s), provided that when W' is an oxygen atom, then R⁷ should be absent, and R⁸ is hydrogen, alkyl or hydroxyalkyl,
an optically active form thereof, a pharmaceutically acceptable salt thereof, a hydrate thereof or a water adduct thereof.

29. A poly(ADP-ribose) synthase inhibitor comprising a fused heterocyclic compound represented by the formula (I): 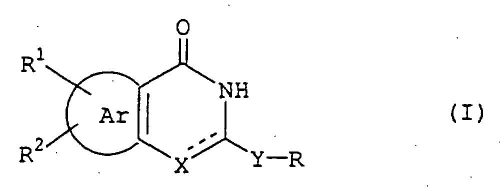 wherein
a dotted line part is a single bond or a double bond;
ring Ar is a benzene ring, a naphthalene ring or an aromatic heterocycle;
X is a carbon atom optionally substituted by an alkyl, an aromatic heterocyclic group optionally having substituent(s) or a phenyl optionally having substituent(s), or a nitrogen atom;
Y is
-(CH₂)ₘ-,
- (CH₂)ₘ-N(R⁴)-CO-(CH₂)ₙ-,
-(CH₂)ₘ-CO-N(R⁴)-(CH₂)ₙ-,
- (CH₂)ₘCO-O-(CH₂)ₙ- or
- (CH₂)ₘ-O-(CH₂)ₙ-
wherein m and n are the same or different and each is 0 or an integer of 1-10, R⁴ is hydrogen or alkyl, and -(CH₂)ₘ- is linked with a parent-nucleus;
R¹ and R² are the same or different and each is a hydrogen, a halogen, an alkyl, an alkoxy, a haloalkyl, a hydroxy, an amino, a dialkylamino, a nitro, a cyano, an acyl, a carboxy, an ester, a carbamoyl, an N-alkylcarbamoyl, an N,N-dialkylcarbamoyl, an acylamino, a diacylamino, a thiol, an alkylthio, an alkoxycarbonylamino, a sulfamoyl, an N-alkylsulfamoyl or an N,N-dialkylsulfamoyl; and
R is an amino, a monoalkylamino, a dialkylamino, a morpholino or a thiomorpholino, or represented by the following formula (a)-(e): 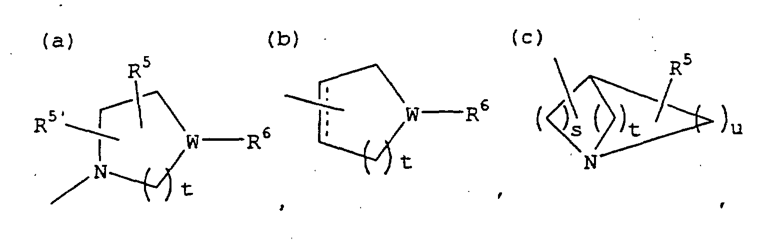 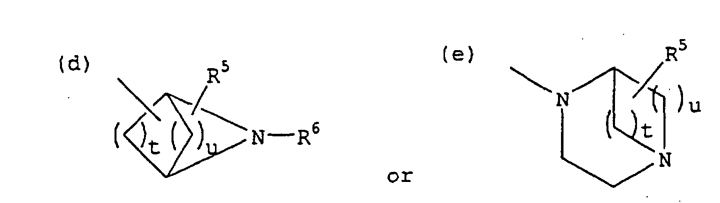 wherein a dotted line part is a single bond or a double bond, W is CH or a nitrogen atom, s is an integer of 1-3, t is an integer of 0-3, u is an integer of 1-3, R⁵ and R^{5'} are the same or different and each is hydrogen, alkyl, hydroxyalkyl, alkoxycarbonyl, dialkylaminoalkyl or dialkylcarbamoyl, or R⁵ and R^{5'} in combination form ketone, and R⁶ is hydrogen, amino, monoalkylamino, dialkylamino, alkyl, alkoxycarbonyl, alkylsulfonyl, acyl, acylamino, benzoylamino optionally having substituent(s), hydroxy, arylalkyl, sulfamoyl or alkylsulfonylamino, or represented by the following formula (f)-(i): 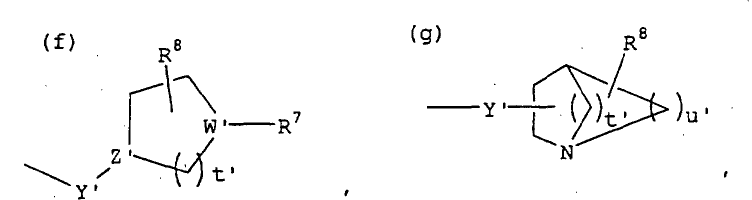 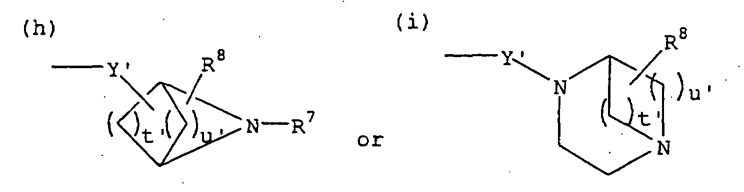
wherein Y' is as defined for Y above, Z' is CH or a nitrogen atom, W' is CH or a nitrogen atom, t' is an integer of 1-3, u' is an integer of 1-3, and R⁷ is hydrogen, amino, monoalkylamino, dialkylamino, alkyl, alkoxycarbonyl, alkylsulfonyl, acyl, hydroxyalkyl, acylamino or benzoylamino optionally having substituent(s), and R⁸ is hydrogen, alkyl or hydroxyalkyl,
provided that (1) when Y is -(CH₂)ₘ- (m=0) and R is 4-methylpiperazin-1-yl, 1-piperidino, 4-morpholino or 4-(2-hydroxyethyl)piperazin-1-yl, then R¹ should be halogen, alkyl, alkoxy, haloalkyl, hydroxy, amino, dialkylamino, nitro, cyano, acyl, carboxy, ester, carbamoyl, N-alkylcarbamoyl, N,N-dialkylcarbamoyl, acylamino, diacylamino, thiol, alkylthio, alkoxycarbonylamino, sulfamoyl, N-alkylsulfamoyl or N,N-dialkylsulfamoyl, and (2) when X is a nitrogen atom and Y is - (CH₂)ₘ- (m=0), then R should be represented by any of the above formulas (b)-(d) and Z should be CH,
an optically active form thereof, a pharmaceutically acceptable salt thereof, a hydrate thereof or a water adduct thereof.
